# EUROPEAN PATENT APPLICATION

(11) **EP 4 681 777 A2**
(43) Date of publication of application: **21.01.2026**
(21) Application number: 25220921.8
(22) Date of filing: 08.01.2019
(51) Int. Cl.: A61P 25/24

(54) **PRODRUGS OF KETAMINE, COMPOSITIONS AND USES THEREOF**

(30) Priority: 10.01.2018 US 201862615948 P
(62) Divisional of application: 19738848.1
(71) Applicant: XWPharma Ltd., Grand Cayman KY1-1205 (KY)
(72) Inventor: XIANG, Jia-Ning, Wuhan, 430079 (CN); XU, Xuesong, Wuhan, 430079 (CN); ENG, Wai-si, Wuhan, 430079 (CN); SHIH, Hao-wei, 221 New Taipei City (TW)
(74) Representative: Marks & Clerk LLP

(57) **Abstract**

The present invention relates to prodrugs of (*S*)- or (*R*)-ketamine, including isotopically labeled ketamine, compositions and uses thereof. Compounds having formula (Ia) or (Ib) as the prodrugs of (*S*)- or (*R*)-ketamine, including isotopically labeled ketamine, and pharmaceutical compositions comprising the compounds provided herein are used for treating or preventing a CNS disease. More particularly, the related diseases include depression and pain.

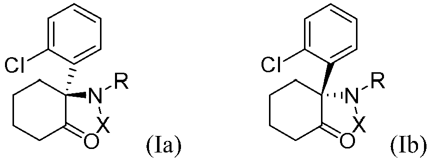

## Description

### RELATED APPLICATIONS

This application claims priority and benefits of US Patent Application No. 62/615,948, filed with United States Patent and Trademark Office on 10 January 2018, the entire content of which is incorporated herein by reference.

### FIELD OF THE INVENTION

This invention relates to the field of pharmaceutical technology, and more specifically relates to prodrugs of (*S*)- or (*R*)-ketamine, including isotopically labeled ketamine, compositions and uses thereof. More specifically, the compounds disclosed herein can be used as NMDA (*N*-methyl-D-aspartate) receptor antagonists, for treating, preventing or lessening neurological and psychiatric disorder or disease of the central nervous system related to NMDA receptor, and the pharmaceutical compositions disclosed herein also have functions of prevention, treatment or lessening of a disease related to NMDA receptor. More particularly, the related diseases include depression and pain.

### BACKGROUND OF THE INVENTION

Antidepressants are central nervous system therapeutics used to treat diseases such as major depressive disorder (MDD), dysthymic disorder, and seasonal affective disorder. MDD, also known as clinical depression, is a condition that lasts two weeks or more and interferes with a person's ability to carry on daily tasks and to enjoy activities that previously brought pleasure.

Glutamate is the major excitatory neurotransmitter in the brain. Similar to classic neurotransmitter, glutamate is released from nerve cells, binds to receptors and is removed by reuptake transporters. Glutamate receptor systems are very complex and can be segregated into various distinct receptor subtypes according to their molecular and pharmacological properties. Most clinical studies across multiple CNS indications have focused on drugs that modulate glutamate function via NMDA receptors. Glutamate and its receptor subtypes play fundamental roles in synaptic plasticity and impact basic human processes of mood, cognition and reward. Additional roles include neurodevelopmental and neurotrophic effects as well as neurodegeneration.

Ketamine is classified as an NMDA receptor antagonist, although its pharmacological profile is complex and it binds to numerous receptors. It was first approved by the US FDA 50 years ago as a general anesthetic. Chemically, ketamine is a racemic mixture of (*R*)- and (*S*)-ketamine. In 1998, (*S*)-ketamine was approved in EU for general anesthesia.

Repeated-dose ketamine is a potential antidepressant continuation strategy for patients who show initial response to ketamine infusion. Repeated-dose *i.v*. ketamine over two weeks (six infusions) in ten patients with TRD who failed to respond to a mean of eight antidepressants in their lifetime led to a mean reduction of 85% in the MADRS (Mongomery-Asberg Depression Rating Scale) score after the sixth infusion.

Despite ketamine's efficacious effect via *i.v*. infusion and advantageous position compared to other drugs in treating treatment-resistant depression, this dosing regimen requires patients obtain the treatment in clinics. Ketamine has been studied in human to evaluate its oral bioavailability. It was found that ketamine only showed 17% oral bioavailability in man as a result of extensive first-pass metabolism, which prevents it from developing an oral regimen. In addition, ketamine's side effects might be associated with high Cmax following bolus dosing.

To overcome the pharmacokinetics deficiencies of ketamine, the prodrug approach will be utilized to identify feasible ketamine derivatives that can significantly improve the pharmacokinetics profile of ketamine via oral administration. A sustained release formulation may be desirable to remove the peak and trough of drug plasma concentration in order to avoid potential side effects.

### SUMMARY OF THE INVENTION

The following is only an overview of some aspects of the present invention, but is not limited thereto. All references of this specification are incorporated herein by reference in their entirety. When the disclosure of this specification is different with citations, the disclosure of this specification shall prevail. The present invention provides compounds and pharmaceutical compositions, which modulate antagonize NMDA receptor, their preparation, and the corresponding pharmaceutical compositions. The compounds and / or pharmaceutical compositions of the present invention can be potentially used in the manufacture of a medicament for preventing, treating, ameliorating certain disorder or a disease related to NMDA receptor in a patient, which includes, depression and pain.

Specifically, in one aspect, the present invention relates to a compound having the structure of Formula (Ia) or (Ib), or a stereoisomer, an N-oxide, a solvate, a metabolite, a pharmaceutically acceptable salt or a prodrug thereof: 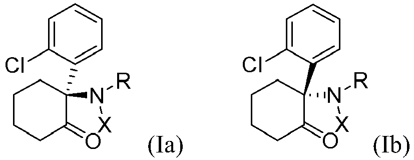
wherein R is -C(=O)R¹, -C(=O)OR², -C(=O)O(CHR³)OC(=O)R⁴ or -CD₃; and X is -CH₃ or - CD₃;
wherein R¹ is, optionally, substituted or unsubstituted aryl-OH, aryl-NH₂, alkenyl-OH, alkenyl-NH₂, alkyl-NH₂, alkyl-OH, carbocyclyl or heterocyclyl containing one or more N or O; and X is -CH₃ or -CD₃;
wherein R² is optionally substituted or unsubstituted alkyl, aryl, carbocyclyl or heterocyclyl containing one or more O; and X is -CH₃ or -CD₃;
wherein R⁴ is independently substituted or unsubstituted alkyl, aryl, azaaryl, carbocyclyl or heterocyclyl containing one or more O or N, while R³ is H or substituted or unsubstituted alkyl; and X is -CH₃ or -CD₃.

It is an object of the present invention that the compound having the structure of Formula (Ia) or (Ib) is a prodrug of (*S*)- or (*R*)-ketamine, wherein the moiety of "N-R" can be cleavable *in vivo* through chemical hydrolysis or metabolic process by endogenous enzymes. The compound having Formula (Ia) or (Ib) may possess characteristics of i) significantly improving the oral bioavailability compared to ketamine; and ii) being amenable for sustained-release formulation suitable for QD or BID to meet patient's compliance and convenience.

In another aspect, provided herein is a compound having the structure of Formula (IIa) or (IIb), or a stereoisomer, an N-oxide, a solvate, a metabolite, a pharmaceutically acceptable salt or a prodrug thereof: 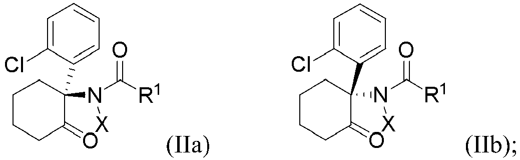 wherein R¹ is, optionally, substituted or unsubstituted aryl-OH, aryl-NH₂, alkenyl-OH, alkenyl-NH₂, alkyl-NH₂, alkyl-OH, carbocyclyl or heterocyclyl containing one or more N or O; and X is -CH₃ or -CD₃.

In one embodiment, R¹ is aminoC₁₋₆ alkyl, -R^{1a}NHCOR^{1b}, -R^{1a}OCOR^{1b}, -R^{1a}COOR^{1b}, 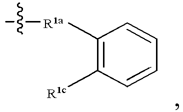 or C₃₋₆ heterocyclyl,
wherein R¹ is optionally substituted with C₁₋₆ alkyl, -OH or oxo(=O),
wherein R^{1a} and R^{1b} is independently H, C₁₋₆ alkyl or C₂₋₆ alkenyl, and
R^{1c} is -OH, C₁₋₃ hydroxyalkyl, -OCOR^{1b} or -CH₂ OCOR^{1b}.

In one embodiment, the heterocyclyl containing one or more N or O is

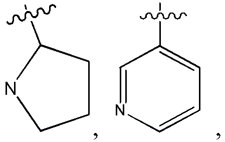

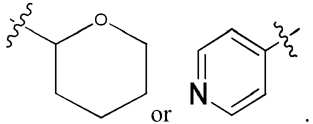

In another aspect, provided herein is a compound having the structure of Formula (IIIa) or (IIIb), or a stereoisomer, an N-oxide, a solvate, a metabolite, a pharmaceutically acceptable salt or a prodrug thereof: 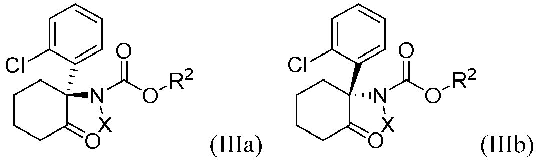 wherein R² is optionally substituted or unsubstituted alkyl, aryl, carbocyclyl or heterocyclyl containing one or more O; and X is -CH₃ or -CD₃.

In one embodiment, R² is C₁₋₆ alkyl, C₁₋₆ hydroxyalkyl, aminoC₁₋₆ alkyl, -R^{2a}S(O)ₙ₁R^{2b}, - R^{2a}COOR^{2b}, C₃₋₆ aryl or C₃₋₆ heterocyclyl,
wherein R² is optionally substituted with C₁₋₆ alkyl, -OH, C₁₋₆ hydroxyalkyl, 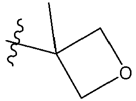 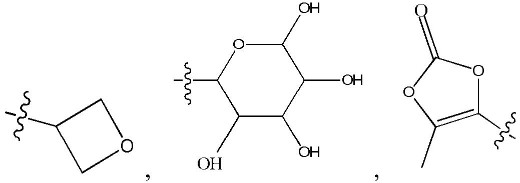 or -R^{2a}COOR^{2b}, given that C₁₋₆ alkyl is substituted with 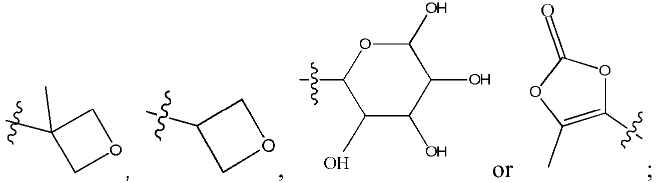
R^{2a} is C₁₋₆ alkyl, wherein R^{2a} is optionally substituted with C₁₋₆ alkyl or -NH₂;
R^{2b} is H or C₁₋₆ alkyl; and
n₁ is 0, 1, 2.

In another aspect, provided herein is a compound having the structure of Formula (IVa) or (IVb), or a stereoisomer, an N-oxide, a solvate, a metabolite, a pharmaceutically acceptable salt or a prodrug thereof: 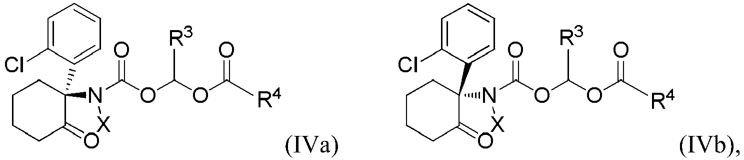 wherein R⁴ is independently substituted or unsubstituted alkyl, aryl, azaaryl, carbocyclyl or heterocyclyl containing one or more O or N, while R³ is H or substituted or unsubstituted alkyl; and X is -CH₃ or -CD₃.

In one embodiment, R³ is H or C₁₋₆ alkyl.

In one embodiment, R⁴ is C₁₋₆ alkyl, aminoC₁₋₆ alkyl, C₁₋₆ hydroxyalkyl, -R^{4a}NCOR^{4b}, - R^{4a}OCOR^{4b}, -R^{4a}S(O)ₙ₂R^{4b}, C₁₋₆ heterocyclyl, C₁₋₅ azaaryl or 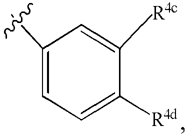
wherein R⁴ is optionally substituted with C₁₋₆ alkyl, -NH₂, oxo(=O), C₁₋₆ hydroxyalkyl, 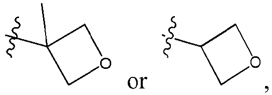 given that C₁₋₆ alkyl is substituted with 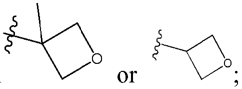
wherein R^{4a} is C₁₋₆ alkyl, R^{4b} is C₁₋₆ alkyl or C₁₋₆ haloalkyl;
R^{4c} is benzyl, R^{4d} is H, or R^{4c} and R^{4d}, together with the carbon atoms to which they are attached, form a C₅₋₆ heterocyclyl; and
n₂ is 0,1 or 2.

In one embodiment, C₁₋₆ heterocyclyl is

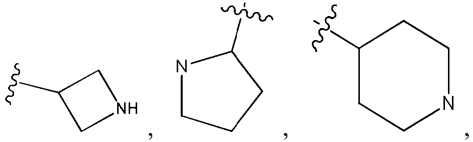

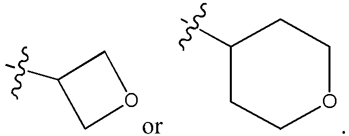

In one embodiment, C₁₋₅ azaaryl is 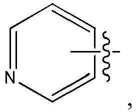 wherein 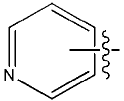 is optionally substituted with one or more methyl or -NH₂ , or the combination thereof.

In one embodiment, 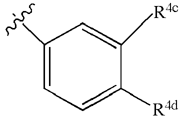 is 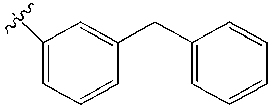 or

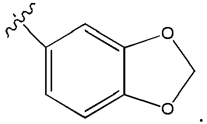

In another aspect, provided herein is a compound having the structure of Formula (Va) or (Vb) or (Vc) or (Vd), or a stereoisomer, an N-oxide, a solvate, a metabolite, a pharmaceutically acceptable salt or a prodrug thereof:

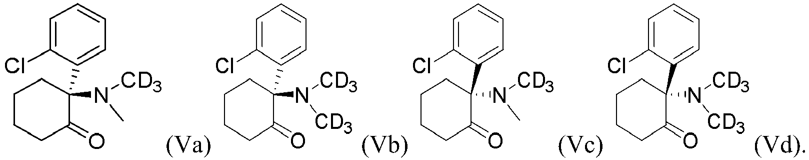

In another aspect, provided herein is a pharmaceutical composition comprising the compound of the present invention.

In one embodiment, the pharmaceutical composition further comprising at least one pharmaceutically acceptable excipient carrier, adjuvant, vehicle or a combination thereof.

In one embodiment, the pharmaceutical composition further comprising one or more adjunctive therapeutic agents in a pharmaceutically effective amount, and wherein the adjunctive therapeutic agent is used in treating a neurological and psychiatric disorder or disease of the central nervous system.

In one embodiment, the neurological and psychiatric disorder or disease of the central nervous system is depression or pain.

In one embodiment, the adjunctive therapeutic agent is selected from the group consisting of at least one member of lithium, a pharmaceutical or an herbal antidepressant, an anticonvulsant, a mood stabilizer, an antipsychotic agent, and a benzodiazepine.

In another aspect, provided herein is use of the compound or the pharmaceutical composition in the manufacture of a medicament for preventing, managing, treating or lessening a neurological and psychiatric disorder or disease of the central nervous system in a patient.

In another aspect, provided herein is use of the compound or the pharmaceutical composition in the manufacture of a medicament for antagonizing the NMDA receptor.

In another aspect, provided herein is the compound or the pharmaceutical composition for use in preventing, managing, treating or lessening a neurological and psychiatric disorder or disease of the central nervous system in a patient.

In another aspect, provided herein is the compound or the pharmaceutical composition for use in antagonizing the NMDA receptor.

In another aspect, provided herein is a method of preventing, managing, treating or lessening a neurological and psychiatric disorder or disease of the central nervous system in a patient, comprising administering to the patient in need thereof a therapeutically effective amount of the compound or the pharmaceutical composition.

In another aspect, provided herein is a method of antagonizing the NMDA receptor in a patient, comprising administering to the patient in need thereof a therapeutically effective amount of the compound or the pharmaceutical composition.

In another aspect, provided herein is the method for preparing, separating, and purifying the compounds represented by Formula (Ia) ~ (Vd).

Biological test results show that the compounds provided herein have a good antagonism for NMDA receptor and show better pharmacokinetic properties and bioavailability.

In certain embodiments of the compounds, pharmaceutical compositions, and methods of the invention, the compound of Formula (Ia) ~ (Vd) is a compound selected from those species described or exemplified in the detailed description below, or is a pharmaceutically acceptable salt of such a compound.

Another preferred embodiment, the present invention is directed to methods of preparing pharmaceutical compositions each comprising an effective amount of at least one compound of Formula (Ia) ~ (Vd) or a pharmaceutically acceptable salt of a compound of Formula (Ia) ~ (Vd). Pharmaceutical compositions according to the invention may further comprise at least one pharmaceutically acceptable excipient, carrier, adjuvant, solvent, support or a combination thereof.

If formulated as a fixed dose, such combination products employ the compounds of this invention within the dosage range described herein (or as known to those skilled in the art) and the other pharmaceutically active agents or treatments within its dosage range. The compounds of the invention may also be administered sequentially with known anti-depression and pain agents when a combination formulation is inappropriate. In any combination treatment, the invention is not limited in the sequence of administration; compounds of Formula (Ia) ~ (Vd) may be administered either prior to or after administration of the known anti-depression and pain agent. Such techniques are within the skills of persons skilled in the art as well as attending physicians.

Yet another embodiment is a method for administering a compound of the instant invention to a subject (*e.g.,* a human) in need thereof by administering to the subject the pharmaceutical formulation of the present invention.

Yet another embodiment is a method of preparing a pharmaceutical formulation of the present invention by mixing at least one pharmaceutically acceptable compound of the present invention, and, optionally, one or more pharmaceutically acceptable additives or excipients.

For preparing pharmaceutical compositions from the compounds described by this invention, inert, pharmaceutically acceptable carriers can be either solid or liquid. Solid form preparations include powders, tablets, dispersible granules, capsules, beads, cachets and suppositories. The powders and tablets may be comprised of from about 5 to about 95 percent active ingredient. Suitable solid carriers are known in the art, e.g., magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington's Pharmaceutical Sciences, 18th Edition, (1990), Mack Publishing Co., Easton, Pa.

Liquid form preparations include solutions, suspensions and emulsions. For example, there are water or water-propylene glycol solutions for parenteral injection or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, *e.g.,* nitrogen.

Also included are solid form preparations that are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and / or emulsions and can be included in a transdermal patch of the matrix or reservoir type as are conventional in the art for this purpose.

The compounds of this invention may also be delivered subcutaneously.

Preferably the compound is administered orally or intravenously.

Preferably, the pharmaceutical preparation is in a unit dosage form. In such form, the preparation is subdivided into suitably sized unit doses containing appropriate quantities of the active component, e.g., an effective amount to achieve the desired purpose.

The quantity of active compound in a unit dose of preparation may be varied or adjusted from about 1 mg to about 1000 mg, preferably from about 1 mg to about 500 mg, more preferably from about 1 mg to about 300 mg, still more preferably from about 1 mg to about 200 mg, according to the particular application.

The actual dosage employed may be varied depending upon the requirements of the patient and the severity of the condition being treated. Determination of the proper dosage regimen for a particular situation is within the skill of the art. For convenience, the total daily dosage may be divided and administered in portions during the day as required. The amount and frequency of administration of the compounds of the invention and / or the pharmaceutically acceptable salts thereof will be regulated according to the judgment of the attending clinician considering such factors as age, condition and size of the patient as well as severity of the symptoms being treated. A typical recommended daily dosage regimen for oral administration can range from about 1 mg/day to about 300 mg/day, preferably 10 mg/day to 200 mg/day, in one to two divided doses.

Any embodiment disclosed herein can be combined with other embodiments as long as they are not contradictory to one another, even though the embodiments are described under different aspects of the invention. In addition, any technical feature in one embodiment can be applied to the corresponding technical feature in other embodiments as long as they are not contradictory to one another, even though the embodiments are described under different aspects of the invention.

The foregoing merely summarizes certain aspects disclosed herein and is not intended to be limiting in nature. These aspects and other aspects and embodiments are described more fully below.

### DETAILED DESCRIPTION AND PARTICULAR EMBODIMENTS

For the sake of brevity, the disclosures of the publications cited in this specification, including patents and patent applications, are herein incorporated by reference in their entirety.

Most chemical names were generated using IUPAC nomenclature herein. Some chemical names were generated using different nomenclatures or alternative or commercial names known in the art. In the case of conflict between names and structures, the structures prevail.

### Definitions and General Terminology

Reference will now be made in detail to certain embodiments of the invention, examples of which are illustrated in the accompanying structures and formulas. The invention is intended to cover all alternatives, modifications and equivalents which may be included within the scope of the present invention as defined by the claims. One skilled in the art will recognize many methods and materials similar or equivalent to those described herein, which could be used in the practice of the present invention. The present invention is in no way limited to the methods and materials described herein. In the event that one or more of the incorporated literatures, patents, and similar materials differs from or contradicts this application, including but not limited to defined terms, term usage, described techniques, or the like, this application controls.

It is further appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

Unless defined otherwise, all technical and scientific terms used herein have the same meaning as are commonly understood by one skilled in the art to which this invention belongs. All patents and publications referred to herein are incorporated by reference in their entirety.

As used herein, the following definitions shall apply unless otherwise indicated. For purposes of this invention, the chemical elements are identified in accordance with the Periodic Table of the Elements, CAS version, and the Handbook of Chemistry and Physics, 75th Ed. 1994. Additionally, general principles of organic chemistry are described in "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are hereby incorporated by reference.

As used above, and throughout this disclosure, the following terms, unless otherwise indicated, shall be understood to have the following meanings. If a definition is missing, the conventional definition as known to one skilled in the art controls. If a definition provided herein conflicts or is different from a definition provided in any cited publication, the definition provided herein controls.

As used herein, the terms "including", "containing", and "comprising" are used in their open, non-limiting sense.

As used herein, the singular forms "a", "an", and "the" include plural referents unless the context clearly dictates otherwise.

To provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and / or measurement conditions for such given value. Whenever a yield is given as a percentage, such yield refers to a mass of the entity for which the yield is given with respect to the maximum amount of the same entity that could be obtained under the particular stoichiometric conditions. Concentrations that are given as percentages refer to mass ratios, unless indicated differently.

The term "optional" or "optionally" refers to that a subsequently described event or circumstance may but need not occur, and that the description includes instances where the event or circumstance occurs and instances in which it does not.

The term "optionally substituted" and "unsubstituted or substituted" can be used interchangeably herein, which means that the structure is unsubstituted or substituted by one or more substituents disclosed herein, wherein the substitution occurs at any valence allowable and reasonable site of structures or groups provided herein.

In general, the term "substituted" refers to the replacement of one or more hydrogen radicals in a given structure or group with the radical of a specified substituent. Unless otherwise indicated, a substituent may have a substituent at each substitutable and reasonable position of the group. When more than one position in a given structure can be substituted with more than one substituent selected from a specified group, the substituent may be either the same or different at each position. The substituents disclosed herein including, but not limited to D, F, Cl, Br, I, -N₃, -CN, -NO₂, -OH, -SH, -NH₂, alkyl, alkenyl, alkynyl, haloalkyl, alkoxy, alkylthio, aminoalkyl, cycloalkyl, heterocyclyl, aryl, heteroaryl, and the like.

### Chemical Definitions

As used herein, "alkyl" refers to a saturated, straight- or branched-chain hydrocarbon group having from 1 to 12 carbon atoms. Representative alkyl groups include, but are not limited to, methyl, ethyl, n-propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, n-pentyl, isopentyl, neopentyl, n-hexyl, and the like, and longer alkyl groups, such as heptyl, octyl, and the like.

At various places in the present specification, substituents of compounds disclosed herein are disclosed in groups or in ranges. It is specifically intended that the invention include each and every individual subcombination of the members of such groups and ranges. For example, the term "C₁₋₆ alkyl" is specifically intended to individually disclose methyl, ethyl, C₃ alkyl, C₄ alkyl, C₅ alkyl, and C₆ alkyl.

The term "D" refers to a single deuterium atom.

The term "alkenyl" refers to a linear or branched-chain monovalent hydrocarbon radical containing 2 to 12 carbon atoms and at least one carbon-carbon, sp2 double bond, and includes radicals having *"cis"* and *"trans"* orientations, or alternatively, "*E*" and "*Z*" orientations. The alkenyl radical may be optionally substituted with one or more substituents described herein.

The term "alkynyl" refers to a linear or branched monovalent hydrocarbon radical containing 2 to 12 carbon atoms and at least one carbon-carbon, sp triple bond, wherein the alkynyl radical may be optionally substituted with one or more substituents described herein.

The term "halogen" or "halo" are used interchangeably in this invention, and refers to Fluoro (F), Chloro (Cl), Bromo (Br), or Iodo (I).

The term "alkoxy" refers to an alkyl group, as previously defined, attached to the parent molecular moiety via an oxygen atom. Unless otherwise specified, the alkoxy group contains 1-12 carbon atoms. In one embodiment, the alkoxy group contains 1-6 carbon atoms. In other embodiment, the alkoxy group contains 1-4 carbon atoms. In still other embodiment, the alkoxy group contains 1-3 carbon atoms. The alkoxy group may be optionally substituted with one or more substituents disclosed herein. As used herein, "alkoxyalkyl" means -(alkylenyl)-O-(alkyl), wherein each "alkyl" is independently an alkyl group defined above.

"Aryl" means a mono-, bi-, or tricyclic aromatic group, wherein all rings of the group are aromatic. For bi- or tricyclic systems, the individual aromatic rings are fused to one another. Exemplary aryl groups include, but are not limited to, phenyl, naphthalene, and anthracene.

The term "haloalkyl" refers to an alkyl group substituted with one or more halogen atoms, wherein the alkyl group is as defined herein. Some non-limiting examples of such groups include, but are not limited to -CF₃, -CF₂CF₃, -CH₂CF₂CHF₂, and the like. In one embodiment, "haloalkyl" refers to a lower C₁₋₄ haloalkyl, wherein the "C₁₋₄ haloalkyl" includes fluorine-substituted C₁₋₄ haloalkyl, chlorine-substituted C₁₋₄ haloalkyl, bromine-substituted C₁₋₄ haloalkyl, iodine-substituted C₁₋₄ haloalkyl, and the like. Specifically, fluorine-substituted C₁₋₄ haloalkyl includes - CH₂F, -CHF₂, -CF₃, -CH₂Cl, -CHCl₂, -CCl₃, -CH₂Br, -CHBr₂, -CBr₃, -CH₂CH₂F, -CH₂CHF₂, - CH₂CF₃, -CF₂CH₂F, -CF₂CHF₂, -CF₂CF₃, -CHFCF₃, -CHFCHF₂, -CHFCH₂F, -CH₂CH₂CF₃, - CH₂CF₂CHF₂ and the like. The haloalkyl is optionally substituted with one or more substituents described herein.

The term "aminoalkyl" refers to an alkyl group substituted with one or more amino groups, wherein the alkyl group is as defined herein, and the amino group is optionally substituted.

The term "hydroxy-substituted alkyl" or "hydroxyalkyl" refers to an alkyl group substituted with one or more hydroxy groups, wherein the alkyl group is as defined herein. Some non-limiting examples of such group include, but are not limited to hydroxymethyl, hydroxyethyl, 1,2-dihydroxyethyl, and the like.

The term "deuterium" as used herein means a stable isotope of hydrogen having one proton and one neutron.

The terms "carbocyclyl" and "carbocycle" as used interchangeably herein, refer to a monovalent or multivalent ring having 3 to 12 carbon atoms as a monocyclic, bicyclic or tricyclic ring system, which is saturated or contains one or more degrees of unsaturation, but an aromatic ring can not exist in the carbocyclyl group.

The term "hydroxy" means an -OH group.

The terms "heterocyclyl" and "heterocycle" as used interchangeably herein refer to a monovalent or multivalent monocyclic, bicyclic or tricyclic ring containing 3-12 carbon atoms, wherein each one or more atoms in the ring is independently replaced with heteroatom, the heteroatom is as defined herein, and the ring may be saturated or contains one or more degrees of unsaturations, but an aromatic ring can not exist in the aromatic ring.

The term "cycloalkyl" refers to a monovalent or multivalent saturated ring having 3 to 12 ring carbon atoms as a monocyclic, bicyclic, or tricyclic ring system.

Those skilled in the art will recognize that the species of heteroaryl, and cycloalkyl groups listed or illustrated above are not exhaustive, and that additional species within the scope of these defined terms may also be selected.

As described herein, compounds disclosed herein may optionally be substituted with one or more substituents, or as exemplified by particular classes, subclasses, and species of the invention.

As used herein, the term "substituted" means that the specified group or moiety bears one or more suitable substituents. As used herein, the term "unsubstituted" means that the specified group bears no substituents. As used herein, the term "optionally substituted" means that the specified group is unsubstituted or substituted by the specified number of substituents. Where the term "substituted" is used to describe a structural system, the substitution is meant to occur at any valency-allowed position on the system.

As used herein, the expression "one or more substituents" denotes one to maximum possible number of substitution(s) that can occur at any valency-allowed position on the system. In a certain embodiment, one or more substituent means 1, 2, 3, 4, or 5 substituents. In another embodiment, one or more substituent means 1, 2, or 3 substituents.

Any atom that is represented herein with an unsatisfied valence is assumed to have the sufficient number of hydrogen atoms to satisfy the atom's valence.

When any variable (e.g., alkyl, alkylenyl, heteroaryl, R¹, R², or R^{a}) appears in more than one place in any formula or description provided herein, the definition of that variable on each occurrence is independent of its definition at every other occurrence.

Numerical ranges, as used herein, are intended to include sequential whole numbers. For example, a range expressed as "from 0 to 4" or "0-4" includes 0, 1, 2, 3 and 4, while a range expressed as "10-20%" includes 10%, 11%, 12%, 13%, 14%, 15%, 16%, 17%, 18%, 19% and 20%. Similarly, numerical ranges are also intended to include sequential fractional integers. For example, a range expressed as "1-2%" would include 1.0%, 1.1%, 1.2%, 1.3%, 1.4%, 1.5%, 1.6%, 1.7%, 1.8%, 1.9% and 2.0%.

When a multifunctional moiety is shown, the point of attachment to the core is indicated by a line or hyphen. For example, aryloxy- refers to a moiety in which an oxygen atom is the point of attachment to the core molecule while aryl is attached to the oxygen atom.

### Additional Definitions

As used herein, the term "subject" encompasses mammals and non-mammals. Examples of mammals include, but are not limited to, any member of the Mammalian class: humans; non-human primates such as chimpanzees, and other apes and monkey species; farm animals such as cattle, horses, sheep, goats, swine; domestic animals such as rabbits, dogs, and cats; and laboratory animals including rodents, such as rats, mice and guinea pigs, and the like. Examples of non-mammals include, but are not limited to, birds, fish and the like. In one embodiment of the present invention, the mammal is a human.

"Patient" includes both human and animals.

The term "inhibitor" refers to a molecule such as a compound, a drug, an enzyme activator, or a hormone that blocks or otherwise interferes with a particular biologic activity.

The term "modulator" refers to a molecule, such as a compound of the present invention, that increases or decreases, or otherwise affects the activity of a given protein, receptor and / or ion channels.

The terms "effective amount" or "therapeutically effective amount" refer to a sufficient amount of the agent to provide the desired biological result. That result can be reduction and/or alleviation of the signs, symptoms, or causes of a disease or medical condition, or any other desired alteration of a biological system. For example, an "effective amount" for therapeutic use is the amount of a compound, or of a composition comprising the compound, that is required to provide a clinically relevant change in a disease state, symptom, or medical condition. An appropriate "effective" amount in any individual case may be determined by one of ordinary skill in the art using routine experimentation. Thus, the expression "effective amount" generally refers to the quantity for which the active substance has a therapeutically desired effect.

As used herein, the terms "treat" or "treatment" encompass both "preventative" and "curative" treatment. "Preventative" treatment is meant to indicate a postponement of development of a disease, a symptom of a disease, or medical condition, suppressing symptoms that may appear, or reducing the risk of developing or recurrence of a disease or symptom. "Curative" treatment includes reducing the severity of or suppressing the worsening of an existing disease, symptom, or condition. Thus, treatment includes ameliorating or preventing the worsening of existing disease symptoms, preventing additional symptoms from occurring, ameliorating or preventing the underlying metabolic causes of symptoms, inhibiting the disorder or disease, e.g., arresting the development of the disorder or disease, relieving the disorder or disease, causing regression of the disorder or disease, relieving a condition caused by the disease or disorder, or stopping the symptoms of the disease or disorder.

As used herein, the terms "administration of" and "administering a" compound should be understood to mean providing a compound of the invention, pharmaceutical composition comprising a compound or a prodrug of a compound of the invention to an individual in need thereof. It is recognized that one skilled in the non-limiting art can treat a patient presently afflicted with neurological and psychiatric disorders or by prophylactically treat a patient afflicted with the disorders with an effective amount of the compound of the present invention.

The term "composition" as used herein is intended to encompass a product comprising the specified ingredients in the specified amounts, as well as any product which results, directly or indirectly, from combinations of the specified ingredients in the specified amounts. Such term in relation to pharmaceutical composition, is intended to encompass a product comprising the active ingredient(s) and the inert ingredient(s) that make up the carrier, as well as any product which results, directly or indirectly, from a combination, complexation or aggregation of any two or more of the ingredients, or from the other types of reactions or interactions such as to cause the dissociation of one or more of the ingredients. Accordingly, the pharmaceutical compositions of the present invention encompass any composition made by mixing a compound of the present invention and a pharmaceutically acceptable carrier.

### Additional Chemical Descriptions

Any formula given herein is intended to represent compounds having structures depicted by the structural formula as well as certain variations or forms. For example, compounds of any formula given herein may have asymmetric or chiral centers and therefore exist in different stereoisomeric forms. All stereoisomers, including optical isomers, enantiomers, and diastereomers, of the compounds of the general formula, and mixtures thereof, are considered to fall within the scope of the formula. Furthermore, certain structures may exist as geometric isomers (*i.e., cis* and *trans* isomers), as tautomers, or as atropisomers. All such isomeric forms, and mixtures thereof, are contemplated herein as part of the present invention. Thus, any formula given herein is intended to represent a racemate, one or more enantiomeric forms, one or more diastereomeric forms, one or more tautomeric or atropisomeric forms, and mixtures thereof.

"Stereoisomer" refers to compounds which have identical chemical constitution, but differ with regard to the arrangement of the atoms or groups in space. Stereoisomers include enantiomer, diastereomers, conformer (rotamer), geometric (cis / trans) isomer, atropisomer etc.

"Chiral" refers to molecules which have the property of non-superimposability of the mirror image partner, while the term "achiral" refers to molecules which are superimposable on their mirror image partner.

"Enantiomers" refers to two stereoisomers of a compound which are non-superimposable mirror images of one another.

"Diastereomer" refers to a stereoisomer with two or more centers of chirality and whose molecules are not mirror images of one another. Diastereomers have different physical properties, *e.g*., melting points, boiling points, spectral properties or biological activities. A mixture of diastereomers may be separated under high resolution analytical procedures such as electrophoresis and chromatography such as HPLC.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994.

Many organic compounds exist in optically active forms, *i.e.,* they have the ability to rotate the plane of polarized light. In describing an optically active compound, the prefixes D and L, or *R* and *S*, are used to denote the absolute configuration of the molecule about its chiral center(s). The prefixes *d* and *l* or (+) and (-) are employed to designate the sign of rotation of plane-polarized light by the compound, with (-) or *l* meaning that the compound is levorotatory. A compound prefixed with (+) or *d* is dextrorotatory. A specific stereoisomer may be referred to as an enantiomer, and a mixture of such stereoisomers is called an enantiomeric mixture. A 50:50 mixture of enantiomers is referred to as a racemic mixture or a racemate, which may occur where there has been no stereoselection or stereospecificity in a chemical reaction or process.

Any asymmetric atom (*e.g*., carbon or the like) of the compound(s) disclosed herein can be in racemic or enantiomerically enriched, for example the (*R*)-*,* (*S*)- or (*R*,*S*)- configuration. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess in the (*R*)*-* or (*S*)- configuration.

Depending on the choice of the starting materials and procedures, the compounds can be present in the form of one of the possible stereoisomers or as mixtures thereof, such as racemates and diastereoisomer mixtures, depending on the number of asymmetric carbon atoms. Optically active (*R*)- and (*S*)- isomers may be prepared using chiral synthons or chiral reagents, or resolved using conventional techniques. If the compound contains a double bond, the substituent may be *E* or *Z* configuration. If the compound contains a disubstituted cycloalkyl, a cycloalkyl substituent may have a *cis-* or *trans*-configuration relative to another substituent of the same cycloalkyl frame.

Any resulting mixtures of stereoisomers can be separated on the basis of the physicochemical differences of the constituents, into the pure or substantially pure geometric isomers, enantiomers, diastereomers, for example, by chromatography and / or fractional crystallization. Any resulting racemates of final products or intermediates can be resolved into the optical antipodes by methods known to those skilled in the art, *e.g.,* by separation of the diastereomeric salts thereof. Racemic products can also be resolved by chiral chromatography, e.g., high performance liquid chromatography (HPLC) using a chiral adsorbent. Preferred enantiomers can also be prepared by asymmetric syntheses. See, for example, Jacques, et al., Enantiomers, Racemates and Resolutions (Wiley Interscience, New York, 1981); Principles of Asymmetric Synthesis (2nd Ed. Robert E. Gawley, Jeffrey Aubé, Elsevier, Oxford, UK, 2012); Eliel, E.L. Stereochemistry of Carbon Compounds (McGraw-Hill, NY, 1962); Wilen, S.H. Tables of Resolving Agents and Optical Resolutions p. 268 (E.L. Eliel, Ed., Univ. of Notre Dame Press, Notre Dame, IN 1972); Chiral Separation Techniques: A Practical Approach (Subramanian, G. Ed., Wiley-VCH Verlag GmbH & Co. KGaA, Weinheim, Germany, 2007).

Diastereomeric mixtures may be separated into their individual diastereomers on the basis of their physical chemical differences by methods well known to those skilled in the art, such as, for example, by chromatography and/or fractional crystallization. Enantiomers may be separated by converting the enantiomeric mixture into a diastereomeric mixture by reaction with an appropriate optically active compound (*e.g*., chiral auxiliary such as a chiral alcohol or Mosher's acid chloride, or formation of a mixture of diastereomeric salts), separating the diastereomers and converting (*e.g*., hydrolyzing or de-salting) the individual diastereomers to the corresponding pure enantiomers. Enantiomers may also be separated by use of chiral HPLC column.

The compounds of the invention can form pharmaceutically acceptable salts, which are also within the scope of this invention. A "pharmaceutically acceptable salt" refers to a salt of a free acid or base of a compound of Formula A that is non-toxic, is physiologically tolerable, is compatible with the pharmaceutical composition in which it is formulated, and is otherwise suitable for formulation and / or administration to a subject. Reference to a compound herein is understood to include reference to a pharmaceutically acceptable salt of said compound unless otherwise indicated.

Compound salts include acidic salts formed with inorganic and / or organic acids, as well as basic salts formed with inorganic and / or organic bases. In addition, where a given compound contains both a basic moiety, such as, but not limited to, a pyridine or imidazole, and an acidic moiety, such as, but not limited to, a carboxylic acid, one of skill in the art will recognize that the compound may exist as a zwitterion ("inner salt"); such salts are included within the term "salt" as used herein. Salts of the compounds of the invention may be prepared, for example, by reacting a compound with an amount of a suitable acid or base, such as an equivalent amount, in a medium such as one in which the salt precipitates or in an aqueous medium followed by lyophilization.

Exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate ("mesylate"), ethanesulfonate, benzenesulfonate, p-toluenesulfonate, and pamoate (*i.e.,* 1,1'-methylene-bis(2-hydroxy-3-naphthoate)) salts. A pharmaceutically acceptable salt may involve the inclusion of another molecule such as an acetate ion, a succinate ion or other counterion. The counterion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counterions. Hence, a pharmaceutically acceptable salt can have one or more charged atoms and/or one or more counter ion.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates,) and the like.

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamines, *tert*-butyl amines, and salts with amino acids such as arginine, lysine and the like. Basic nitrogen-containing groups may be quarternized with agents such as lower alkyl halides (*e.g*., methyl, ethyl, and butyl chlorides, bromides and iodides), dialkyl sulfates (*e.g*., dimethyl, diethyl, and dibutyl sulfates), long chain halides (*e.g.,* decyl, lauryl, and stearyl chlorides, bromides and iodides), aralkyl halides (*e.g.,* benzyl and phenethyl bromides), and others.

Additionally, acids and bases which are generally considered suitable for the formation of pharmaceutically useful salts from pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, MD, available from FDA). These disclosures are incorporated herein by reference thereto.

Additionally, any compound described herein is intended to refer also to any unsolvated form, or a hydrate, solvate, or polymorph of such a compound, and mixtures thereof, even if such forms are not listed explicitly. "Solvate" means a physical association of a compound of the invention with one or more solvent molecules. This physical association involves varying degrees of ionic and covalent bonding, including hydrogen bonding. In certain instances, the solvate will be capable of isolation, for example when one or more solvent molecules are incorporated in the crystal lattice of a crystalline solid. "Solvate" encompasses both solution-phase and isolatable solvates. Suitable solvates include those formed with pharmaceutically acceptable solvents such as water, ethanol, and the like. In some embodiments, the solvent is water and the solvates are hydrates.

One or more compounds of the invention may optionally be converted to a solvate. Methods for the preparation of solvates are generally known. Thus, for example, M. Caira et al., J. Pharmaceutical Sci., 93(3), 601-611 (2004), describes the preparation of the solvates of the antifungal fluconazole in ethyl acetate as well as from water. Similar preparations of solvates, hemisolvate, hydrates, and the like are described by E. C. van Tonder et al, AAPS PharmSciTech., 5(1), article 12 (2004); and A. L. Bingham et al, Chem. Commun., 603-604 (2001). A typical, non-limiting process involves dissolving the compound of the invention in a suitable amount of the solvent (organic solvent or water or a mixture thereof) at a higher than ambient temperature and cooling the solution at a rate sufficient to form crystals which are then isolated by standard methods. Analytical techniques such as, for example, infrared spectroscopy, show the presence of the solvent (or water) in the crystals as a solvate (or hydrate).

The present invention also relates to pharmaceutically active metabolites of compounds of Formula (A), and uses of such metabolites in the methods of the invention. A "pharmaceutically active metabolite" means a pharmacologically active product of metabolism in the body of a compound of Formula (A) or salt thereof. Active metabolites of a compound may be determined using routine techniques known or available in the art. See, *e.g.,* Bertolini et al., J. Med. Chem. 1997, 40, 2011-2016; Shan et al., J. Pharm. Sci. 1997, 86 (7), 765-767; Bagshawe, Drug Dev. Res. 1995, 34, 220-230; Bodor, Adv. Drug Res. 1984, 13, 255-331; Bundgaard, Design of Prodrugs (Elsevier Press, 1985); and Larsen, Design and Application of Prodrugs, Drug Design and Development (Krogsgaard-Larsen et al., eds., Harwood Academic Publishers, 1991).

Any formula given herein is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the invention include isotopes of hydrogen, carbon, nitrogen, oxygen, phosphorous, fluorine, chlorine, and iodine, such as ²H, ³H, ¹¹C, ¹³C, ¹⁴C, ¹⁵N, ¹⁸O, ¹⁷O, ³¹P, ³²P, ³⁵S, ¹⁸F , ³⁶Cl, and ¹²⁵I, respectively. Such isotopically labelled compounds are useful in metabolic studies (for example with ¹⁴C), reaction kinetic studies (with, for example ²H or ³H), detection or imaging techniques [such as positron emission tomography (PET) or single-photon emission computed tomography (SPECT) including drug or substrate tissue distribution assays, or in radioactive treatment of patients. In particular, an ¹⁸F or ¹¹C labeled compound may be particularly suitable for PET or SPECT studies. Further, substitution with heavier isotopes such as deuterium (*i*.*e*., ²H) may afford certain therapeutic advantages resulting from greater metabolic stability, for example increased *in vivo* half-life or reduced dosage requirements. Isotopically labeled compounds of this invention can generally be prepared by carrying out the procedures disclosed in the schemes or in the examples and preparations described below by substituting a readily available isotopically labeled reagent for a non-isotopically labeled reagent.

The use of the terms "salt," "solvate," "polymorph," and the like, with respect to the compounds described herein is intended to apply equally to the salt, solvate, and polymorph forms of enantiomers, stereoisomers, rotamers, tautomers, atropisomers, and racemates of the compounds of the invention.

### DESCRIPTION OF COMPOUNDS OF THE INVENTION

Provided herein are prodrugs of (*S*)- or (*R*)-ketamine, including isotopically labeled ketamine, compositions and uses thereof. More specifically, the compounds having Formula (Ia) ~ (Vd) disclosed herein as prodrugs of (*S*)- or (*R*)-ketamine, including isotopically labeled ketamine, can be used as NMDA receptor antagonists for treating, preventing or lessening neurological and psychiatric disorder or disease of the central nervous system related to NMDA receptor, and the pharmaceutical compositions disclosed herein also have functions of prevention, treatment or lessening of a disease related to NMDA receptor.

In one embodiment of the present invention, there is provided a compound having the structure of Formula (Ia) or (Ib), or a stereoisomer, an N-oxide, a solvate, a metabolite, a pharmaceutically acceptable salt or a prodrug thereof: 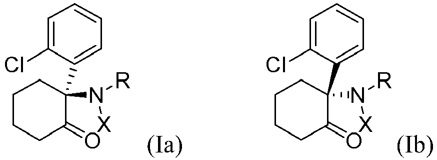
wherein R is -C(=O)R¹, -C(=O)OR², -C(=O)O(CHR³)OC(=O)R⁴ or -CD₃; and X is -CH₃ or - CD₃.
wherein R¹ is, optionally, substituted or unsubstituted aryl-OH, aryl-NH₂, alkenyl-OH, alkenyl-NH₂, alkyl-NH₂, alkyl-OH, carbocyclyl or heterocyclyl containing one or more N or O; and X is -CH₃ or -CD₃;
wherein R² is optionally substituted or unsubstituted alkyl, aryl, carbocyclyl or heterocyclyl containing one or more O; and X is -CH₃ or -CD₃;
wherein R⁴ is independently substituted or unsubstituted alkyl, aryl, azaaryl, carbocyclyl or heterocyclyl containing one or more O or N, while R³ is H or substituted or unsubstituted alkyl; and X is -CH₃ or -CD₃.

In another aspect, provided herein is a compound having the structure of Formula (IIa) or (IIb), or a stereoisomer, an N-oxide, a solvate, a metabolite, a pharmaceutically acceptable salt or a prodrug thereof: 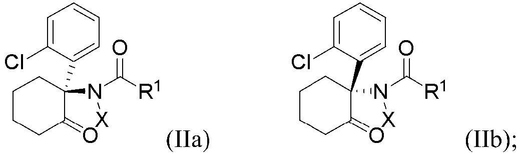 wherein R¹ is, optionally, substituted or unsubstituted aryl-OH, aryl-NH₂, alkenyl-OH, alkenyl-NH₂, alkyl-NH₂, alkyl-OH, carbocyclyl or heterocyclyl containing one or more N or O; and X is -CH₃ or -CD₃.

In one embodiment, R¹ is aminoC₁₋₆ alkyl, -R^{1a}NHCOR^{1b}, -R^{1a}OCOR^{1b}, -R^{1a}COOR^{1b}, 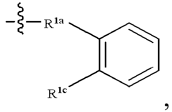 or C₃₋₆ heterocyclyl,
wherein R¹ is optionally substituted with C₁₋₆ alkyl, -OH or oxo(=O),
wherein R^{1a} and R^{1b} is independently H, C₁₋₆ alkyl or C₂₋₆ alkenyl, and
R^{1c} is -OH, C₁₋₃ hydroxyalkyl, -OCOR^{1b} or -CH₂ OCOR^{1b}.

In one embodiment, the heterocyclyl containing one or more N or O is

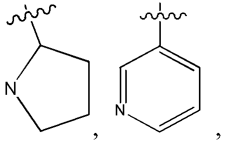

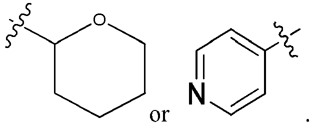

In another aspect, provided herein is a compound selected from the group consisting of 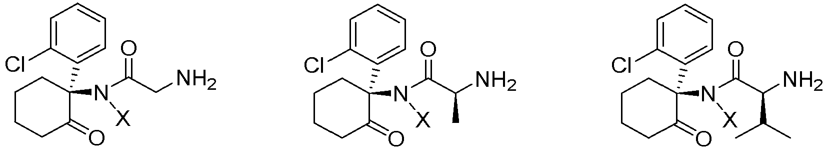 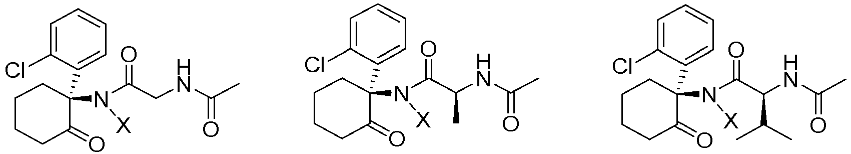 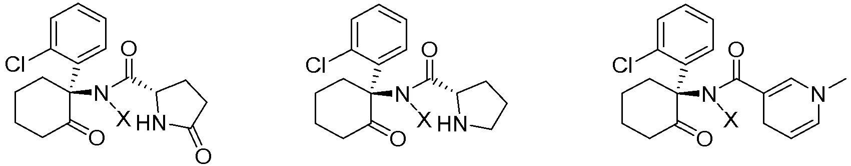 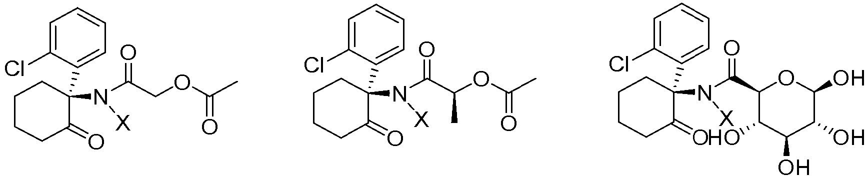 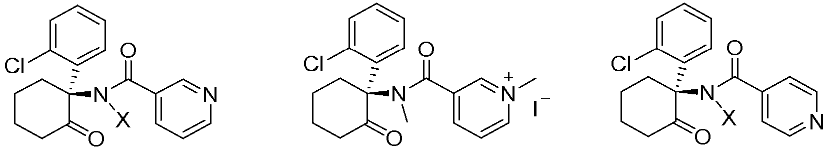 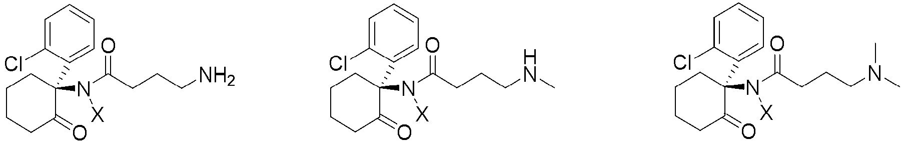 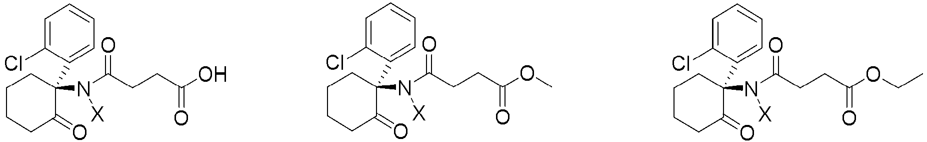 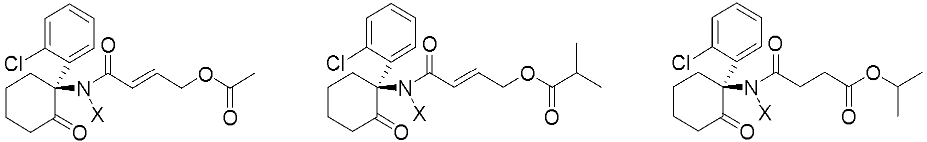 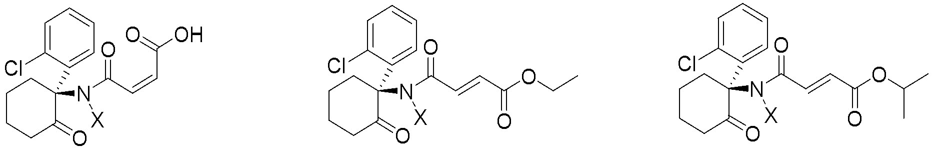 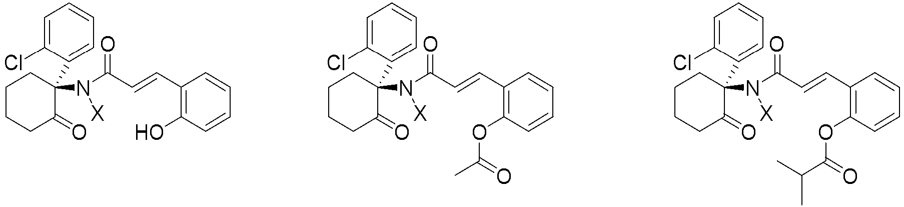 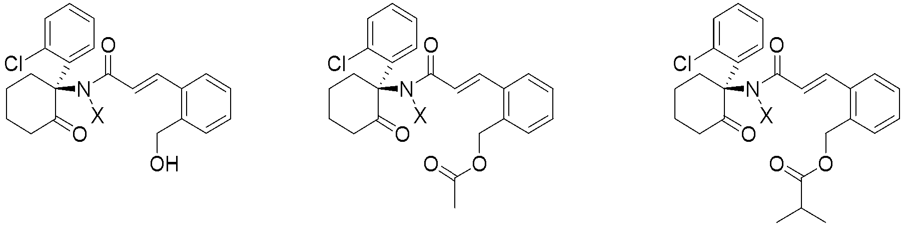 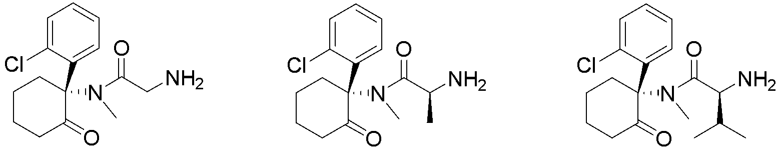 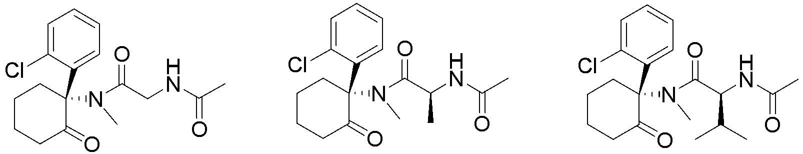 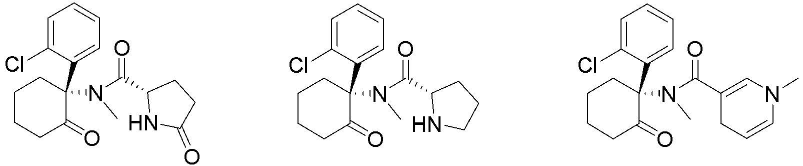 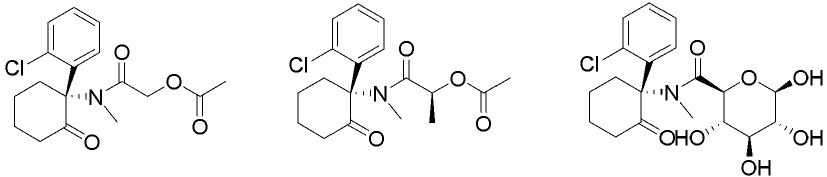 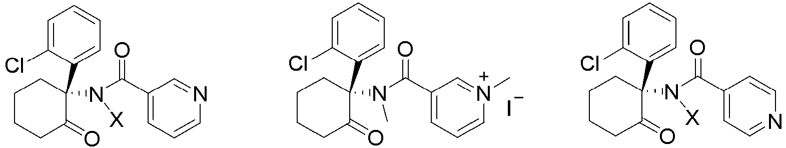 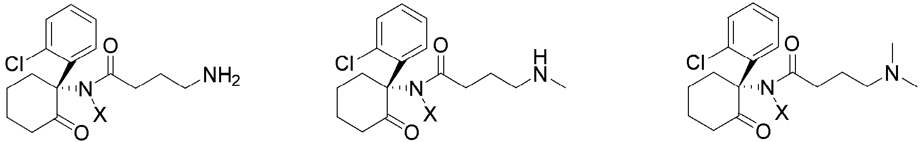 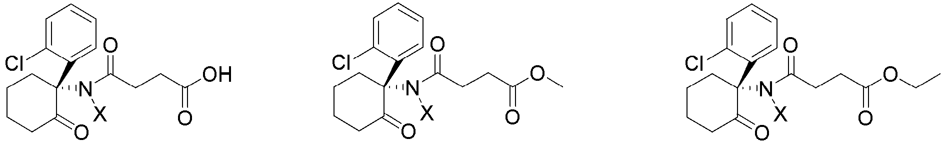 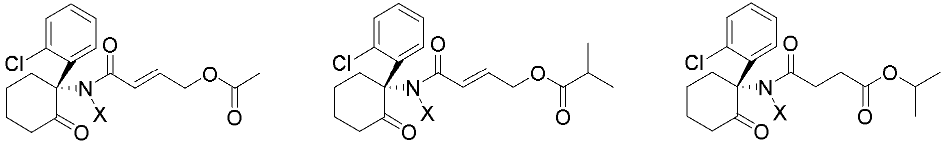 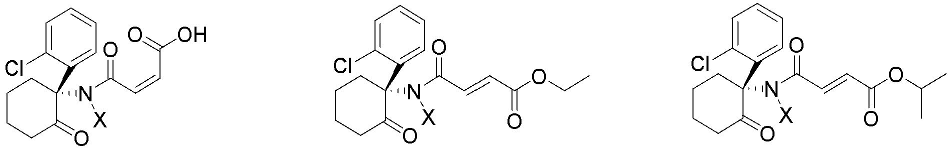 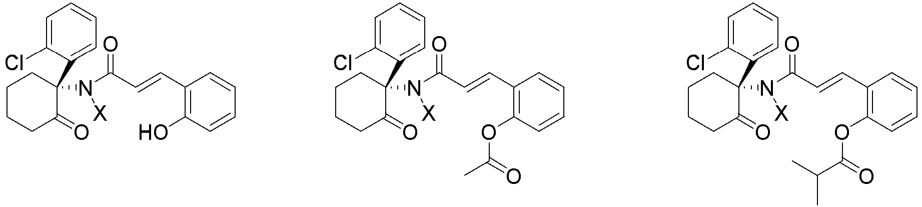 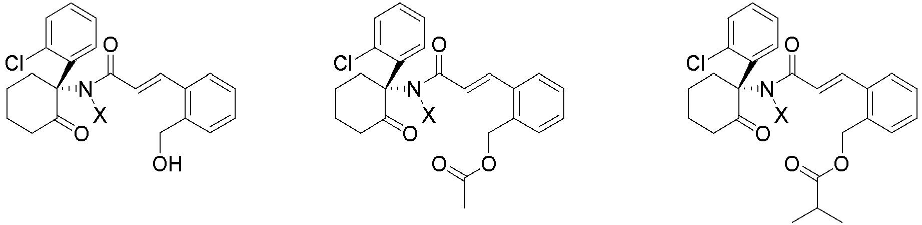 wherein X is -CH₃ or -CD₃.

In another aspect, provided herein is a compound having the structure of Formula (IIIa) or (IIIb), or a stereoisomer, an *N*-oxide, a solvate, a metabolite, a pharmaceutically acceptable salt or a prodrug thereof: 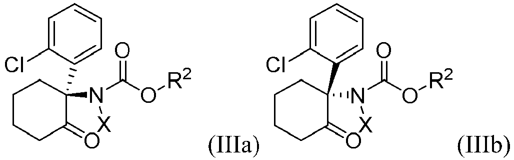 wherein R² is optionally substituted or unsubstituted alkyl, aryl, carbocyclyl or heterocyclyl containing one or more O; and X is -CH₃ or -CD₃.

In one embodiment, R² is C₁₋₆ alkyl, C₁₋₆ hydroxyalkyl, aminoC₁₋₆ alkyl, -R^{2a}S(O)ₙ₁R^{2b}, - R^{2a}COOR^{2b}, C₃₋₆ aryl or C₃₋₆ heterocyclyl,
wherein R² is optionally substituted with C₁₋₆ alkyl, -OH, C₁₋₆ hydroxyalkyl, 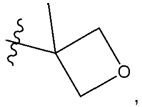 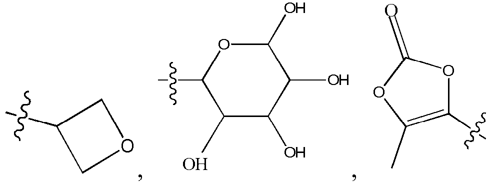 or -R^{2a}COOR^{2b}, given that C₁₋₆ alkyl is substituted with 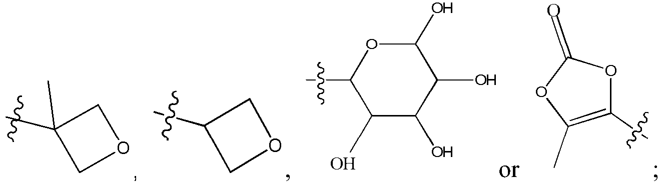
R^{2a} is C₁₋₆ alkyl, wherein R^{2a} is optionally substituted with C₁₋₆ alkyl or -NH₂;
R^{2b} is H or C₁₋₆ alkyl; and
n₁ is 0, 1, 2.

In another aspect, provided herein is a compound selected from the group consisting of 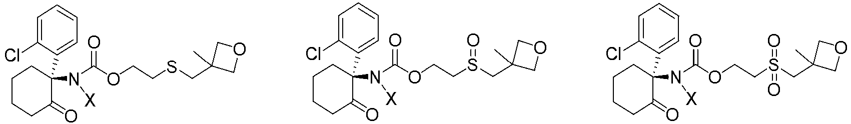 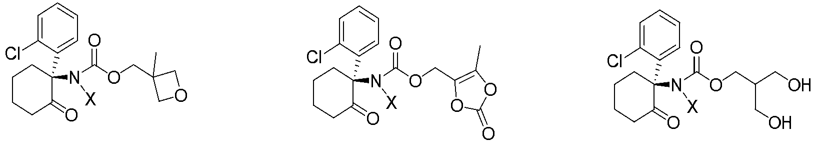 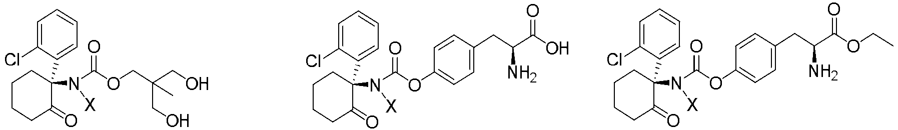 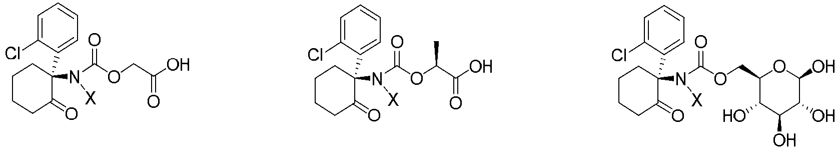 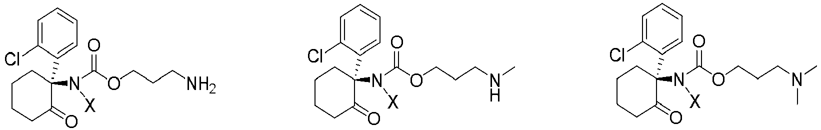 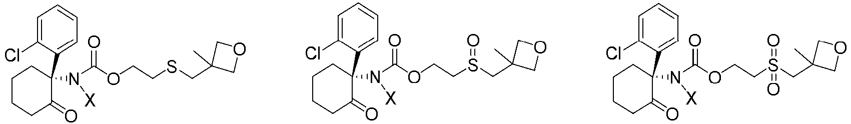 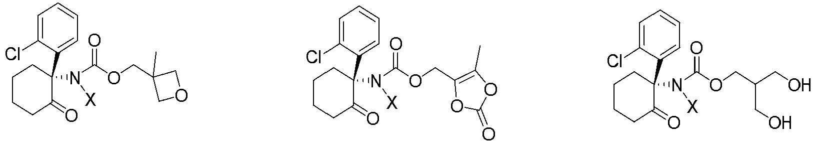 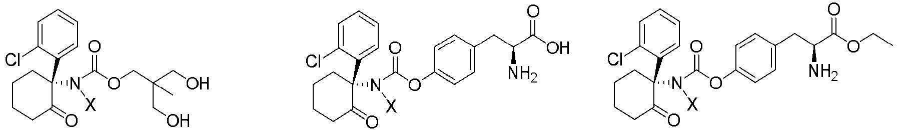 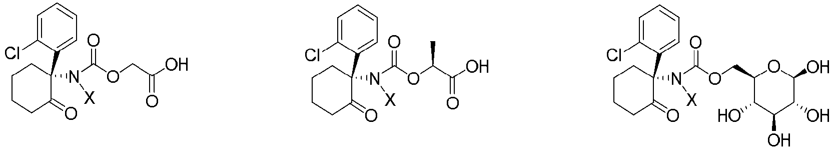 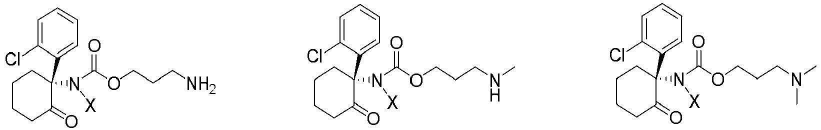 wherein X is -CH₃ or -CD₃.

In another aspect, provided herein is a compound having the structure of Formula (IVa) or (IVb), or a stereoisomer, an N-oxide, a solvate, a metabolite, a pharmaceutically acceptable salt or a prodrug thereof: 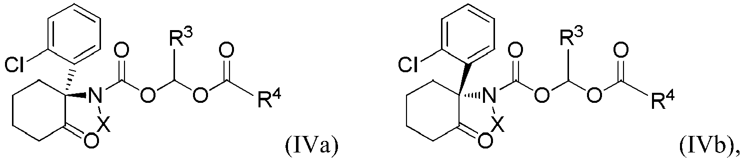 wherein R⁴ is independently substituted or unsubstituted alkyl, aryl, azaaryl, carbocyclyl or heterocyclyl containing one or more O or N, while R³ is H or substituted or unsubstituted alkyl; and X is -CH₃ or -CD₃.

In one embodiment, R³ is H or C₁₋₆ alkyl.

In one embodiment, R⁴ is C₁₋₆ alkyl, aminoC₁₋₆ alkyl, C₁₋₆ hydroxyalkyl, -R^{4a}NCOR^{4b}, - R^{4a}OCOR^{4b}, -R^{4a}S(O)ₙ₂R^{4b}, C₁₋₆ heterocyclyl, C₁₋₅ azaaryl or 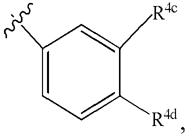
wherein R⁴ is optionally substituted with C₁₋₆ alkyl, -NH₂, oxo(=O), C₁₋₆ hydroxyalkyl, 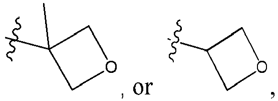 given that C₁₋₆ alkyl is substituted with 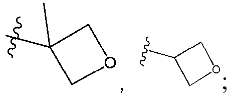
wherein R^{4a} is C₁₋₆ alkyl, R^{4b} is C₁₋₆ alkyl or C₁₋₆ haloalkyl;
R^{4c} is benzyl, R^{4d} is H, or R^{4c} and R^{4d}, together with the carbon atoms to which they are attached, form a C₅₋₆ heterocyclyl; and
n₂ is 0,1 or 2.

In one embodiment, C₁₋₆ heterocyclyl is

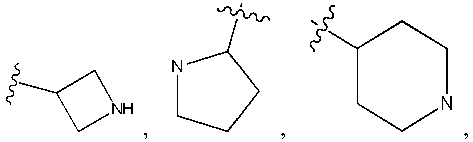

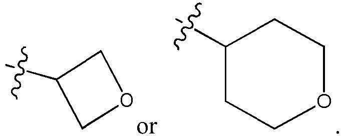

In one embodiment, C₁₋₅ azaaryl is 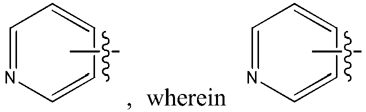 is optionally substituted with one or more methyl or -NH₂., or the combination thereof.

In one embodiment, 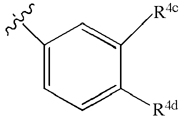 is 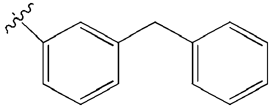 or 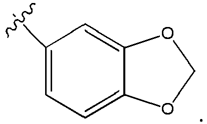 In another aspect, provided herein is a compound selected from the group consisting of 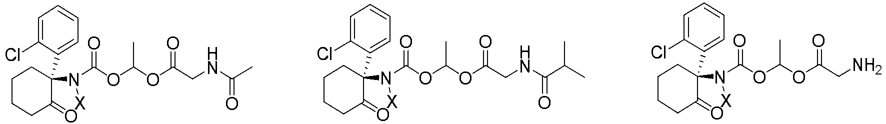 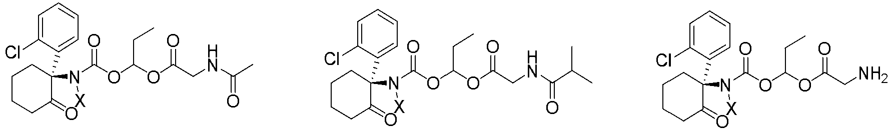 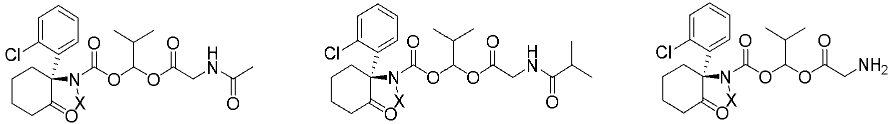 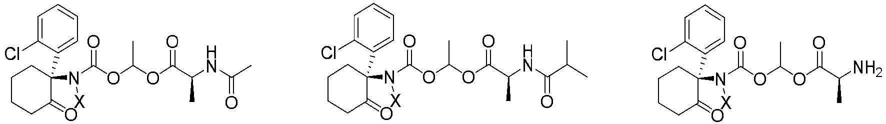 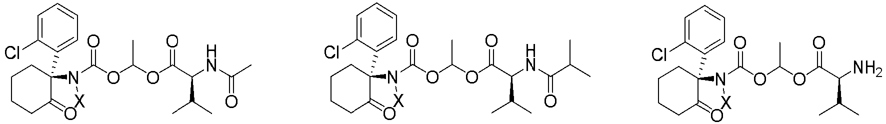 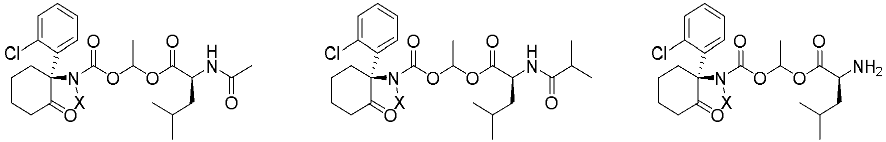 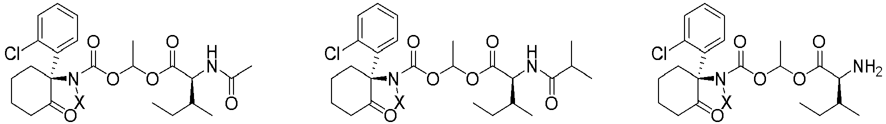 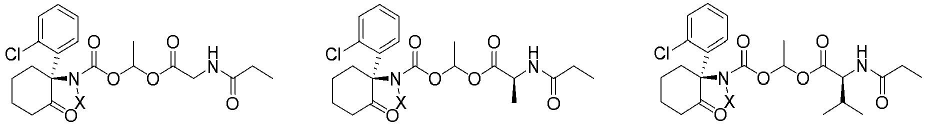 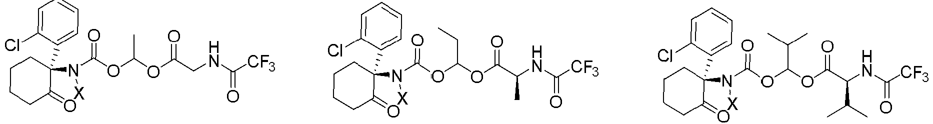 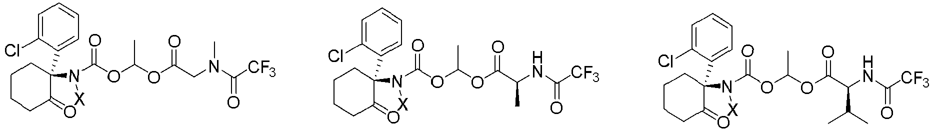 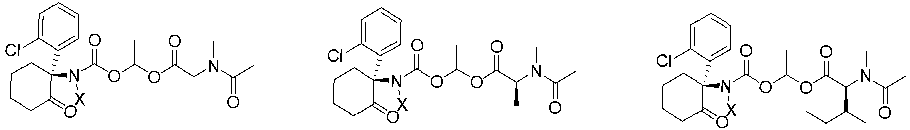 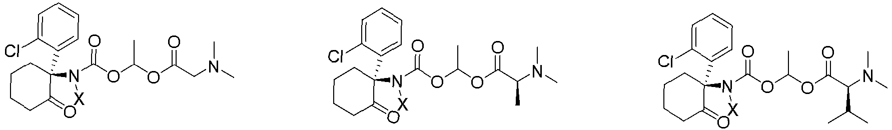 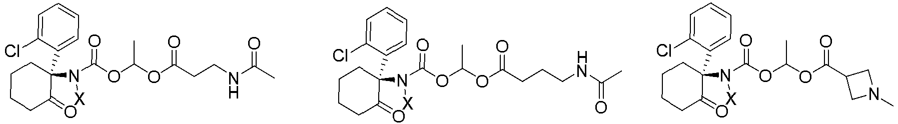 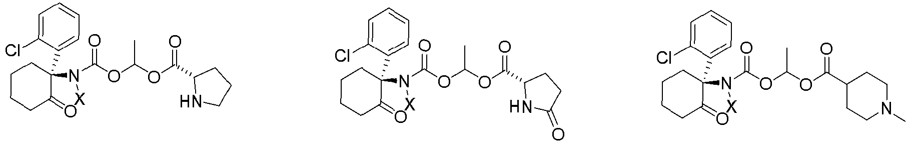 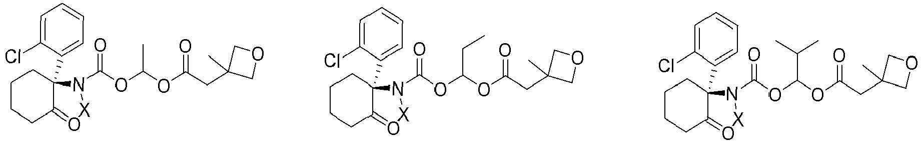 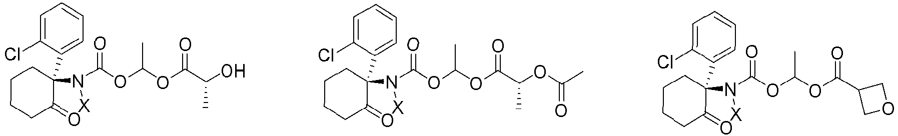 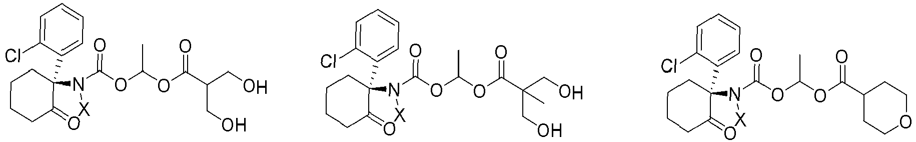 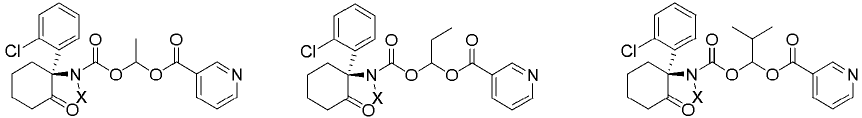 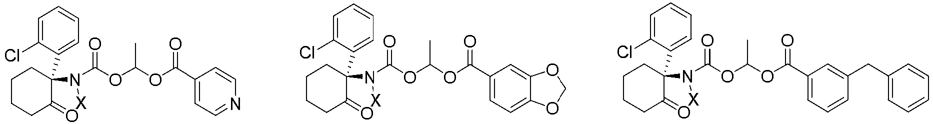 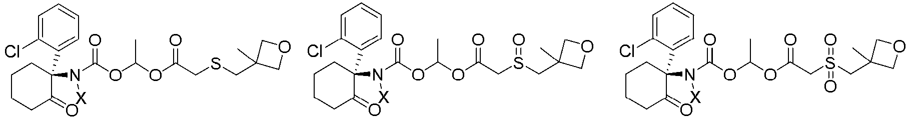 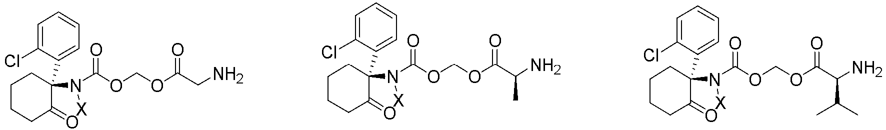 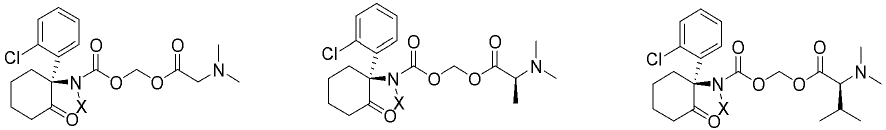 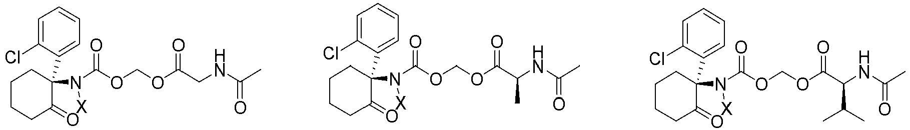 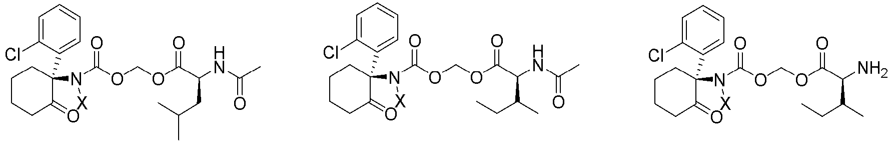 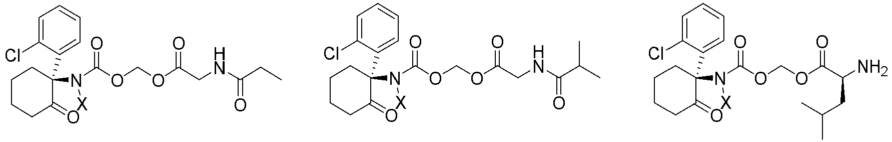 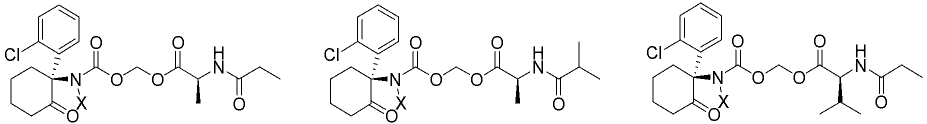 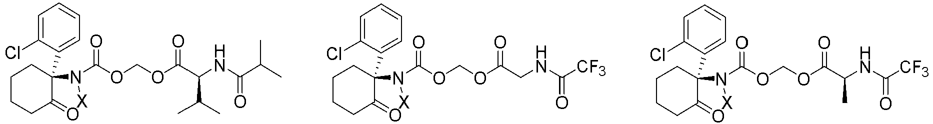 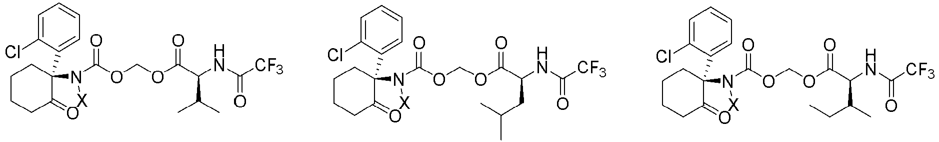 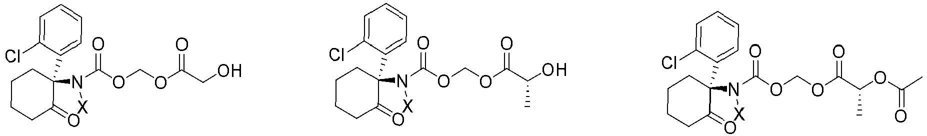 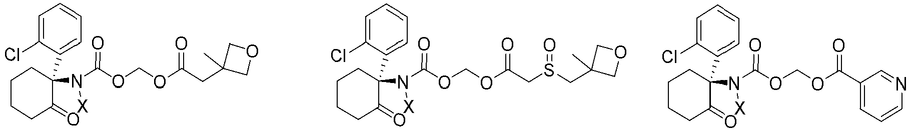 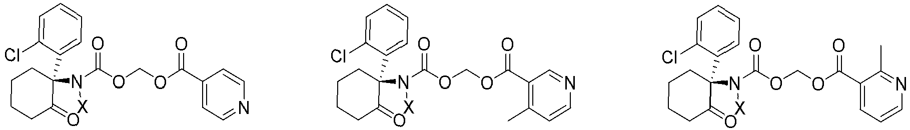 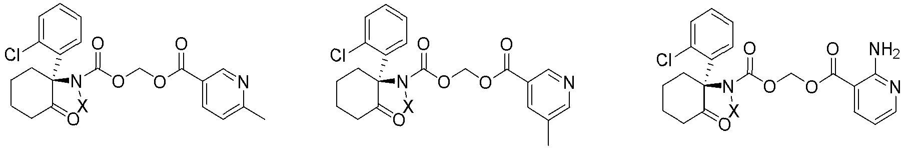 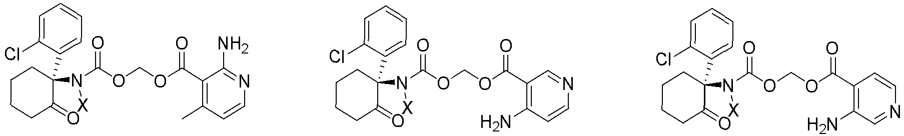 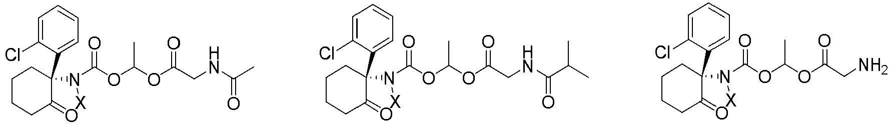 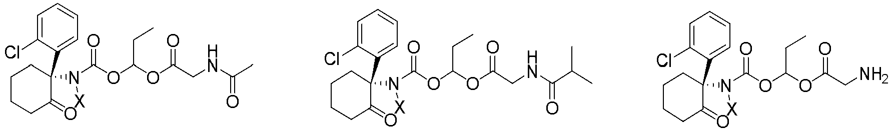 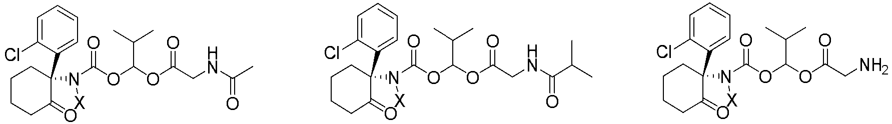 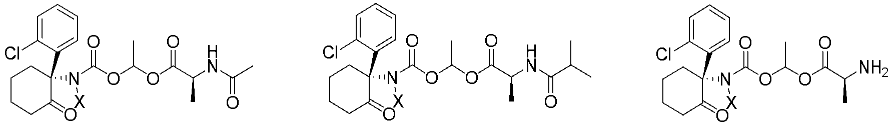 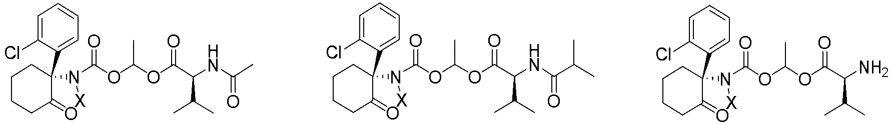 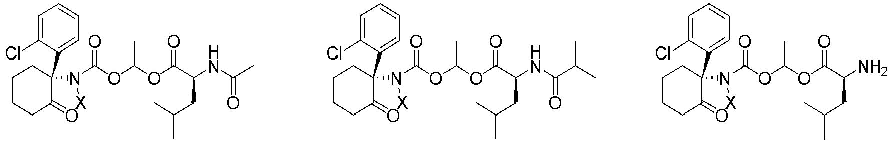 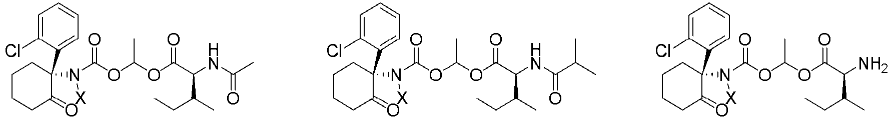 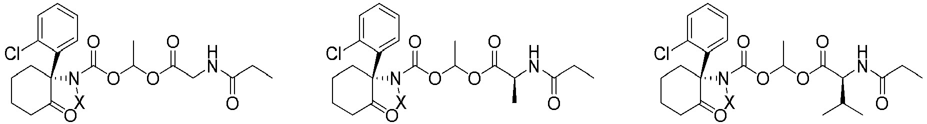 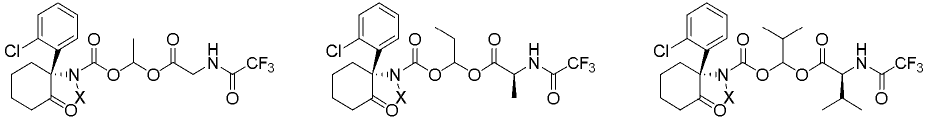 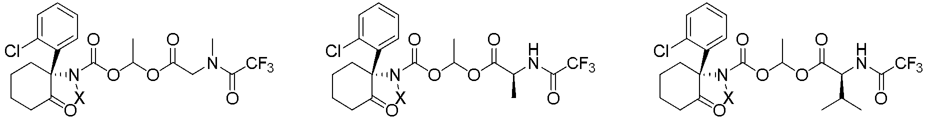 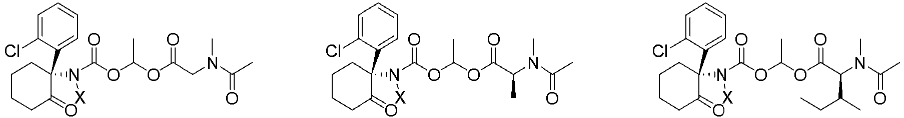 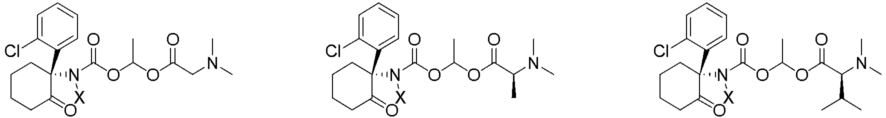 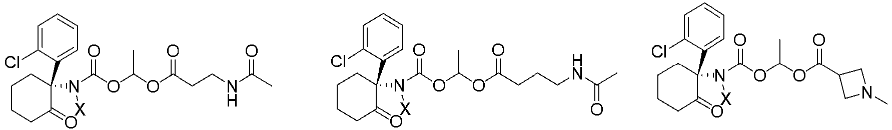 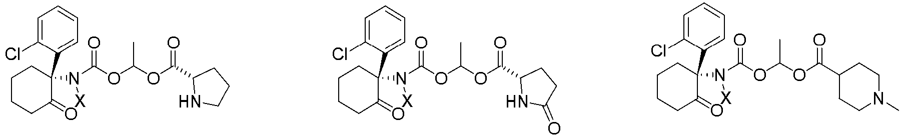 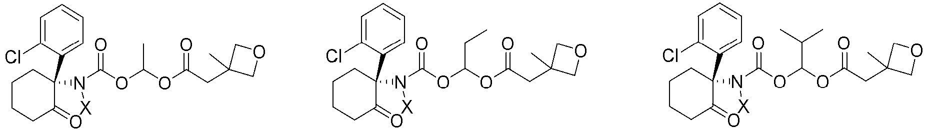 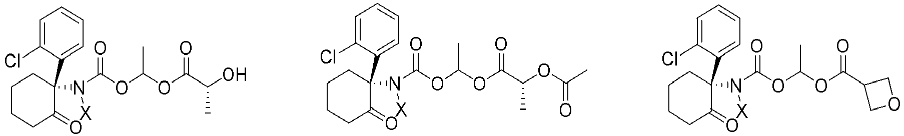 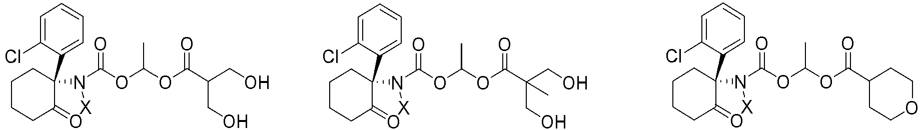 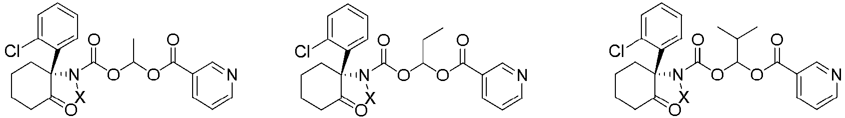 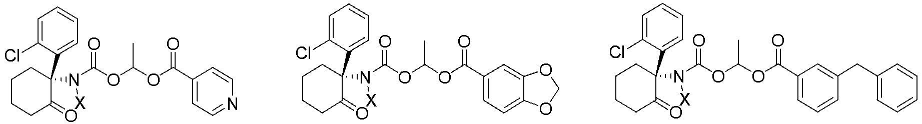 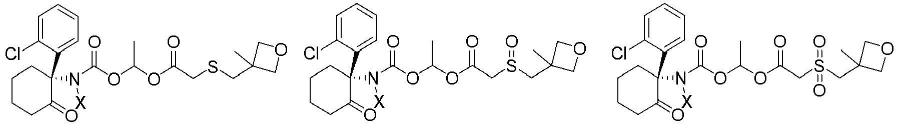 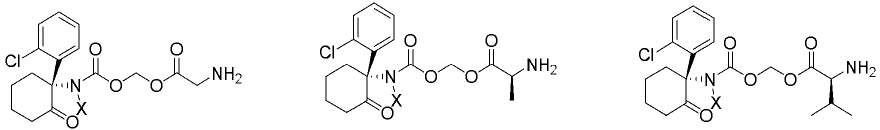 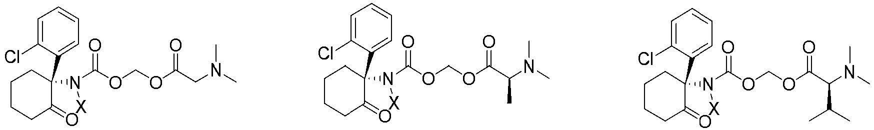 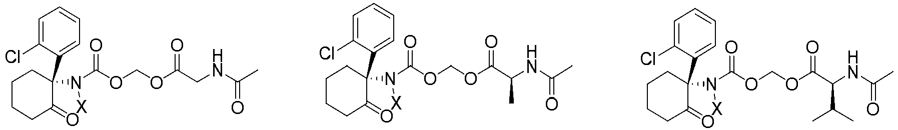 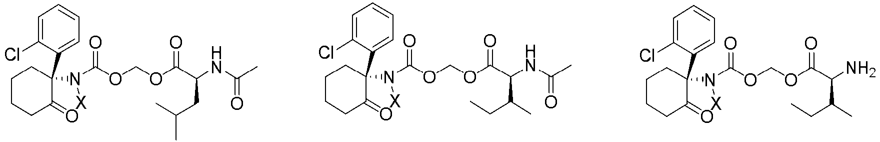 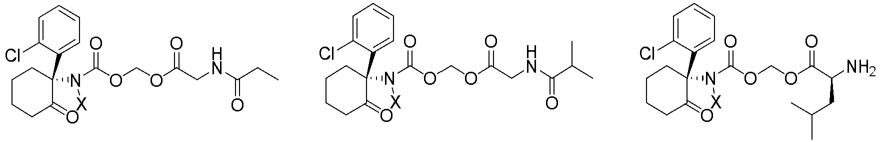 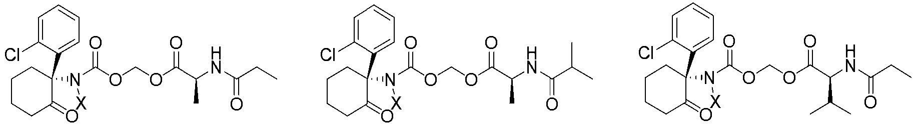 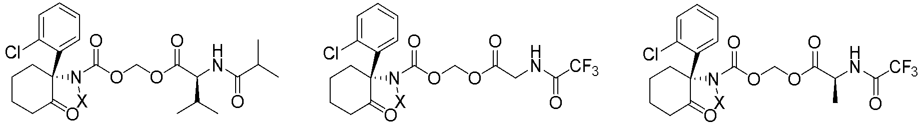 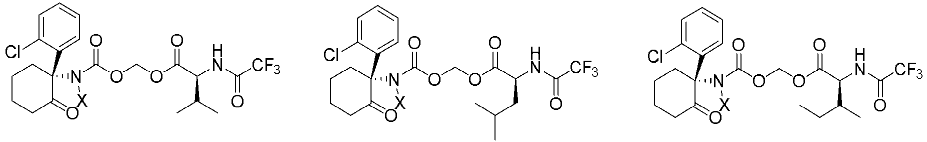 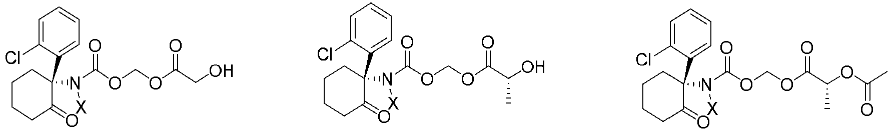 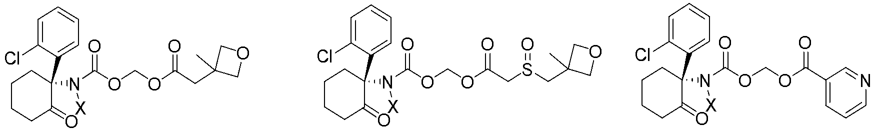 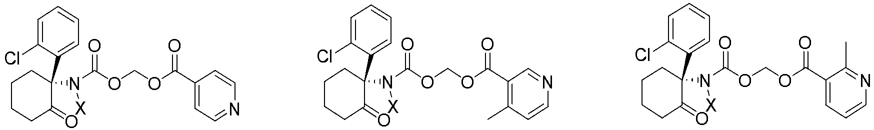 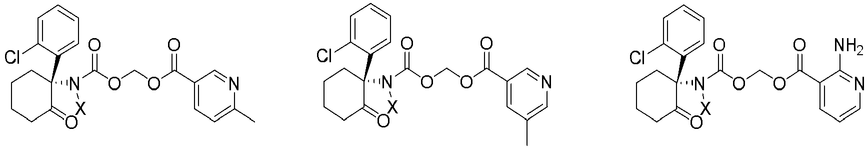 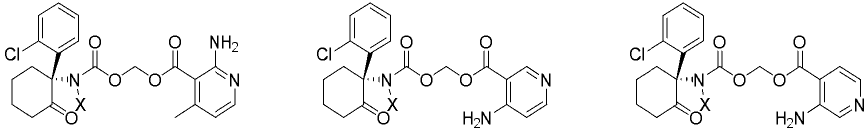 wherein X is -CH₃ or -CD₃.

In another aspect, provided herein is a compound having the structure of Formula (Va) or (Vb) or (Vc) or (Vd), or a stereoisomer, an N-oxide, a solvate, a metabolite, a pharmaceutically acceptable salt or a prodrug thereof:

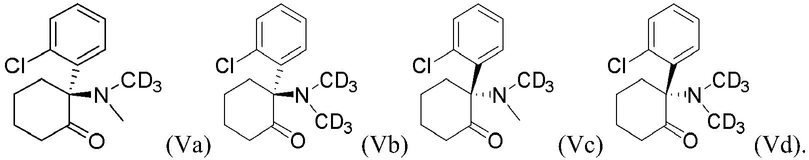

Unless otherwise stated, all suitable isotope changes, stereoisomers, tautomers, solvates, metabolites, salts and pharmaceutically acceptable prodrugs of the compounds disclosed herein are within the scope of the invention.

The compounds shown above in Formula (Ia) ~ (Vd) may exist in different tautomeric forms, and all of these tautomers are contemplated within the scope of the present invention.

N-Oxides of the compounds disclosed herein are also within the scope of the invention. *N-*Oxides of the compounds disclosed herein may be prepared by oxidation of the corresponding nitrogen base using a conventional oxidizing agent (such as hydrogen peroxide) in the presence of an acid such as acetic acid at an elevated temperature, or by reaction with a peracid such as peracetic acid in a suitable solvent, e.g. DCM, ethyl acetate or methyl acetate, or in chloroform or DCM with 3-chloroperoxybenzoic acid.

Moreover, when compounds disclosed herein form hydrates or solvates, which are within the scope of the invention. Similarly, the pharmaceutical acceptable salts of hydratas and solvates of compounds disclosed herein are also within the scope of the invention.

The compounds of Formula (Ia) ~ (Vd) can exist in the form of salts. In some embodiments, the salt is a pharmaceutically acceptable salt. The pharmaceutically acceptable salts of the present invention can be synthesized from a basic or acidic moiety by conventional chemical methods. Generally, such salts can be prepared by reacting free acid forms of these compounds with a stoichiometric amount of the appropriate base (such as Na, Ca, Mg, or K hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of additional suitable salts can be found, *e.g.,* in *"*Remington's Pharmaceutical Sciences", 20th ed., Mack Publishing Company, Easton, Pa., (1985); and in *"*Handbook of Pharmaceutical Salts: Properties, Selection, and Use" by Stahl and Wermuth (Wiley-VCH, Weinheim, Germany, 2002).

Compounds of the present invention are basic, thus pharmaceutically acceptable acid addition salts can be formed generally by processing a suitable acid. The suitable acid includes pharmaceutically acceptable inorganic acid and organic acid. Representative pharmaceutically acceptable acid addition salts include hydrochloride, hydrobromide, nitrate, methylnitrate, sulfate, hydrosulfate, sulfamate, phosphate, acetate, glycolate, phenyl acetate, propionate, butyrate, isobutyrate, valerate, maleate, hydroxymaleate, acrylate, fumarate, malate, tartrate, citrate, salicylate, para-amino salicylate, glycollate, lactate, enantate, phthalate, oxalate, succinate, benzoate, acetoxybenzoate, chlorobenzoate, methylbenzoate, binitrobenzoate, hydroxybenzoate, methoxybenzoate, mandelate, tannate, formate, stearate, ascorbate, palmitate, oleate, pyruvate, pamoate, malonate, laurate, glutarate, glutamate, estolate, mesylate, ethyl sulfate, 2-hydroxyesilate, benzene sulfonate, para-amino benzene sulfonate, para-methylbenzene sulfonate and naphthalene-2-sulfonate, and the like.

Any formula given herein is also intended to represent isotopically unenriched forms as well as isotopically enriched forms of the compounds. Isotopically enriched compounds have the structure represented by the general formula of the present invention, but for the fact that one or more atoms are replaced by an atom having a selected atomic mass or mass number. Examples of isotopes that can be incorporated into compounds of the invention include isotopes of hydrogen, carbon, nitrogen, oxygen, phosphorous, sulfur, fluorine and chlorine, such as ²H, ³H, ¹¹C, ¹³C, ¹⁴C, ¹⁵N, ¹⁷O, ¹⁸O, ¹⁸F, ³¹P, ³²P, ³⁵S, ³⁶Cl and ¹²⁵I.

In another aspect, the compounds of the invention include isotopically enriched compounds as defined herein, for example, wherein radioisotopes exist, such as ³H, ¹⁴C and ¹⁸F, or wherein non-radioactive isotopes exist, such as ²H and ¹³C. Such isotopically enriched compounds are useful in metabolic studies (with ¹⁴C), reaction kinetic studies (with, for example ²H or ³H), detection or imaging techniques, such as positron emission tomography (PET) or single-photon emission computed tomography (SPECT) including drug or substrate tissue distribution assays, or in radioactive treatment of patients. ¹⁸F-enriched compounds are particularly desirable for PET or SPECT studies. Isotopically-enriched compounds of Formula (I) can generally be prepared by conventional techniques known to those skilled in the art or by processes analogous to those described in the accompanying Examples and Preparations using an appropriate isotopically-labeled reagent in place of the non-labeled reagent previously employed.

In another aspect, provided herein is a pharmaceutical composition comprising the compound of the present invention.

In one embodiment, the pharmaceutical composition further comprising at least one pharmaceutically acceptable excipient carrier, adjuvant, vehicle or a combination thereof.

In one embodiment, the pharmaceutical composition further comprising one or more adjunctive therapeutic agents in a pharmaceutically effective amount, and wherein the adjunctive therapeutic agent is used in treating a neurological and psychiatric disorder or disease of the central nervous system.

In one embodiment, the neurological and psychiatric disorder or disease of the central nervous system is depression or pain.

In one embodiment, the adjunctive therapeutic agent is selected from the group consisting of at least one member of lithium, a pharmaceutical or an herbal antidepressant, an anticonvulsant, a mood stabilizer, an antipsychotic agent, and a benzodiazepine.

In another aspect, provided herein is use of the compound or the pharmaceutical composition in the manufacture of a medicament for preventing, managing, treating or lessening a neurological and psychiatric disorder or disease of the central nervous system in a patient.

In another aspect, provided herein is use of the compound or the pharmaceutical composition in the manufacture of a medicament for antagonizing the NMDA receptor.

In another aspect, provided herein is the compound or the pharmaceutical composition for use in preventing, managing, treating or lessening a neurological and psychiatric disorder or disease of the central nervous system in a patient.

In another aspect, provided herein is the compound or the pharmaceutical composition for use in antagonizing the NMDA receptor.

In another aspect, provided herein is a method of preventing, managing, treating or lessening a neurological and psychiatric disorder or disease of the central nervous system in a patient, comprising administering to the patient in need thereof a therapeutically effective amount of the compound or the pharmaceutical composition.

In another aspect, provided herein is a method of antagonizing the NMDA receptor in a patient, comprising administering to the patient in need thereof a therapeutically effective amount of the compound or the pharmaceutical composition.

In yet another aspect, the present invention is directed to methods of making compounds of Formula (Ia) ~ (Vd) and pharmaceutically acceptable salts thereof.

### PHARMACEUTICAL COMPOSITION OF THE COMPOUND OF THE INVENTION AND PREPARATIONS AND ADMINISTRATION

In one aspect, provided herein is a pharmaceutical composition including compounds of Formula (Ia) ~ (Vd) or a stereoisomer, a tautomer, an N-oxide, a solvate, a metabolite, a pharmaceutically acceptable salt or a prodrug thereof. Optionally, the pharmaceutical compositions further comprise at least a pharmaceutically acceptable carrier, an adjuvant, or an excipient, and optionally other therapeutic and/or prophylactic ingredients.

Suitable carriers, adjuvants and excipients are well known to those skilled in the art and described in detail in such as Ansel H. C. et al., Ansel's Pharmaceutical Dosage Forms and Drug Delivery Systems (2004) Lippincott, Williams & Wilkins, Philadelphia; Gennaro A. R. et al., Remington: The Science and Practice of Pharmacy (2000) Lippincott, Williams & Wilkins, Philadelphia; and Rowe R. C., Handbook of Pharmaceutical Excipients (2005) Pharmaceutical Press, Chicago.

"Pharmaceutically acceptable excipient" as used herein means a pharmaceutically acceptable material, composition or vehicle involved in giving form or consistency to the pharmaceutical composition. Each excipient must be compatible with the other ingredients of the pharmaceutical composition when commingled, such that interactions which would substantially reduce the efficacy of the compound of the invention when administered to a patient and would result in pharmaceutically unacceptable compositions are avoided. In addition, each excipient must of course be of sufficiently high purity to render it pharmaceutically acceptable.

Suitable pharmaceutically acceptable excipients will vary depending upon the particular dosage form chosen. In addition, suitable pharmaceutically acceptable excipients may be chosen for a particular function that they may serve in the composition. For example, certain pharmaceutically acceptable excipients may be chosen for their ability to facilitate the production of uniform dosage forms. Certain pharmaceutically acceptable excipients may be chosen for their ability to facilitate the production of stable dosage forms. Certain pharmaceutically acceptable excipients may be chosen for their ability to facilitate the carrying or transporting the compound of the present invention once administered to the patient from one organ, or portion of the body, to another organ, or portion of the body. Certain pharmaceutically acceptable excipients may be chosen for their ability to enhance patient compliance.

Suitable pharmaceutically acceptable excipients include the following types of excipients: diluents, fillers, binders, disintegrants, lubricants, glidants, granulating agents, coating agents, wetting agents, solvents, co-solvents, suspending agents, emulsifiers, sweetners, flavoring agents, flavor masking agents, coloring agents, anticaking agents, humectants, chelating agents, plasticizers, viscosity increasing agents, antioxidants, preservatives, stabilizers, surfactants, and buffering agents. One skilled in the art will appreciate that certain pharmaceutically acceptable excipients may serve more than one function and may serve alternative functions depending on how much of the excipient is present in the formulation and what other ingredients are present in the formulation.

Skilled artisans possess the knowledge and skill in the art to enable them to select suitable pharmaceutically acceptable excipients in appropriate amounts for use in the invention. In addition, there are a number of resources that are available to the skilled artisan which describe pharmaceutically acceptable excipients and may be useful in selecting suitable pharmaceutically acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

In Remington: The Science and Practice of Pharmacy, 21st edition, 2005, ed. D.B. Troy, Lippincott Williams & Wilkins, Philadelphia, and Encyclopedia of Pharmaceutical Technology, eds. J. Swarbrick and J. C. Boylan, 1988-1999, Marcel Dekker, New York, the contents of each of which is incorporated by reference herein, are disclosed various carriers used in formulating pharmaceutically acceptable compositions and known techniques for the preparation thereof. Except insofar as any conventional carrier medium is incompatible with the compounds of the invention, such as by producing any undesirable biological effect or otherwise interacting in a deleterious manner with any other component(s) of the pharmaceutically acceptable composition, its use is contemplated to be within the scope of this invention.

The compound of the invention will typically be formulated into a dosage form adapted for administration to the patient by the desired route of administration. For example, dosage forms include those adapted for (1) oral administration such as tablets, capsules, caplets, pills, troches, powders, syrups, elixers, suspensions, solutions, emulsions, sachets, and cachets; (2) parenteral administration such as sterile solutions, suspensions, and powders for reconstitution; (3) transdermal administration such as transdermal patches; (4) rectal administration such as suppositories; (5) inhalation such as aerosols, solutions, and dry powders; and (6) topical administration such as creams, ointments, lotions, solutions, pastes, sprays, foams, and gels.

It will also be appreciated that certain of the compounds of present invention can exist in free form for treatment, or where appropriate, as a pharmaceutically acceptable derivative or a prodrug thereof. According to the present invention, a pharmaceutically acceptable derivative or a prodrug includes, but is not limited to, pharmaceutically acceptable prodrugs, salts, esters, salts of such esters, or any other adduct or derivative which upon administration to a patient in need thereof is capable of providing, directly or indirectly, a compound as otherwise described herein, or a metabolite or residue thereof.

In one embodiment, the compounds disclosed herein can be prepared to oral dosage forms. In one embodiment, the compounds disclosed herein can be prepared to inhalation dosage forms. In one embodiment, the compounds disclosed herein can be prepared to dosage forms of nasal administration. In one embodiment, the compounds disclosed herein can be prepared to transdermal dosage forms. In one embodiment, the compounds disclosed herein can be prepared to dosage forms of topical administration.

The pharmaceutical compositions provided herein may be provided as compressed tablets, tablet triturates, chewable lozenges, rapidly dissolving tablets, multiple compressed tablets, or enteric-coating tablets, sugar-coated, or film-coated tablets. Enteric-coated tablets are compressed tablets coated with substances that resist the action of stomach acid but dissolve or disintegrate in the intestine, thus protecting the active ingredients from the acidic environment of the stomach. Enteric-coatings include, but are not limited to, fatty acids, fats, phenylsalicylate, waxes, shellac, ammoniated shellac, and cellulose acetate phthalates. Sugar-coated tablets are compressed tablets surrounded by a sugar coating, which may be beneficial in covering up objectionable tastes or odors and in protecting the tablets from oxidation. Film-coated tablets are compressed tablets that are covered with a thin layer or film of a water-soluble material. Film coatings include, but are not limited to, hydroxyethylcellulose, sodium carboxymethylcellulose, polyethylene glycol 4000, and cellulose acetate phthalate. Film coating imparts the same general characteristics as sugar coating. Multiple compressed tablets are compressed tablets made by more than one compression cycle, including layered tablets, and press-coated or dry-coated tablets.

The tablet dosage forms may be prepared from the active ingredient in powdered, crystalline, or granular forms, alone or in combination with one or more carriers or excipients described herein, including binders, disintegrants, controlled-release polymers, lubricants, diluents, and/or colorants. Flavoring and sweetening agents are especially useful in the formation of chewable tablets and lozenges.

The pharmaceutical compositions provided herein may be provided as soft or hard capsules, which can be made from gelatin, methylcellulose, starch, or calcium alginate. The hard gelatin capsule, also known as the dry-filled capsule (DFC), consists of two sections, one slipping over the other, thus completely enclosing the active ingredient. The soft elastic capsule (SEC) is a soft, globular shell, such as a gelatin shell, which is plasticized by the addition of glycerin, sorbitol, or a similar polyol. The soft gelatin shells may contain a preservative to prevent the growth of microorganisms. Suitable preservatives are those as described herein, including methyl- and propyl-parabens, and sorbic acid. The liquid, semisolid, and solid dosage forms provided herein may be encapsulated in a capsule. Suitable liquid and semisolid dosage forms include solutions and suspensions in propylene carbonate, vegetable oils, or triglycerides. Capsules containing such solutions can be prepared as described in U.S. Pat. Nos. 4,328,245; 4,409,239; and 4,410,545. The capsules may also be coated as known by those of skill in the art in order to modify or sustain dissolution of the active ingredient.

The pharmaceutical compositions provided herein may be provided in liquid and semisolid dosage forms, including emulsions, solutions, suspensions, elixirs, and syrups. An emulsion is a two-phase system, in which one liquid is dispersed in the form of small globules throughout another liquid, which can be oil-in-water or water-in-oil. Emulsions may include a pharmaceutically acceptable non-aqueous liquids or solvent, emulsifying agent, and preservative. Suspensions may include a pharmaceutically acceptable suspending agent and preservative. Aqueous alcoholic solutions may include a pharmaceutically acceptable acetal, such as a di(lower alkyl) acetal of a lower alkyl aldehyde, e.g., acetaldehyde diethyl acetal; and a water-miscible solvent having one or more hydroxy groups, such as propylene glycol and ethanol. Elixirs are clear, sweetened, and hydroalcoholic solutions. Syrups are concentrated aqueous solutions of a sugar, for example, sucrose, and may also contain a preservative. For a liquid dosage form, for example, a solution in a polyethylene glycol may be diluted with a sufficient quantity of a pharmaceutically acceptable liquid carrier, e.g., water, to be measured conveniently for administration.

Provided herein is a pharmaceutical composition which can be prepared to a dosage form adapted for administration to a patient by inhalation, for example as a dry powder, an aerosol, a suspension, or a solution composition. In one embodiment, the invention is directed to a dosage form adapted for administration to a patient by inhalation as a dry powder. In one embodiment, the invention is directed to a dosage form adapted for administration to a patient by inhalation as a dry powder. Dry powder compositions for delivery to the lung by inhalation typically comprise a compound disclosed herein or a pharmaceutically acceptable salt thereof as a finely divided powder together with one or more pharmaceutically-acceptable excipients as finely divided powders. Pharmaceutically-acceptable excipients particularly suited for use in dry powders are known to those skilled in the art and include lactose, starch, mannitol, and mono-, di-, and polysaccharides. The finely divided powder may be prepared by, for example, micronisation and milling. Generally, the size-reduced (e.g. micronised) compound can be defined by a D₅₀ value of about 1 to 10 microns (for example as measured using laser diffraction).

Pharmaceutical compositions adapted for transdermal administration may be presented as discrete patches intended to remain in intimate contact with the epidermis of the patient for a prolonged period of time. For example, the active ingredient may be delivered from the patch by iontophoresis as generally described in Pharmaceutical Research, 3(6), 318 (1986).

Pharmaceutical compositions adapted for topical administration may be formulated as ointments, creams, suspensions, lotions, powders, solutions, pastes, gels, sprays, aerosols or oils. Ointments, creams and gels, may, for example, be formulated with an aqueous or oily base with the addition of suitable thickening and / or gelling agent and/or solvents. Such bases may thus, for example, include water and / or oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and / or glyceryl monostearate and / or non-ionic emulsifying agents.

The compounds disclosed herein can also be coupled to soluble polymers as targeted medicament carriers. Such polymers may encompass polyvinylpyrrolidone, pyran copolymer, polyhydroxypropylmethacrylamidophenol, polyhydroxyethylaspartamidophenol or polyethylene oxide polylysine, substituted by palmitoyl radicals. The compounds may furthermore be coupled to a class of biodegradable polymers which are suitable for achieving controlled release of a medicament, for example polylactic acid, poly-epsilon-caprolactone, polyhydroxybutyric acid, polyorthoesters, polyacetals, polydihydroxypyrans, polycyanoacrylates and crosslinked or amphipathic block copolymers of hydrogels.

The pharmaceutical compositions provided herein may be administered parenterally by injection, infusion, or implantation, for local or systemic administration. Parenteral administration, as used herein, include intravenous, intraarterial, intraperitoneal, intrathecal, intraventricular, intraurethral, intrasternal, intracranial, intramuscular, intrasynovial, and subcutaneous administration.

The pharmaceutical compositions provided herein may be formulated in any dosage forms that are suitable for parenteral administration, including solutions, suspensions, emulsions, micelles, liposomes, microspheres, nanosystems, and solid forms suitable for solutions or suspensions in liquid prior to injection. Such dosage forms can be prepared according to conventional methods known to those skilled in the art of pharmaceutical science (see, *Remington: The Science and Practice of Pharmacy,* supra).

The pharmaceutical compositions intended for parenteral administration may include one or more pharmaceutically acceptable carriers and excipients, including, but not limited to, aqueous vehicles, water-miscible vehicles, non-aqueous vehicles, antimicrobial agents or preservatives against the growth of microorganisms, stabilizers, solubility enhancers, isotonic agents, buffering agents, antioxidants, local anesthetics, suspending and dispersing agents, wetting or emulsifying agents, complexing agents, sequestering or chelating agents, cryoprotectants, lyoprotectants, thickening agents, pH adjusting agents, and inert gases.

The pharmaceutical compositions provided herein may be formulated as immediate or modified release dosage forms, including delayed-, sustained, pulsed-, controlled, targeted-, and programmed-release forms.

The pharmaceutical compositions provided herein may be formulated for single or multiple dosage administration. The single dosage formulations are packaged in an ampoule, a vial, or a syringe. The multiple dosage parenteral formulations must contain an antimicrobial agent at bacteriostatic or fungistatic concentrations. All parenteral formulations must be sterile, as known and practiced in the art.

The pharmaceutical compositions provided herein may be co-formulated with other active ingredients which do not impair the desired therapeutic action, or with substances that supplement the desired action.

In one embodiment, the therapeutic methods disclosed herein comprise administrating to a patient in need of the treatment a safe and effective amount of the compound of the invention or the pharmaceutical composition containing the compound of the invention. Each example disclosed herein comprises treating the above disorders or diseases by administrating to a patient in need of the treatment a safe and effective amount of the compound of the invention or the pharmaceutical composition containing the compound of the invention.

In one embodiment, the compound of the invention or the pharmaceutical composition thereof may be administered by any suitable route of administration, including both systemic administration and topical administration. Systemic administration includes oral administration, parenteral administration, transdermal administration and rectal administration. Parenteral administration refers to routes of administration other than enteral or transdermal, and is typically by injection or infusion. Parenteral administration includes intravenous, intramuscular, and subcutaneous injection or infusion. Topical administration includes application to the skin as well as intraocular, otic, intravaginal, inhaled and intranasal administration. In one embodiment, the compound of the invention or the pharmaceutical composition thereof may be administered orally. In one embodiment, the compound of the invention or the pharmaceutical composition thereof may be administered by inhalation. In a further embodiment, the compound of the invention or the pharmaceutical composition thereof may be administered intranasally.

In one embodiment, the compound of the invention or the pharmaceutical composition thereof may be administered once or according to a dosing regimen wherein a number of doses are administered at varying intervals of time for a given period of time. For example, doses may be administered one, two, three, or four times per day. In one embodiment, a dose is administered once per day. In a further embodiment, a dose is administered twice per day. Doses may be administered until the desired therapeutic effect is achieved or indefinitely to maintain the desired therapeutic effect. Suitable dosing regimens for the compound of the invention or the pharmaceutical composition thereof depend on the pharmacokinetic properties of that compound, such as absorption, distribution, and half-life, which can be determined by the skilled artisan. In addition, suitable dosing regimens, including the duration such regimens are administered, for the compound of the invention or the pharmaceutical composition thereof depend on the disorder being treated, the severity of the disorder being treated, the age and physical condition of the patient being treated, the medical history of the patient to be treated, the nature of concurrent therapy, the desired therapeutic effect, and like factors within the knowledge and expertise of the skilled artisan. It will be further understood by such skilled artisans that suitable dosing regimens may require adjustment given an individual patient's response to the dosing regimen or over time as individual patient needs change.

The compounds of the present invention may be administered either simultaneously with, or before or after, one or more other therapeutic agents. The compounds of the present invention may be administered separately, by the same or different route of administration, or together in the same pharmaceutical composition as the other agents.

Compounds provided herein can used in combination with sedative, hypnotic, anxiolytic, antipsychotic, antianxiety agent, cyclopyrrolidone, imidazopyridine, pyrazolopyrimidines, minor tranquilizer, melatonin agonist and antagonist, melatoninergic agent, benzodiazepine, barbiturate, 5HT-2 antagonist, and the like. For example: adinazolan, allobarbital, alonimid, alprazolam, amitriptyline, amobarbital, amoxapine, bentazepam, tacitin, brotizolam, bupropion, buspirone, butabarbital, butalbital, capuride, carbocloral, chloral betaine, chloral hydrate, chlorodyne, clomipramine, clonazepam, domperidone, methaminodiazepoxide, cloretate, clozapine, cyprazepam, desipramine, dexclamo, diazepam, chloralsalicylamide, divalproic acid, diphenhydramine, doxepin, estazolam, ethchlorvynol, etomidate, fenobam, flunitrazepam, flurazepam, fluvoxamine, fluoxetine, fosazepam, glutethimide, halazepam, hydroxyzine, imipramine, lithium, orazepam, lormetazepam, maprotiline, mecloqualone, melatonin, methylphenobarbital, meprobamate, methaqualone, midaflur, midazolam, nefazodone, nisobamate, nitrazepam, nortriptyline, oxezepam, paraaldehyde, paroxetine, pentobarbital, perlapine, perphenazine, phenelzine, phenobarbital, Prazepam, promethazine, isopropylphenol, protriptyline, quazepam, reclazepam, rolipram, secobarbital, sertraline, suproclone, temazepam, thioridazine, tracazolate, tranylcypromine, trazodone, triazole benzodiazepine, trepipam, tricetamide, trichloroethyl phosphate, trifluoperazine, trimetozine, trimeprimine, uldazepam, venlafaxine, zaleplon, zolazepam, zolpidem and the salts and compositions thereof, and the like. Alternatively, physical methods such as light therapy or electrical stimulation can be used during administration of compounds disclosed herein.

Additionally, the compounds of the invention may be administered as prodrugs. As used herein, a "prodrug" of a compound of the invention is a functional derivative of the compound which, upon administration to a patient, eventually liberates the compound of the invention *in vivo.* Administration of a compound of the invention as a prodrug may enable the skilled artisan to do one or more of the following: (a) modify the onset of action of the compound *in vivo;* (b) modify the duration of action of the compound in vivo; (c) modify the transportation or distribution of the compound *in vivo;* (d) modify the solubility of the compound *in vivo;* and (e) overcome a side effect or other difficulty encountered with the compound. Typical functional derivatives used to prepare prodrugs include modifications of the compound that are chemically or enzymatically cleaved *in vivo.* Such modifications, which include the preparation of phosphates, amides, esters, thioesters, carbonates, and carbamates, are well known to those skilled in the art.

### USE OF THE COMPOUNDS AND PHARMACEUTICAL COMPOSITIONS

Compounds or pharmaceutical compositions disclosed herein are efficient as NMDA receptors antagonists for treating or preventing neurological and psychiatric disorder disease related to NMDA receptors and may be used in preparation of a medicament antagonizing to NMDA receptors.

All diseases related to NMDA receptors are selectable from all types of neurological and psychiatric disorder or disease.

In one embodiment, the disease related to NMDA receptors comprises depression, an anxiety disorder, a seasonal affective disorder, mania, a bipolar disorder, obsessive-compulsive disorder, insomnia and fatigue resulting from jet lag, mental schizophrenia, seizure, panic attack, melancholia, alcohol addiction, drug addiction, alcoholism, substance abuse, drug addiction withdrawal symptoms, insomnia, a psychotic disorder, epilepsy, somnipathy, sleep disorder, sleep apnea syndrome, a mandatory eating disorder, fibromyalgia, stress, obesity, Parkinson's disease, a cognitive disorder, a memory disorder, premenstrual tension syndrome, a migraine headache, memory loss, Alzheimer silent disease or a disorder related to normal or pathological aging.

It should be understood that any of above symptoms or diseases is promoted or accelerated under certain environmental conditions such as pressure or fear (wherein, pressure may generated from social source such as social pressure or physical source such as physical pressure, which comprises pressure generated by fear), and compounds disclosed herein particularly useful in the treatment of symptoms and diseases adjusted by these environment.

Besides being useful for human treatment, the compounds of the present invention and the compositions thereof are also useful for veterinary treatment of animals such as companion animals, exotic animals and mammals of farm animals. In other embodiments, the animals disclosed herein include horses, dogs, and cats. As used herein, the compounds disclosed herein include the pharmaceutically acceptable derivatives thereof.

### PREFERRED EMBODIMENT OF THE INVENTION

### General synthetic procedures

The following examples are provided so that the invention might be more fully understood. However, it should be understood that these embodiments merely provide a method of practicing the present invention, and the present invention is not limited to these embodiments.

Generally, the compounds disclosed herein may be prepared by methods described herein, wherein the substituents are as defined for Formula (Ia) or Formula (Ib) above, except where further noted. The following non-limiting schemes and examples are presented to further exemplify the invention.

Professionals skilled in the art will recognize that the chemical reactions described may be readily adapted to prepare a number of other compounds disclosed herein, and alternative methods for preparing the compounds disclosed herein are deemed to be within the scope disclosed herein. Those having skill in the art will recognize that the starting materials may be varied and additional steps employed to produce compounds encompassed by the present inventions, as demonstrated by the following examples. In some cases, protection of certain reactive functionalities may be necessary to achieve some of the above transformations. In general, such need for protecting groups, as well as the conditions necessary to attach and remove such groups, will be apparent to those skilled in the art of organic synthesis. For example, the synthesis of non-exemplified compounds according to the invention may be successfully performed by modifications apparent to those skilled in the art, *e.g.,* by appropriately protecting interfering groups, by utilizing other suitable reagents known in the art other than those described, and / or by making routine modifications of reaction conditions. Alternatively, the known reaction conditions or the reaction disclosed in the present invention will be recognized as having applicability for preparing other compounds disclosed herein.

In the examples described below, unless otherwise indicated all temperatures are set forth in degrees Celsius. Reagents were purchased from commercial suppliers such as Aldrich Chemical Company, Arcos Chemical Company, Alfa Aesar Chemical Company and J&K Chemical Company, and were used without further purification unless otherwise indicated.

### Preparation of compounds

Compounds of the present invention, including salts, esters, hydrates, or solvates thereof, can be prepared using any known organic synthesis techniques and can be synthesized according to any of numerous possible synthetic routes.

The reactions for preparing compounds of the present invention can be carried out in suitable solvents, which can be readily selected by one skilled in the art of organic synthesis. Suitable solvents can be substantially non-reactive with the starting materials (reactants), the intermediates, or products at the temperatures at which the reactions are carried out, *e.g*., temperatures that can range from the solvent's freezing temperature to the solvent's boiling temperature. A given reaction can be carried out in one solvent or a mixture of more than one solvent. Depending on the particular reaction step, suitable solvents for a particular reaction step can be selected by a skilled artisan.

Reactions can be monitored according to any suitable method known in the art. For example, product formation can be monitored by spectroscopic means, such as nuclear magnetic resonance spectroscopy (e.g., ¹H or ¹³C), infrared spectroscopy, spectrophotometry (e.g., UV-visible), mass spectrometry, or by chromatographic methods such as high-performance liquid chromatography (HPLC), liquid chromatography-mass spectroscopy (LCMS), or thin layer chromatography (TLC). Compounds can be purified by those skilled in the art by a variety of methods, including high performance liquid chromatography (HPLC) ("Preparative LC-MS Purification: Improved Compound Specific Method Optimization" Karl F. Blom, Brian Glass, Richard Sparks, Andrew P. Combs J. Combi. Chem. 2004, 6(6), 874-883, which is incorporated herein by reference in its entirety) and normal phase silica chromatography.

Compounds of the present invention can be synthesized using the methods described below, together with synthetic methods known in the art of synthetic organic chemistry, or variations thereon as appreciated by those skilled in the art. Preferred methods include but are not limited to those methods described below. Specifically, the compounds of the present invention of Formula (Ia) ~ (Vd) can be synthesized by following the steps outlined in the exemplary general synthetic schemes listed below, and the abbreviations for the reactants or for the chemical groups of the reactants included in the synthetic schemes are defined in the Examples.

Generally, the synthesis towards compounds having Formula (IIa) or (IIb) can be conducted according to the below synthetic methods, but is not limited to these described methods. The followings are illustrated for Formula (IIa).

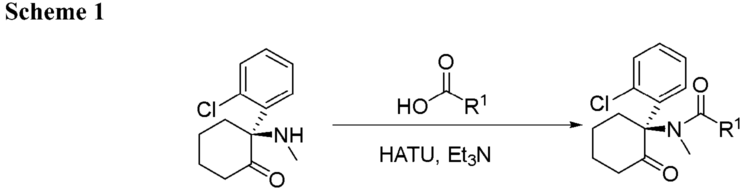

R¹ can be

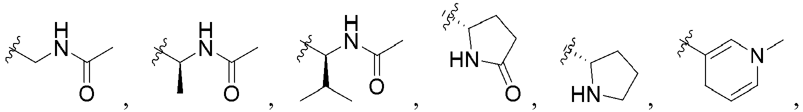

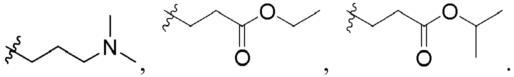

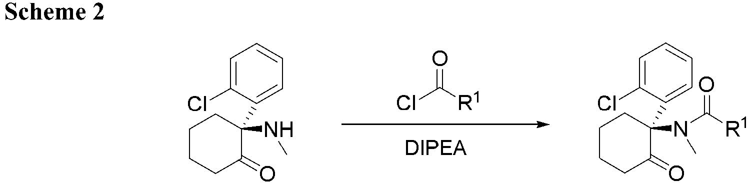

R¹ can be

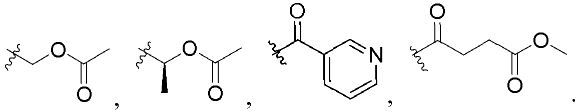

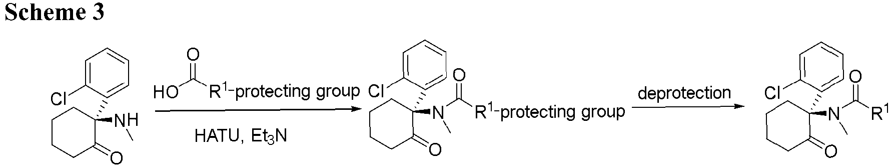

R¹ can be

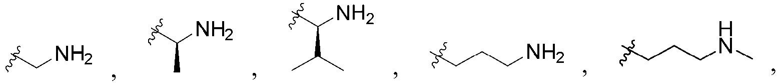

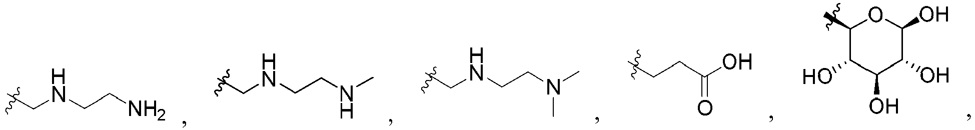

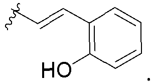

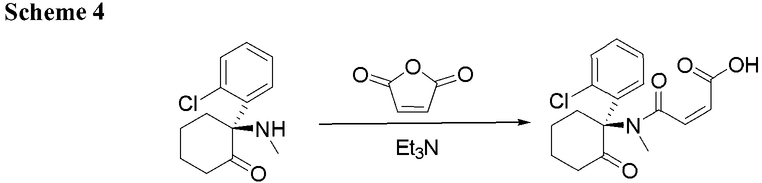

R¹ can be

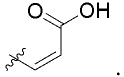

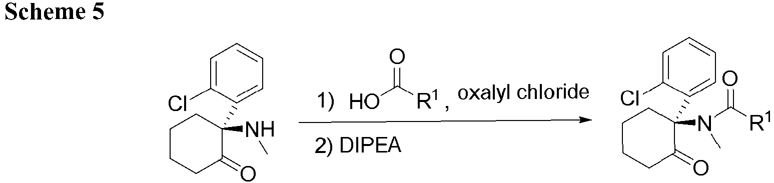

R¹ can be

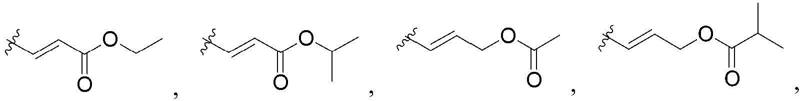

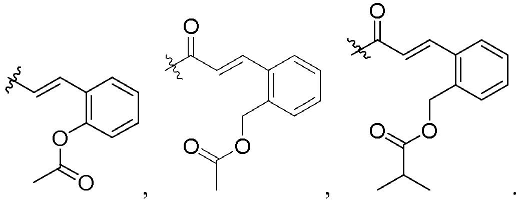

Generally, the synthesis towards compounds having Formula (IIIa) or (IIIb) can be conducted according to the below synthetic methods, but is not limited to these described methods. The followings are illustrated for Formula (IIIa).

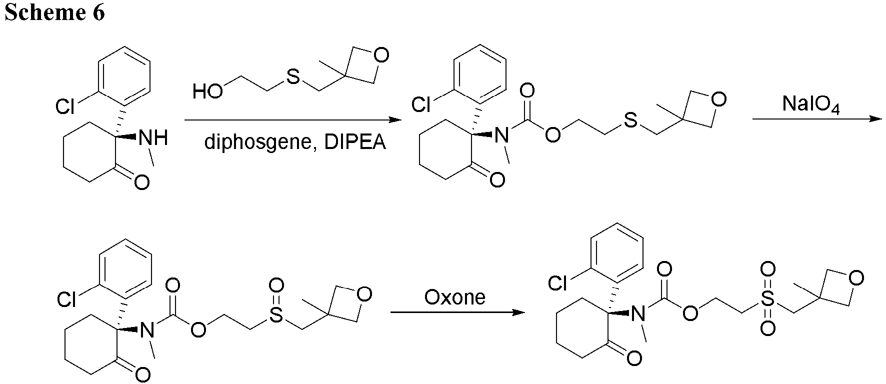

R² can be

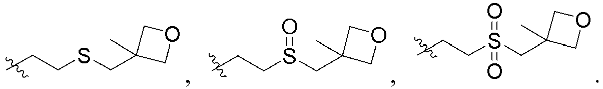

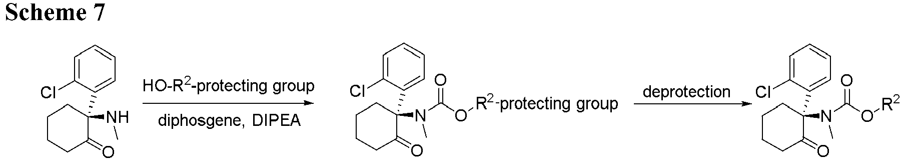

R² can be

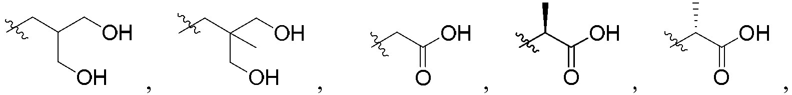

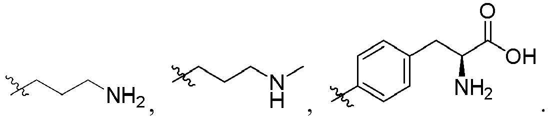

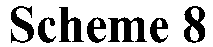

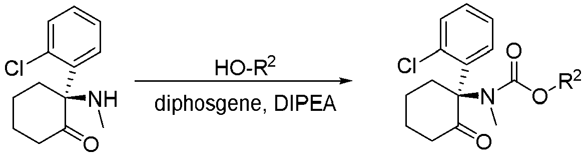

R² can be

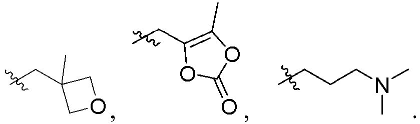

Generally, the synthesis towards compounds having Formula (IVa) or (IVb) can be conducted according to the below synthetic methods, but is not limited to these described methods. The followings are illustrated for Formula (IVa). 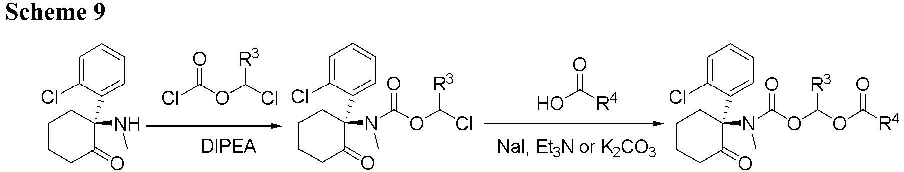 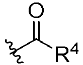 can be 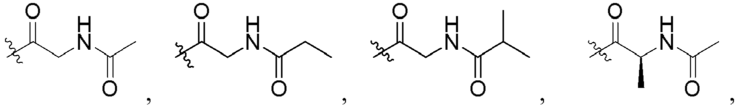 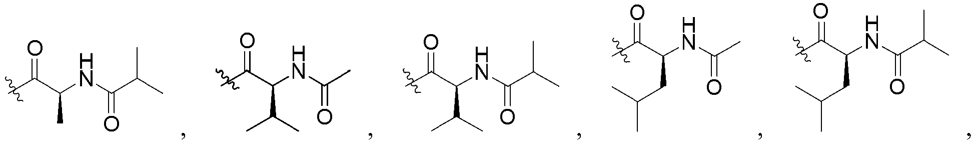 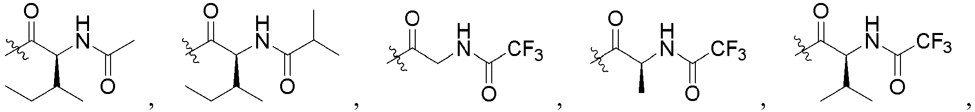 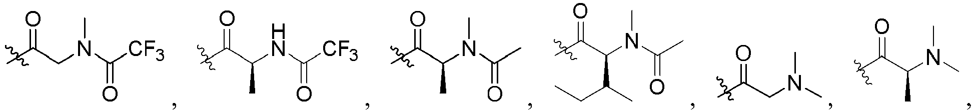 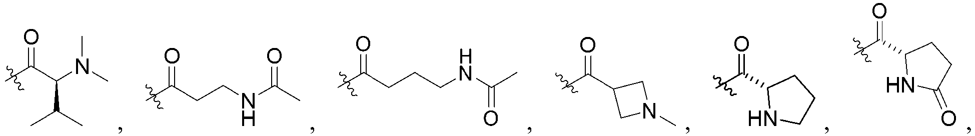 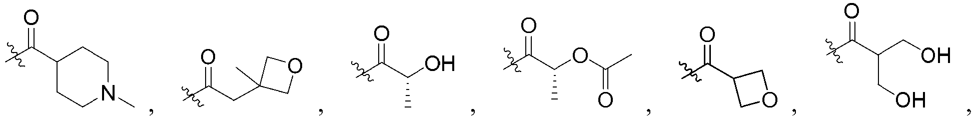 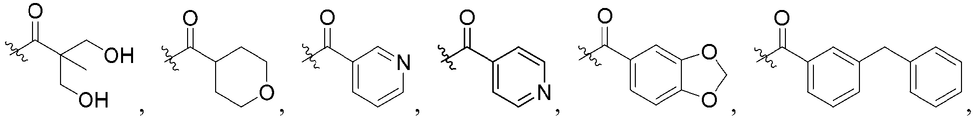 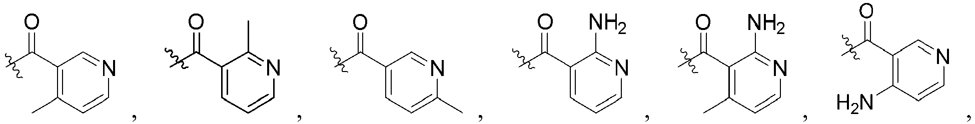 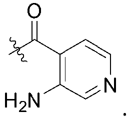 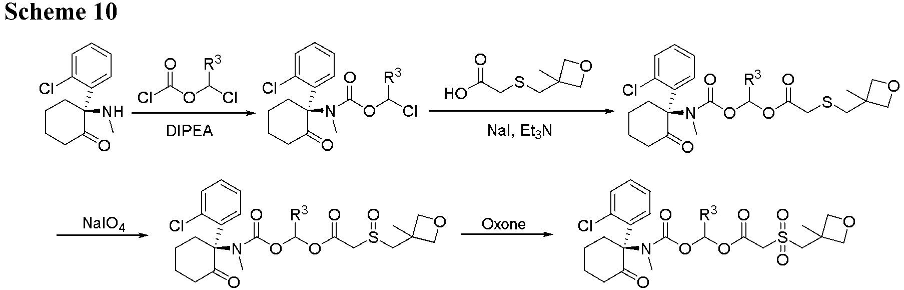 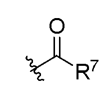 can be 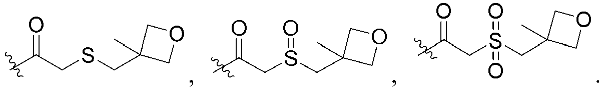 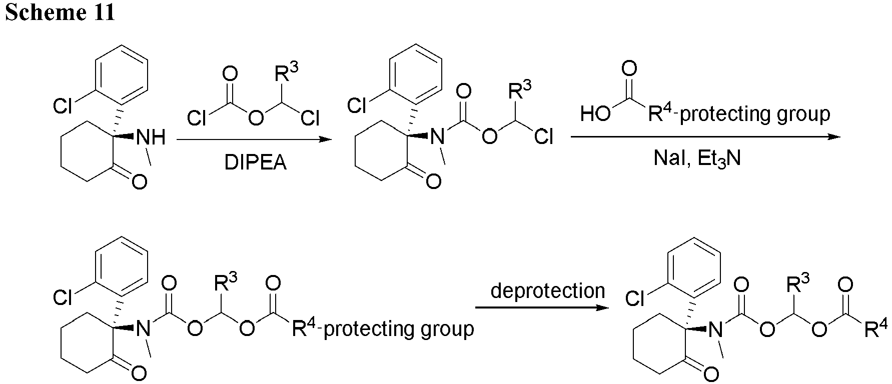 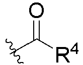 can be

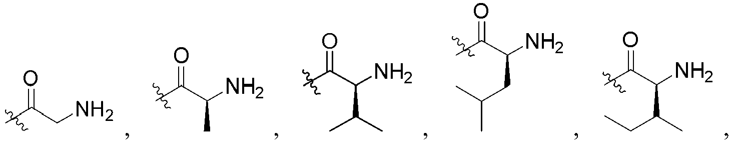

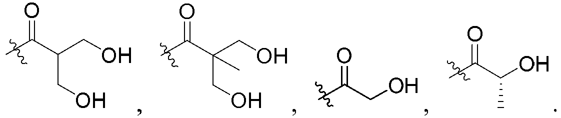

Generally, the synthesis towards compounds having Formula (Va) ~ (Vd) can be conducted according to the below synthetic methods, but is not limited to these described methods. The followings are illustrated for Formula (Va) or Formula (Vb)

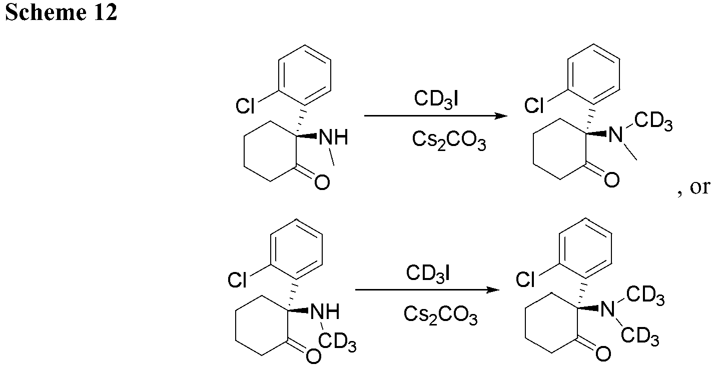

### Preparation and characterization of exemplary compounds

Compounds encompassed in the present disclosure may be prepared *via* different schemes. Detailed preparation processes of 108 exemplary compounds *via* various schemes are described below and the characterization results are listed as well.

Unless stated otherwise, all reagents were purchased from commercial suppliers without further purification. Solvent drying by standard methods was employed when necessary. The plates used for thin-layer chromatography (TLC) were E. Merck silica gel 60F254 (0.24 nm thickness) precoated on aluminum plates, and then visualized under UV light (365 nm and 254 nm) or through staining with a 5% of dodecamolybdophosphoric acid in ethanol and subsequent heating. Column chromatography was performed using silica gel (200-400 mesh) from commercial suppliers. ¹HNMR spectra were recorded on a BRUKER AVANCE III HD 500MHz NMR spectrometer and BRUKER AVANCE III HD 600MHz at room temperature. Solvent signal was used as reference for ¹HNMR (CDCl₃, 7.26 ppm; CD₃OD, 3.31 ppm; DMSO-*d₆*, 2.50 ppm; acetone-*d₆*, 2.05 ppm; D₂O, 4.79 ppm). The following abbreviations were used to explain the multiplicities: s = singlet, d = doublet, t = triplet, q = quartet, br. s = broad singlet, dd = double doublet, td = triple doublet, dt = double triplet, dq = double quartet, m = multiplet. Other abbreviations used in the experimental details are as follows: δ = chemical shift in parts per million downfield from tetramethylsilane, Ar = aryl, Ac = acyl, Boc = *tert*-butyloxy carbonyl, Bn = benzyl, DCM = dichloromethane, DCE = dichloroethane, DMF = *N,N*'-dimethylformamide, NMP = *N-*methyl-2-pyrrolidone, DIBAL-H = diisobutyl aluminium hydride, DIPEA = diisopropylethylamine, DMAP = 4-(dimethylamino)pyridine, DMSO = dimethyl sulphoxide, HATU = 1-[bis(dimethylamino)methylene]-1*H*-1,2,3-triazolo[4,5-*b*]pyridinium 3-oxid hexafluorophosphate, HOBT = 1-hydroxybenzotriazole, EA = ethyl acetate, Et = ethyl, Me = methyl, Hz = hertz, HPLC = high performance liquid chromatography, *J* = coupling constant (in NMR), min = minute(s), h = hour(s), NMR = nuclear magnetic resonance, NBS = *N*-bromosuccinimide, NCS = *N-*chlorosuccinimide, prep = preparative, PE = petroleum ether, *s*-Bu = *sec*-butyl, *t*-Bu = *tert*-butyl, *i*Pr = isopropyl, TBAF = tetrabutylammonium fluoride, *tert* = tertiary, TFA = trifluoroacetic acid, THF = tetrahydrofuran, MTBE = methyl *tert*-butyl ether, TLC = thin-layer chromatography.

### Examples

It should be noted that embodiments of the present invention described in detail below are exemplary for explaining the present invention only, and not be construed as limiting the present invention. Examples without a specific technology or condition can be implemented according to technology or condition in the documentation of the art or according to the product instructions. The reagents or instruments without manufacturers are available through conventional purchase. Those having skill in the art will recognize that the starting materials may be varied and additional steps employed to produce compounds encompassed by the present inventions, as demonstrated by the following examples.

| Compound | Chemical Structure | Compound | Chemical Structure |
|---|---|---|---|
| A-1 | 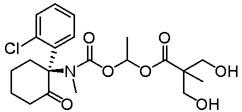 | A-2 | 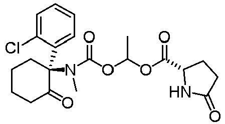 |
| A-3 | 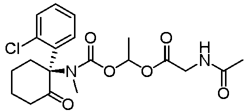 | A-4 | 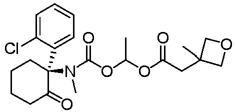 |
| A-5 | 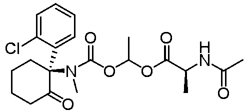 | A-6 | 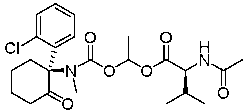 |
| A-7 | 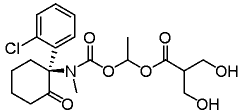 | A-8 | 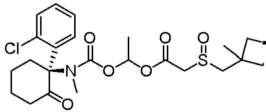 |
| A-9 | 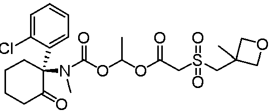 | A-10 | 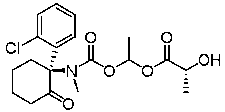 |
| A-11 | 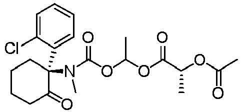 | A-12 | 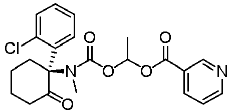 |
| A-13 | 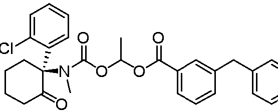 | A-14 | 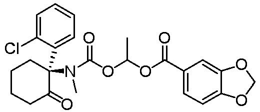 |
| A-15 | 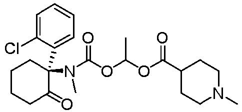 | A-16 | 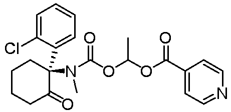 |
| A-17 | 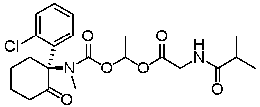 | A-18 | 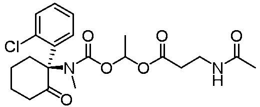 |
| A-19 | 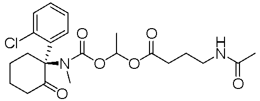 | A-20 | 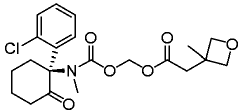 |
| A-21 | 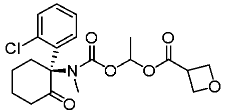 | A-22 | 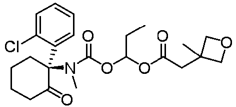 |
| A-23 | 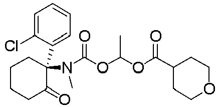 | A-24 | 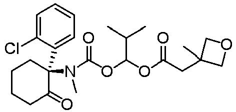 |
| A-25 | 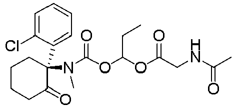 | A-26 | 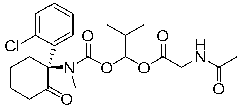 |
| A-27 | 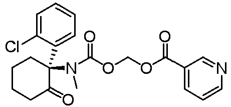 | A-28 | 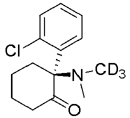 |
| A-29 | 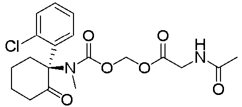 | A-30 | 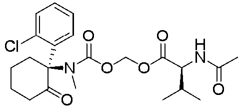 |
| A-31 | 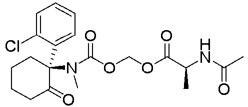 | A-32 | 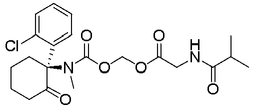 |
| A-33 | 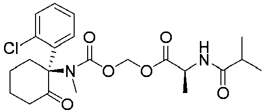 | A-34 | 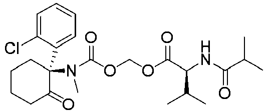 |
| A-35 | 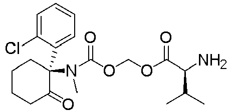 | A-36 | 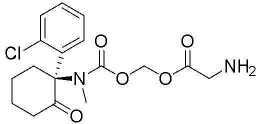 |
| A-37 | 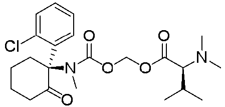 | A-38 | 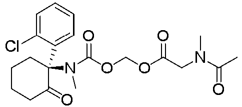 |
| A-39 | 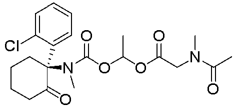 | A-40 | 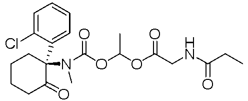 |
| A-41 | 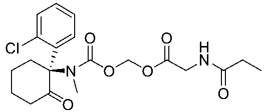 | A-42 | 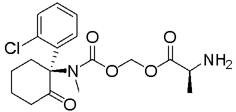 |
| A-43 | 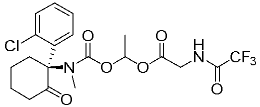 | A-44 | 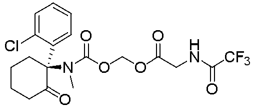 |
| A-45 | 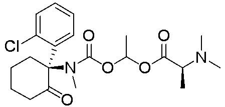 | A-46 | 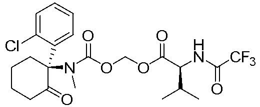 |
| A-47 | 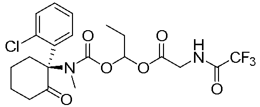 | A-48 | 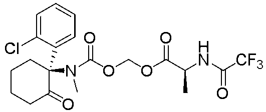 |
| A-49 | 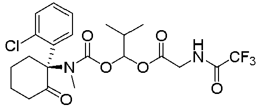 | A-50 | 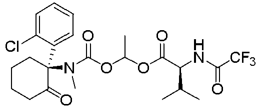 |
| A-51 | 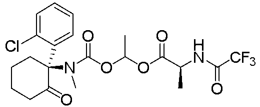 | A-52 | 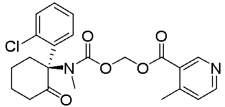 |
| A-53 | 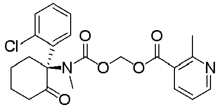 | A-54 | 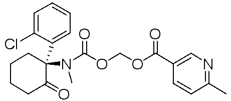 |
| A-55 | 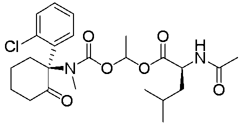 | A-56 | 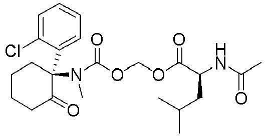 |
| A-57 | 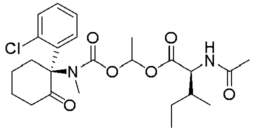 | A-58 | 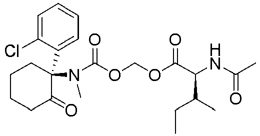 |
| A-59 | 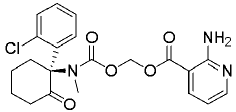 | A-60 | 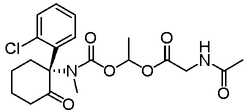 |
| A-61 | 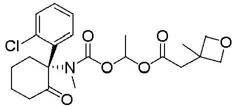 | A-62 | 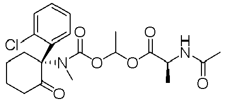 |
| A-63 | 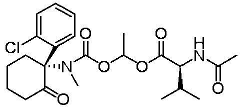 | A-64 | 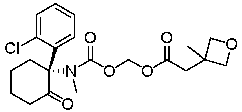 |
| A-65 | 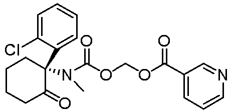 | A-66 | 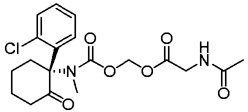 |
| A-67 | 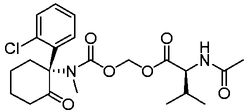 | A-68 | 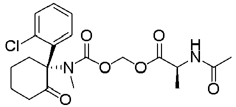 |
| A-69 | 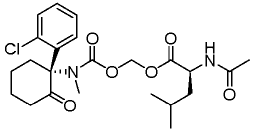 | A-70 | 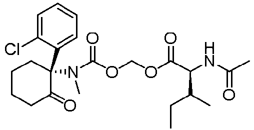 |
| A-71 | 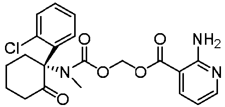 | A-72 | 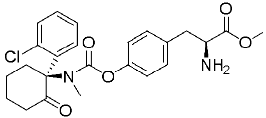 |
| A-73 | 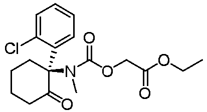 | A-74 | 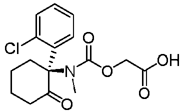 |
| A-75 | 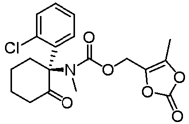 | A-76 | 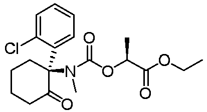 |
| A-77 | 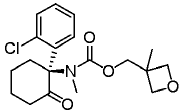 | A-78 | 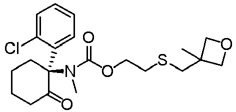 |
| A-79 | 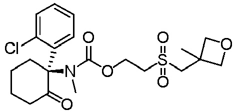 | A-80 | 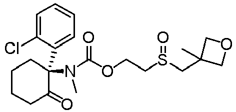 |
| A-81 | 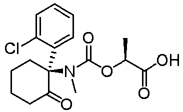 | A-82 | 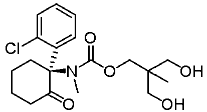 |
| A-83 | 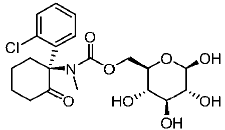 | A-84 | 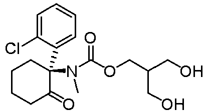 |
| A-85 | 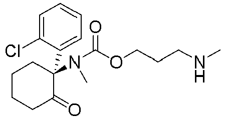 | A-86 | 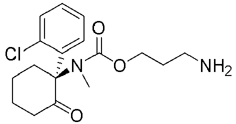 |
| A-87 | 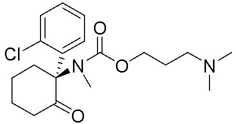 | A-88 | 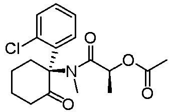 |
| A-89 | 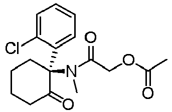 | A-90 | 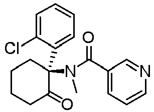 |
| A-91 | 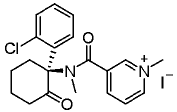 | A-92 | 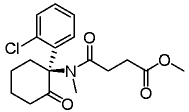 |
| A-93 | 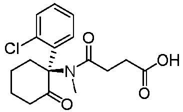 | A-94 | 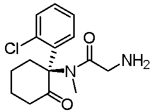 |
| A-95 | 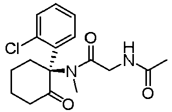 | A-96 | 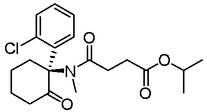 |
| A-97 | 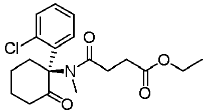 | A-98 | 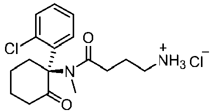 |
| A-99 | 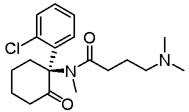 | A-100 | 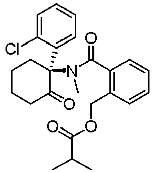 |
| A-101 | 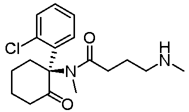 | A-102 | 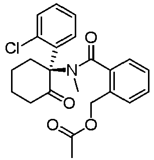 |
| A-103 | 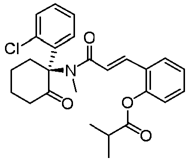 | A-104 | 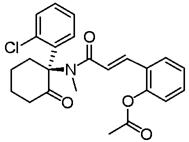 |
| A-105 | 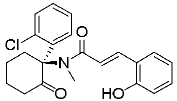 | A-106 | 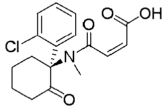 |
| A-107 | 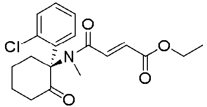 | A-108 | 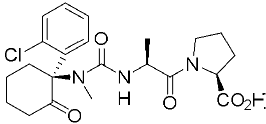 |

### Example 1: 1-((((S)-1-(2-chlorophenyl)-2-oxocyclohexyl)(methyl)carbamoyl)oxy)ethyl 3-hydroxy-2-(hydroxymethyl)-2-methylpropanoate (A-1)

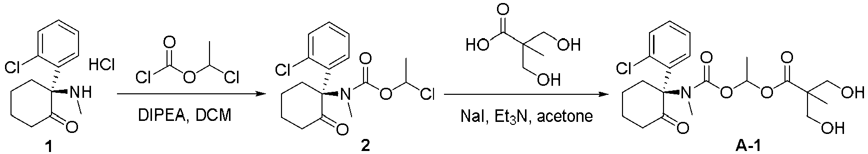

To a solution of S-ketamine hydrochloride **1** (274 mg, 1.0 mmol) and DIPEA (260 mg, 1.0 mmol) in DCM (10 mL) was added 1-chloroethyl carbonochloridate (172 mg, 1.2 mmol) slowly at 0 °C. The reaction was stirred at 25 °C for 1.5 h. The reaction mixture was diluted with DCM (10 mL) and washed with water (10 mL) and brine (10 mL). The organic layer was dried over MgSO₄, filtered and concentrated to get an oil, which was purified on silica gel column eluting with hexane/EA (1/1 to 5/1) to afford 276 mg (79% yield) of compound **2** as a white solid.

**¹HNMR** (500MHz, CDCl₃) δ = 1.60-1.96 (m, 6H), 1.99-2.10 (m, 1H), 2.32-2.56 (m, 1H), 2.57-2.63 (m, 1H), 2.67-2.84 (m, 1H), 3.01-3.07 (m, 3H), 3.22-3.40 (m, 1H), 6.48-6.60 (m, 1H), 6.91-7.04 (m, 1H), 7.22-7.30 (m, 2H), 7.43-7.49 (m, 1H).

To a solution of compound **2** (150 mg, 0.44 mmol), NaI (65 mg, 0.44 mmol) and 3-hydroxy-2-(hydroxymethyl)-2-methylpropanoic acid (292 mg, 2.18 mmol) in acetone (1.7 mL) was added Et₃N (0.31 mL, 2.18 mmol). The reaction was stirred at 25 °C for 5 h. The reaction mixture was concentrated and re-dissolved in EA (20 mL), washed with H₂O (8 mL), aqueous saturated NaHCO₃ solution (2 mL) and brine (5 mL). The organic layer was dried over MgSO₄, filtered and concentrated to get an oil, which was purified on silica gel column eluting with hexane/EA (100% hexane to 4/6) to afford 95 mg (49% yield) of titled compound (**A-1**) as a colorless oil.

**¹HNMR** (500MHz, DMSO-*d₆*) δ = 1.02 (br. s, 3H), 1.46 (br. s, 3H), 1.68 (br. s, 3H), 1.99 (br. s, 1H), 2.26-2.37 (m, 2H), 2.50-2.65 (m, 1H), 2.95 (d, *J =* 9.0 Hz, 3H), 3.10-3.18 (m, 1H), 3.44-3.52 (m, 4H), 4.72 (m, 2H), 6.61 (q, *J =* 5.4 Hz, 1H), 6.96 (d, *J =* 7.1 Hz, 1H), 7.30 (m, 2H), 7.46 (m, 1H).

**MS (ESI):** [M + H]⁺ = 442.2.

### Example 2: 1-((((S)-1-(2-chlorophenyl)-2-oxocyclohexyl)(methyl)carbamoyl)oxy)ethyl (2S)-5-oxopyrrolidine-2-carboxylate (A-2)

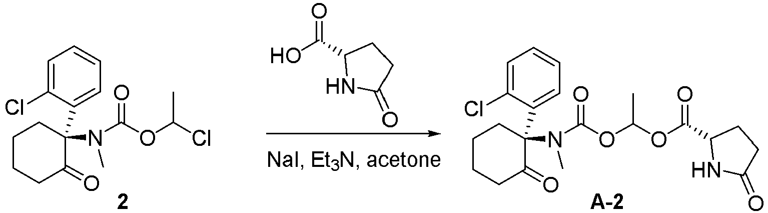

To a solution of compound **2** (100 mg, 0.29 mmol), NaI (43 mg, 0.29 mmol) and (*S*)-5-oxopyrrolidine-2-carboxylic acid (188 mg, 1.46 mmol) in acetone (1.2 mL) was added Et₃N (0.20 mL, 1.46 mmol). The reaction was stirred at 25 °C for 5 h and then concentrated. The mixture was diluted with EA (20 mL) and filtered. The filtrate was concentrated and then purified on silica gel column eluting with hexane/EA (100% hexane to 4/6) to afford 50 mg (39% yield) of the titled compound (**A-2**) as a white foam.

**¹HNMR** (500MHz, CDCl₃) δ = 1.48 (br. s, 3H), 1.73 (m, 2H), 1.88 (br. s, 1H), 2.01 (m, 1H), 2.27-2.44 (m, 5H), 2.54-2.59 (m, 1H), 2.66-2.71 (m, 1H), 3.02 (br. s, 3H), 3.29-3.33 (m, 1H), 4.19-4.25 (m, 1H), 6.22-6.57 (m, 1H), 6.73-6.79 (m, 1H), 6.97 (br. s, 1H), 7.23-7.27 (m, 2H), 7.44 (m, 1H).

**MS (ESI):** [M + H]⁺= 437.2.

### Example 3: 1-((((S)-1-(2-chlorophenyl)-2-oxocyclohexyl)(methyl)carbamoyl)oxy)ethyl acetylglycinate (A-3)

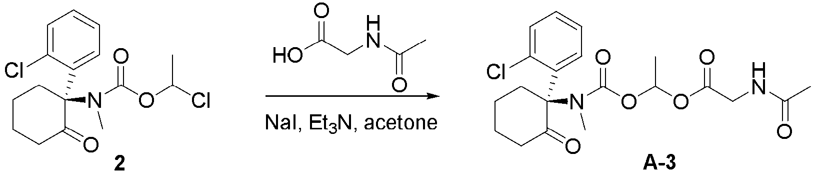

To a solution of compound **2** (172 mg, 0.5 mmol), NaI (75 mg, 0.5 mmol) and acetylglycine (176 mg, 1.5 mmol) in acetone (6 mL) was added Et₃N (0.35 mL, 2.5 mmol). The reaction was heated to 70 °C for 16 h. The reaction was concentrated and re-dissolved in DCM (10 mL), washed with aqueous saturated NaHCO₃ solution (10 mL) and brine (10 mL). The organic layer was dried over MgSO₄, filtered and concentrated to get an oil, which was purified on silica gel column eluting with hexane/EA (100% hexane to 1/2) to afford 110 mg (35% yield) of the titled compound (**A-3**) as a white foam.

**¹HNMR** (500 MHz, CD₃OD) δ = 1.51 (br. s, 3H), 1.77-1.83 (m, 3H), 1.99-2.01 (m, 3H), 2.01-2.06 (m, 1H), 2.33-2.46 (m, 2H), 2.67-2.82 (m, 1H), 3.03-3.05 (m, 3H), 3.36 (m, 1H), 3.87-3.97 (m, 2H), 6.73-6.77 (m, 1H), 7.03-7.05 (m, 1H), 7.39-7.45 (m, 2H), 7.45-7.47 (m, 1H).

**MS (ESI):** [M + H]⁺= 425.3.

### Example 4: 1-((((S)-1-(2-chlorophenyl)-2-oxocyclohexyl)(methyl)carbamoyl)oxy)ethyl 2-(3-methyloxetan-3-yl)acetate (A-4)

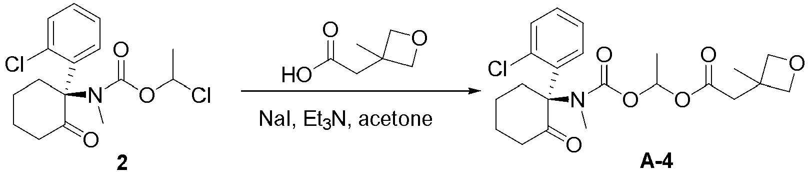

To a solution of compound 2 (262 mg, 0.76 mmol), NaI (114 mg, 0.76 mmol) and 2-(3-methyloxetan-3-yl)acetic acid (296 mg, 2.28 mmol) in acetone (9 mL) was added Et₃N (0.53 mL, 3.8 mmol). The reaction was heated to 70 °C for 3 h. The reaction was concentrated and re-dissolved in DCM (5 mL), washed with H₂O (5 mL) and brine (5 mL). The organic layer was dried over MgSO₄, filtered and concentrated to get an oil, which was purified on silica gel column eluting with hexane/EA (100% hexane to 3/1) to afford a yellow oil. Ether (3 mL) was added, filtered and the solid was washed with cold ether to afford 102 mg (31% yield) of the titled compound (**A-4**) as a white solid.

**¹HNMR** (500 MHz, CD₃OD) δ = 1.38 (s, 3H), 1.48 (br. s, 3H), 1.76-1.84 (m, 3H), 2.03-2.06 (m, 1H), 2.36 (d, *J =* 15.2 Hz, 1H), 2.46 (d, *J =* 13.5 Hz, 1H), 2.70-2.73 (m, 1H), 2.73 (s, 2H), 3.04 (s, 3H), 3.31-3.36 (m, 1H), 4.36-4.38 (m, 2H), 4.59-4.61 (m, 2H), 6.69-6.72 (m, 1H), 7.05-7.08 (m, 1H), 7.29-7.31 (m, 2H), 7.45-7.47 (m, 1H).

**MS (ESI):** [M + H]⁺ = 438.4.

The filtrate was concentrated to get an oil and stored at -20 °C to get a sticky solid. The mixture was diluted with ether (2 mL) and collected the filtrate. The filtrate was concentrated to afford 40 mg (12 % yield) of **A-4 isomer** as a colorless oil.

**¹HNMR** (500 MHz, CD₃OD) δ = 1.32-1.40 (m, 3H), 1.49 (br. s, 3H), 1.72-1.92 (m, 3H), 2.03-2.06 (m, 1H), 2.41 (d, *J =* 11.8 Hz, 1H), 2.49 (d, *J =* 11.7 Hz, 1H), 2.63-2.74 (m, 2H), 2.75-2.84 (m, 1H), 3.03 (s, 3H), 3.33-3.36 (m, 1H), 4.33-4.36 (m, 2H), 4.57-4.60 (m, 2H), 6.71-6.73 (m, 1H), 7.02-7.04 (m, 1H), 7.29-7.31 (m, 2H), 7.45-7.47 (m, 1H).

### Example 5: 1-((((S)-1-(2-chlorophenyl)-2-oxocyclohexyl)(methyl)carbamoyl)oxy)ethyl acetyl-L-alaninate (A-5)

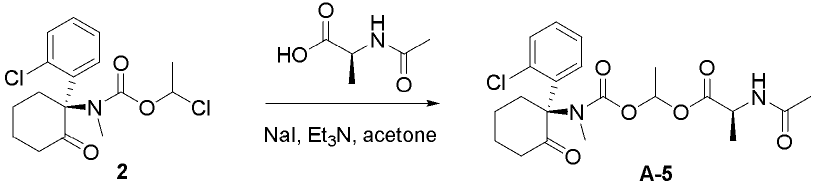

To a solution of compound **2** (172 mg, 0.5 mmol), NaI (150 mg, 1.0 mmol) and (*S*)-2-acetamidopropanoic acid (328 mg, 2.5 mmol) in acetone (6 mL) was added Et₃N (0.35 mL, 2.5 mmol). The reaction was heated to 70 °C for 16 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/1) to afford 149 mg (68% yield) of the titled compound (**A-5**) as a white foam.

**¹HNMR** (500 MHz, DMSO-*d₆*) δ = 1.13-1.28 (m, 3H), 1.31-1.59 (m, 3H), 1.60-1.79 (m, 3H), 1.80-1.90 (m, 4H), 2.21-2.42 (m, 2H), 2.53-2.75 (m, 1H), 2.96-2.98 (m, 3H), 3.06-3.21 (m, 1H), 4.14-4.29 (m, 1H), 6.55-6.65 (m, 1H), 6.91-7.01 (m, 1H), 7.28-7.40 (m, 2H), 7.42-7.53 (m, 1H), 8.15-8.41 (m, 1H).

**MS (ESI):** [M + H]⁺= 439.1.

### Example 6: 1-((((S)-1-(2-chlorophenyl)-2-oxocyclohexyl)(methyl)carbamoyl)oxy)ethyl acetyl-L-valinate (A-6)

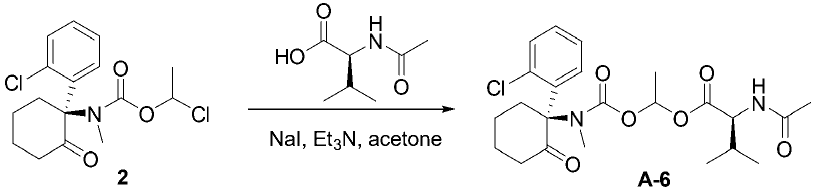

To a solution of compound **2** (172 mg, 0.5 mmol), NaI (150 mg, 1.0 mmol) and (S)-2-acetamido-3-methylbutanoic acid (239 mg, 1.5 mmol) in acetone (6 mL) was added Et₃N (0.35 mL, 2.5 mmol). The reaction was heated to 70 °C for 16 h. The reaction was concentrated and re-dissolved in DCM (5 mL), washed with aqueous saturated NaHCO₃ solution (5 mL) and brine (5 mL). The organic layer was dried over MgSO₄, filtered concentrated to get an oil, which was purified on silica gel column eluting with hexane/EA (100% hexane to 1/1) to afford 159 mg (68% yield) of the titled compound (**A-6**) as a white foam.

**¹HNMR** (500 MHz, DMSO-*d₆*) δ = 0.70-0.95 (m, 6H), 1.30-1.55 (m, 3H), 1.60-1.79 (m, 3H), 1.88 (s, 3H), 1.90-2.08 (m, 1H), 2.22-2.41 (m, 2H), 2.55-2.70 (m, 1H), 2.95-2.97 (m, 3H), 3.05-3.20 (m, 2H), 4.10-4.25 (m, 1H), 6.55-6.78 (m, 1H), 6.92-7.05 (m, 1H), 7.25-7.43 (m, 2H), 7.45-7.55 (m, 1H), 8.10-8.35 (m, 1H).

**MS (ESI):** [M + H]⁺= 467.3.

### Example 7: 1-((((S)-1-(2-chlorophenyl)-2-oxocyclohexyl)(methyl)carbamoyl)oxy)ethyl 3-hydroxy-2-(hydroxymethyl)propanoate (A-7)

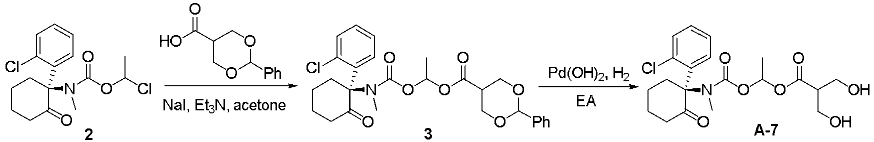

To a solution of compound **2** (172 mg, 0.5 mmol), NaI (75 mg, 0.5 mmol) and 2-phenyl-1,3-dioxane-5-carboxylic acid (520 mg, 2.5 mmol) in acetone (6 mL) was added Et₃N (0.35 mL, 2.5 mmol). The reaction was heated to 70 °C for 16 h. The reaction was concentrated and re-dissolved in DCM (10 mL), washed with aqueous saturated NaHCO₃ solution (10 mL) and brine (10 mL). The organic layer was dried over MgSO₄, filtered and concentrated to get an oil, which was purified on silica gel column eluting with hexane/EA (100% hexane to 4/1) to afford 175 mg (68% yield) of compound **3** as a white foam.

**¹HNMR** (500 MHz, CD₃OD) δ = 1.51 (br. s, 3H), 1.76-1.81 (m, 3H), 2.05-2.08 (m, 1H), 2.35-2.50 (m, 2H), 2.70-2.81 (m, 1H), 3.04-3.10 (m, 4H), 3.36-3.39 (m, 1H), 3.93-4.02 (m, 2H), 4.36-4.39 (m, 2H), 5.42 (s, 1H), 6.70-6.73 (m, 1H), 7.04-7.08 (m, 1H), 7.29-7.35 (m, 5H), 7.42-7.47 (m, 3H).

**MS (ESI):** [M + H]⁺= 516.3.

To a solution of compound 3 (100 mg, 0.19 mmol) in EA (10 mL) was added Pd(OH)₂/C (11 mg). The reaction was stirred at 25 °C under H₂ atmosphere for 50 min. The reaction was filtered through a pad of Celite and concentrated to get an oil, which was purified on silica gel column eluting with hexane/EA (100% hexane to 1/4) to afford 40 mg (49% yield) of the titled compound (**A-7**) as a white foam.

**¹HNMR** (500 MHz, acetone-*d₆*) δ = 1.47 (br. s, 3H), 1.76-1.80 (m, 3H), 2.37-2.40 (m, 2H), 2.70-2.73 (m, 2H), 2.87-3.02 (m, 3H), 3.23-3.34 (m, 1H), 3.77-3.83 (m, 5H), 6.72-6.75 (m, 1H), 7.09-7.11 (m, 1H), 7.28-7.34 (m, 2H), 7.43-7.45 (m, 1H).

**MS (ESI):** [M + H]⁺= 428.1.

### Example 8: 1-((((S)-1-(2-chlorophenyl)-2-oxocyclohexyl)(methyl)carbamoyl)oxy)ethyl 2-(((3-methyloxetan-3-yl)methyl)sulfinyl)acetate (A-8)

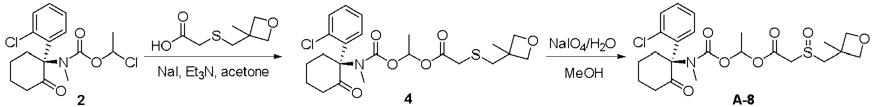

To a solution of compound **2** (172 mg, 0.5 mmol), NaI (75 mg, 0.5 mmol) and2-(((3-methyloxetan-3-yl)-methyl)thio)acetic acid (264 mg, 1.5 mmol) in acetone (6 mL) was added triethylamine (0.35 mL, 2.5 mmol). The reaction was heated to 70 °C for 2 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 2/1) to afford 180 mg (74% yield) of compound **4** as a yellow oil.

**¹HNMR** (500 MHz, acetone-*d₆*) δ = 1.20-1.35 (m, 3H), 1.40-1.65 (m, 3H), 1.70-1.90 (m, 3H), 2.30-2.60 (m, 3H), 2.65-2.80 (m, 1H), 2.95-3.00 (m, 2H), 3.04-3.07 (m, 3H), 3.20-3.45 (m, 3H), 4.20-4.30 (m, 2H), 4.35-4.50 (m, 2H), 6.75-6.85 (m, 1H), 7.05-7.15 (m, 1H), 7.25-7.40 (m, 2H), 7.45-7.50 (m, 1H).

**MS (ESI):** [M + H]⁺= 484.1.

To a solution of compound **4** (140 mg, 0.29 mmol) in MeOH (1.4 mL) was added a solution of NaIO₄ (62 mg, 0.29 mmol) in H₂O (0.7 mL) dropwise at 0 °C. The reaction was stirred at 25 °C for 16 h, filtered and collected the filtrate. The filtrate was concentrated and purified on silica gel column eluting with DCM/MeOH (100% DCM to 98/2) to afford 38 mg (26% yield) of the titled compound (**A-8**) as a white foam.

**¹HNMR** (500 MHz, DMSO-*d₆*) δ = 1.43-1.51 (m, 6H), 1.64-1.70 (m, 3H), 1.95-1.99 (m, 1H), 2.30-2.33 (m, 2H), 2.55-2.61 (m, 1H), 2.96-3.03 (m, 4H), 3.11-3.14 (m, 1H), 3.40-3.44 (m, 1H), 3.98-4.10 (m, 2H), 4.21-4.28 (m, 2H), 4.48-4.50 (m, 1H), 4.59-4.62 (m, 1H), 6.69-6.71 (m, 1H), 6.97-6.99 (m, 1H), 7.33-7.35 (m, 2H), 7.46-7.48 (m, 1H).

**MS (ESI):** [M + H]⁺= 500.1.

### Example 9: 1-((((S)-1-(2-chlorophenyl)-2-oxocyclohexyl)(methyl)carbamoyl)oxy)ethyl 2-(((3-methyloxetan-3-yl)methyl)sulfonyl)acetate (A-9)

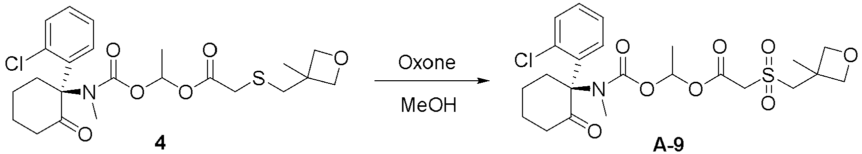

To a solution of compound **4** (141 mg, 0.29 mmol) in MeOH (1.1 mL) was added a solution of Oxone (356 mg, 0.58 mmol) in H₂O (0.9 mL) dropwise at 0 °C. The reaction was stirred at 25 °C for 16 h. The reaction was concentrated and re-dissolved in DCM (5 mL), washed with H₂O (5 mL). The organic layer was dried over MgSO4, filtered concentrated to get an oil, which was purified on silica gel column eluting with hexane/EA (100% hexane to 1/1) to afford 42 mg (29% yield) of the titled compound (**A-9**) as a white foam.

**¹HNMR** (500 MHz, acetone-*d₆*) δ = 1.19-1.21 (m, 2H), 1.59-1.62 (m, 6H), 1.78-1.93 (m, 4H), 2.45-2.57 (m, 2H), 3.04-3.08 (m, 3H), 3.20-3.40 (m, 1H), 3.79-3.82 (m, 2H), 4.25-4.30 (m, 3H), 4.53-4.67 (m, 2H), 6.81-6.84 (m, 1H), 7.09-7.11 (m, 1H), 7.31-7.37 (m, 2H), 7.37-7.46 (m, 1H).

**MS (ESI):** [M + H]⁺= 516.2.

### Example 10: 1-((((S)-1-(2-chlorophenyl)-2-oxocyclohexyl)(methyl)carbamoyl)oxy)ethyl (2R)-2-hydroxypropanoate (A-10)

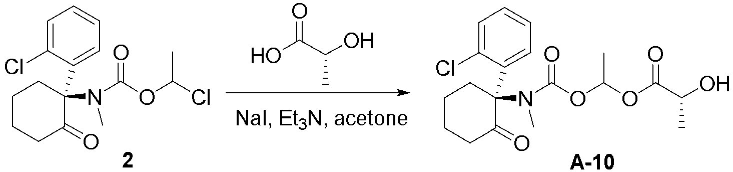

To a solution of compound **2** (172 mg, 0.5 mmol), NaI (75 mg, 0.5 mmol) and *R*-lactic acid (227 mg, 2.5 mmol) in acetone (6 mL) was added Et₃N (0.35 mL, 2.5 mmol). The reaction was heated to 70 °C for 3.5 h. The reaction was concentrated and re-dissolved in DCM (10 mL), washed with H₂O (10 mL) and brine (10 mL). The organic layer was dried over MgSO₄, filtered and concentrated to get an oil, which was purified on silica gel column eluting with hexane/EA (100% hexane to 2/1) to afford 100 mg (50% yield) of the titled compound (**A-10**) as a white foam.

**¹HNMR** (500 MHz, DMSO-*d₆*) δ = 1.20-1.26 (m, 3H), 1.45-1.47 (m, 3H), 1.65-1.69 (m, 3H), 1.98 (br. s, 1H), 2.29-2.36 (m, 2H), 2.58 (br. s, 1H), 2.95-2.98 (m, 3H), 3.12-3.16 (m, 1H), 4.11-4.13 (m, 1H), 5.47-5.56 (m, 1H), 6.62-6.65 (m,1H), 6.93-6.95 (m, 1H), 7.31-7.34 (m, 2H), 7.45-7.48 (m, 1H).

**MS (ESI):** [M + H]⁺= 398.1.

### Example 11: 1-((((S)-1-(2-chlorophenyl)-2-oxocyclohexyl)(methyl)carbamoyl)oxy)ethyl (2R)-2-acetoxypropanoate (A-11)

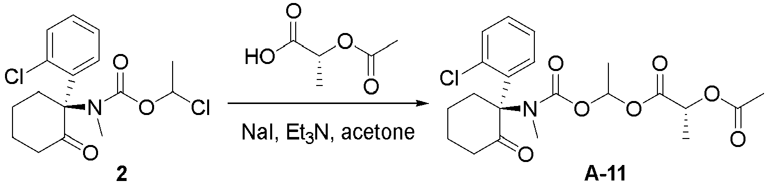

To a solution of compound **2** (172 mg, 0.5 mmol), NaI (79 mg, 0.525 mmol) and (*R*)-2-acetoxypropanoic acid (172 mg, 0.5 mmol) in acetone (6 mL) was added Et₃N (0.35 mL, 2.5 mmol). The reaction was heated to 70 °C for 16 h. The reaction was concentrated and dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 65/35) to afford 204 mg (93% yield) of the titled compound (**A-11**) as a white foam.

**¹HNMR** (500 MHz, DMSO-*d₆*) δ = 1.20-1.55 (m, 6H), 1.56-1.69 (m, 4H), 2.06-2.10 (m, 3H), 2.25-2.42 (m, 2H), 2.55-2.65 (m, 1H), 2.97 (d, *J =* 6.4 Hz, 3H), 3.05-3.21 (m, 1H), 4.85-5.02 (m, 1H), 6.60-6.70 (m, 1H), 6.90-7.05 (m, 1H), 7.21-7.39 (m, 2H), 7.41-7.48 (m, 1H).

**MS (ESI):** [M + H]⁺= 440.0.

### Example 12: 1-((((S)-1-(2-chlorophenyl)-2-oxocyclohexyl)(methyl)carbamoyl)oxy)ethyl nicotinate (A-12)

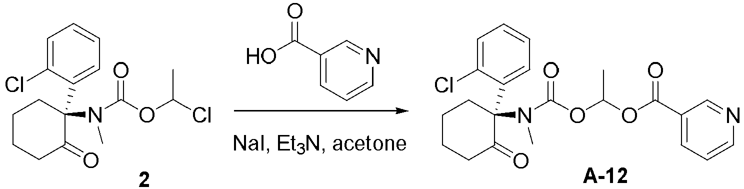

To a solution of compound **2** (86 mg, 0.25 mmol), NaI (75 mg, 0.5 mmol) and nicotinic acid (92 mg, 0.75 mmol) in acetone (3 mL) was added Et₃N (0.18 mL, 1.25 mmol). The reaction was heated to 70 °C for 3 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 3/1) to afford 47 mg (47% yield) of the titled compound (**A-12**) as a white solid.

**¹HNMR** (500 MHz, acetone-*d₆*) δ = 1.46-1.88 (m, 6H), 2.28-2.62 (m, 3H), 2.66-2.78 (m, 1H), 3.07-3.11 (m, 3H), 3.18-3.38 (m, 1H), 6.94-7.06 (m, 1H), 7.08-7.18 (m, 1H), 7.22-7.36 (m, 2H), 7.40-7.50 (m, 1H), 7.54-7.62 (m, 1H), 8.18-8.40 (m, 1H), 8.78-8.88 (m, 1H), 9.04-9.24 (m, 1H).

**MS (ESI):** [M + H]⁺= 431.2.

### Example 13: 1-((((S)-1-(2-chlorophenyl)-2-oxocyclohexyl)(methyl)carbamoyl)oxy)ethyl 3-benzylbenzoate (A-13)

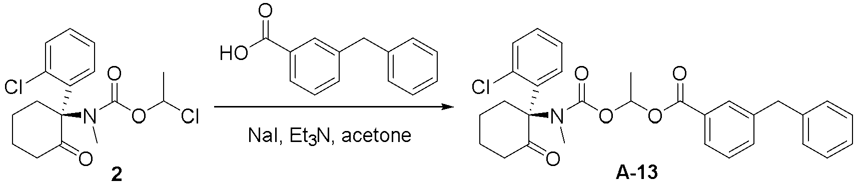

To a solution of compound **2** (54 mg, 0.16 mmol), NaI (25 mg, 0.17 mmol) and 3-benzylbenzoic acid (100 mg, 0.47 mmol) in acetone (2 mL) was added Et₃N (0.11 mL, 0.78 mmol). The reaction was heated to 70 °C for 16 h. The reaction was concentrated and dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 4/1) to afford 60 mg (74% yield) of the titled compound (**A-13**) as a colorless solid.

**¹HNMR** (500 MHz, CD₃OD) δ = 1.51-1.69 (m, 2H), 1.70-1.88 (m, 3H), 1.97-2.12 (m, 1H), 2.29-2.48 (m, 2H), 2.68-2.80 (m, 1H), 3.05 (d, *J =* 12.9 Hz, 3H), 3.24-3.30 (m, 1H), 3.32-3.44 (m, 1H), 4.05 (d, *J =* 4.3 Hz, 2H), 6.92-6.97 (m, 1H), 7.01-7.08 (m, 1H), 7.13-7.22 (m, 4H), 7.23-7.33 (m, 3H), 7.35-7.45 (m, 2H), 7.46-7.51 (m, 1H), 7.76-7.92 (m, 2H).

**MS (ESI):** [M + H]⁺= 520.4.

### Example 14: 1-((((S)-1-(2-chlorophenyl)-2-oxocyclohexyl)(methyl)carbamoyl)oxy)ethyl benzo[d][1,3]dioxole-5-carboxylate (A-14)

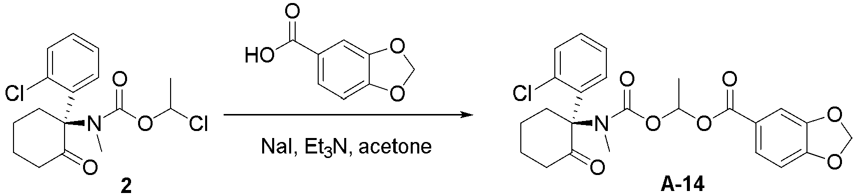

To a solution of compound **2** (86 mg, 0.25 mmol), NaI (39 mg, 0.26 mmol) and benzo[*d*][1,3]dioxole-5-carboxylic acid (125 mg, 0.75 mmol) in acetone (3 mL) was added Et₃N (0.18 mL, 1.25 mmol). The reaction was heated to 70 °C for 16 h. The reaction was concentrated and dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 7/3) to afford 110 mg (93% yield) of the titled compound (**A-14**) as a white solid.

**¹HNMR** (500 MHz, CD₃OD) δ = 1.43-1.70 (m, 3H), 1.71-1.90 (m, 3H), 2.03-2.15 (m, 1H), 2.28-2.52 (m, 2H), 2.65-2.87 (m, 1H), 3.07 (d, *J =* 15.4 Hz, 3H), 3.34-3.45 (m, 1H), 6.08 (s, 2H), 6.87-6.96 (m, 2H), 7.02-7.12 (m, 1H), 7.22-7.33 (m, 2H), 7.34-7.50 (m, 2H), 7.57-7.72 (m, 1H).

**MS (ESI):** [M + H]⁺= 474.3.

### Example 15: 1-((((S)-1-(2-chlorophenyl)-2-oxocyclohexyl)(methyl)carbamoyl)oxy)ethyl 1-methylpiperidine-4-carboxylate (A-15)

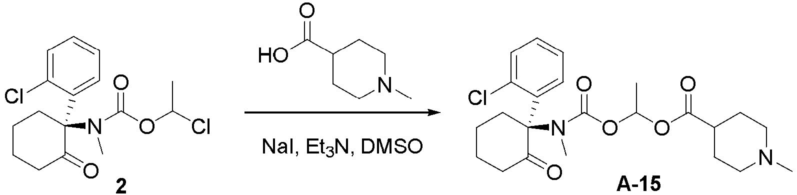

To a solution of compound **2** (86 mg, 0.25 mmol), NaI (75 mg, 0.5 mmol) and 1-methylpiperidine-4-carboxylic acid (117 mg, 0.82 mmol) in DMSO (1 mL) was added Et₃N (0.18 mL, 1.25 mmol). The reaction was stirred at 25 °C for 3 h. The reaction was concentrated and then purified on silica gel column eluting with DCM/MeOH (100% hexane to 95/5) to afford 23 mg (20% yield) of the titled compound (**A-15**) as a yellow oil.

**¹HNMR** (600 MHz, CD₃OD) δ = 1.22-1.36 (m, 1H), 1.38-1.66 (m, 3H), 1.72-1.90 (m, 5H), 1.92-2.02 (m, 2H), 2.06-2.12 (m, 1H), 2.28-2.62 (m, 8H), 2.68-2.82 (m, 1H), 2.86-3.14 (m, 2H), 3.05 -3.07 (m, 2H), 3.31-3.40 (m, 1H), 6.68-6.77 (m, 1H), 6.98-7.08 (m, 1H), 7.26-7.37 (m, 2H), 7.44-7.50 (m, 1H).

**MS (ESI):** [M + H]⁺= 451.2.

### Example 16: 1-(isonicotinoyloxy)ethyl (S)-1-(2-chlorophenyl)-2-oxocyclohexylmethylcarbamate (A-16)

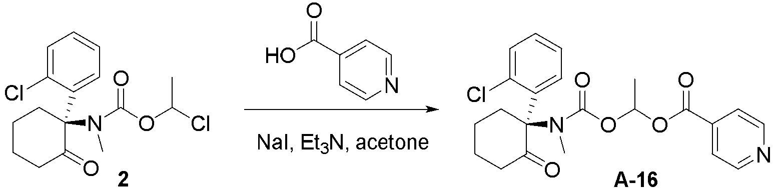

To a solution of compound **2** (86 mg, 0.25 mmol), NaI (75 mg, 0.5 mmol) and isonicotinic acid (92 mg, 0.75 mmol) in acetone (3 mL) was added Et₃N (0.18 mL, 1.25 mmol). The reaction was heated to 70 °C for 3 h. The reaction was concentrated and dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 4/1) to afford 50 mg (46% yield) of the titled compound (**A-16**) as a white solid.

**¹HNMR** (500 MHz, acetone-*d₆*) δ = 1.50-1.87 (m, 6H), 2.32-2.54 (m, 3H), 2.65-2.78 (m, 1H), 3.04-3.13 (m, 3H), 3.17-3.35 (m, 1H), 6.96-7.04 (m, 1H), 7.07-7.17 (m, 1H), 7.23-7.36 (m, 2H), 7.39-7.48 (m, 1H), 7.77-7.92 (m, 2H), 8.78-8.86 (m, 2H).

**MS (ESI):** [M + H]⁺= 430.8.

### Example 17: 1-(2-(isobutyramido)acetoyloxy)ethyl (S)-1-(2-chlorophenyl)-2-oxocyclohexylmethylcarbamate (A-17)

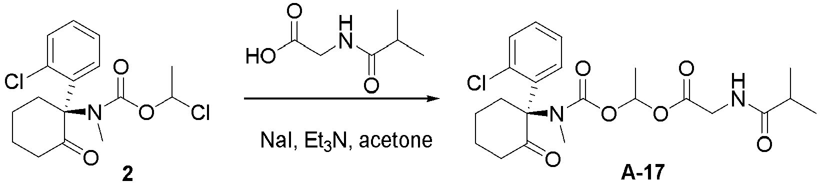

To a solution of compound **2** (86 mg, 0.25 mmol), NaI (75 mg, 0.5 mmol) and 2-(isobutyramido)acetic acid (109 mg, 0.75 mmol) in acetone (3 mL) was added Et₃N (0.18 mL, 1.25 mmol). The reaction was heated to 70 °C for 16 h. The reaction was concentrated and dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/1) to afford 57 mg (50% yield) of the titled compound (**A-17**) as a white solid.

**¹HNMR** (500 MHz, CD₃OD) δ = 1.12-1.16 (m, 6H), 1.52 (s, 2H), 1.72-1.89 (m, 3H), 2.03-2.12 (m, 1H), 2.32-2.56 (m, 3H), 2.66-2.85 (m, 1H), 3.00-3.08 (m, 3H), 3.25-3.40 (m, 2H), 3.84-4.01 (m, 2H), 6.70-6.78 (m, 1H), 7.01-7.10 (m, 1H), 7.26-7.35 (m, 2H), 7.44-7.47 (m, 1H).

**MS (ESI):** [M + H]⁺= 452.6.

### Example 18: 1-(3-acetamidopropanoyloxy)ethyl (S)-1-(2-chlorophenyl)-2-oxocyclohexylmethylcarbamate (A-18)

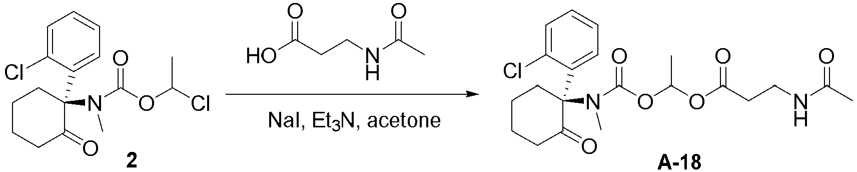

To a solution of compound **2** (86 mg, 0.25 mmol), NaI (75 mg, 0.5 mmol) and 3-acetamidopropanoic acid (98 mg, 0.75 mmol) in acetone (3 mL) was added Et₃N (0.18 mL, 1.25 mmol). The reaction was heated to 70 °C for 22 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/2) to afford 20 mg (18% yield) of the titled compound (**A-18**) as a light-yellow oil.

**¹HNMR** (600 MHz, DMSO-*d₆*) δ = 1.30-1.75 (m, 7H), 1.78 (d, *J =* 2.4 Hz, 3H), 1.90-2.05 (m, 1H), 2.20-2.45 (m, 3H), 2.55-2.65 (m, 1H), 2.95-2.97 (m, 3H), 3.00-3.25 (m, 3H), 6.55-6.70 (m, 1H), 6.90-7.10 (m, 1H), 7.25-7.60 (m, 3H), 7.80-7.90 (m, 1H).

**MS (ESI):** [M + H]⁺= 438.9.

### Example 19: 1-(4-acetamidobutanoyloxy)ethyl (S)-1-(2-chlorophenyl)-2-oxocyclohexylmethylcarbamate (A-19)

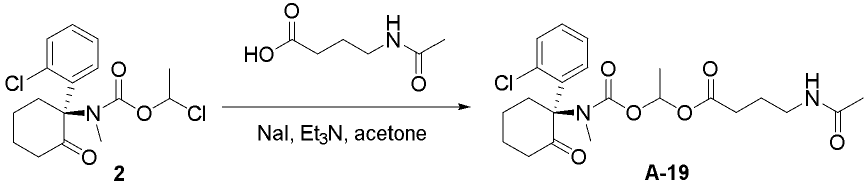

To a solution of compound **2** (86 mg, 0.25 mmol), NaI (75 mg, 0.5 mmol) and 4-acetamidobutanoic acid (109 mg, 0.75 mmol) in acetone (3 mL) was added Et₃N (0.18 mL, 1.25 mmol). The reaction was heated to 70 °C for 22 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/2) to afford 72 mg (64% yield) of the titled compound (**A-19**) as a white foam.

**¹HNMR** (600 MHz, DMSO-*d₆*) δ = 1.30-1.55 (m, 3H), 1.56-1.76 (m, 5H), 1.78 (d, *J =* 2.7 Hz, 3H), 1.94-2.04 (m, 1H), 2.23-2.40 (m, 4H), 2.54-2.65 (m, 1H), 2.95-2.97 (m, 3H), 2.99-3.07 (m, 2H), 3.09-3.19 (m, 1H), 6.58-6.66 (m, 1H), 6.92-7.00 (m, 1H), 7.28-7.36 (m, 2H), 7.44-7.49 (m, 1H), 7.80-7.88 (m, 1H).

**MS (ESI):** [M + H]⁺= 452.9.

### Example 20: (2-(3-methyloxetan-3-yl)acetoyloxy)methyl (S)-1-(2-chlorophenyl)-2-oxocyclohexylmethylcarbamate (A-20)

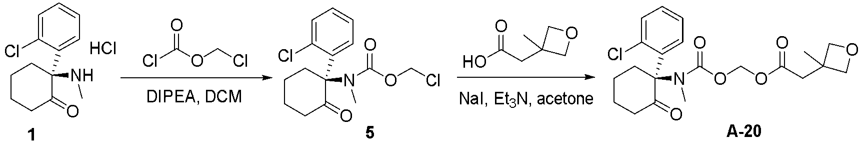

To a solution of S-ketamine hydrochloride **1** (102 mg, 0.375 mmol) and DIPEA (97 mg, 0.75 mmol) in DCM (3.75 mL) was added chloromethyl chloroformate (121 mg, 0.94 mmol) slowly at 0 °C. The reaction was stirred at 25 °C for 24 h. The reaction was diluted with DCM (5 mL) and washed with water (5 mL) and brine (5 mL). The organic layer was dried over MgSO4, filtered and concentrated to get an oil, which was purified on silica gel column eluting with hexane/EA (100% hexane to 9/1) to afford 93 mg (75% yield) of compound **5** as a white solid.

**¹HNMR** (500 MHz, acetone-*d₆*) δ = 1.68-1.90 (m, 4H), 2.42-2.49 (m, 1H), 2.50-2.59 (m, 1H), 2.65-2.75 (m, 1H), 3.07 (s, 3H), 3.20-3.33 (m, 1H), 5.88 (s, 2H), 7.05-7.13 (m, 1H), 7.28-7.36 (m, 2H), 7.43-7.50 (m, 1H).

**MS (ESI):** [M + H]⁺= 330.2.

To a solution of compound **5** (82 mg, 0.25 mmol), NaI (75 mg, 0.5 mmol) and 2-(3-methyloxetan-3-yl)acetic acid (98 mg, 0.75 mmol) in acetone (3 mL) was added K₂CO₃ (173 mg, 1.25 mmol). The reaction was heated to 70 °C for 2 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/2) to afford 84 mg (80% yield) of the titled compound (**A-20**) as a white solid.

**¹HNMR** (500 MHz, acetone-*d₆*) δ = 1.39 (s, 3H), 1.68-1.88 (m, 3H), 1.98-2.09 (m, 1H), 2.41-2.53 (m, 2H), 2.65-2.73 (m, 1H), 2.77 (s, 2H), 3.03 (s, 3H), 3.19-3.32 (m, 1H), 4.28 (d, *J* = 5.85 Hz, 2H), 4.50 (d, *J =* 5.85 Hz, 2H), 5.66-5.86 (m, 2H), 7.05-7.11 (m, 1H), 7.28-7.35 (m, 2H), 7.42-7.48 (m, 1H).

**MS (ESI):** [M + H]⁺= 424.5.

### Example 21: 1-(oxetane-3-carboxyloyloxy)ethyl (S)-1-(2-chlorophenyl)-2-oxocyclohexylmethylcarbamate (A-21)

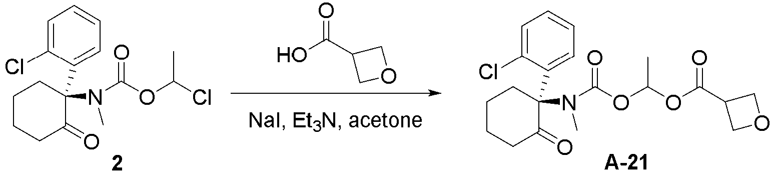

To a solution of compound **2** (86 mg, 0.25 mmol), NaI (75 mg, 0.5 mmol) and oxetane-3-carboxylic acid (77 mg, 0.75 mmol) in acetone (3 mL) was added Et₃N (0.18 mL, 1.25 mmol). The reaction was heated to 70 °C for 16 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (7/3) to afford 40 mg (49% yield) of the titled compound (**A-21**) as a light yellow oil.

**¹HNMR** (600 MHz, acetone-*d₆*) δ = 1.33-1.64 (m, 3H), 1.68-1.90 (m, 4H), 2.34-2.53 (m, 2H), 2.65-2.77 (m, 1H), 3.04-3.06 (m, 3H), 3.20-3.34 (m, 1H), 3.82-3.94 (m, 1H), 4.55-4.82 (m, 4H), 6.75-6.82 (m, 1H), 7.02-7.11 (m, 1H), 7.26-7.36 (m, 2H), 7.41-7.48 (m, 1H).

**MS (ESI)**: [M + H]⁺ = 409.9.

### Example 22: 1-((((S)-1-(2-chlorophenyl)-2-oxocyclohexyl)(methyl)carbamoyl)oxy)propyl 2-(3-methyloxetan-3-yl)acetate (A-22)

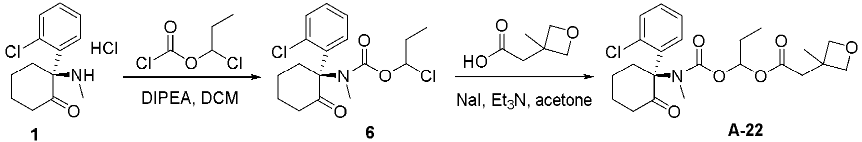

To a solution of S-ketamine hydrochloride **1** (137 mg, 0.5 mmol) and DIPEA (130 mg, 1.0 mmol) in DCM (5 mL) was added 1-chloroethyl carbonochloridate (94 mg, 0.6 mmol) slowly at 0 °C. The reaction was stirred at 25 °C for 16 h. The reaction was diluted with DCM (5 mL) and washed with water (5 mL) and brine (5 mL). The organic layer was dried over MgSO4, filtered and concentrated to get an oil, which was purified on silica gel column eluting with hexane/EA (100% hexane to 10/1) to afford 133 mg (74% yield) of compound **6** as a colorless oil.

**¹HNMR** (600 MHz, CDCl₃) δ = 1.04-1.06 (m, 3H), 1.75-1.89 (m, 4H), 2.02-2.05 (m, 2H), 2.37-2.50 (m, 1H), 2.55-2.59 (m, 1H), 2.70-2.73 (m, 1H), 3.01-3.08 (m, 3H), 3.27-3.35 (m, 1H), 6.36-6.40 (m, 1H), 6.94-7.00 (m, 1H), 7.22-7.25 (m, 2H), 7.41-7.45 (m, 1H).

To a solution of compound **6** (90 mg, 0.25 mmol), NaI (37 mg, 0.25 mmol), and 2-(3-methyloxetan-3-yl)acetic acid (98 mg, 0.75 mmol) in acetone (1 mL) was added Et₃N (0.18 mL, 1.25 mmol). The reaction was heated to 70 °C for 10 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 2/1) to afford 32 mg (28% yield) of the titled compound (**A-22**) as a yellow oil.

**¹HNMR** (600 MHz, acetone-*d₆*) δ = 0.89-1.04 (m, 3H), 1.38-1.41 (m, 3H), 1.88-1.78 (m, 5H), 2.41-2.55 (m, 2H), 2.67-2.84 (m, 4H), 3.06-3.09 (m, 3H), 3.20-3.37 (m, 1H), 4.29-4.31 (m, 2H), 4.50-4.54 (m, 2H), 6.63-6.67 (m, 1H), 7.09-7.014 (m, 1H), 7.32-7.35 (m, 2H), 7.49-7.46 (m, 1H).

**MS (ESI):** [M + H]⁺= 452.0.

### Example 23: 1-(tetrahydro-2H-pyran-4-carboxyloyloxy)ethyl (S)-1-(2-chlorophenyl)-2-oxocyclohexylmethylcarbamate (A-23)

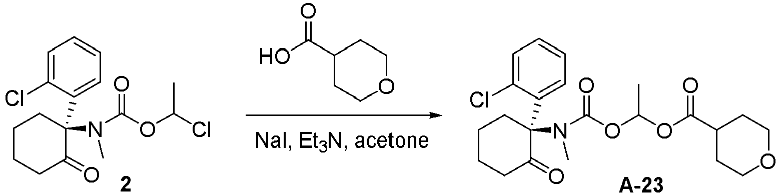

To a solution of compound **2** (86 mg, 0.25 mmol), NaI (75 mg, 0.5 mmol) and tetrahydro-2*H-*pyran-4-carboxylic acid (98 mg, 0.75 mmol) in acetone (3 mL) was added Et₃N (0.18 mL, 1.25 mmol). The reaction was heated to 70 °C for 16 h. The reaction was concentrated and dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (7/3) to afford 72 mg (66% yield) of the titled compound (**A-23**) as a white foam.

**¹HNMR** (500 MHz, CD₃OD) δ = 1.51 (s, 3H), 1.61-1.91 (m, 7H), 2.03-2.12 (m, 1H), 2.32-2.52 (m, 2H), 2.56-2.65 (m, 1H), 2.67-2.83 (m, 1H), 3.05 (d, *J =* 11.5 Hz, 3H), 3.32-3.39 (m, 1H), 3.40-3.50 (m, 2H), 3.81-3.94 (m, 2H), 6.69-6.75 (m, 1H), 6.99-7.08 (m, 1H), 7.26-7.32 (m, 2H), 7.43-7.48 (m, 1H).

**MS (ESI):** [M + H]⁺= 438.1.

### Example 24: 1-(2-(3-methyloxetan-3-yl)acetoyloxy)-2-methylpropyl (S)-1-(2-chlorophenyl)-2-oxocyclohexylmethylcarbamate (A-24)

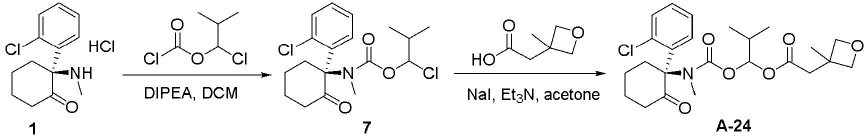

To a solution of S-ketamine hydrochloride **1** (200 mg, 0.73 mmol) and DIPEA (0.25 mL, 1.46 mmol) in DCM (8 mL) was added 1-chloro-2-methylpropyl chloroformate (312 mg, 1.83 mmol) slowly at 0 °C and then stirred at 25 °C for 1 h. The reaction was diluted with DCM (5 mL) and washed with water (5 mL) and brine (5 mL). The organic layer was dried over MgSO₄, filtered and concentrated to get an oil, which was purified on silica gel column eluting with hexane/EA (100% hexane to 9/1) to afford 230 mg (85% yield) of compound 7 as a white solid.

**¹HNMR** (600 MHz, acetone-*d₆*) δ = 0.75-1.27 (m, 6H), 1.68-1.90 (m, 3H), 2.38-2.58 (m, 2H), 2.65-2.77 (m, 1H), 2.83-2.85 (m, 2H), 3.08-3.12 (m, 3H), 3.18-3.36 (m, 1H), 6.35 (d, *J* = 4.3 Hz, 1H), 7.01-7.11 (m, 1H), 7.28-7.35 (m, 2H), 7.44-7.49 (m, 1H).

**MS (ESI):** [M + H]⁺= 371.8.

To a solution of compound **7** (93 mg, 0.25 mmol), NaI (75 mg, 0.5 mmol) and 2-(3-methyloxetan-3-yl)acetic acid (98 mg, 0.75 mmol) in acetone (3 mL) was added Et₃N (0.18 mL, 1.25 mmol). The reaction was heated to 70 °C for 5 h. The reaction was concentrated and dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (7/3) to afford 15 mg (13% yield) of the titled compound (**A-24**) as a white foam.

**¹HNMR** (600 MHz, CD₃OD) δ = 1.01 (s, 6H), 1.40 (s, 3H), 1.72-1.90 (m, 3H), 1.99-2.16 (m, 3H), 2.32-2.52 (m, 2H), 2.64-2.88 (m, 3H), 3.05 (d, *J =* 20.2 Hz, 3H), 3.33-3.43 (m, 1H), 4.38 (dd, *J* = 1.8, 6.0 Hz, 2H), 4.6 (d, *J* = 5.8 Hz, 2H), 6.50 (dd, *J* = 4.8, 7.8 Hz, 1H), 7.01-7.10 (m, 1H), 7.27-7.32 (m, 2H), 7.43-7.48 (m, 1H).

**MS (ESI):** [M + H]⁺= 466.1.

### Example 25: 1-((((S)-1-(2-chlorophenyl)-2-oxocyclohexyl)(methyl)carbamoyl)oxy)propyl acetylglycinate (A-25)

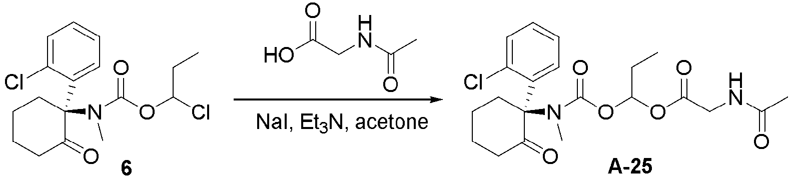

To a solution of compound **6** (90 mg, 0.25 mmol), NaI (37 mg, 0.25 mmol), and acetylglycine (88 mg, 0.75 mmol) in acetone (1 mL) was added Et₃N (0.18 mL, 1.25 mmol). The reaction was heated to 70 °C for 10 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (1/0 to 1/2) to afford 18 mg (16% yield) of the titled compound (**A-25**) as a white solid.

**¹HNMR** (600 MHz, acetone-*d₆*) δ = 0.86-1.04 (m, 3H), 1.74-1.84 (m, 4H), 1.94-1.95 (m, 3H), 2.06-2.07 (m, 3H), 2.37-2.49 (m, 2H), 2.67-2.79 (m, 1H), 3.03-3.07 (m, 3H), 3.21-3.34 (m, 1H), 3.85-3.94 (m, 1H), 3.99-4.05 (m, 1H), 6.62-6.66 (m, 1H), 7.06-7.09 (m, 1H), 7.28-7.35 (m, 2H), 7.45-7.44 (m, 1H).

**MS (ESI):** [M + H]⁺= 439.0.

### Example 26: 1-(2-acetamidoacetoyloxy)-2-methylpropyl (S)-1-(2-chlorophenyl)-2-oxocyclohexylmethylcarbamate (A-26)

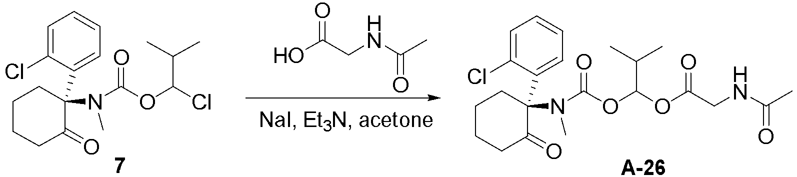

To a solution of compound **7** (93 mg, 0.25 mmol), NaI (75 mg, 0.5 mmol) and acetylglycine (88 mg, 0.75 mmol) in acetone (3 mL) was added Et₃N (0.18 mL, 1.25 mmol). The reaction was heated to 70 °C for 16 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (2/3) to afford 29 mg (28% yield) of the titled compound (**A-26**) as a colorless oil.

**¹HNMR** (600 MHz, DMSO-*d₆*) δ = 0.76-1.11 (m, 6H), 1.60-1.78 (m, 3H), 1.86 (d, *J =* 2.0 Hz, 3H), 1.95-2.05 (m, 1H), 2.26-2.40 (m, 2H), 2.52-2.68 (m, 1H), 2.65-2.98 (m, 3H), 3.04-3.20 (m, 1H), 3.71-3.97 (m, 3H), 6.38-6.47 (m, 1H), 6.89-6.99 (m, 1H), 7.28-7.36 (m, 2H), 7.43-7.49 (m, 1H), 8.33-8.43 (m, 1H).

**MS (ESI):** [M + H]⁺= 453.3.

### Example 27: (nicotinoyloxy)methyl (S)-1-(2-chlorophenyl)-2-oxocyclohexylmethylcarbamate (A-27)

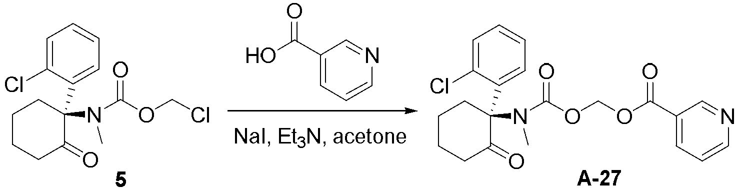

To a solution of compound **5** (82 mg, 0.25 mmol), NaI (75 mg, 0.5 mmol) and nicotinic acid (92 mg, 0.75 mmol) in acetone (3 mL) was added Et₃N (0.18 mL, 1.25 mmol). The reaction was heated to 70 °C for 2 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (3/2) to afford 32 mg (31% yield) of the titled compound (**A-27**) as a white solid.

**¹HNMR** (600 MHz, acetone-*d₆*) δ = 1.66-1.87 (m, 3H), 1.97-2.20 (m, 1H), 2.36-2.55 (m, 2H), 2.67-2.75 (m, 1H), 3.07 (s, 3H), 3.20-3.32 (m, 1H), 5.86-6.18 (m, 2H), 7.07-7.13 (m, 1H), 7.21-7.32 (m, 2H), 7.40-7.46 (m, 1H), 7.56-7.62 (m, 1H), 8.30-8.39 (m, 1H), 8.82-8.89 (m, 1H), 9.12-9.20 (m, 1H).

**MS (ESI):** [M + H]⁺ = 416.9.

### Example 28: 2-(2-chlorophenyl)-2-(methyl(methyl-d3)amino)cyclohexan-1-one (A-28)

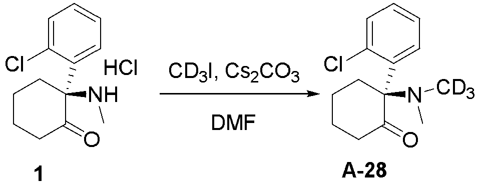

To a solution of S-ketamine hydrochloride **1** (68 mg, 0.25 mmol) and iodomethane-*d₃* (109 mg, 0.75 mmol) and Cs₂CO₃ (163 mg, 0.5 mmol) in DMF (5 mL). The reaction was stirred at 25 °C for 4 h. The reaction was diluted with DCM (5 mL) and washed with water (5 mL) and brine (5 mL). The organic layer was dried over MgSO₄, filtered and concentrated to get an oil, which was purified on silica gel column eluting with hexane/EA (100% hexane to 3/1) to afford 16 mg (23% yield) of the titled compound (**A-28**) as a yellow solid.

**¹HNMR** (500 MHz, CD₃OD) δ = 1.58-1.48 (m, 1H), 1.80-1.62 (m, 3H), 2.03-1.95 (m, 1H), 2.19 (s, 3H), 2.51-2.40 (m, 1H), 2.66-2.56 (m, 1H), 3.20-3.09 (m, 1H), 7.41-7.35 (m, 1H), 7.52-7.43 (m, 2H), 7.62-7.56 (m, 1H).

**MS (ESI):** [M + H]⁺ = 255.0.

### Example 29: (2-acetamidoacetoyloxy)methyl (S)-1-(2-chlorophenyl)-2-oxocyclohexylmethylcarbamate (A-29)

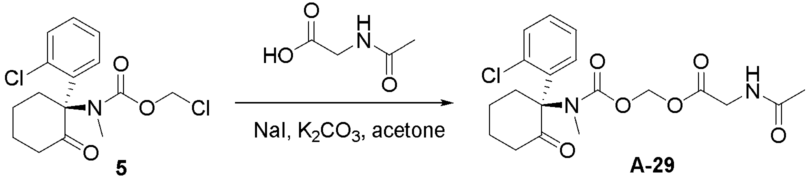

To a solution of compound **5** (50 mg, 0.15 mmol), NaI (45 mg, 0.30 mmol) and 2-acetamidoacetic acid (53 mg, 0.45 mmol) in acetone (2 mL) was added K₂CO₃ (105 mg, 0.76 mmol). The reaction was heated to 70 °C for 3 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/4) to afford 25 mg (40% yield) of the titled compound (**A-29**) as a colorless gum.

**¹HNMR** (600 MHz, DMSO-*d₆*) δ = 1.61-1.77 (m, 3H), 1.87 (s, 3H), 1.95-2.00 (m, 1H), 2.29-2.37 (m, 2H), 2.54-2.63 (m, 1H), 2.96 (s, 3H), 3.08-3.17 (m, 1H), 3.81-3.90 (m, 2H), 5.62-5.78 (m, 2H), 6.93-6.98 (m, 1H), 7.29-7.38 (m, 2H), 7.44-7.48 (m, 1H), 8.36-8.43 (m, 1H).

**MS (ESI):** [M + H]⁺= 411.1.

### Example 30: ((S)-2-acetamido-3-methylbutanoyloxy)methyl (S)-1-(2-chlorophenyl)-2-oxocyclohexylmethylcarbamate (A-30)

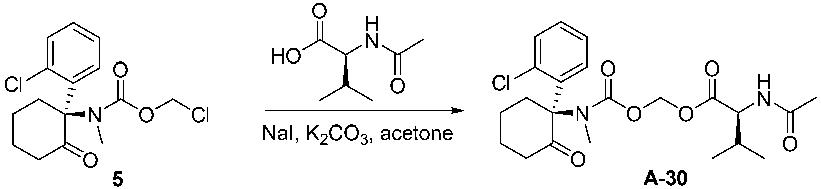

To a solution of compound **5** (50 mg, 0.15 mmol), NaI (45 mg, 0.30 mmol) and (S)-2-acetamido-3-methylbutanoic acid (72 mg, 0.45 mmol) in acetone (2 mL) was added K₂CO₃ (105 mg, 0.76 mmol). The reaction was heated to 70 °C for 3 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/4) to afford 65 mg (95% yield) of the titled compound (**A-30**) as a white foam.

**¹HNMR** (600 MHz, DMSO-*d₆*) δ = 0.82-0.97 (m, 6H) 1.54-1.77 (m, 3H), 1.88 (s, 3H), 1.92-2.07 (m, 2H), 2.29-2.37 (m, 2H), 2.52-2.60 (m, 1H), 2.95 (s, 3H), 3.06-3.17 (m, 1H), 4.09-4.16 (m, 1H), 5.60-5.85 (m, 2H), 6.89-6.99 (m, 1H), 7.26-7.37 (m, 2H), 7.42-7.50 (m, 1H), 8.19-8.29 (m, 1H).

**MS (ESI):** [M + H]⁺= 453.1.

### Example 31: ((S)-2-acetamidopropanoyloxy)methyl (S)-1-(2-chlorophenyl)-2-oxocyclohexylmethylcarbamate (A-31)

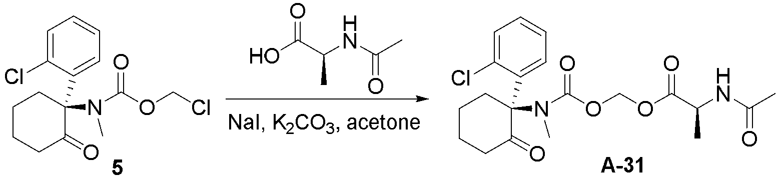

To a solution of compound **5** (50 mg, 0.15 mmol), NaI (46 mg, 0.3 mmol) and (*S*)-2-acetamidopropanoic acid (60 mg, 0.46 mmol) in acetone (1.8 mL) was added K₂CO₃ (105 mg, 0.76 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 13/7) to afford 52 mg (81% yield) of the titled compound (**A-31**) as a white foam.

**¹HNMR** (600 MHz, DMSO-*d₆*) δ = 1.22-1.30 (m, 3H), 1.60-1.82 (m, 3H), 1.84 (s, 3H), 1.93-2.00 (m, 1H), 2.30-2.37 (m, 2H), 2.54-2.60 (m, 1H), 2.96 (s, 3H), 3.09-3.16 (m, 1H), 4.16-4.24 (m, 1H), 5.60-5.80 (m, 2H), 6.94-7.00 (m, 1H), 7.30-7.36 (m, 2H), 7.44-7.49 (m, 1H), 8.38 (d, *J* = 6.0 Hz, 1H).

**MS (ESI):** [M + H]⁺= 424.8.

### Example 32: (2-(isobutyramido)acetoyloxy)methyl (S)-1-(2-chlorophenyl)-2-oxocyclohexylmethylcarbamate (A-32)

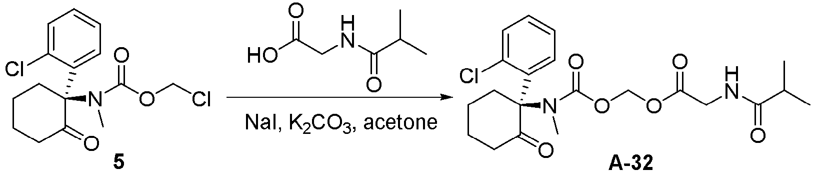

To a solution of compound **5** (50 mg, 0.15 mmol), NaI (46 mg, 0.3 mmol) and 2-(isobutyramido)acetic acid (66 mg, 0.46 mmol) in acetone (1.8 mL) was added K₂CO₃ (105 mg, 0.76 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/1) to afford 47 mg (70% yield) of the titled compound (**A-32**) as a white foam.

**¹HNMR** (600 MHz, DMSO-*d₆*) δ = 1.01 (d, *J =* 6.8 Hz, 6H), 1.62-1.76 (m, 3H), 1.94-2.01 (m, 1H), 2.29-2.38 (m, 2H), 2.39-2.46 (m, 1H), 2.55-2.63 (m, 1H), 2.99 (s, 3H), 3.08-3.16 (m, 1H), 3.85 (d, *J =* 5.3 Hz, 2H), 5.60-5.80 (m, 2H), 6.94-6.98 (m, 1H), 7.30-7.37 (m, 2H), 7.44-7.48 (m, 1H), 8.27 (t, *J =* 5.64 Hz, 1H).

**MS (ESI):** [M + H]⁺= 439.2.

### Example 33: ((S)-2-(isobutyramido)propanoyloxy)methyl (S)-1-(2-chlorophenyl)-2-oxocyclohexylmethylcarbamate (A-33)

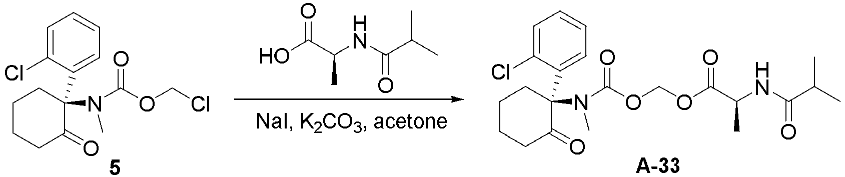

To a solution of compound **5** (50 mg, 0.15 mmol), NaI (45 mg, 0.30 mmol) and (*S*)-2-(isobutyramido)propanoic acid (72 mg, 0.45 mmol) in acetone (2 mL) was added K₂CO₃ (105 mg, 0.76 mmol). The reaction was heated to 70 °C for 4 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 3/2) to afford 30 mg (44% yield) of the titled compound (**A-33**) as a white foam.

**¹HNMR** (600 MHz, DMSO-*d₆*) δ = 0.96-1.03 (m, 6H) 1.23-1.32 (m, 3H), 1.58-1.77 (m, 3H), 1.89-2.03 (m, 1H), 2.28-2.37 (m, 2H), 2.37-2.46 (m, 1H), 2.54-2.64 (m, 1H), 2.96 (s, 3H), 3.08-3.17 (m, 1H), 4.17-4.26 (m, 1H), 5.58-5.82 (m, 2H), 6.95-7.04 (m, 1H), 7.29-7.36 (m, 2H), 7.43-7.50 (m, 1H), 8.17-8.29 (m, 1H).

**MS (ESI):** [M + H]⁺= 453.0.

### Example 34: ((S)-2-(isobutyramido)-3-methylbutanoyloxy)methyl (S)-1-(2-chlorophenyl)-2-oxocyclohexylmethylcarbamate (A-34)

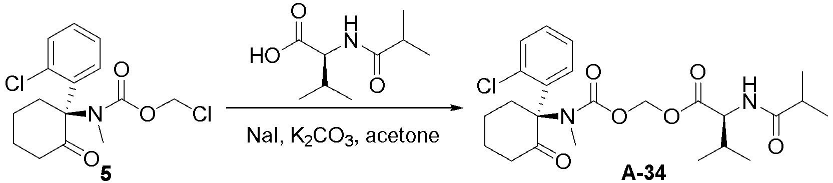

To a solution of compound 5 (50 mg, 0.15 mmol), NaI (46 mg, 0.3 mmol) and (*S*)-2-(isobutyramido)-3-methylbutanoic acid (102 mg, 0.46 mmol) in acetone (1.8 mL) was added K₂CO₃ (105 mg, 0.76 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 7/3) to afford 67 mg (93% yield) of the titled compound (**A-34**) as a yellow foam.

**¹HNMR** (600 MHz, DMSO-*d₆*) δ = 0.85-0.94 (m, 6H), 0.96-1.02 (m, 6H), 1.55-1.65 (m, 1H), 1.66-1.76 (m, 2H), 1.92-1.99 (m, 1H), 2.00-2.08 (m, 1H), 2.31-2.40 (m, 2H), 2.52-2.60 (m, 2H), 2.95 (s, 3H), 3.08-3.17 (m, 1H), 4.14 (t, *J =* 6.8 Hz, 1H), 5.60-5.88 (m, 2H), 6.95-7.00 (m, 1H), 7.30-7.36 (m, 2H), 7.44-7.49 (m, 1H), 8.12 (d, *J =* 7.6 Hz, 1H).

**MS (ESI):** [M + H]⁺= 481.1.

### Example 35: ((((S)-1-(2-chlorophenyl)-2-oxocyclohexyl)(methyl)carbamoyl)oxy)methyl L-valinate (A-35)

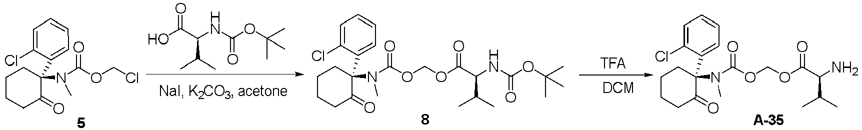

To a solution of compound 5 (150 mg, 0.46 mmol), NaI (137 mg, 0.9 mmol) and ***N*-(*tert-*butoxycarbonyl)-*L*-valine** (297 mg, 1.4 mmol) in acetone (5.4 mL) was added K₂CO₃ (315 mg, 2.3 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 4/1) to afford 191 mg (82% yield) of compound **8** as a white foam.

**¹HNMR** (600 MHz, acetone-*d₆*) δ = 0.94-1.02 (m, 6H), 1.40 (s, 9H), 1.72-1.87 (m, 3H), 2.00-2.03 (m, 1H), 2.11-2.19 (m, 1H), 2.39-2.51 (m, 2H), 2.65-2.72 (m, 1H), 3.05 (s, 3H), 3.23-3.32 (m, 1H), 4.07-4.12 (m, 1H), 5.70-5.94 (m, 2H), 6.31 (br. s, 1H), 7.05-7.12 (m, 1H), 7.28-7.36 (m, 2H), 7.43-7.48 (m, 1H).

**MS (ESI):** [M + H]⁺= 511.3.

To a solution of compound **8** (71 mg, 0.14 mmol) in DCM (5 mL) was added TFA (0.19 mL, 2.5 mmol). The reaction was stirred at 25 °C for 16 h. The reaction was concentrated to afford 67 mg of the titled compound (**A-35**) in TFA salt form as a colorless gum.

**¹HNMR** (600 MHz, DMSO-*d₆*) δ = 0.92-0.99 (m, 6H), 1.56-1.78 (m, 3H), 1.92-1.99 (m, 1H), 2.10-2.20 (m, 1H), 2.32-2.43 (m, 2H), 2.53-2.62 (m, 1H), 2.97 (s, 3H), 3.06-3.17 (m, 1H), 4.02-4.10 (m, 1H), 5.68-5.86 (m, 1H), 5.87-6.05 (m, 1H), 6.95-7.01 (m, 1H), 7.30-7.37 (m, 2H), 7.45-7.51 (m, 1H), 8.45 (br. s, 3H).

**MS (ESI):** [M + H]⁺= 411.2.

### Example 36: (S)-(((1-(2-chlorophenyl)-2-oxocyclohexyl)(methyl)carbamoyl)oxy)methyl glycinate (A-36)

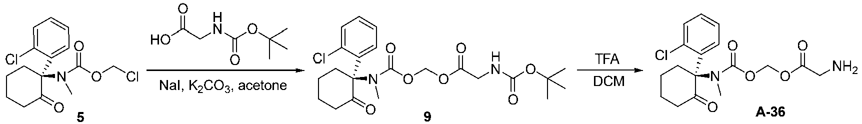

To a solution of compound **5** (50 mg, 0.15 mmol), NaI (46 mg, 0.3 mmol) and ***N-*(*tert-*butoxycarbonyl)-*L*-glycine** (102 mg, 0.46 mmol) in acetone (1.8 mL) was added K₂CO₃ (105 mg, 0.76 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 7/3) to afford 54 mg (76% yield) of compound **9** as a white foam.

**¹HNMR** (600 MHz, acetone-*d₆*) δ = 1.42 (s, 9H), 1.69-1.87 (m, 3H), 1.99-2.03 (m, 1H), 2.38-2.51 (m, 2H), 2.66-2.74 (m, 1H), 3.04 (s, 3H), 3.22-3.32 (m, 1H), 3.82-3.92 (m, 2H), 5.70-5.88 (m, 2H), 6.44 (br. s, 1H), 7.05-7.11 (m, 1H), 7.28-7.37 (m, 2H), 7.43-7.48 (m, 1H).

**MS (ESI):** [M + H]⁺= 469.1.

To a solution of compound **9** (25 mg, 0.05 mmol) in DCM (1.9 mL) was added TFA (0.07 mL, 0.96 mmol). The reaction was stirred at 25 °C for 16 h. The reaction was concentrated to afford 25 mg of the titled compound (**A-36**) in TFA salt form as a colorless gum.

**¹HNMR** (500 MHz, DMSO-*d₆*) δ = 1.60-1.80 (m, 3H), 1.92-2.01 (m, 1H), 2.33-2.43 (m, 2H), 2.54-2.65 (m, 1H), 2.98 (s, 3H), 3.08-3.17 (m, 1H), 3.92 (br. s, 2H), 5.72-5.92 (m, 2H), 6.96-7.02 (m, 1H), 7.30-7.38 (m, 2H), 7.45-7.51 (m, 1H), 8.31 (br. s, 3H).

**MS (ESI):** [M + H]⁺= 368.9.

### Example 37: ((((S)-1-(2-chlorophenyl)-2-oxocyclohexyl)(methyl)carbamoyl)oxy)methyl dimethyl-L-valinate (A-37)

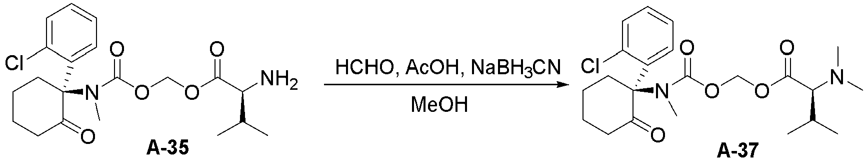

Compound (**A-35**) TFA salt (52 mg, 0.1 mmol) was dissolved in MeOH (5.8 mL) and cooled to 0 °C in an ice bath. Acetic acid (0.02 mL, 0.4 mmol) and NaBH₃CN (13 mg, 0.2 mmol) was added to the above solution and stirred at 0 °C for 5 min. Formaldehyde (37% in H₂O, 0.02 mmol) was added at 0 °C and the reaction mixture was stirred at 25 °C for 2.5 h. The reaction was quenched with aqueous saturated NaHCO₃ solution (5 mL) and diluted with water (5 mL). The aqueous layer was extracted with DCM (5 mL) and the organic layer was washed with brine (5 mL). The organic layer was dried over MgSO₄, filtered and concentrated to get a solid. The solid was washed with hexane and then recrystallized from DCM and hexane at 4 °C. After 16 h, the mixture was filtered and collected the filtrate, concentrated to afford 20 mg (46% yield) of the titled compound (**A-37**) as a white solid.

**¹HNMR** (600 MHz, acetone-*d₆*) δ = 0.89 (d, *J =* 6.5 Hz, 3H), 0.97 (d, *J =* 6.6 Hz, 3H), 1.72-1.87 (m, 3H), 1.96-2.03 (m, 2H), 2.30 (s, 6H), 2.40-2.46 (m, 1H), 2.46-2.53 (m, 1H), 2.66-2.73 (m, 1H), 2.74-2.78 (m, 1H), 3.04 (s, 3H), 3.22-3.29 (m, 1H), 5.80-5.90 (m, 2H), 7.05-7.09 (m, 1H), 7.27-7.34 (m, 2H), 7.44-7.48 (m, 1H).

**MS (ESI):** [M + H]⁺= 439.5.

### Example 38: (2-(N-methylacetamido)acetoyloxy)methyl (S)-1-(2-chlorophenyl)-2-oxocyclohexylmethylcarbamate (A-38)

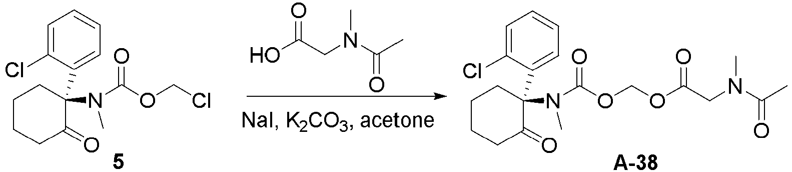

To a solution of compound **5** (50 mg, 0.15 mmol), NaI (45 mg, 0.30 mmol) and 2-(N-methylacetamido)acetic acid (99 mg, 0.76 mmol) in acetone (2 mL) was added K₂CO₃ (105 mg, 0.76 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/4) to afford 25 mg (39% yield) of the titled compound (**A-38**) as a white foam.

**¹HNMR** (600 MHz, DMSO-*d₆*) δ = 1.61-1.76 (m, 3H) 1.89-2.01 (m, 2H), 2.01-2.05 (m, 2H), 2.30-2.40 (m, 2H), 2.54-2.73 (m, 2H), 2.80 (s, 1H), 2.96 (s, 3H), 3.03 (s, 2H), 3.08-3.18 (m, 1H), 4.06-4.37 (m, 2H), 5.61-5.86 (m, 2H), 6.93-7.00 (m, 1H), 7.29-7.38 (m, 2H), 7.43-7.50 (m, 1H).

**MS (ESI):** [M + H]⁺= 425.3.

### Example 39: 1-(2-(N-methylacetamido)acetoyloxy)ethyl (S)-1-(2-chlorophenyl)-2-oxocyclohexylmethylcarbamate (A-39)

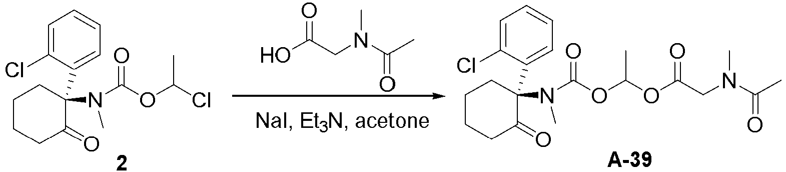

To a solution of compound **2** (50 mg, 0.15 mmol), NaI (43 mg, 0.29 mmol) and 2-(N-methylacetamido) acetic acid (95 mg, 0.73 mmol) in acetone (2 mL) was added Et₃N (0.10 mL, 0.73 mmol). The reaction was heated to 70 °C for 16 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/4) to afford 36 mg (57% yield) of the titled compound (**A-39**) as a white foam.

**¹HNMR** (600 MHz, DMSO-*d₆*) δ = 1.29-1.59 (m, 3H), 1.59-1.78 (m, 3H), 1.81-1.90 (m, 1H), 1.94-2.07 (m, 3H), 2.25-2.42 (m, 2H), 2.53-2.68 (m, 1H), 2.78 (s, 1H), 2.92-3.03 (m, 5H), 3.06-3.19 (m, 1H), 3.99-4.29 (m, 2H), 6.61-6.72 (m, 1H), 6.91-7.02 (m, 1H), 7.28-7.37 (m, 2H), 7.43-7.50 (m, 1H).

**MS (ESI):** [M + H]⁺= 439.3.

### Example 40: 1-(2-(propionamido)acetoyloxy)ethyl (S)-1-(2-chlorophenyl)-2-oxocyclohexylmethylcarbamate (A-40)

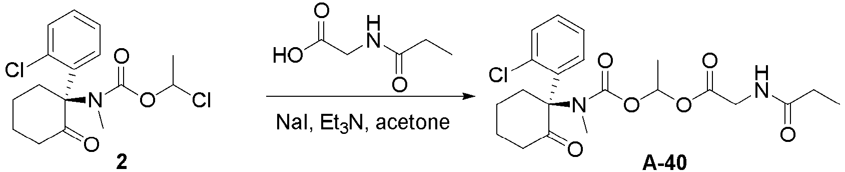

To a solution of compound **2** (50 mg, 0.145 mmol), NaI (23 mg, 0.15 mmol) and 2-(propionamido)acetic acid (57 mg, 0.435 mmol) in acetone (1.8 mL) was added Et₃N (0.1 mL, 0.725 mmol). The reaction was heated to 70 °C for 16 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/1) to afford 41 mg (65% yield) of the titled compound (**A-40**) as a white foam.

**¹HNMR** (600 MHz, DMSO-*d₆*) δ = 1.00 (t, *J =* 7.6 Hz, 3H), 1.34-1.59 (m, 3H), 1.60-1.77 (m, 3H), 1.94-2.03 (m, 1H), 2.10-2.18 (m, 2H), 2.27-2.41 (m, 2H), 2.53-2.67 (m, 1H), 2.94-2.97 (m, 3H), 3.06-3.19 (m, 1H), 3.71-3.82 (m, 1H), 3.82-3.96 (m, 1H), 6.61-6.68 (m, 1H), 6.92-7.00 (m, 1H), 7.29-7.37 (m, 2H), 7.43-7.49 (m, 1H), 8.22-8.32 (m, 1H).

**MS (ESI):** [M + H]⁺= 439.2.

### Example 41: (2-(propionamido)acetoyloxy)methyl (S)-1-(2-chlorophenyl)-2-oxocyclohexylmethylcarbamate (A-41)

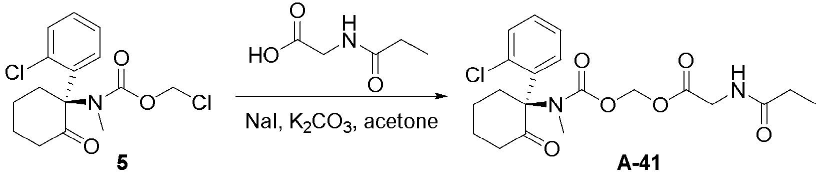

To a solution of compound **5** (50 mg, 0.15 mmol), NaI (23 mg, 0.3 mmol) and 2-(propionamido)acetic acid (60 mg, 0.46 mmol) in acetone (1.8 mL) was added K₂CO₃ (105 mg, 0.76 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 2/3) to afford 45 mg (69% yield) of the titled compound (**A-41)** as a white foam.

**¹HNMR** (600 MHz, DMSO-*d₆*) δ = 1.00 (t, *J =* 7.6 Hz, 3H), 1.62-1.78 (m, 3H), 1.97-2.04 (m, 1H), 2.15 (q, *J =* 7.6 Hz, 2H), 2.30-2.39 (m, 2H), 2.55-2.63 (m, 1H), 2.96 (s, 3H), 3.08-3.16 (m, 1H), 3.80-3.92 (m, 2H), 5.60-5.80 (m, 2H), 6.93-6.99 (m, 1H), 7.30-7.38 (m, 2H), 7.44-7.49 (m, 1H), 8.30 (t, *J =* 5.5 Hz, 1H).

**MS (ESI):** [M + H]⁺ = 425.4.

### Example 42: ((((S)-1-(2-chlorophenyl)-2-oxocyclohexyl)(methyl)carbamoyl)oxy)methyl L-alaninate (A-42)

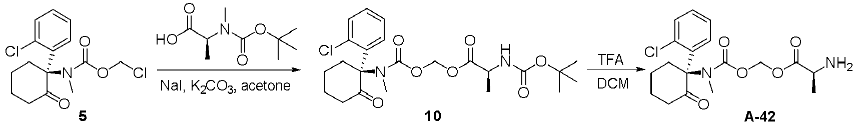

To a solution of compound **5** (150 mg, 0.45 mmol), NaI (136 mg, 0.91 mmol) and *N-*(*tert-*butoxycarbonyl)-*N*-methyl-*L*-alanine (258 mg, 1.36 mmol) in acetone (5 mL) was added K₂CO₃ (314 mg, 2.27 mmol). The reaction was heated to 70 °C for 1 h. The reaction was concentrated and re-dissolved in DCM (10 mL), washed with aqueous saturated NaHCO₃ solution (10 mL) and brine (10 mL). The organic layer was dried over MgSO₄, filtered and concentrated to get an oil, which was purified on silica gel column eluting with hexane/EA (100% hexane to 3/2) to afford 200 mg (91% yield) of compound **10** as a white foam.

**¹HNMR** (500 MHz, DMSO-*d₆*) δ = 1.19-1.28 (m, 3H) 1.28-1.46 (m, 9H), 1.57-1.79 (m, 3H), 1.93-2.02 (m, 1H), 2.28-2.42 (m, 2H), 2.54-2.62 (m, 1H), 2.96 (s, 3H), 3.07-3.19 (m, 1H), 3.97-4.08 (m, 1H), 5.60-5.84 (m, 2H), 6.92-7.05 (m, 1H), 7.29-7.37 (m, 2H), 7.37-7.44 (m, 1H), 7.44-7.51 (m, 1H).

**MS (ESI):** [M + H]⁺= 483.3.

To a solution of compound **10** (200 mg, 0.41 mmol) in DCM (15 mL) was added TFA (0.57 mL, 7.5 mmol). The reaction was stirred at 25 °C for 16 h. The reaction was concentrated to afford 250 mg of the titled compound (**A-42**) as a colorless gum.

**¹HNMR** (600 MHz, DMSO-*d₆*) δ = 1.29-1.43 (m, 3H) 1.58-1.79 (m, 3H), 1.90-2.02 (m, 1H), 2.31-2.43 (m, 2H), 2.54-2.62 (m, 1H), 2.98 (s, 3H), 3.07-3.17 (m, 1H), 4.12-4.26 (m, 1H), 5.65-5.98 (m, 2H), 6.93-7.02 (m, 1H), 7.28-7.38 (m, 2H), 7.43-7.51 (m, 1H), 8.26-8.48 (m, 3H).

**MS (ESI):** [M + H]⁺= 383.6.

### Example 43: 1-(2-(propionamido)acetoyloxy)ethyl (S)-1-(2-chlorophenyl)-2-oxocyclohexylmethylcarbamate (A-43)

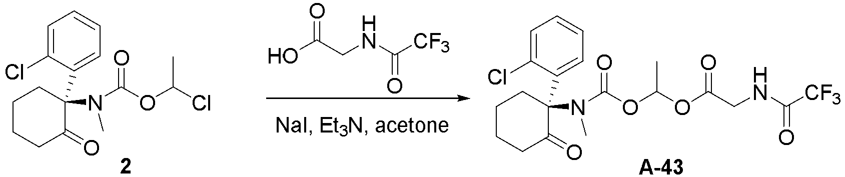

To a solution of compound 2 (103 mg, 0.3 mmol), NaI (47 mg, 0.315 mmol) and 2-(2,2,2-trifluoroacetamido)-acetic acid (154 mg, 0.9 mmol) in acetone (4 mL) was added Et₃N (0.21 mL, 1.5 mmol). The reaction was heated to 70 °C for 16 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 3/1) to afford 113 mg (79% yield) of the titled compound (**A-43**) as a white solid.

**¹HNMR** (500 MHz, DMSO-*d₆*) δ = 1.37-1.58 (m, 3H), 1.60-1.78 (m, 3H), 1.94-2.03 (m, 1H), 2.28-2.40 (m, 2H), 2.53-2.68 (m, 1H), 2.94-2.97 (m, 3H), 3.06-3.20 (m, 1H), 3.90-3.99 (m, 1H), 4.00-4.16 (m, 1H), 6.65-6.72 (m, 1H), 6.93-7.01 (m, 1H), 7.27-7.36 (m, 2H), 7.44-7.49 (m, 1H), 9.99 (t, *J =* 5.8 Hz, 1H).

**MS (ESI):** [M + H]⁺= 479.1.

### Example 44: (2-(2,2,2-trifluoroacetamido)acetoyloxy)methyl (S)-1-(2-chlorophenyl)-2-oxocyclohexylmethylcarbamate (A-44)

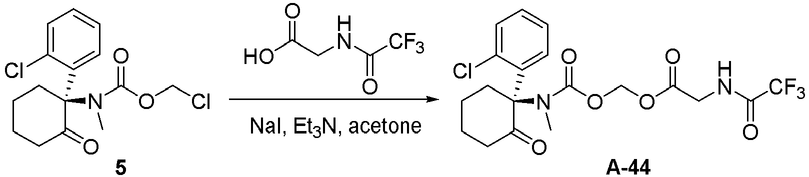

To a solution of compound **5** (50 mg, 0.15 mmol), NaI (46 mg, 0.3 mmol) and 2-(2,2,2-trifluoroacetamido)-acetic acid (78 mg, 0.46 mmol) in acetone (4 mL) was added Et₃N (0.1 mL, 0.76 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 7/3) to afford 14 mg (20% yield) of the titled compound (**A-44**) as a white solid.

**¹HNMR** (600 MHz, DMSO-*d₆*) δ = 1.60-1.78 (m, 3H), 1.92-2.01 (m, 1H), 2.30-2.39 (m, 2H), 2.55-2.63 (m, 1H), 2.97 (s, 3H), 3.08-3.17 (m, 1H), 4.07 (t, *J =* 4.8 Hz, 2H), 5.64-5.86 (m, 2H), 6.93-6.99 (m, 1H), 7.30-7.36 (m, 2H), 7.44-7.49 (m, 1H), 10.04 (t, *J =* 5.4 Hz, 1H).

**MS (ESI):** [M + H]⁺= 465.6.

### Example 45: 1-((((S)-1-(2-chlorophenyl)-2-oxocyclohexyl)(methyl)carbamoyl)oxy)ethyl dimethyl-L-alaninate (A-45)

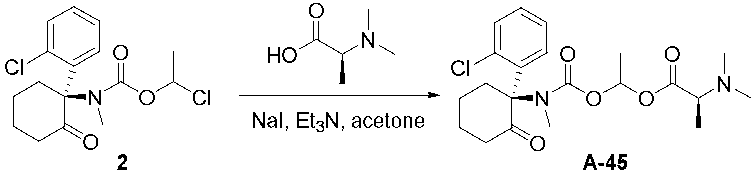

To a solution of compound **2** (31 mg, 0.09 mmol), NaI (27 mg, 0.18 mmol) and (S)-2-(dimethylamino)-propanoic acid (32 mg, 0.27 mmol) in acetone (1 mL) was added Et₃N (0.06 mL, 0.45 mmol). The reaction was heated to 70 °C for 20 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/3) to afford 14 mg (37% yield) of the titled compound (**A-45**) as a yellow gum.

**¹HNMR** (600 MHz, DMSO-*d₆*) δ = 1.14 (d, *J =* 7.0 Hz, 3H), 1.37-1.58 (m, 3H), 1.60-1.77 (m, 3H), 1.95-2.03 (m, 1H), 2.14-2.26 (m, 6H), 2.28-2.36 (m, 2H), 2.56-2.70 (m, 1H), 2.94-2.97 (m, 3H), 3.05-3.19 (m, 1H), 3.20-3.28 (m, 1H), 6.62-6.70 (m, 1H), 6.93-7.02 (m, 1H), 7.26-7.36 (m, 2H), 7.44-7.49 (m, 1H).

**MS (ESI):** [M + H]⁺= 425.5.

### Example 46: ((S)-2-(2,2,2-trifluoroacetamido)-3-methylbutanoyloxy)methyl (S)-1-(2-chlorophenyl)-2-oxocyclohexylmethylcarbamate (A-46)

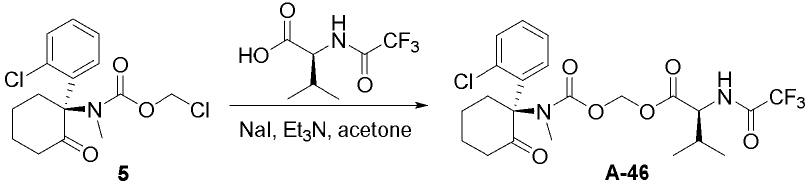

To a solution of compound **5** (50 mg, 0.15 mmol), NaI (45 mg, 0.30 mmol) and (*S*)-2-(2,2,2-trifluoroacetamido)-3-methylbutanoic acid (97 mg, 0.45 mmol) in acetone (2 mL) was added Et₃N (0.11 mL, 0.76 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 4/1) to afford 34 mg (44% yield) of the titled compound (**A-46**) as a white gum.

**¹HNMR** (500 MHz, DMSO-*d₆*) δ = 0.88-0.99 (m, 6H) 1.56-1.78 (m, 3H), 1.89-2.00 (m, 1H), 2.13-2.26 (m, 1H), 2.30-2.42 (m, 2H), 2.52-2.62 (m, 1H), 2.95 (s, 3H), 3.07-3.18 (m, 1H), 4.17-4.29 (m, 1H), 5.70-5.88 (m, 2H), 6.92-7.01 (m, 1H), 7.28-7.38 (m, 2H), 7.43-7.50 (m, 1H), 9.89 (d, *J =* 7.5 Hz, 1H).

**MS (ESI):** [M + H]⁺= 507.5.

### Example 47: 1-(2-(2,2,2-trifluoroacetamido)acetoyloxy)propyl (S)-1-(2-chlorophenyl)-2-oxocyclohexylmethylcarbamate (A-47)

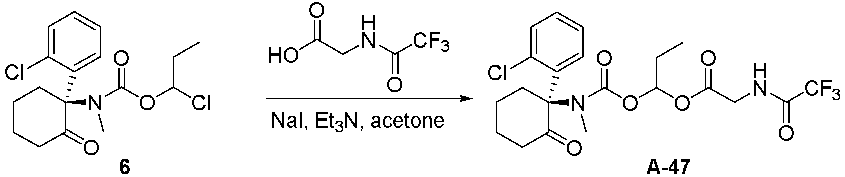

To a solution of compound **6** (50 mg, 0.14 mmol), NaI (22 mg, 0.15 mmol) and 2-(2,2,2-trifluoroacetamido)-acetic acid (72 mg, 0.42 mmol) in acetone (1.8 mL) was added Et₃N (0.1 mL, 0.7 mmol). The reaction was heated to 70 °C for 16 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 3/1) to afford 28 mg (41% yield) of the titled compound (**A-47**) as a white foam.

**¹HNMR** (500 MHz, DMSO-*d₆*) δ = 0.80-1.02 (m, 3H), 1.58-1.92 (m, 5H), 1.93-2.04 (m, 1H), 2.27-2.41 (m, 2H), 2.53-2.70 (m, 1H), 2.95-2.98 (m, 3H), 3.04-3.20 (m, 1H), 3.92-4.02 (m, 1H), 4.03-4.16 (m, 1H), 6.57 (q, *J =* 5.6 Hz, 1H), 6.93-7.00 (m, 1H), 7.26-7.36 (m, 2H), 7.43-7.50 (m, 1H), 9.93-10.06 (m, 1H).

**MS (ESI):** [M + H]⁺= 493.4.

### Example 48: ((S)-2-(2,2,2-trifluoroacetamido)propanoyloxy)methyl (S)-1-(2-chlorophenyl)-2-oxocyclohexylmethylcarbamate (A-48)

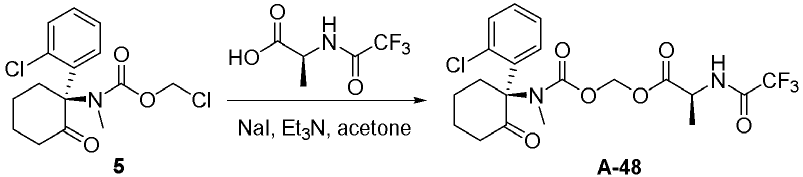

To a solution of compound **5** (50 mg, 0.15 mmol), NaI (45 mg, 0.30 mmol) and (*S*)-2-(2,2,2-trifluoro-acetamido)propanoic acid (84 mg, 0.45 mmol) in acetone (2 mL) was added Et₃N (0.11 mL, 0.76 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 4/1) to afford 13 mg (18% yield) of the titled compound (**A-48**) as a white solid.

**¹HNMR** (500 MHz, DMSO-*d₆*) δ = 1.34-1.45 (m, 3H), 1.59-1.78 (m, 3H), 1.92-2.01 (m, 1H), 2.30-2.41 (m, 2H), 2.52-2.62 (m, 1H), 2.96 (s, 3H), 3.07-3.18 (m, 1H), 4.40-4.49 (m, 1H), 5.66-5.86 (m, 2H), 6.92-7.01 (m, 1H), 7.28-7.38 (m, 2H), 7.43-7.50 (m, 1H), 9.91-10.02 (m, 1H).

**MS (ESI):** [M + H]⁺= 479.2.

### Example 49: 1-(2-(2,2,2-trifluoroacetamido)acetoyloxy)-2-methylpropyl (S)-1-(2-chlorophenyl)-2-oxocyclohexylmethylcarbamate (A-49)

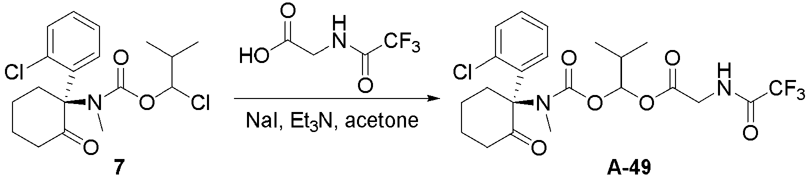

To a solution of compound **7** (93 mg, 0.25 mmol), NaI (39 mg, 0.26 mmol) and 2-(2,2,2-trifluoro-acetamido)acetic acid (128 mg, 0.75 mmol) in acetone (3 mL) was added Et₃N (0.17 mL, 1.25 mmol). The reaction was heated to 70 °C for 16 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 3/1) to afford 51 mg (40% yield) of the titled compound (**A-49**) as a white foam.

**¹HNMR** (500 MHz, DMSO-*d₆*) δ = 0.80-1.08 (m, 6H), 1.60-1.78 (m, 3H), 1.93-2.15 (m, 2H), 2.28-2.41 (m, 2H), 2.64-2.72 (m, 1H), 2.96-2.99 (m, 3H), 3.03-3.21 (m, 1H), 3.92-4.03 (m, 1H), 4.03-4.18 (m, 1H), 6.45 (d, *J =* 4.9 Hz, 1H), 6.92-6.99 (m, 1H), 7.27-7.36 (m, 2H), 7.44-7.49 (m, 1H), 10.01 (br. s, 1H).

**MS (ESI):** [M + H]⁺= 507.4.

### Example 50: 1-((S)-2-(2,2,2-trifluoroacetamido)-3-methylbutanoyloxy)ethyl (S)-1-(2-chlorophenyl)-2-oxocyclohexylmethylcarbamate (A-50)

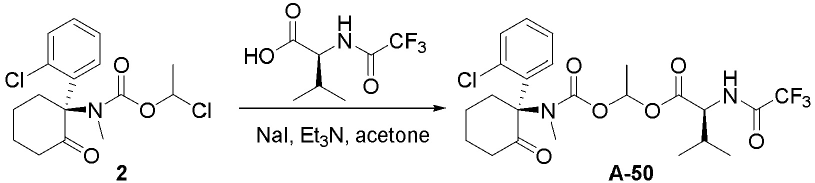

To a solution of compound **2** (103 mg, 0.3 mmol), NaI (47 mg, 0.315 mmol) and (*S*)-2-(2,2,2-trifluoro-acetamido)-3-methylbutanoic acid (192 mg, 0.9 mmol) in acetone (4 mL) was added Et₃N (0.21 mL, 1.5 mmol). The reaction was heated to 70 °C for 16 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 17/3) to afford 128 mg (82% yield) of the titled compound (**A-50**) as a white foam.

**¹HNMR** (500 MHz, DMSO-*d₆*) δ = 0.82-0.99 (m, 6H), 1.38-1.55 (m, 3H), 1.56-1.78 (m, 3H), 1.91-2.00 (m, 1H), 2.10-2.20 (m, 1H), 2.26-2.39 (m, 2H), 2.58-2.69 (m, 1H), 2.94-2.97 (m, 3H), 3.05-3.16 (m, 1H), 4.12 (t, *J =* 7.6 Hz, 1H), 6.72 (q, *J =* 5.4 Hz, 1H), 6.91-7.01 (m, 1H), 7.27-7.36 (m, 2H), 7.43-7.48 (m, 1H), 9.76-9.88 (m, 1H).

**MS (ESI):** [M + H]⁺= 521.5.

### Example 51: 1-((S)-2-(2,2,2-trifluoroacetamido)propanoyloxy)ethyl (S)-1-(2-chlorophenyl)-2-oxocyclohexylmethylcarbamate (A-51)

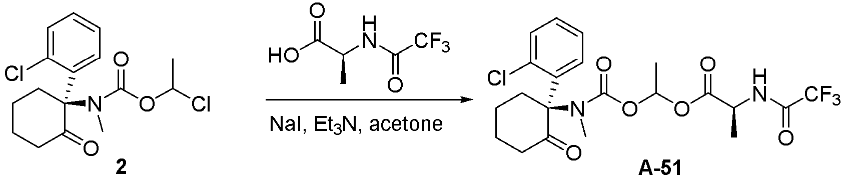

To a solution of compound **2** (103 mg, 0.3 mmol), NaI (47 mg, 0.315 mmol) and (*S*)-2-(2,2,2-trifluoro-acetamido)propanoic acid (167 mg, 0.9 mmol) in acetone (4 mL) was added Et₃N (0.21 mL, 1.5 mmol). The reaction was heated to 70 °C for 16 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 4/1) to afford 81 mg (55% yield) of the titled compound (**A-51**) as a white foam.

**¹HNMR** (500 MHz, DMSO-*d₆*) δ = 1.32 (d, *J =* 7.3 Hz, 3H), 1.40-1.58 (m, 3H), 1.62-1.78 (m, 3H), 1.95-2.03 (m, 1H), 2.28-2.39 (m, 2H), 2.55-2.65 (m, 1H), 2.95-2.98 (m, 3H), 3.10-3.19 (m, 1H), 4.38-4.47 (m, 1H), 6.66 (q, *J =* 5.4 Hz, 1H), 6.91-7.00 (m, 1H), 7.28-7.36 (m, 2H), 7.44-7.50 (m, 1H), 9.90 (d, *J =* 6.9 Hz, 1H).

**MS (ESI):** [M + H]⁺= 493.4.

### Example 52: (4-methylpyridine-3-carboxyloyloxy)methyl (S)-1-(2-chlorophenyl)-2-oxocyclohexylmethylcarbamate (A-52)

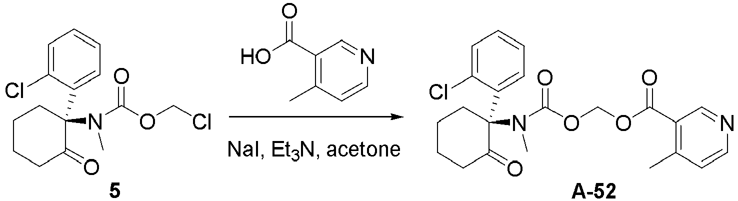

To a solution of compound **5** (100 mg, 0.3 mmol), NaI (90 mg, 0.6 mmol) and 4-methylpyridine-3-carboxylic acid (123 mg, 0.9 mmol) in acetone (4 mL) was added Et₃N (0.21 mL, 1.5 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 3/2) to afford 45 mg (35% yield) of the titled compound (**A-52**) as a white solid.

**¹HNMR** (600 MHz, DMSO-*d₆*) δ = 1.62-1.77 (m, 3H), 1.92-2.00 (m, 1H), 2.31-2.40 (m, 2H), 2.55 (s, 3H), 2.58-2.66 (m, 1H), 3.00 (s, 3H), 3.08-3.16 (m, 1H), 5.80-6.12 (m, 2H), 6.98-7.02 (m, 1H), 7.22-7.27 (m, 1H), 7.28-7.34 (m, 1H), 7.41-7.44 (m, 1H), 7.45-7.48 (m, 1H), 8.64 (d, *J* = 5.0 Hz, 1H), 8.92 (s, 1H).

**MS (ESI):** [M + H]⁺= 431.1.

### Example 53: (2-methylpyridine-3-carboxyloyloxy)methyl (S)-1-(2-chlorophenyl)-2-oxocyclohexylmethylcarbamate (A-53)

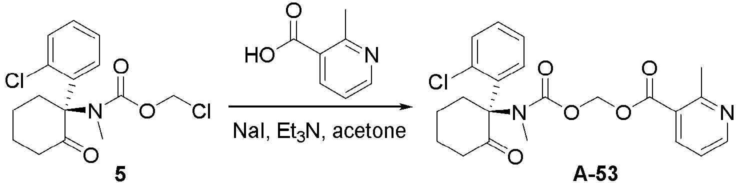

To a solution of compound **5** (100 mg, 0.3 mmol), NaI (90 mg, 0.6 mmol) and 2-methylpyridine-3-carboxylic acid (123 mg, 0.9 mmol) in acetone (4 mL) was added Et₃N (0.21 mL, 1.5 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 3/2) to afford 56 mg (43% yield) of the titled compound (**A-53**) as a white foam.

**¹HNMR** (600 MHz, DMSO-*d₆*) δ = 1.62-1.77 (m, 3H), 1.92-1.99 (m, 1H), 2.31-2.40 (m, 2H), 2.57-2.65 (m, 1H), 2.72 (s, 3H), 3.00 (s, 3H), 3.07-3.16 (m, 1H), 5.80-6.08 (m, 2H), 6.97-7.02 (m, 1H), 7.22-7.27 (m, 1H), 7.28-7.34 (m, 1H), 7.40-7.48 (m, 2H), 8.12-8.22 (m, 1H), 8.65-8.70 (m, 1H).

**MS (ESI):** [M + H]⁺= 431.1.

### Example 54: (6-methylpyridine-3-carboxyloyloxy)methyl (S)-1-(2-chlorophenyl)-2-oxocyclohexylmethylcarbamate (A-54)

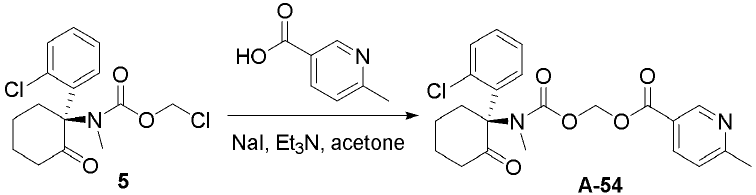

To a solution of compound **5** (100 mg, 0.3 mmol), NaI (90 mg, 0.6 mmol) and 6-methylpyridine-3-carboxylic acid (123 mg, 0.9 mmol) in acetone (4 mL) was added Et₃N (0.21 mL, 1.5 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (1/0 to 3/2) to afford 44 mg (34% yield) of the titled compound (**A-54**) as a white foam.

**¹HNMR** (600 MHz, DMSO-*d₆*) δ = 1.61-1.76 (m, 3H), 1.92-1.99 (m, 1H), 2.30-2.40 (m, 2H), 2.55-2.63 (m, 1H), 2.58 (s, 3H), 2.99 (s, 3H), 3.05-3.15 (m, 1H), 5.82-6.08 (m, 2H), 6.96-7.01 (m, 1H), 7.21-7.27 (m, 1H), 7.28-7.34 (m, 1H), 7.42-7.49 (m, 2H), 8.12-8.24 (m, 1H), 8.97 (s, 1H).

**MS (ESI):** [M + H]⁺= 431.1.

### Example 55: 1-((S)-2-acetamido-4-methylpentanoyloxy)ethyl (S)-1-(2-chlorophenyl)-2-oxocyclohexylmethylcarbamate (A-55)

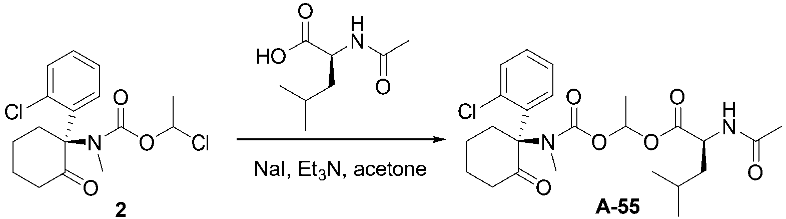

To a solution of compound **2** (103 mg, 0.3 mmol), NaI (47 mg, 0.315 mmol) and (S)-2-acetamido-4-methylpentanoic acid (156 mg, 0.9 mmol) in acetone (4 mL) was added Et₃N (0.21 mL, 1.5 mmol). The reaction was heated to 70 °C for 16 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 11/9) to afford 102 mg (71 % yield) of the titled compound (**A-55**) as a white foam.

**¹HNMR** (600 MHz, DMSO-*d₆*) δ = 0.79-0.91 (m, 6H), 1.36-1.77 (m, 9H), 1.84 (s, 3H), 1.94-2.01 (m, 1H), 2.27-2.38 (m, 2H), 2.56-2.70 (m, 1H), 2.94-2.96 (m, 3H), 3.06-3.16 (m, 1H), 4.14-4.25 (m, 1H), 6.60-6.67 (m, 1H), 6.92-6.98 (m, 1H), 7.29-7.36 (m, 2H), 7.44-7.49 (m, 1H), 8.26 (d, *J =* 7.6 Hz, 1H).

**MS (ESI):** [M + H]⁺= 481.1.

### Example 56: ((S)-2-acetamido-4-methylpentanoyloxy)methyl (S)-1-(2-chlorophenyl)-2-oxocyclohexylmethylcarbamate (A-56)

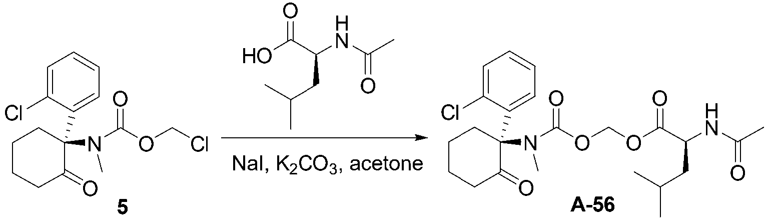

To a solution of compound **5** (100 mg, 0.3 mmol), NaI (90 mg, 0.6 mmol) and (S)-2-acetamido-4-methylpentanoic acid (156 mg, 0.9 mmol) in acetone (4 mL) was added K₂CO₃ (207 mg, 1.5 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 3/2) to afford 120 mg (86% yield) of the titled compound (**A-56**) as a white foam.

**¹HNMR** (500 MHz, DMSO-*d₆*) δ = 0.85 (d, *J =* 6.5 Hz, 3H), 0.89 (d, *J =* 6.6 Hz, 3H), 1.40-1.59 (m, 2H), 1.60-1.79 (m, 4H), 1.86 (s, 3H), 1.93-2.00 (m, 1H), 2.31-2.40 (m, 2H), 2.53-2.62 (m, 1H), 2.95 (s, 3H), 3.07-3.17 (m, 1H), 4.19-4.27 (m, 1H), 5.62-5.80 (m, 2H), 6.95-7.01 (m, 1H), 7.30-7.37 (m, 2H), 7.44-7.49 (m, 1H), 8.31 (d, *J =* 7.0 Hz, 1H).

**MS (ESI):** [M + H]⁺= 467.2.

### Example 57: 1-((((S)-1-(2-chlorophenyl)-2-oxocyclohexyl)(methyl)carbamoyl)oxy)ethyl 2-(3-methyloxetan-3-yl)acetate (A-57)

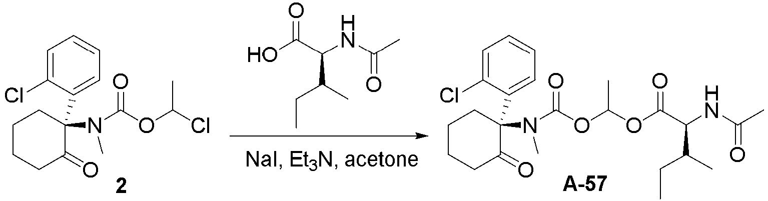

To a solution of compound **2** (100 mg, 0.29 mmol), NaI (87 mg, 0.58 mmol) and (2S,3R)-2-acetamido-3-methylpentanoic acid (151 mg, 0.87 mmol) in acetone (4 mL) was added Et₃N (0.163 mL, 1.17 mmol). The reaction was heated to 70 °C for 1 h. The reaction was concentrated and re-dissolved in DCM (5 mL), washed with H₂O (5 mL) and brine (5 mL). The organic layer was dried over MgSO₄, filtered and concentrated to get an oil, which was purified on silica gel column eluting with hexane/EA (100% hexane to 2/3) to afford 88 mg (63% yield) of the titled compound (**A-57**) as a white solid.

**¹HNMR** (500 MHz, DMSO-*d₆*) δ = 0.65-0.78 (m, 2H), 0.78-0.89 (m, 4H), 1.12-1.21 (m, 1H), 1.28-1.59 (m, 4H), 1.62-1.78 (m, 4H), 1.87 (s, 3H), 1.92-2.03 (m, 1H), 2.28-2.39 (m, 2H), 2.55-2.65 (m, 1H), 2.91-2.99 (m, 3H), 3.04-3.20 (m, 1H), 4.08-4.25 (m, 1H), 6.62-6.74 (m, 1H), 6.90-7.01 (m, 1H), 7.27-7.38 (m, 2H), 7.42-7.51 (m, 1H), 8.09-8.22 (m, 1H).

**MS (ESI):** [M + H]⁺= 481.2.

### Example 58: ((2S,3R)-2-acetamido-3-methylpentanoyloxy)methyl (S)-1-(2-chlorophenyl)-2-oxocyclohexylmethylcarbamate (A-58)

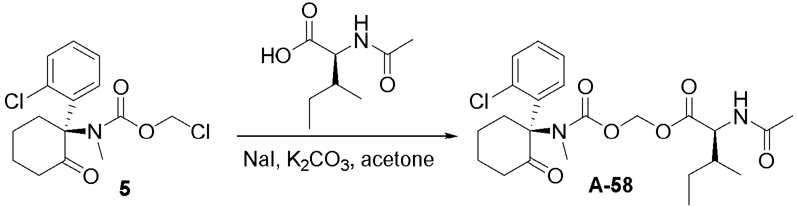

To a solution of compound **5** (100 mg, 0.30 mmol), NaI (91 mg, 0.60 mmol) and (2*S*,3*R*)-2-acetamido-3-methylpentanoic acid (157 mg, 0.91 mmol) in acetone (4 mL) was added K₂CO₃ (209 mg, 1.51 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 3/2) to afford 130 mg (92% yield) of the titled compound (**A-58**) as a white solid.

**¹HNMR** (500 MHz, DMSO-*d₆*) δ = 0.79-0.92 (m, 6H), 1.17-1.28 (m, 1H), 1.37-1.49 (m, 1H), 1.56-1.81 (m, 4H), 1.88 (s, 3H), 1.92-2.02 (m, 1H), 2.30-2.41 (m, 2H), 2.53-2.62 (m, 1H), 2.95 (s, 3H), 3.07-3.19 (m, 1H), 4.14-4.24 (m, 1H), 5.64-5.83 (m, 2H), 6.92-7.00 (m, 1H), 7.28-7.38 (m, 2H), 7.43-7.51 (m, 1H), 8.19-8.29 (m, 1H).

**MS (ESI):** [M + H]⁺= 467.2.

### Example 59: (S)-(((1-(2-chlorophenyl)-2-oxocyclohexyl)(methyl)carbamoyl)oxy)methyl 2-aminonicotinate (A-59)

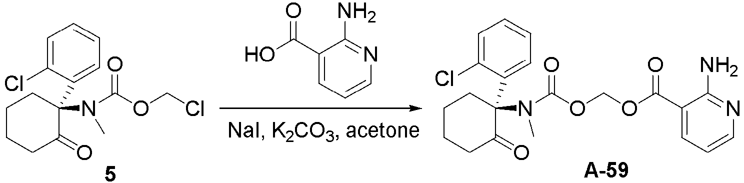

To a solution of compound **5** (100 mg, 0.15 mmol), NaI (45 mg, 0.30 mmol) and 2-aminopyridine-3-carboxylic acid (63 mg, 0.45 mmol) in acetone (2 mL) was added K₂CO₃ (105 mg, 0.76 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 2/3) to afford 40 mg (60% yield) of the titled compound (**A-59**) as a pale-yellow foam.

**¹HNMR** (600 MHz, DMSO-*d₆*) δ = 1.59-1.76 (m, 3H), 1.90-2.00 (m, 1H), 2.29-2.40 (m, 2H), 2.55-2.63 (m, 1H), 2.98 (s, 3H), 3.05-3.16 (m, 1H), 5.80-6.04 (m, 2H), 6.63-6.71 (m, 1H), 6.93-6.99 (m, 1H), 7.18-7.27 (m, 3H), 7.28-7.34 (m, 1H), 7.42-7.48 (m, 1H), 7.99-8.07 (m, 1H), 8.26 (dd, *J =* 1.9, 4.6 Hz, 1H).

**MS (ESI):** [M + H]⁺= 432.0.

### Example 60: 1-(2-acetamidoacetoyloxy)ethyl (R)-1-(2-chlorophenyl)-2-oxocyclohexyl-methylcarbamate (A-60)

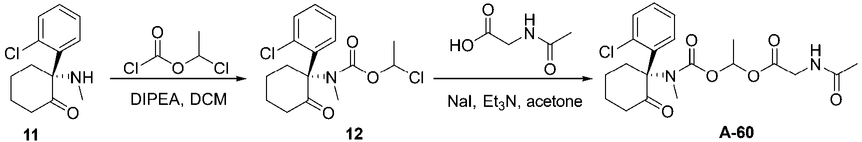

To a solution of *R*-ketamine **11** (1.0 g, 4.2 mmol) and DIPEA (1.36 g, 10.5 mmol) in DCM (42 mL) was added 1-chloroethyl carbonochloridate (1.50 g, 10.5 mmol) slowly at 0 °C. The reaction was stirred at 25 °C for 1.5 h. The reaction was diluted with DCM (10 mL) and washed with water (20 mL) and brine (20 mL). The organic layer was dried over MgSO₄, filtered and concentrated to get an oil. The oil was diluted with ice MeOH and filtered to afford 1.14 g (80% yield) of compound **12** as a white solid.

**¹HNMR** (600 MHz, CDCl₃) δ = 1.60-1.96 (m, 6H), 2.00-2.09 (m, 1H), 2.30-2.56 (m, 1H), 2.57-2.63 (m, 1H), 2.67-2.86 (m, 1H), 3.02-3.07 (m, 3H), 3.24-3.39 (m, 1H), 6.48-6.60 (m, 1H), 6.90-7.03 (m, 1H), 7.22-7.28 (m, 2H), 7.42-7.48 (m, 1H).

To a solution of compound **12** (52 mg, 0.15 mmol), NaI (24 mg, 0.16 mmol) and acetylglycine (53 mg, 0.45 mmol) in acetone (1 mL) was added Et₃N (0.1 mL, 0.75 mmol). The reaction was heated to 70 °C for 16 h. The reaction was concentrated and re-dissolved in DCM (10 mL), washed with aqueous saturated NaHCO₃ solution (10 mL) and brine (10 mL). The organic layer was dried over MgSO₄, filtered and concentrated to get an oil, which was purified on silica gel column eluting with hexane/EA (1/0 to 1/2) to afford 39 mg (61% yield) of the titled compound (**A-60**) as a white foam.

**¹HNMR** (500 MHz, DMSO-*d₆*) δ = 1.36-1.56 (m, 3H), 1.60-1.78 (m, 3H), 1.86 (d, *J =* 3.0 Hz, 3H), 1.95-2.03 (m, 1H), 2.28-2.36 (m, 2H), 2.55-2.62 (m, 1H), 2.94-2.97 (m, 3H), 3.06-3.20 (m, 1H), 3.70-3.79 (m, 1H), 3.81-3.94 (m, 1H), 6.61-6.69 (m, 1H), 6.92-7.00 (m, 1H), 7.29-7.37 (m, 2H), 7.43-7.49 (m, 1H), 8.28-8.37 (m, 1H).

**MS (ESI):** [M + H]⁺= 425.2.

### Example 61: 1-(2-(3-methyloxetan-3-yl)acetoyloxy)ethyl (R)-1-(2-chlorophenyl)-2-oxocyclohexylmethylcarbamate (A-61)

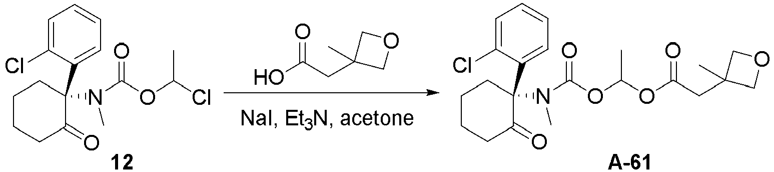

To a solution of compound **12** (121 mg, 0.35 mmol), NaI (105 mg, 0.7 mmol) and 2-(3-methyloxetan-3-yl)acetic acid (137 mg, 1.05 mmol) in acetone (5 mL) was added K₂CO₃ (242 mg, 1.75 mmol). The reaction was heated to 70 °C for 4 h. The reaction was concentrated and re-dissolved in DCM (5 mL), washed with H₂O (5 mL) and brine (5 mL). The organic layer was dried over MgSO₄, filtered and concentrated to get an oil, which was purified on silica gel column eluting with hexane/EA (100% hexane to 3/1) to afford a yellow oil. Ether (3 mL) was added, filtered and the solid was washed with cold ether to afford 15 mg (10% yield) of the titled compound (**A-61**) as a white solid.

**¹HNMR** (600 MHz, CD₃OD) δ = 1.38 (s, 3H), 1.41-1.64 (m, 3H), 1.72-1.88 (m, 3H), 2.04-2.10 (m, 1H), 2.32-2.39 (m, 1H), 2.43-2.51 (m, 1H), 2.66-2.72 (m, 1H), 2.73 (s, 2H), 3.05 (s, 3H), 3.32-3.34 (m, 1H), 4.34-4.39 (m, 2H), 4.57-4.64 (m, 2H), 6.68-6.75 (m, 1H), 7.01-7.15 (m, 1H), 7.27-7.34 (m, 2H), 7.44-7.48 (m, 1H).

**MS (ESI):** [M + H]⁺= 438.2.

### Example 62: 1-((S)-2-acetamidopropanoyloxy)ethyl (S)-1-(2-chlorophenyl)-2-oxocyclohexylmethylcarbamate (A-62)

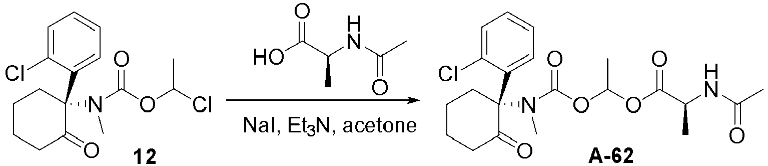

To a solution of compound **12** (52 mg, 0.15 mmol), NaI (24 mg, 0.16 mmol) and (S)-2-acetamidopropanoic acid (59 mg, 0.45 mmol) in acetone (1 mL) was added Et₃N (0.1 mL, 0.75 mmol). The reaction was heated to 70 °C for 16 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/1) to afford 47 mg (72% yield) of the titled compound (**A-62**) as a white foam.

**¹HNMR** (600 MHz, DMSO-*d₆*) δ = 1.15-1.30 (m, 3H), 1.34-1.58 (m, 3H), 1.60-1.77 (m, 3H), 1.84 (d, *J =* 14.2 Hz, 3H), 1.96-2.03 (m, 1H), 2.26-2.38 (m, 2H), 2.51-2.64 (m, 1H), 2.94-2.97 (m, 3H), 3.06-3.20 (m, 1H), 4.10-4.26 (m, 1H), 6.60-6.65 (m, 1H), 6.92-7.02 (m, 1H), 7.25-7.36 (m, 2H), 7.44-7.49 (m, 1H), 8.25-8.37 (m, 1H).

**MS (ESI):** [M + H]⁺ = 439.3.

### Example 63: 1-((S)-2-acetamido-3-methylbutanoyloxy)ethyl (R)-1-(2-chlorophenyl)-2-oxocyclohexylmethylcarbamate (A-63)

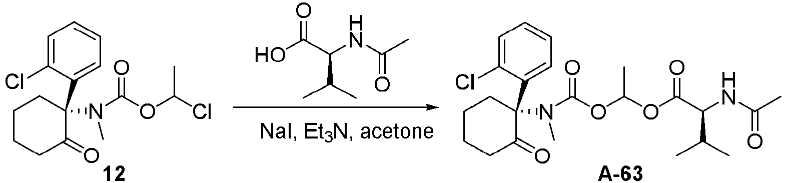

To a solution of compound **12** (52 mg, 0.15 mmol), NaI (24 mg, 0.16 mmol) and (S)-2-acetamido-3-methylbutanoic acid (72 mg, 0.45 mmol) in acetone (1 mL) was added Et₃N (0.1 mL, 0.75 mmol). The reaction was heated to 70 °C for 16 h. The reaction was concentrated and re-dissolved in DCM (5 mL), washed with aqueous saturated NaHCO₃ solution (5 mL) and brine (5 mL). The organic layer was dried over MgSO₄, filtered concentrated to get an oil, which was purified on silica gel column eluting with hexane/EA (100% hexane to 1/1) to afford 49 mg (70% yield) of the titled compound (**A-63**) as a white foam.

**¹HNMR** (500 MHz, DMSO-*d₆*) δ = 0.82-0.93 (m, 6H), 1.36-1.56 (m, 3H), 1.60-1.77 (m, 3H), 1.88 (d, *J =* 21.0 Hz, 3H), 1.94-2.06 (m, 1H), 2.27-2.35 (m, 2H), 2.54-2.62 (m, 1H), 2.94-2.97 (m, 3H), 3.06-3.20 (m, 2H), 4.09-4.20 (m, 1H), 6.55-6.70 (m, 1H), 6.92-7.01 (m, 1H), 7.25-7.36 (m, 2H), 7.44-7.49 (m, 1H), 8.10-8.20 (m, 1H).

**MS (ESI):** [M + H]⁺= 467.2.

### Example 64: (2-(3-methyloxetan-3-yl)acetoyloxy)methyl (R)-1-(2-chlorophenyl)-2-oxocyclohexylmethylcarbamate (A-64)

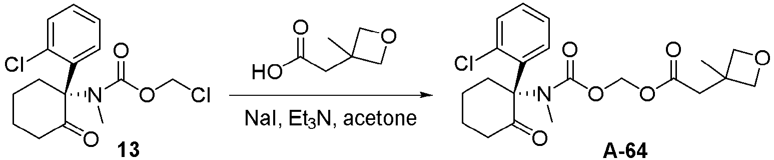

To a solution of compound **13** (152 mg, 0.46 mmol), NaI (138 mg, 0.92 mmol) and 2-(3-methyloxetan-3-yl)acetic acid (120 mg, 0.92 mmol) in acetone (3 mL) was added K₂CO₃ (254 mg, 1.84 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/2) to afford 74 mg (38% yield) of the titled compound (**A-64**) as a colorless oil.

**¹HNMR** (500 MHz, DMSO-*d₆*) δ = 1.32 (s, 3H), 1.60-1.79 (m, 3H), 1.92-2.02 (m, 1H), 2.30-2.41 (m, 2H), 2.53-2.62 (m, 1H), 2.76 (s, 2H), 2.95 (s, 3H), 3.07-3.15 (m, 1H), 4.23 (d, *J* = 5.8 Hz, 2H), 4.45 (d, *J* = 5.7 Hz, 2H), 5.62-5.74 (m, 2H), 6.94-6.99 (m, 1H), 7.29-7.36 (m, 2H), 7.45-7.50 (m, 1H).

**MS (ESI):** [M + H]⁺= 424.2.

### Example 65: (nicotinoyloxy)methyl (R)-1-(2-chlorophenyl)-2-oxocyclohexylmethylcarbamate (A-65)

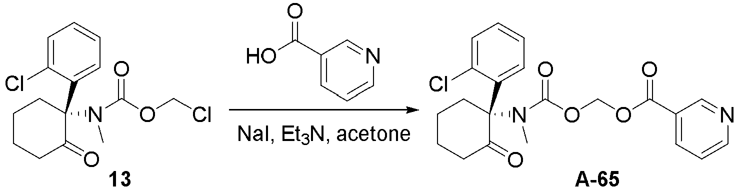

To a solution of 13 (495 mg, 1.5 mmol), NaI (450 mg, 3.0 mmol) and nicotinic acid (554 mg, 4.5 mmol) in acetone (18 mL) was added Et₃N (1.05 mL, 7.5 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (3/2) to afford 188 mg (30% yield) of the titled compound (**A-65**) as a white solid.

**¹HNMR** (500 MHz, DMSO-*d₆*) δ = 1.60-1.77 (m, 3H), 1.90-2.00 (m, 1H), 2.30-2.40 (m, 2H), 2.56-2.65 (m, 1H), 2.99 (s, 3H), 3.06-3.15 (m, 1H), 5.88-6.08 (m, 2H), 6.97-7.02 (m, 1H), 7.21-7.26 (m, 1H), 7.27-7.33 (m, 1H), 7.42-7.47 (m, 1H), 7.60-7.65 (m, 1H), 8.28-8.35 (m, 1H), 8.86-8.90 (m, 1H), 9.07-9.13 (m, 1H).

**MS (ESI):** [M + H]⁺= 417.2.

### Example 66: (2-acetamidoacetoyloxy)methyl 1-(2-chlorophenyl)-2-oxocyclohexy-lmethylcarbamate (A-66)

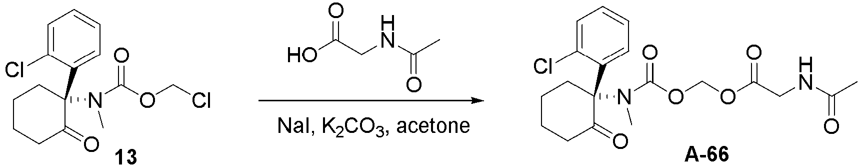

To a solution of **13** (50 mg, 0.15 mmol), NaI (45 mg, 0.30 mmol) and 2-acetamidoacetic acid (53.2 mg, 0.45 mmol) in acetone (2 mL) was added K₂CO₃ (105 mg, 0.76 mmol). The reaction was heated to 70 °C for 3 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/4) to afford 17 mg (27% yield) of the titled compound (**A-66**) as a white foam.

**¹HNMR** (500 MHz, DMSO-*d₆*) δ = 1.62-1.77 (m, 3H), 1.87 (s, 3H), 1.94-2.04 (m, 1H), 2.29-2.41 (m, 2H), 2.55-2.65 (m, 1H), 2.96 (s, 3H), 3.06-3.18 (m, 1H), 3.79-3.93 (m, 2H), 5.62-5.79 (m, 2H), 6.93-7.01 (m, 1H), 7.29-7.39 (m, 2H), 7.43-7.50 (m, 1H), 8.38 (t, *J =* 5.8 Hz, 1H).

**MS (ESI):** [M + H]⁺= 411.2.

### Example 67: ((S)-2-acetamido-3-methylbutanoyloxy)methyl 1-(2-chlorophenyl)-2-oxocyclohexylmethylcarbamate (A-67)

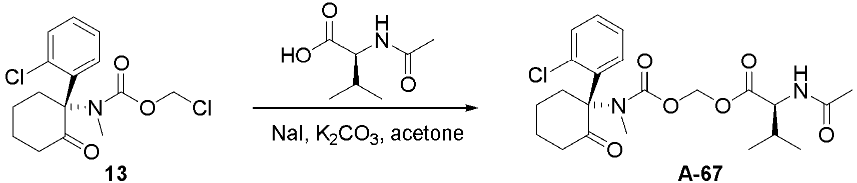

To a solution of compound **13** (50 mg, 0.15 mmol), NaI (45 mg, 0.30 mmol) and (S)-2-acetamido-3-methylbutanoic acid (72 mg, 0.45 mmol) in acetone (2 mL) was added K₂CO₃ (105 mg, 0.76 mmol). The reaction was heated to 70 °C for 3 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 1/4) to afford 56 mg (82% yield) of the titled compound (**A-67**) as a white foam.

**¹HNMR** (600 MHz, DMSO-*d₆*) δ = 0.89-0.96 (m, 6H), 1.59-1.78 (m, 3H), 1.88 (s, 3H), 1.92-2.08 (m, 2H), 2.30-2.39 (m, 2H), 2.54-2.62 (m, 1H), 2.95 (s, 3H), 3.08-3.16 (m, 1H), 4.09-4.17 (m, 1H), 5.66-5.83 (m, 2H), 6.91-6.98 (m, 1H), 7.26-7.38 (m, 2H), 7.44-7.51 (m, 1H), 8.23 (d, *J =* 7.3 Hz, 1H).

**MS (ESI): [M** + H]⁺= 453.2.

### Example 68: ((S)-2-acetamidopropanoyloxy)methyl (S)-1-(2-chlorophenyl)-2-oxocyclohexylmethylcarbamate (A-68)

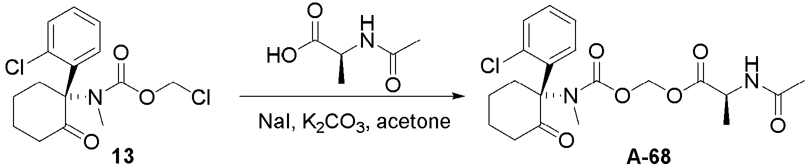

To a solution of **13** (50 mg, 0.15 mmol), NaI (46 mg, 0.30 mmol) and (*S*)-2-acetamidopropanoic acid (60 mg, 0.45 mmol) in acetone (2 mL) was added K₂CO₃ (105 mg, 0.76 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 13/7) to afford 54 mg (84% yield) of the titled compound (**A-68**) as a white foam.

**¹HNMR** (600 MHz, DMSO-*d₆*) δ = 1.22-1.33 (m, 3H), 1.63-1.76 (m, 3H), 1.84 (s, 3H), 1.95-2.01 (m, 1H), 2.30-2.41 (m, 2H), 2.55-2.63 (m, 1H), 2.95 (s, 3H), 3.07-3.16 (m, 1H), 4.18-4.26 (m, 1H), 5.65-5.78 (m, 2H), 6.90-7.00 (m, 1H), 7.26-7.38 (m, 2H), 7.43-7.50 (m, 1H), 8.38 (d, *J* = 6.1 Hz, 1H).

**MS (ESI):** [M + H]⁺= 425.2.

### Example 69: ((S)-2-acetamido-4-methylpentanoyloxy)methyl (R)-1-(2-chlorophenyl)-2-oxocyclohexylmethylcarbamate (A-69)

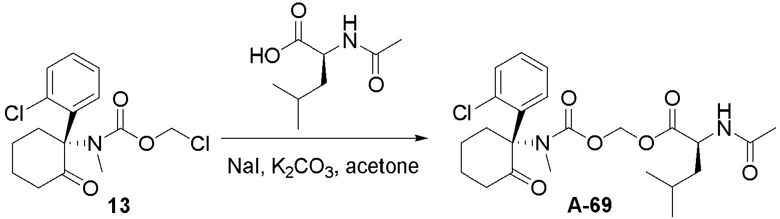

To a solution of compound **13** (50 mg, 0.15 mmol), NaI (45 mg, 0.3 mmol) and (*S*)-2-acetamido-4-methylpentanoic acid (78 mg, 0.45 mmol) in acetone (2 mL) was added K₂CO₃ (104 mg, 0.75 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 3/2) to afford 55 mg (79% yield) of the titled compound (**A-69**) as a white foam.

**¹HNMR** (500 MHz, DMSO-*d₆*) δ = 0.85 (d, *J =* 6.5 Hz, 3H), 0.90 (d, *J =* 6.5 Hz, 3H), 1.42-1.60 (m, 2H), 1.60-1.78 (m, 4H), 1.85 (s, 3H), 1.92-2.00 (m, 1H), 2.31-2.41 (m, 2H), 2.54-2.63 (m, 1H), 2.94 (s, 3H), 3.06-3.15 (m, 1H), 4.20-4.27 (m, 1H), 5.65-5.80 (m, 2H), 6.94-7.00 (m, 1H), 7.28-7.37 (m, 2H), 7.45-7.49 (m, 1H), 8.31 (d, *J* = 7.1 Hz, 1H).

**MS (ESI):** [M + H]⁺= 467.2.

### Example 70: ((2S,3R)-2-acetamido-3-methylpentanoyloxy)methyl 1-(2-chlorophenyl)-2-oxocyclohexylmethylcarbamate (A-70)

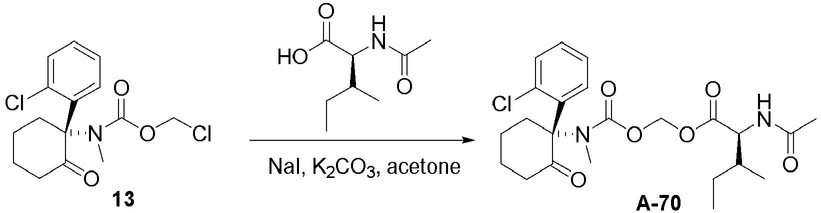

To a solution of compound **13** (50 mg, 0.15 mmol), NaI (45 mg, 0.30 mmol) and (2*S*,3*R*)-2-acetamido-3-methylpentanoic acid (79 mg, 0.45 mmol) in acetone (2 mL) was added K₂CO₃ (105 mg, 0.76 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 3/2) to afford 60 mg (85% yield) of the titled compound (**A-70**) as a white foam.

**¹HNMR** (600 MHz, DMSO-*d₆*) δ = 0.81-0.92 (m, 6H), 1.18-1.26 (m, 1H), 1.38-1.49 (m, 1H), 1.59-1.81 (m, 4H), 1.87 (s, 3H), 1.93-2.01 (m, 1H), 2.30-2.40 (m, 2H), 2.54-2.62 (m, 1H), 2.95 (s, 3H), 3.08-3.16 (m, 1H), 4.14-4.20 (m, 1H), 5.66-5.82 (m, 2H), 6.93-6.98 (m, 1H), 7.29-7.37 (m, 2H), 7.45-7.50 (m, 1H), 8.26 (d, *J=* 7.2 Hz, 1H).

**MS (ESI):** [M + H]⁺= 467.1.

### Example 71: (R)-(((1-(2-chlorophenyl)-2-oxocyclohexyl)(methyl)carbamoyl)oxy)methyl 2-aminonicotinate (A-71)

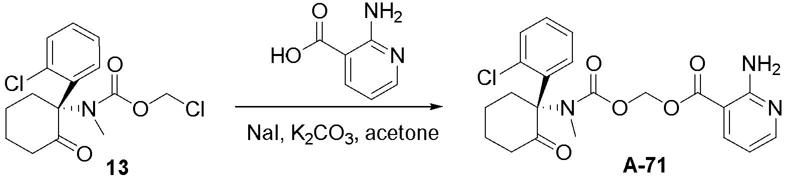

To a solution of compound **13** (50 mg, 0.15 mmol), NaI (45 mg, 0.30 mmol) and 2-aminopyridine-3-carboxylic acid (63 mg, 0.45 mmol) in acetone (2 mL) was added K₂CO₃ (105 mg, 0.76 mmol). The reaction was heated to 70 °C for 1 h. The reaction was concentrated and re-dissolved in DCM (5 mL), washed with aqueous saturated NaHCO₃ solution (5 mL) and brine (5 mL). The organic layer was dried over MgSO₄, filtered and concentrated to get an oil, which was purified on silica gel column eluting with hexane/EA (100% hexane to 2/3) to afford 42 mg (64% yield) of the titled compound (**A-71**) as a pale-yellow foam.

**¹HNMR** (600 MHz, DMSO-*d₆*): 1.59-1.76 (m, 3H), 1.90-1.99 (m, 1H), 2.28-2.40 (m, 2H), 2.55-2.63 (m, 1H), 2.98 (s, 3H), 3.06-3.15 (m, 1H), 5.81-6.05 (m, 2H), 6.63-6.71 (m, 1H), 6.90-7.00 (m, 1H), 7.18-7.27 (m, 3H), 7.28-7.34 (m, 1H), 7.41-7.49 (m, 1H), 7.99-8.08 (m, 1H), 8.26 (dd, *J =* 1.9, 4.6 Hz, 1H).

**MS (ESI):** [M + H]⁺= 432.1.

### Example 72: ethyl (S)-2-((((9H-fluoren-9-yl)methoxy)carbonyl)amino)-3-(4-((((S)-1-(2-chlorophenyl)-2-oxocyclohexyl)(methyl)carbamoyl)oxy)phenyl)propanoate (A-72)

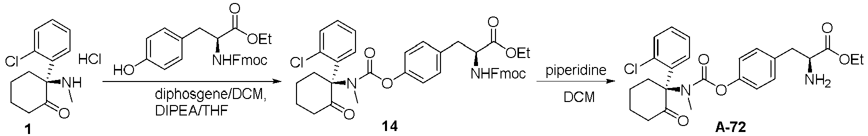

To a solution of diphosgene (237 mg, 1.2 mmol) in DCM (12 mL) was added a solution of ethyl (((9H-fluoren-9-yl)methoxy)carbonyl)-*L*-tyrosinate (863 mg, 2 mmol) in DCM (12 mL) slowly at 0 °C followed by the addition of a solution of DIPEA (1.05 mL, 6 mmol) in THF (24 mL) dropwise at 0 °C within 30 min. The reaction was stirred at 0 °C for 30 min and then a solution of compound **1** (274 mg, 1 mmol) and DIPEA (0.18 mL, 1 mmol) in DCM (15 mL) was added. The reaction was warmed to 25 °C and stirred for 16 h. The reaction was poured into H₂O (50 mL) and extracted with DCM (50 mL×2). The organic layer was dried over MgSO₄, filtered and concentrated to get an oil, which was purified on silica gel column eluting with hexane/EA (100% hexane to 4/1) to afford 590 mg (85% yield) of compound **14** as a white foam.

**¹HNMR** (500 MHz, DMSO-*d₆*) δ = 1.12 (t, *J =* 6.0 Hz, 3H), 1.75 (m, 3H), 2.01 (m, 1H), 2.38-2.41 (m, 2H), 2.71 (m, 1H), 2.86-2.91 (m, 1H), 3.01-3.04 (m, 1H), 3.13 (s, 4H), 4.06 (q, *J =* 7.0 Hz, 2H), 4.16-4.27 (m, 4H), 7.04-7.14 (m, 3H), 7.25-7.42 (m, 8H), 7.47-7.49 (m, 1H), 7.64 (t, *J =* 8.0 Hz, 2H), 7.86-7.89 (m, 3H).

**MS (ESI):** [M + H]⁺= 695.5.

To a solution of compound **14** (150 mg, 0.22 mmol) in DCM (4.5 mL) was added piperidine (0.21 mL, 2.2 mmol). The reaction was stirred at 25 °C for 3 h. The reaction was washed with H₂O (5 mL) and brine (5 mL). The organic layer was dried over MgSO₄, filtered and concentrated to get an oil, which was purified on silica gel column eluting with DCM/MeOH (100% DCM to 97/3) to afford 75 mg (74% yield) of the titled compound (**A-72**) as a yellow gum.

**¹HNMR** (500 MHz, DMSO-*d₆*) δ = 1.12 (t, *J =* 6.0 Hz, 3H), 1.73-1.77 (m, 3H), 2.02 (m, 1H), 2.39-2.42 (m, 2H), 2.73-2.85 (m, 3H), 3.13 (s, 4H), 3.51 (t, *J =* 6.5 Hz, 1H), 4.01 (q, *J =* 7.0 Hz, 2H), 7.02-7.19 (m, 5H), 7.33-7.41 (m, 2H), 7.48 (d, *J =* 7.5 Hz, 1H).

**MS (ESI):** [M + H]⁺ = 473.1.

### Example 73: ethyl (S)-2-(((1-(2-chlorophenyl)-2-oxocyclohexyl)(methyl)carbamoyl)oxy)acetate (A-73)

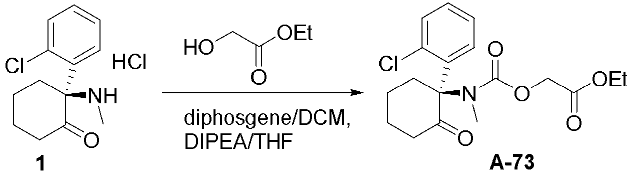

To a solution of diphosgene (119 mg, 0.6 mmol) in DCM (6 mL) was added a solution of ethyl 2-hydroxyacetate (104 mg, 1 mmol) in DCM (6 mL) slowly at 0 °C followed by the addition of a solution of DIPEA (0.52 mg, 3 mmol) in THF (6 mL) dropwise at 0 °C within 30 min. The reaction was stirred at 0 °C for 1 h and then a solution of compound **1** (137 mg, 0.5 mmol) and DIPEA (0.087 mL, 0.5 mmol) in DCM (6 mL) was added. The reaction was warmed to 25 °C and stirred for 16 h. The reaction was poured into H₂O (20 mL) and extracted with DCM (20 mL×2). The organic layer was dried over MgSO₄, filtered and concentrated to get an oil, which was purified on silica gel column eluting with hexane/EA (100% hexane to 2/1) to afford 70 mg (38% yield) of the titled compound (**A-73**) as a white solid.

**¹HNMR** (500 MHz, DMSO-*d₆*) δ = 1.19 (t, *J =* 7.0 Hz, 3H), 1.68 (t, *J =* 11.0 Hz, 3H), 1.98-2.01 (m, 1H), 2.26-2.35 (m, 2H), 2.63-2.65 (m, 1H), 3.04 (s, 3H), 3.14-3.17 (m, 1H), 4.13 (q, *J =* 7.0 Hz, 2H), 4.66 (s, 2H), 7.02-7.05 (m, 1H), 7.30-7.33 (m, 2H), 7.45-7.47 (m, 1H).

**MS (ESI):** [M + H]⁺ = 368.3.

### Example 74: (S)-2-(((1-(2-chlorophenyl)-2-oxocyclohexyl)(methyl)carbamoyl)oxy)acetic acid (A-74)

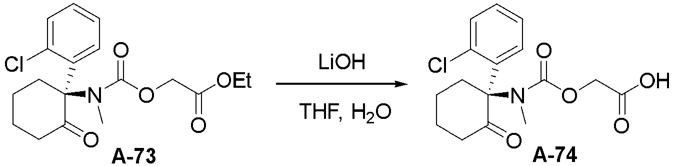

To a solution of compound (**A-73**) (74 mg, 0.2 mmol) in THF (3 mL) was added aqueous LiOH solution (0.5 mL, 1 M) at 0 °C. The reaction was stirred at 25 °C for 1.5 h and then concentrated. The mixture was diluted with H₂O (3 mL) and adjusted to pH=3 with aqueous HCl solution (1M) and then extracted with DCM (5 mL). The organic layer was dried over MgSO₄, filtered and concentrated to afford 50 mg (74% yield) of the titled compound (**A-74**) as a white foam.

**¹HNMR** (500 MHz, DMSO-*d₆*) δ = 1.68-1.72 (m, 3H), 1.98-2.02 (m, 1H), 2.24-2.33 (m, 2H), 2.60-2.64 (m, 1H), 3.04 (s, 3H), 3.16-3.21 (m, 1H), 4.55-4.90 (m, 2H), 7.07-7.09 (m, 1H), 7.27-7.33 (m, 2H), 7.44-7.46 (m, 1H).

**MS (ESI):** [M + H]⁺= 340.2.

### Example 75: (5-methyl-2-oxo-1,3-dioxol-4-yl)methyl (S)-(1-(2-chlorophenyl)-2-oxocyclohexyl)-(methyl)carbamate (A-75)

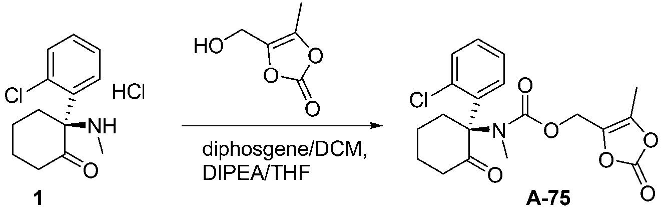

To a solution of diphosgene (119 mg, 0.6 mmol) in DCM (6 mL) was added a solution of 4-(hydroxymethyl)-5-methyl-1,3-dioxol-2-one (130 mg, 1 mmol) in DCM (6 mL) slowly at 0 °C followed by the addition of a solution of DIPEA (0.52 mg, 3 mmol) in THF (6 mL) dropwise at 0 °C within 30 min. The reaction was stirred at 0 °C for 1 h and then a solution of compound **1** (137 mg, 0.5 mmol) and DIPEA (0.087 mL, 0.5 mmol) in DCM (6 mL) was added. The reaction was warmed to 25 °C and stirred for 16 h. The reaction was poured into H₂O (20 mL) and extracted with DCM (20 mL×2). The organic layer was dried over MgSO₄, filtered and concentrated to get an oil, which was purified on silica gel column eluting with hexane/EA (100% hexane to 3/1) to afford 45 mg (23% yield) of the titled compound (**A-75**) as a colorless oil.

**¹HNMR** (500 MHz, DMSO-*d₆*) δ = 1.66 (m, 3H), 2.09 (m, 1H), 2.16 (s, 3H), 2.34 (t, 2H), 2.56-2.59 (m, 1H), 2.97 (s, 3H), 3.12 (m, 1H), 4.96 (s, 2H), 6.95 (m, 1H), 7.29-7.33 (m, 2H), 7.45 (m, 1H).

**MS (ESI):** [M + Na]⁺ = 416.1.

### Example 76: ethyl (S)-2-((((S)-1-(2-chlorophenyl)-2-oxocyclohexyl)(methyl)carbamoyl)oxy)-propanoate (A-76)

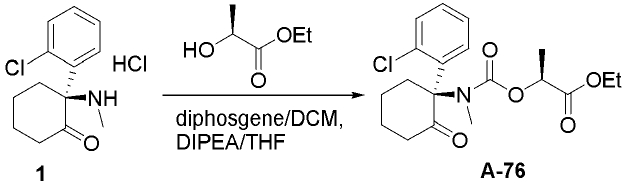

To a solution of diphosgene (89 mg, 0.45 mmol) in DCM (4.5 mL) was added a solution of (*S*)-ethyl 2-hydroxypropanoate (89 mg, 0.75 mmol) in DCM (4.5 mL) slowly at 0 °C followed by the addition of a solution of DIPEA (0.4 mL, 2.25 mmol) in THF (9 mL) dropwise at 0 °C within 30 min. The reaction was stirred at 0 °C for 2 h and then a solution of compound **1** (68 mg, 0.25 mmol) and DIPEA (0.044 mL, 0.25 mmol) in DCM (3.75 mL) was added. The reaction was warmed to 25 °C and stirred for 24 h. The reaction was poured into H₂O (10 mL) and extracted with DCM (10 mL× 2). The organic layer was dried over MgSO₄, filtered and concentrated to get an oil, which was purified on silica gel column eluting with hexane/EA (100% hexane to 4/1) to afford 18 mg (18% yield) of the titled compound (**A-76**) as a light-yellow oil.

**¹HNMR** (500 MHz, acetone-*d₆*) δ = 1.15-1.35 (m, 4H), 1.40-1.60 (m, 3H), 1.70-1.90 (m, 3H), 2.25-2.55 (m, 2H), 2.65-2.75 (m, 1H), 3.09 ( s, 3H), 3.18-3.32 (m, 1H), 4.16 (q, *J* = 7.1 Hz, 2H), 4.94 (q, *J =* 7.1 Hz, 1H), 7.05-7.15 (m, 1H), 7.21-7.31 (m, 2H), 7.40-7.50 (m, 1H).

**MS (ESI):** [M + H]⁺= 382.2.

### Example 77: (3-methyloxetan-3-yl)methyl (S)-(1-(2-chlorophenyl)-2-oxocyclohexyl)(methyl)-carbamate (A-77)

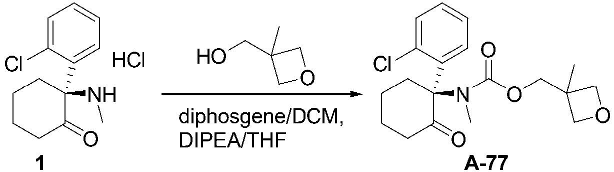

To a solution of diphosgene (59 mg, 0.3 mmol) in DCM (3 mL) was added a solution of (3-methyloxetan-3-yl)methanol (51 mg, 0.5 mmol) in DCM (3 mL) slowly at 0 °C followed by the addition of a solution of DIPEA (0.26 mL, 1.5 mmol) in THF (6 mL) dropwise at 0 °C within 30 min. The reaction was stirred at 0 °C for 2 h and then a solution of compound **1** (46 mg, 0.17 mmol) and DIPEA (0.029 mL, 0.17 mmol) in DCM (3 mL) was added. The reaction was warmed to 25 °C and stirred for 24 h. The reaction was poured into H₂O (20 mL) and extracted with DCM (20 mL×2). The organic layer was dried over MgSO₄, filtered and concentrated to get an oil, which was purified on silica gel column eluting with hexane/EA (100% hexane to 3/1) to afford 46 mg (76% yield) of the titled compound (**A-77**) as a light yellow oil.

**¹HNMR** (500 MHz, DMSO-*d₆*) δ = 1.04-1.24 (m, 3H), 1.58-1.79 (m, 3H), 1.92-2.05 (m, 1H), 2.26-2.37 (m, 2H), 2.55-2.68 (m, 1H), 3.01 (s, 3H), 3.08-3.18 (m, 1H), 4.02-4.47 (m, 6H), 6.88-6.98 (m, 1H), 7.25-7.36 (m, 2H), 7.42-7.50 (m, 1H).

**MS (ESI):** [M + H]⁺= 366.1.

### Example 78: 2-(((3-methyloxetan-3-yl)methyl)thio)ethyl (S)-(1-(2-chlorophenyl)-2-oxocyclohexyl)(methyl)carbamate (A-78)

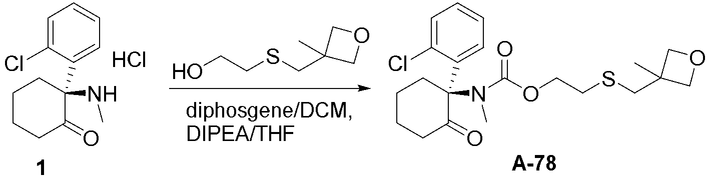

To a solution of diphosgene (356 mg, 1.8 mmol) in DCM (18 mL) was added a solution of 2-((3-methyloxetan-3-yl)methylthio)ethanol (486 mg, 3 mmol) in DCM (18 mL) slowly at 0 °C followed by the addition of a solution of DIPEA (1.57 mL, 9 mmol) in THF (36 mL) dropwise at 0 °C within 30 min. The reaction was stirred at 0 °C for 2 h and then a solution of compound **1** (273 mg, 1.0 mmol) and DIPEA (0.175 mL, 1.0 mmol) in DCM (15 mL) was added. The reaction was warmed to 25 °C and stirred for 24 h. The reaction was poured into H₂O (20 mL) and extracted with DCM (20 mL×2). The organic layer was dried over MgSO₄, filtered and concentrated to get an oil, which was purified on silica gel column eluting with hexane/EA (100% hexane to 3/1) to afford 238 mg (56% yield) of the titled compound (**A-78**) as a yellow oil.

**¹HNMR** (600 MHz, CD₃OD) δ = 1.36 (s, 3H), 1.73-1.90 (m, 3H), 2.04-2.12 (m, 1H),2.33-2.41 (m, 1H), 2.42-2.50 (m, 1H), 2.71-2.99 (m, 5H), 3.09 (s, 3H), 3.32-3.38 (m, 1H), 4.20-4.37 (m, 4H), 4.45-4.52 (m, 2H), 7.04-7.09 (m, 1H), 7.26-7.32 (m, 2H), 7.43-7.48 (m, 1H).

**MS (ESI):** [M + H]⁺= 425.9.

### Example 79: 2-(((3-methyloxetan-3-yl)methyl)sulfonyl)ethyl (S)-(1-(2-chlorophenyl)-2-oxocyclohexyl)(methyl)carbamate (A-79)

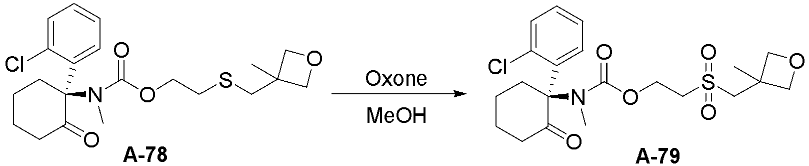

To a solution of compound (**A-78**) (33 mg, 0.08 mmol) in MeOH (0.3 mL) was added a solution of Oxone (96 mg, 0.16 mmol) in H₂O (0.3 mL) dropwise at 0 °C. The reaction was stirred at 25 °C for 16 h. The reaction was concentrated and re-dissolved in DCM (5 mL), washed with H₂O (5 mL). The organic layer was dried over MgSO₄, filtered concentrated to get an oil, which was purified on silica gel column eluting with hexane/EA (1/2) to afford 17 mg (47% yield) of the titled compound (**A-79**) as a white foam.

**¹HNMR** (500 MHz, acetone-*d₆*) δ = 1.32 (s, 3H), 1.61-1.81 (m, 4H), 2.38-2.52 (m, 2H), 2.70-2.80 (m, 1H), 3.10 (s, 3H), 3.15-3.80 (m, 5H), 4.26 (s, 2H), 4.56-4.62 (m, 4H), 7.13-7.15 (m, 1H), 7.29-7.32 (m, 2H), 7.44-7.46 (m, 1H).

**MS (ESI):** [M + H]⁺ = 457.5.

### Example 80: 2-(((3-methyloxetan-3-yl)methyl)sulfinyl)ethyl ((S)-1-(2-chlorophenyl)-2-oxocyclohexyl)(methyl)carbamate (A-80)

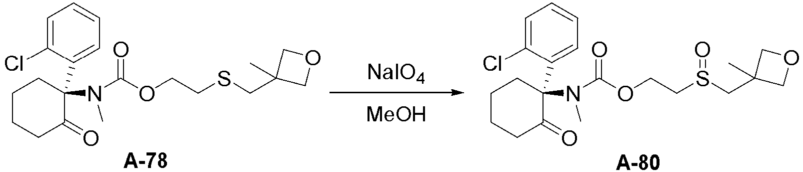

To a solution of compound (**A-78**) (85 mg, 0.2 mmol) in MeOH (0.8 mL) was added a solution of NaIO₄ (43 mg, 0.2 mmol) in H₂O (0.4 mL) dropwise at 0 °C. The reaction was stirred at 25 °C for 3 h. The reaction was concentrated and re-dissolved in DCM (5 mL), washed with H₂O (5 mL). The organic layer was dried over MgSO4, filtered concentrated to get an oil, which was purified on silica gel column eluting with DCM/MeOH (100% DCM to 97/3) to afford 79 mg (89% yield) of compound (**A-80**) as a white foam.

**¹HNMR** (600 MHz, CD₃OD) δ = 1.48-1.62 (m, 3H), 1.71-1.91 (m, 3H), 2.03-2.11 (m, 1H),2.33--2.52 (m, 2H), 2.71-2.83 (m, 1H), 2.88-3.29 (m, 3H), 3.09 ( s, 3H), 3.30-3.38 (m, 2H), 4.33-4.58 (m, 4H), 4.59-4.67 (m, 1H), 4.71-4.81 (m, 1H), 7.02-7.10 (m, 1H), 7.26-7.34 (m, 2H), 7.43-7.49 (m, 1H).

**MS (ESI):** [M + H]⁺ = 442.0.

### Example 81: (S)-2-((((S)-1-(2-chlorophenyl)-2-oxocyclohexyl)(methyl)carbamoyl)oxy)propanoic acid (A-81)

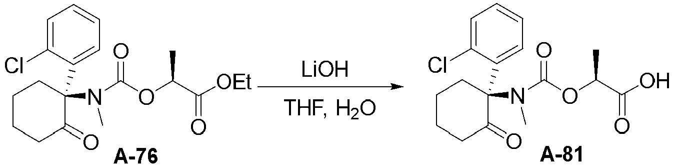

To a solution of compound (**A-76**) (46 mg, 0.12 mmol) in THF (1.8 mL) was added aqueous LiOH solution (0.3 mL, 1 M) at 0 °C. The reaction was stirred at 25 °C for 4 h and then concentrated. The mixture was diluted with H₂O (3 mL) and adjusted to pH=3 with aqueous HCl (1 M) and then extracted with DCM (5 mL). The organic layer was dried over MgSO₄, filtered and concentrated to afford 16 mg (37% yield) of the titled compound (**A-81**) as a white solid.

**¹HNMR** (500 MHz, acetone-*d₆*) δ = 1.43-1.59 (m, 3H), 1.70-1.86 (m, 4H),2.28-2.35 (m, 1H), 2.45-2.53 (m, 1H), 2.65-2.85 (m, 1H), 3.10 (s, 3H), 3.20-3.28 (m, 1H), 4.95 (q, *J =* 6.9 Hz, 1H), 7.09-7.18 (m, 1H), 7.24-7.34 (m, 2H), 7.39-7.48 (m, 1H).

**MS (ESI):** [M + H]⁺= 354.4.

### Example 82: 3-hydroxy-2-(hydroxymethyl)-2-methylpropyl (S)-(1-(2-chlorophenyl)-2-oxocyclohexyl)(methyl)carbamate (A-82)

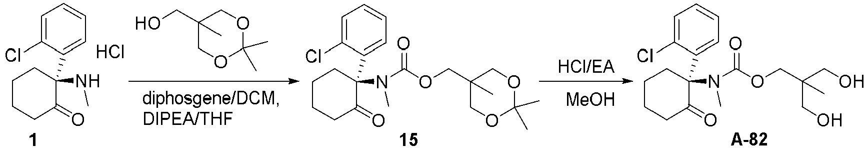

To a solution of diphosgene (267 mg, 1.35 mmol) in DCM (13.5 mL) was added a solution of (2,2,5-trimethyl-1,3-dioxan-5-yl)methanol (361 mg, 2.25 mmol) in DCM (13.5 mL) slowly at 0 °C followed by the addition of a solution of DIPEA (1.18 mL, 6.75 mmol) in THF (27 mL) dropwise at 0 °C within 30 min. The reaction was stirred at 0 °C for 2 h and then a solution of compound **1** (206 mg, 0.75 mmol) and DIPEA (0.131 mL, 0.75 mmol) in DCM (11 mL) was added. The reaction was warmed to 25 °C and stirred for 16 h. The reaction was poured into H₂O (20 mL) and extracted with DCM (20 mL×2). The organic layer was dried over MgSO₄, filtered and concentrated to get an oil, which was purified on silica gel column eluting with hexane/EA (9/1) to afford 141 mg (44% yield) of compound **15** as a white foam.

**¹HNMR** (500 MHz, acetone-*d₆*) δ = 1.29 (s, 6H), 1.36 (s, 3H), 1.72-1.86 (m, 3H), 2.34-2.46 (m, 2H), 2.67-2.76 (m, 1H), 3.10 (s, 3H), 3.20-3.34 (m, 1H), 3.40-3.80 (m, 5H), 4.01-4.24 (m, 2H), 6.98-7.08 (m, 1H), 7.26-7.33 (m, 2H), 7.41-7.47 (m, 1H).

**MS (ESI):** [M + H]⁺= 424.3.

To a solution of compound **15** (140 mg, 0.33 mmol) in MeOH (28 mL) was added HCl solution (0.42 mL, 0.2 M in EA). The reaction was stirred at 25 °C for 2 h. The reaction was concentrated and then purified on silica gel column eluting with hexane/EA (1/1) to afford 116 mg (92% yield) of the titled compound (**A-82**) as a white foam.

**¹HNMR** (500 MHz, acetone-*d₆*) δ = 0.86 (s, 3H), 1.70-1.90 (m, 3H), 2.35-2.50 (m, 2H), 2.65-2.80 (m, 1H), 3.09 (s, 3H), 3.25-3.85 (m, 6H), 4.00-4.20 (m, 2H), 7.05-7.15 (m, 1H), 7.25-7.40 (m, 2H), 7.42-7.52 (m, 1H).

**MS (ESI):** [M + H]⁺= 384.3.

### Example 83: ((2R,3S,4S,5R,6R)-3,4,5,6-tetrahydroxytetrahydro-2H-pyran-2-yl)methyl ((S)-1-(2-chlorophenyl)-2-oxocyclohexyl)(methyl)carbamate (A-83)

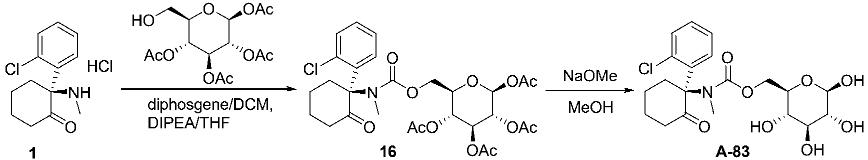

To a solution of diphosgene (267 mg, 1.35 mmol) in DCM (13.5 mL) was added a solution of 1,2,3,4-Tetra-*O*-acetyl-β-D-glucopyranose (361 mg, 2.25 mmol) in DCM (13.5 mL) slowly at 0 °C followed by the addition of a solution of DIPEA (1.18 mL, 6.75 mmol) in THF (27 mL) dropwise at 0 °C within 30 min. The reaction was stirred at 0 °C for 2 h and then a solution of compound **1** (206 mg, 0.75 mmol) and DIPEA (0.131 mL, 0.75 mmol) in DCM (11 mL) was added. The reaction was warmed to 25 °C and stirred for 16 h. The reaction was poured into H₂O (20 mL) and extracted with DCM (20 mL×2). The organic layer was dried over MgSO₄, filtered and concentrated to get a crude oil **16,** which was used directly in the following reaction.

To a solution of compound **16** in MeOH (40 mL) was added 3% NaOMe (in MeOH, 0.9 mL) at 25 °C. The reaction was stirred at 25 °C for 2 h and then adjusted to pH=3 with Dowex^{®} 50WX4 hydrogen form, filtered and concentrated to get an oil, which was purified on silica gel column eluting with DCM/MeOH (100% DCM to 9/1) to afford 40 mg (12% yield) of the titled compound (**A-83**) as a white solid.

**¹HNMR** (500 MHz, CD₃OD) δ = 1.73-1.91 (m, 3H), 2.02-2.15 (m, 1H),2.31-2.48 (m, 2H), 2.70-2.81 (m, 1H), 3.09 (s, 3H), 3.14-3.19 (m, 0.6H), 3.36-3.43 (m, 2.4H), 3.44-3.52 (m, 0.4H), 3.63-3.73 (m, 0.6H), 3.88-4.02 (m, 0.6H), 4.17-4.28 (m, 1H), 4.42-4.54 (m, 1.4H), 4.56-4.66 (m, 0.4H), 5.08-5.16 (m, 0.6H), 7.00-7.08 (m, 1H), 7.24-7.36 (m, 2H), 7.42-7.48 (m, 1H).

**MS (ESI):** [M + H]⁺ = 444.4.

### Example 84: 3-hydroxy-2-(hydroxymethyl)propyl (S)-(1-(2-chlorophenyl)-2-oxocyclohexyl)(methyl)carbamate (A-84)

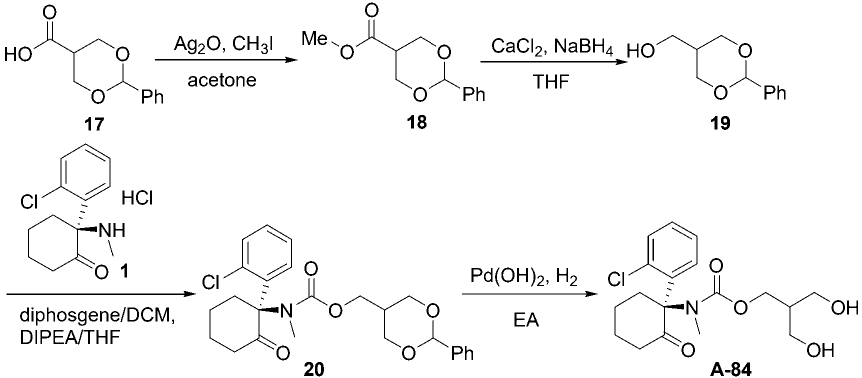

To a solution of compound **17** (1.0 g, 4.8 mmol) in acetone (25.5 mL) were added CH₃I (1.01 g, 71.1 mmol) and Ag₂O (1.16 g, 5.0 mmol). The suspension was stirred at 25 °C for 16 h. The mixture was filtered through Celite and concentrated to get an oil, which was purified on silica gel column eluting with hexane/EA (100% hexane to 95/5) to afford 965 mg (90% yield) of compound **18** as a white solid.

**¹HNMR** (500 MHz, CDCl₃) δ = 3.10-3.20 (m, 1H), 3.72 (s, 3H), 4.01 (t, *J =* 11.5 Hz, 2H), 4.47 (dd, *J* = 4.8, 11.9 Hz, 2H), 5.44 (s, 1H), 7.30-7.45 (m, 3H), 7.46-7.55 (m, 2H).

To a solution of CaCl₂ (4.3 g, 39.2 mmol) in THF (110 mL) was added NaBH₄ (3.0 g, 78.3 mmol). The reaction was stirred at 25 °C for 4 h and then compound **18** (965 mg, 4.35 mmol) was added. The reaction was warmed to reflux and stirred for 16 h. The reaction was poured into ice- water and extracted with DCM. The organic layer was dried over MgSO₄, filtered and evaporated to afford 836 mg (99% yield) of compound **19** as a white solid.

**¹HNMR** (500 MHz, acetone-*d₆*) δ = 2.15-2.30 (m, 1H), 3.40-3.50 (m, 2H), 3.65-3.80 (m, 2H), 4.24 (dd, *J* = 4.6*,* 11.6 Hz, 2H), 5.43 (s, 1H), 7.25-7.40 (m, 3H), 7.41-7.50 (m, 2H).

To a solution of diphosgene (267 mg, 1.35 mmol) in DCM (13.5 mL) was added a solution of **19** (437 mg, 2.25 mmol) in DCM (13.5 mL) slowly at 0 °C followed by the addition of a solution of DIPEA (1.18 mL, 6.75 mmol) in THF (27 mL) dropwise at 0 °C within 30 min. The reaction was stirred at 0 °C for 2 h and then a solution of compound **1** (206 mg, 0.75 mmol) and DIPEA (0.131 mL, 0.75 mmol) in DCM (11 mL) was added. The reaction was warmed to 25 °C and stirred for 16 h. The reaction was poured into H₂O (20 mL) and extracted with DCM (20 mL×2). The organic layer was dried over MgSO₄, filtered and concentrated to get an oil, which was purified on silica gel column eluting with DCM/EA (100% DCM to 10/1) to afford 243 mg (71% yield) of compound **20** as a white foam.

**¹HNMR** (500 MHz, acetone-*d₆*) δ = 1.70-1.88 (m, 4H), 2.34-2.48 (m, 3H), 2.68-2.78 (m, 1H), 3.10 (s, 3H), 3.23-3.34 (m, 1H), 3.60-3.84 (m, 2H), 3.90-4.08 (m, 2H), 4.10-4.32 (m, 2H), 5.43 (s, 1H), 7.02-7.10 (m, 1H), 7.27-7.39 (m, 5H), 7.40-7.50 (m, 3H).

**MS (ESI):** [M + H]⁺ = 457.9.

To a solution of compound **20** (100 mg, 0.22 mmol) in EA (10 mL) was added Pd(OH)₂/C (15 mg). The reaction was stirred at 25 °C under H₂ atmosphere for 30 min. The reaction was filtered through a pad of Celite and concentrated to get an oil, which was purified on silica gel column eluting with hexane/EA (100% hexane to EA) to afford 70 mg (86% yield) of the titled compound (**A-84**) as a light yellow oil.

**¹HNMR** (500 MHz, acetone-*d₆*) δ = 1.68-1.87 (m, 4H), 2.34-2.45 (m, 2H), 2.65-2.75 (m, 1H), 3.07 (s, 3H), 3.22-3.33 (m, 1H), 3.50-3.74 (m, 5H), 4.12-4.26 (m, 2H), 7.01-7.08 (m, 1H), 7.24-7.33 (m, 2H), 7.41-7.47 (m, 1H).

**MS (ESI):** [M + H]⁺ = 370.1.

### Example 85: 3-(methylamino)propyl (S)-1-(2-chlorophenyl)-2-oxocyclohexylmethylcarbamate (A-85)

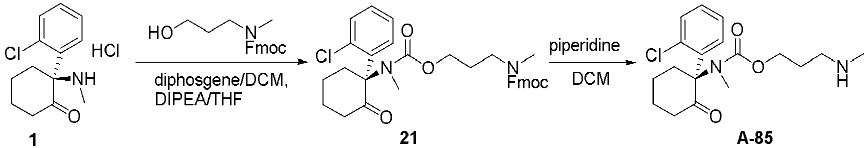

To a solution of diphosgene (89 mg, 0.45 mmol) in DCM (4.5 mL) was added a solution of (9H-fluoren-9-yl)methyl 3-hydroxypropylmethylcarbamate (234 mg, 0.75 mmol) in DCM (4.5 mL) slowly at 0 °C followed by the addition of a solution of DIPEA (0.392 mL, 2.25 mmol) in THF (9.0 mL) dropwise at 0 °C within 30 mins. The reaction was stirred at 0 °C for 1 h and then a solution of compound **1** (134 mg, 0.49 mmol) and DIPEA (0.044 mL, 0.25 mmol) in DCM (4.0 mL) was added. The reaction was warmed to 25 °C and stirred for 16 h. The reaction was poured into H₂O (20 mL) and extracted with DCM (20 mL×2).The organic layer was dried over MgSO₄, filtered and concentrated to get an oil, which was purified on silica gel column eluting with hexane/EA (8/2) to afford 80 mg (28% yield) of compound **21** as a white solid.

**¹HNMR** (500 MHz, CDCl₃) δ = 1.23-1.29 (m, 2H), 1.68-1.78 (m, 2H), 1.78-1.94 (m, 2H), 1.98-2.09 (m, 1H), 2.27-2.39 (m, 1H), 2.48-2.60 (m, 1H), 2.61-2.90 (m, 4H), 2.97-3.12 (m, 3H), 3.16-3.45 (m, 2H), 3.86-4.15 (m, 2H), 4.16-4.54 (m, 3H), 7.15-7.24 (m, 2H), 7.28-7.51 (m, 6H), 7.51-7.64 (m, 2H), 7.70-7.80 (m, 2H).

**MS (ESI):** [M + H]⁺ = 575.3.

To a solution of compound **21** (80 mg, 0.139 mmol) in DCM (5 mL) was added piperidine (1 mL). The reaction was stirred at 25 °C for 1.5 h. The reaction was poured into aqueous HCl solution (20 mL, 1 M) and extracted with DCM (20 mL×2).The organic layer was extracted with aqueous saturated NaHCO₃ solution (20 mL), then the organic layer was dried over MgSO₄, filtered and concentrated to get an oil, which was purified on silica gel column eluting with hexane/EA (100% hexane to 2/5) to afford 28 mg (57% yield) of the titled compound (**A-85**) as a pale yellow solid.

**¹HNMR** (500 MHz, CD₃OD) δ = 1.70-1.98 (m, 5H), 2.01-2.12 (m, 1H), 2.30-2.40 (m, 3H), 2.41-2.50 (m, 2H), 2.52-2.67 (m, 2H), 2.69-2.81 (m, 1H), 3.08 (s, 3H), 3.25-3.40 (m, 1H), 4.05-4.25 (m, 2H), 6.95-7.03 (m, 1H), 7.22-7.38 (m, 2H), 7.41-7.51 (m, 1H).

**MS (ESI):** [M + H]⁺ = 353.1.

### Example 86: 3-aminopropyl (S)-1-(2-chlorophenyl)-2-oxocyclohexylmethylcarbamate (A-86)

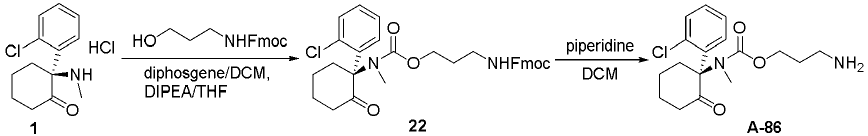

To a solution of diphosgene (166 mg, 0.84 mmol) in DCM (9.0 mL) was added a solution of (9*H*-fluoren-9-yl)methyl 3-hydroxypropylcarbamate (414 mg, 1.39 mmol) in DCM (9 mL) slowly at 0 °C followed by the addition of a solution of DIPEA (0.728 mL, 4.17 mmol) in THF (18 mL) dropwise at 0 °C within 30 min. The reaction was stirred at 0 °C for 1 h and then a solution of compound **1** (127 mg, 0.46 mmol) and DIPEA (0.081 mL, 0.46 mmol) in DCM (7.5 mL) was added. The reaction was warmed to 25 °C and stirred for 16 h. The reaction was poured into H₂O (20 mL) and extracted with DCM (20 mL×2). The organic layer was dried over MgSO₄, filtered and concentrated to get an oil, which was purified on silica gel column eluting with hexane/EA (8/2) to afford 74 mg (29% yield) of compound **22** as a white solid.

**¹HNMR** (500 MHz, CD₃OD) δ = 1.67-1.93 (m, 6H), 1.97-2.09 (m, 3H), 2.30-2.49 (m, 2H), 3.06 (s, 3H), 4.06-4.15 (m, 2H), 4.18-4.24 (m, 1H), 4.29-4.45 (m, 2H), 4.56-4.67 (m, 2H), 6.98-7.35 (m, 5H), 7.35-7.48 (m, 3H), 7.59-7.69 (m, 2H), 7.75-7.84 (m, 2H).

**MS (ESI):** [M + H]⁺ = 561.2.

To a solution of compound **22** (74 mg, 0.13 mmol) in DCM (3.0 mL) was added piperidine (1 mL). The reaction was stirred at 25 °C for 1.5 h. The reaction was poured into aqueous HCl solution (20 mL, 1 M) and extracted with DCM (20 mL×2).The organic layer was extracted with aqueous saturated NaHCO₃ solution (20 mL), then the organic layer was dried over MgSO₄, filtered and concentrated to get an oil, which was purified on silica gel column eluting with hexane/EA (100% hexane to 2/5) to afford 30 mg (67% yield) of the titled compound (**A-86**) as a sticky solid.

**¹HNMR** (500 MHz, CD₃OD) δ = 1.70-1.97 (m, 5H), 2.02-2.16 (m, 1H), 2.32-2.54 (m, 2H), 2.59-2.86 (m, 3H), 3.08 (s, 3H), 3.28-3.40 (m, 1H), 4.08-4.28 (m, 2H), 6.97-7.09 (m, 1H), 7.25-7.38 (m, 2H), 7.42-7.55 (m, 1H).

**MS (ESI):** [M + H]⁺ = 339.4.

### Example 87: 3-(methylamino)propyl (S)-1-(2-chlorophenyl)-2-oxocyclohexylmethylcarbamate (A-87)

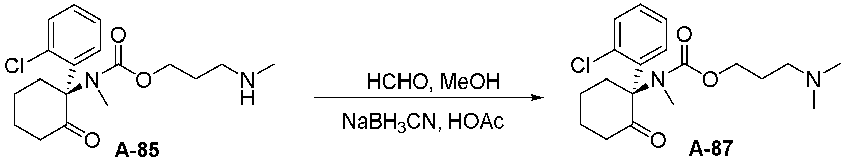

To a solution of compound (**A-85**) (17 mg, 0.048 mmol) in MeOH (2.0 mL) at 0 °C was added acetic acid (0.011 mL, 0.193 mmol) and NaBH₃CN (6.0 mg, 0.096 mmol). The reaction was stirred at 0 °C for 5 mins and then added formaldehyde (0.004 mL, 0.118 mmol). The reaction was warmed to 25 °C and stirred for 2 h. The reaction was poured into aqueous saturated NaHCO₃ solution (20 mL) and extracted with DCM (20 mL×2).The organic layer was dried over MgSO₄, filtered and concentrated to afford 14 mg (79% yield) of the titled compound (**A-87**) as a white solid.

**¹HNMR** (500 MHz, CDCl₃) δ = 1.70-1.82 (m, 4H), 1.84-1.93 (m, 1H), 2.02-2.09 (m, 1H), 2.18-2.38 (m, 9H), 2.49-2.59 (m, 1H), 2.66-2.76 (m, 1H), 3.05 (s, 3H), 3.27-3.38 (m, 1H), 4.08-4.20 (m, 2H), 6.89-6.97 (m, 1H), 7.19-7.25 (m, 2H), 7.42-7.47 (m, 1H).

**MS (ESI):** [M + H]⁺ = 367.4.

### Example 88: (S)-1-(((S)-1-(2-chlorophenyl)-2-oxocyclohexyl)(methyl)amino)-1-oxopropan-2-yl acetate (A-88)

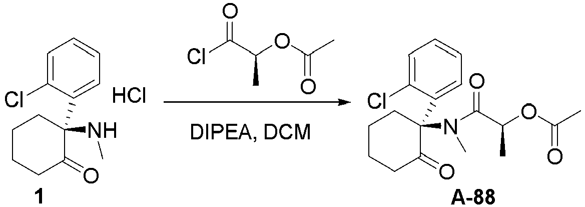

To a solution of compound **1** (150 mg, 0.55 mmol) and DIPEA (0.36 mL, 2.2 mmol) in DCM (3.0 mL) was added (*S*)-1-(chlorocarbonyl)ethyl acetate (0.17 mL, 1.4 mmol) dropwise at 0 °C. The mixture was warmed to 25 °C and stirred for 3 h. The reaction was poured into aqueous saturated NaHCO₃ solution (20 mL) and extracted with DCM (20 mL×2).The organic layer was dried over MgSO₄, filtered and concentrated to get an oil, which was purified on silica gel column eluting with hexane/EA (85/15) to afford 138 mg (72% yield) of the titled compound (**A-88**) as a light yellow foam.

**¹HNMR** (500 MHz, CD₃OD) δ = 1.56 (d, *J =* 6.7 Hz, 3H), 1.70-1.90 (m, 3H), 2.00-2.08 (m, 1H), 2.10 (s, 3H), 2.30-2.40 (m, 1H), 2.45-2.55 (m, 1H), 2.65-2.75 (m, 1H), 3.13 (s, 3H), 3.20-3.30 (m, 1H), 5.46 (q, *J =* 6.7 Hz, 1H), 7.00-7.05 (m, 1H), 7.25-7.35 (m, 2H), 7.40-7.50 (m, 1H).

**MS (ESI):** [M + H]⁺ = 351.9.

### Example 89: (S)-2-((1-(2-chlorophenyl)-2-oxocyclohexyl)(methyl)amino)-2-oxoethyl acetate (A-89)

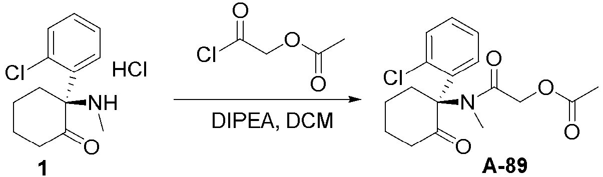

To a solution of compound **1** (150 mg, 0.55 mmol) and DIPEA (0.27 mL, 1.6 mmol) in DCM (3.0 mL), was added (chlorocarbonyl)methyl acetate (0.09 mL, 0.8 mmol) dropwise at 0 °C. The reaction was warmed up to 25 °C and stirred for 3 h. The reaction was poured into aqueous saturated NaHCO₃ solution (20 mL) and extracted with DCM (20 mL×2).The organic layer was dried over MgSO₄, filtered and concentrated to get an oil, which was purified on silica gel column eluting with hexane/EA (85/15) to afford 94 mg (51% yield) of the titled compound (**A-89**) as a white solid.

**¹HNMR** (500 MHz, DMSO-*d*₆) δ = 1.09-1.21 (m, 1H), 1.46-1.72 (m, 4H), 1.77-1.91 (m, 2H), 2.08 (s, 3H), 2.70 (s, 3H), 2.94-3.16 (m, 1H), 5.16 (s, 1H), 5.47 (s, 1H), 7.11-7.25 (m, 1H), 7.27-7.40 (m, 2H), 7.41-7.48 (m, 1H).

**MS (ESI):** [M + H]⁺ = 338.1.

### Example 90: (S)-N-(1-(2-chlorophenyl)-2-oxocyclohexyl)-N-methylnicotinamide (A-90)

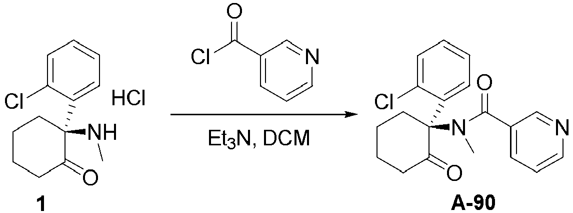

To a solution of compound **1** (237 mg, 1 mmol) and Et₃N (0.63 mL, 4.5 mmol) in DCM (6 mL), was added nicotinoyl chloride hydrochloride (534 mg, 3 mmol) at 0 °C. The reaction was warmed up to 25 °C and stirred for 3 h. The reaction was poured into aqueous saturated NaHCO₃ solution (20 mL) and extracted with DCM (20 mL×2). The organic layer was dried over MgSO₄, filtered and concentrated to get an oil, which was purified on silica gel column eluting with hexane/EA (100% hexane to 3/2) to afford 274 mg (80% yield) of the titled compound (**A-90**) as a light yellow solid.

**¹HNMR** (500 MHz, DMSO-*d*₆) δ = 1.68-1.80 (m, 2H), 1.89-1.98 (m, 1H), 2.02-2.10 (m, 1H), 2.29-2.37 (m, 1H), 2.41-2.49 (m, 1H), 2.58-2.67 (m, 1H), 2.99 (s, 3H), 3.18-3.27 (m, 1H), 7.07-7.14 (m, 1H), 7.30-7.38 (m, 2H), 7.46-7.52 (m, 1H), 7.53-7.59 (m, 1H), 8.05-8.11 (m, 1H), 8.71-8.76 (m, 1H), 8.82-8.86 (m, 1H).

**MS (ESI):** [M + H]⁺ = 343.0.

### Example 91: (S)-3-((1-(2-chlorophenyl)-2-oxocyclohexyl)(methyl)carbamoyl)-1-methylpyridin-1-ium iodide (A-91)

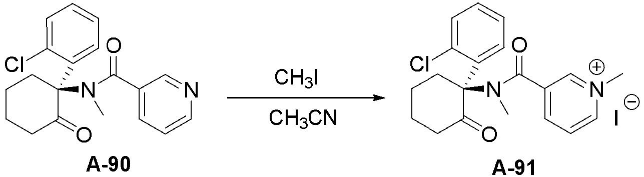

To a solution of compound (**A-90**) (103 mg, 0.3 mmol) in CH₃CN (3 mL) was added CH₃I (0.09 mL, 1.5 mmol). The reaction was heated to 80 °C for 16 h, and the solvent was removed under vacuum. Ether (3 mL) was added, filtered and the solid was washed with cold ether to afford 111 mg (76% yield) of the titled compound (**A-91**) as a white solid.

**¹HNMR** (500 MHz, DMSO-*d*₆) δ = 1.70-1.84 (m, 2H), 1.93-2.08 (m, 2H), 2.34-2.41 (m, 1H), 2.52-2.56 (m, 1H), 2.62-2.63 (m, 1H), 3.02 (s, 3H), 3.17-3.26 (m, 1H), 4.42 (s, 3H), 7.15-7.21 (m, 1H), 7.31-7.40 (m, 2H), 7.49-7.53 (m, 1H), 8.21-8.27 (m, 1H), 8.84 (d, *J =* 8.1 Hz, 1H), 9.09 (d, *J* = 6.1 Hz, 1H), 9.34 (s, 1H).

**MS (ESI):** [M + H]⁺ = 357.4.

### Example 92: methyl (S)-4-((1-(2-chlorophenyl)-2-oxocyclohexyl)(methyl)amino)-4-oxobutanoate (A-92)

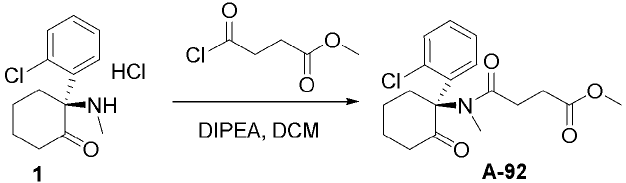

To a solution of compound **1** (150 mg, 0.55 mmol), DIPEA (0.29 mL, 1.65 mmol) in DCM (3.0 mL), was added methyl 3-(chlorocarbonyl)propanoate (0.1 mL, 0.825 mmol) dropwise at 0 °C. The reaction was warmed to 25 °C and stirred for 2 h. The reaction was poured into aqueous saturated NaHCO₃ solution (20 mL) and extracted with DCM (20 mL×2).The organic layer was dried over MgSO₄, filtered and concentrated to get an oil, which was purified on silica gel column eluting with hexane/EA (3/1) to afford 150 mg (78% yield) of the titled compound (**A-92**) as a white solid.

**¹HNMR** (500 MHz, acetone-*d₆*) δ = 1.66-1.86 (m, 4H), 2.20-2.27 (m, 1H), 2.34-2.41 (m, 1H), 2.54-2.68 (m, 3H), 2.86-2.99 (m, 2H), 3.30 (s, 3H), 3.26-3.35 (m, 1H), 3.60 (s, 3H), 7.01-7.08 (m, 1H), 7.20-7.29 (m, 2H), 7.39-7.44 (m, 1H).

**MS (ESI):** [M + H]⁺ = 352.1.

### Example 93: (S)-4-((1-(2-chlorophenyl)-2-oxocyclohexyl)(methyl)amino)-4-oxobutanoic acid (A-93)

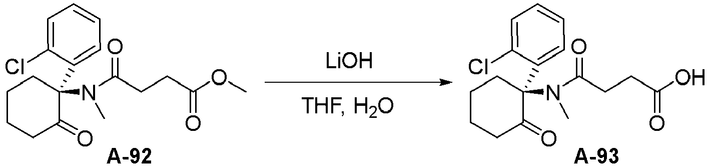

To a solution of compound (**A-92**) (100 mg, 0.285 mmol) in THF (4.3 mL) was added aqueous LiOH solution (0.7 mL, 1 M) at 0 °C. The reaction was stirred at 25 °C for 2 h and then concentrated. The mixture was diluted with H₂O (3 mL) and adjusted to pH=3 with aqueous HCl solution (1 M) and then extracted with DCM (5 mL). The organic layer was dried over MgSO₄, filtered and concentrated to afford 83 mg (83% yield) of the titled compound (**A-93**) as a white solid.

**¹HNMR** (500 MHz, acetone-*d₆*) δ = 1.63-1.83 (m, 4H), 2.16-2.22 (m, 1H), 2.30-2.38 (m, 1H), 2.52-2.67 (m, 3H), 2.84-2.94 (m, 2H), 3.16 (s, 3H), 3.24-3.33 (m, 1H), 7.01-7.06 (m, 1H), 7.16-7.25 (m, 2H), 7.36-7.40 (m, 1H).

**MS (ESI):** [M + H]⁺ = 338.3.

### Example 94: (S)-2-((1-(2-chlorophenyl)-2-oxocyclohexyl)(methyl)amino)-2-oxoethan-1-aminium chloride (A-94)

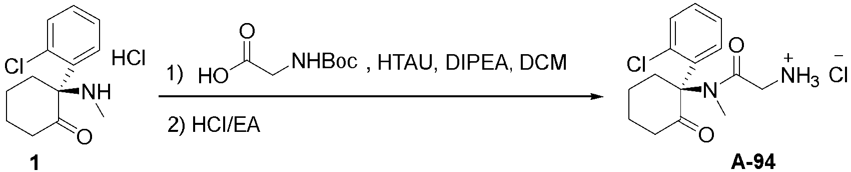

To a solution of compound **1** (68 mg, 0.25 mmol), (*tert*-butoxycarbonyl)glycine (111 mg, 0.375 mmol) and HATU (114 mg, 0.3 mmol) in DCM (2 mL) was added DIPEA (0.13 mL, 0.75 mmol). The reaction mixture was microwave irradiated at 70 °C for 20 min. The reaction was diluted with DCM (5 mL), washed with H₂O (5 mL) and brine (5 mL). The organic layer was dried over MgSO₄, filtered and concentrated to get an oil, which was purified on silica gel column eluting with hexane/EA (1/0 to 3/2) to afford a white foam. The white foam was dissolved in EA (1.5 mL) and HCl (0.15 mL, 2N solution in EA) was added. The reaction was stirred at 25 °C for 16 h, filtered and the solid was washed with cold EA to afford 35 mg (38% yield) of the titled compound (**A-94**) as a white solid.

**¹HNMR** (500 MHz, DMSO-*d*₆) δ = 1.68 (m, 3H), 1.95 (m, 1H), 2.26 (d, *J =* 15 Hz, 1H), 2.35 (d, *J =* 15 Hz, 1H), 2.63 (m, 1H), 3.02 (s, 3H), 3.15 (m, 1H), 4.10 (m, 1H), 4.29 (m, 1H), 7.02-7.04 (m, 1H), 7.25-7.33 (m, 2H), 7.45-7.46 (m, 1H), 8.18 (s, 3H).

**MS (ESI):** [M-H₂O] = 277.1*.*

### Example 95: (S)-2-acetamido-N-(1-(2-chlorophenyl)-2-oxocyclohexyl)-N-methylacetamide (A-95)

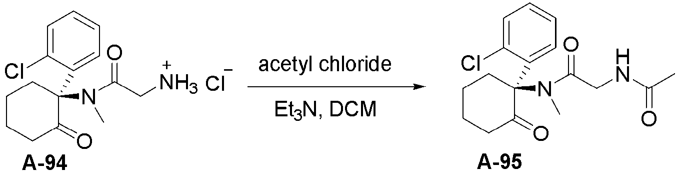

To a solution of compound (**A-94**) (35 mg, 0.12 mmol) and Et₃N (0.033 mL, 0.24 mmol) in DCM (2 mL) was added acetylchloride (0.013 mL, 0.18 mmol). The reaction was stirred at 25 °C for 16 h. The reaction was diluted with DCM (3 mL), washed with H₂O (3 mL) and brine (3 mL). The organic layer was dried over MgSO₄, filtered and concentrated to get an oil, which was purified on silica gel column eluting with hexane/EA (100% hexane to 2/1) to afford 20 mg (50% yield) of the titled compound (**A-95**) as a white solid.

**¹HNMR** (500 MHz, DMSO-*d*₆) δ = 1.67 (m, 3H), 1.86 (s, 3H), 1.95 (m, 1H), 2.17 (d, *J =* 12.9 Hz, 1H), 2.28 (d, *J =* 13.2 Hz, 1H), 2.55 (m, 1H), 3.03 (s, 3H), 3.14 (m, 1H), 4.16-4.21 (m, 2H), 6.97 (m, 1H), 7.24-7.30 (m, 2H), 7.42 (m, 1H), 8.11 (m, 1H).

**MS (ESI):** [M + H]⁺ = 337.2.

### Example 96: isopropyl (S)-4-((1-(2-chlorophenyl)-2-oxocyclohexyl)(methyl)amino)-4-oxobutanoate (A-96)

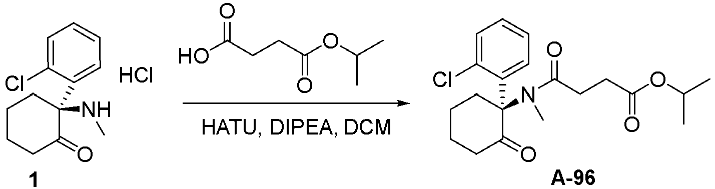

To a solution of compound **1** (136 mg, 0.5mmol), 3-(isopropoxycarbonyl)propanoic acid (121 mg, 0.75 mmol) and HATU (228, 0.6 mmol) in DCM (4mL) was added DIPEA (0.26 mL, 1.5 mmol). The reaction mixture was microwave irradiated at 70 °C for 30 min. The reaction was diluted with DCM (10 mL), washed with H₂O (10 mL) and brine (10 mL). The organic layer was dried over MgSO₄, filtered and concentrated to get an oil, which was purified on silica gel column eluting with DCM/EA (100% DCM to 9/1) to afford 77mg (40% yield) of the titled compound (**A-96**) as a colorless oil.

**¹HNMR** (500 MHz, acetone-*d₆*) δ = 1.18 (d, *J =* 1.8 Hz, 3H), 1.19 (d, *J =* 1.8 Hz, 3H), 1.66-1.86 (m, 4H), 2.20-2.27 (m, 1H), 2.34-2.42 (m, 1H), 2.50-2.67 (m, 3H), 2.87-2.94 (m, 2H), 3.18 (s, 3H), 3.26-3.34 (m, 1H), 4.88-4.97 (m, 1H), 7.03-7.08 (m, 1H), 7.20-7.28 (m, 2H), 7.38-7.43 (m, 1H).

**MS (ESI):** [M + H]⁺= 380.2.

### Example 97: ethyl (S)-4-((1-(2-chlorophenyl)-2-oxocyclohexyl)(methyl)amino)-4-oxobutanoate (A-97)

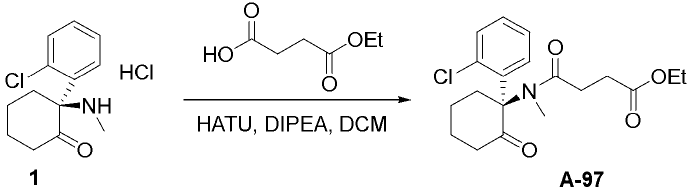

To a solution of compound **1** (68 mg, 0.25 mmol), 3-(isopropoxycarbonyl)propanoic acid (55.1 mg, 0.375 mmol) and HATU (114 mg, 0.3 mmol) in DCM (2 mL) was added DIPEA (0.13 mL, 0.75 mmol). The reaction mixture was microwave irradiated at 70 °C for 30 min. The reaction was diluted with DCM (10 mL), washed with H₂O (10 mL) and brine (10 mL). The organic layer was dried over MgSO₄, filtered and concentrated to get an oil, which was purified on silica gel column eluting with DCM/EA (100% DCM to 9/1) to afford 47 mg (51% yield) of the titled compound (**A-97**) as a light yellow foam.

**¹HNMR** (500 MHz, acetone-*d₆*) δ = 1.20 (t, *J =* 7.1 Hz, 3H), 1.66-1.86 (m, 4H), 2.20-2.26 (m, 1H), 2.34-2.42 (m, 1H), 2.52-2.68 (m, 3H), 2.86-2.98 (m, 2H), 3.18 (s, 3H), 3.26-3.35 (m, 1H), 4.01-4.13 (m, 2H), 7.03-7.08 (m, 1H), 7.20-7.28 (m, 2H), 7.38-7.43 (m, 1H).

**MS (ESI):** [M + H]⁺ = 366.2.

### Example 98: (S)-4-((1-(2-chlorophenyl)-2-oxocyclohexyl)(methyl)amino)-4-oxobutan-1-aminium chloride (A-98)

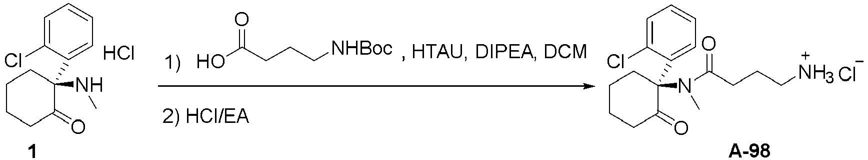

To a solution of compound **1** (137 mg, 0.5 mmol), (4-((tert-butoxycarbonyl)amino)butanoic acid (152 mg, 0.75 mmol) and HATU (228 mg, 0.6 mmol) in DCM (4 mL) was added DIPEA (0.26 mL, 1.5 mmol). The reaction mixture was microwave irradiated at 70 °C for 20 min. The reaction was diluted with DCM (10 mL), washed with H₂O (10 mL) and brine (10 mL). The organic layer was dried over MgSO₄, filtered and concentrated to get an oil, which was purified on silica gel column eluting with hexane/EA (1/0 to 2/1) to afford a white foam. The white foam was dissolved in ether (1.5 mL) and HCl (1.5 mL, 2N solution in ether) was added. The reaction was stirred at 25 °C for 16 h, filtered and the solid was washed with cold ether to afford 69 mg (39% yield) of the titled compound (**A-98**) as a white solid.

**¹HNMR** (500 MHz, DMSO-*d*₆) δ = 1.67 (m, 3H), 1.81 (m, 2H), 1.91 (m, 1H), 2.19 (d, *J =* 12.9 Hz, 1H), 2.26 (d, *J =* 13.9 Hz, 1H), 2.43 (m, 1H), 2.76-2.83 (m, 4H), 3.02 (s, 3H), 3.36 (m, 1H), 6.94 (m, 1H), 7.26-7.31 (m, 2H), 7.44 (m, 1H), 8.07 (s, 3H).

**MS (ESI):** [M + H]⁺ = 323.1.

### Example 99: (S)-N-(1-(2-chlorophenyl)-2-oxocyclohexyl)-4-(dimethylamino)-N-methylbutanamide (A-99)

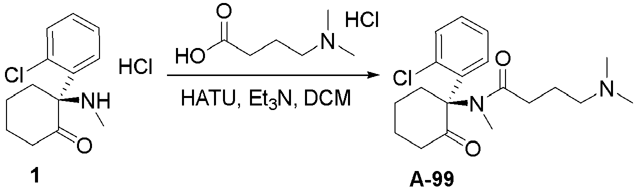

To a solution of compound **1** (68 mg, 0.25 mmol), 4-(dimethylamino)butanoic acid hydrochloride (84 mg, 0.5 mmol) and HATU (190 mg, 0.5 mmol) in DCM (2 mL) was added DIPEA (0.22 mL, 1.25 mmol). The reaction mixture was microwave irradiated at 70 °C for 30 min. The reaction was diluted with DCM (5 mL), washed with H₂O (5 mL) and brine (5 mL). The organic layer was dried over MgSO₄, filtered and concentrated to get an oil, which was purified on silica gel column eluting with DCM/EA (1/1) to afford 13 mg (15% yield) of the titled compound (**A-99**) as a white solid.

**¹HNMR** (500 MHz, acetone-*d₆*) δ = 1.70-1.83 (m, 4H), 2.29-2.36 (m, 1H), 2.39-2.47 (m, 1H), 2.57-2.66 (m, 1H), 2.94-3.08 (m, 3H), 3.13 (s, 6H), 3.16 (s, 3H), 3.21-3.33 (m, 2H), 3.39-3.48 (m, 2H), 7.05-7.10 (m, 1H), 7.21-7.33 (m, 2H), 7.42-7.48 (m, 1H).

**MS (ESI):** [M + H]⁺ = 350.9.

### Example 100: (S)-2-((1-(2-chlorophenyl)-2-oxocyclohexyl)(methyl)carbamoyl)benzyl isobutyrate (A-100)

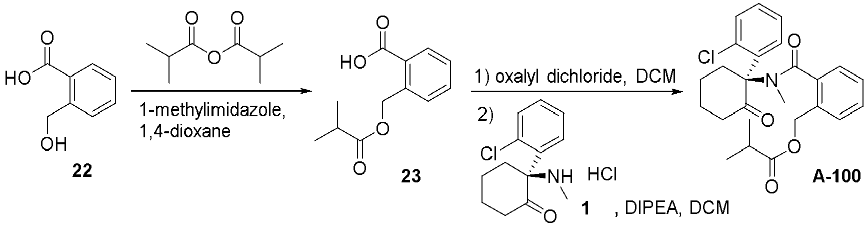

To a solution of compound **22** (304 mg, 2.0 mmol) in 1,4-dioxane (5 mL) was added isobutyric anhydride (0.497 mL, 3.0 mmol) and 1-methylimidazole (0.24 mL, 3.0 mmol). The reaction was stirred at 25 °C for 1 h, and the solvent was removed under vacuum. The residue was diluted with DCM (10 mL), washed with H₂O (10 mL). The organic layer was dried over MgSO₄, filtered and concentrated to get an oil, which was purified on silica gel column eluting with hexane/EA (8/2) to afford 340 mg (77% yield) of compound **23** as a white solid.

**¹HNMR** (500 MHz, DMSO-*d*₆) δ = 1.17-1.19 (d, *J* = 7.0 Hz, 6H), 2.56-2.68 (m, 1H), 5.38-5.45 (m, 2H), 7.39-7.53 (m, 2H), 7.54-7.94 (m, 3H).

**MS (ESI):** [M + Na]⁺ = 245.0.

To a solution of compound **23** (83 mg, 0.374 mmol) in DCM (2 mL) was added oxalyl chloride (0.043 mL, 0.498 mmol). The reaction was stirred at 25 °C for 4 h, and the solvent was removed under vacuum. The residue was dissolved in DCM (2 mL) and a solution of compound **1** (68 mg, 0.25 mmol) and DIPEA (0.065 mL, 0.375 mmol) in DCM (1 mL) was added. The reaction was stirred at 25 °C for 16 h. The reaction was diluted with DCM (10 mL), washed with H₂O (10 mL). The organic layer was dried over MgSO₄, filtered and concentrated to get an oil, which was purified on silica gel column eluting with hexane/EA (85/15) to afford 30 mg (27% yield) of the titled compound (**A-100**) as a white solid.

**¹HNMR** (500 MHz, CD₃OD) δ = 1.17 (dd, *J =* 7.0, 4.3 Hz, 6H), 1.76-1.85 (m, 1H), 1.86-2.00 (m, 2H), 2.10-2.20 (m, 1H), 2.53-2.59 (m, 1H), 2.59-2.68 (m, 2H), 2.75-2.84 (m, 1H), 2.96 (s, 3H), 3.38-3.49 (m, 1H), 5.10-5.20 (m, 1H), 5.24-5.33 (m, 1H), 7.29-7.36 (m, 2H), 7.37-7.42 (m, 1H), 7.47-7.54 (m, 4H), 7.55-7.59 (m, 1H).

**MS (ESI):** [M + H]⁺ = 442.3.

### Example 101: (S)-N-(1-(2-chlorophenyl)-2-oxocyclohexyl)-N-methyl-4-(methylamino)butanamide (A-101)

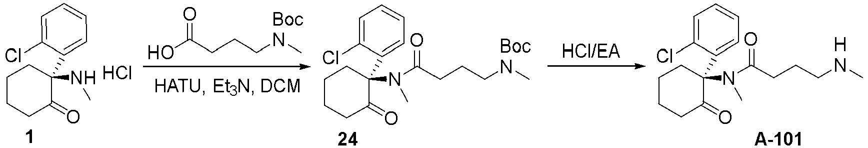

To a solution of compound **1** (68 mg, 0.25 mmol), 4-((*tert-*butoxycarbonyl)(methyl)amino)butanoic acid (81.4 mg, 0.375 mmol) and HATU (114 mg, 0.3 mmol) in DCM (2 mL) was added DIPEA (0.13 mL, 0.75 mmol). The reaction mixture was microwave irradiated at 70 °C for 1 h. The reaction was diluted with DCM (10 mL), washed with H₂O (10 mL) and brine (10 mL). The organic layer was dried over MgSO₄, filtered and concentrated to get an oil, which was purified on silica gel column eluting with hexane/EA (7/3) to afford 45 mg (41% yield) of compound **24** as a white solid.

**¹HNMR** (500 MHz, acetone-*d₆*) δ = 1.46 (s, 9H), 1.69-1.89 (m, 5H), 2.24-2.42 (m, 2H), 2.54-2.68 (m, 3H), 2.84 (s, 3H), 3.12 (s, 3H), 3.23-3.40 (m, 4H), 7.00-7.12 (m, 1H), 7.20-7.29 (m, 2H), 7.38-7.45 (m, 1H).

**MS (ESI):** [M + H]⁺ = 437.4.

To a solution of compound **24** (45 mg, 0.1 mmol) in EA (3 mL) was added HCl (3 mL, 1 M solution in EA). The reaction was stirred at 25 °C for 16 h, filtered and the solid. The solid was poured into aqueous saturated NaHCO₃ solution (20 mL) and extracted with DCM (20 mL×2). The organic layer was dried over MgSO₄, filtered and concentrated to get an oil, which was purified on silica gel column eluting with DCM/EA (1/1) to afford 14 mg (42% yield) of the titled compound **(A-101)** as a white solid.

**¹HNMR** (500 MHz, CD₃OD) δ = 1.72-1.90 (m, 5H), 2.00-2.09 (m, 1H), 2.32-2.44 (m, 2H), 2.40 (s, 3H), 2.56-2.75 (m, 5H), 2.65-2.75 (m, 1H), 3.12 (s, 3H), 3.32-3.38 (m, 1H), 6.94-7.00 (m, 1H), 7.24-7.31 (m, 2H), 7.42-7.48 (m, 1H).

**MS (ESI):** [M + H]⁺ = 337.3.

### Example 102: (S)-2-((1-(2-chlorophenyl)-2-oxocyclohexyl)(methyl)carbamoyl)benzyl acetate (A-102)

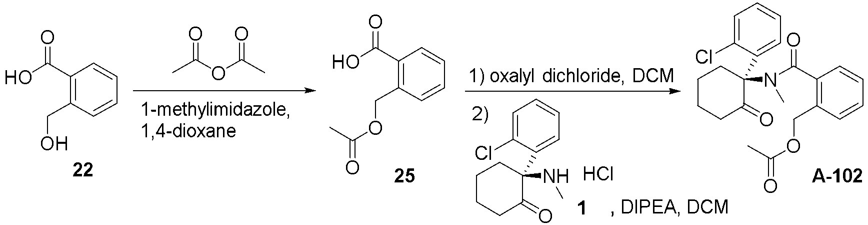

To a solution of compound **22** (304 mg, 2.0 mmol) in 1,4-dioxane (5 mL) was added acetic anhydride (0.28 mL, 3.0 mmol) and 1-methylimidazole (0.240 mL, 3.0 mmol). The reaction was stirred at 25 °C for 1 h, and the solvent was removed under vacuum. The reaction was diluted with DCM (10 mL), washed with H₂O (10 mL). The organic layer was dried over MgSO₄, filtered and concentrated to get an oil, which was purified on silica gel column eluting with hexane/EA (8/2) to afford 100 mg (26% yield) of compound **25** as a white solid.

**¹HNMR** (500 MHz, DMSO-*d*₆) δ = 2.10 (s, 3H), 5.42 (s, 2H), 7.39-7.45 (m, 1H), 7.45-7.52 (m, 1H), 7.53-7.59 (m, 1H), 7.85-7.92 (m, 1H).

**MS (ESI):** [M + Na]⁺ = 217.1.

To a solution of compound **25** (73 mg, 0.375 mmol) in DCM (2 mL) was added oxalyl chloride (0.064 mL, 0.75 mmol). The reaction was stirred at 25 °C for 4 h, and the solvent was removed under vacuum. The residue was dissolved with DCM (2 mL), and a solution of compound **1** (68 mg, 0.25 mmol) and DIPEA (0.065 mL, 0.375 mmol) in DCM (1 mL) was added. The reaction was stirred at 25 °C for 16 h. The reaction was diluted with DCM (10 mL), washed with H₂O (10 mL). The organic layer was dried over MgSO₄, filtered and concentrated to get an oil, which was purified on silica gel column eluting with hexane/EA (7/3) to afford 22 mg (21% yield) of the titled compound (**A-102**) as a sticky oil.

**¹HNMR** (500 MHz, CD₃OD) δ = 1.75-1.85 (m, 1H), 1.88-1.97 (m, 2H), 2.10 (s, 3H), 2.11-2.19 (m, 1H), 2.52-2.58 (m, 1H), 2.60-2.70 (m, 1H), 2.75-2.84 (m, 1H), 2.95 (s, 3H), 3.37-3.49 (m, 1H), 5.11-5.20 (m, 1H), 5.23-5.32 (m, 1H), 7.32-7.35 (m, 2H), 7.37-7.40 (m, 1H), 7.48-7.54 (m, 4H), 7.55-7.59 (m, 1H).

**MS (ESI):** [M + H]⁺ = 414.3.

### Example 103: (S,E)-2-(3-((1-(2-chlorophenyl)-2-oxocyclohexyl)(methyl)amino)-3-oxoprop-1-en-1-yl)phenyl isobutyrate (A-103)

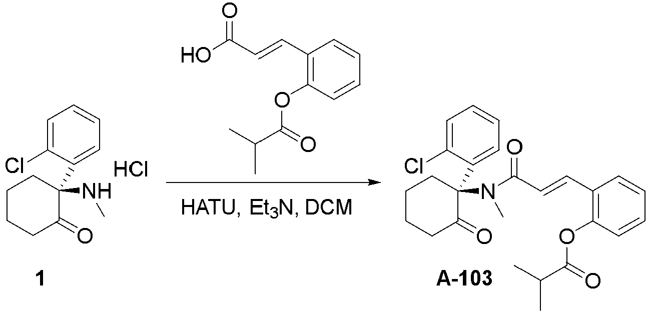

To a solution of compound **1** (170 mg, 0.62 mmol), (*E*)-3-(2-(isobutyryloxy)phenyl)acrylic acid (219 mg, 0.9 mmol) and HATU (285 mg, 0.75 mmol) in DCM (5 mL) was added DIPEA (0.33 mL, 1.9 mmol). The reaction was heated to 40 °C for 3 h. The reaction was diluted with DCM (5 mL), washed with H₂O (5 mL) and brine (5 mL). The organic layer was dried over MgSO₄, filtered and concentrated to get an oil, which was purified on silica gel column eluting with hexane/EA (3/1) to afford 28 mg (10% yield) of the titled compound (**A-103**) as a white solid.

**¹HNMR** (500 MHz, acetone-*d₆*) δ = 1.30 (d, *J =* 1.2 Hz, 3H), 1.32 (d, *J =* 1.2 Hz, 3H), 1.72-1.90 (m, 4H), 2.29-2.37 (m, 1H), 2.44-2.50 (m, 1H), 2.52-2.65 (m, 1H), 2.88-2.97 (m, 1H), 3.31 (s, 3H), 3.32-3.38 (m, 1H), 7.03-7.09 (m, 1H), 7.17 (d, *J =* 8.1 Hz, 1H), 7.23-7.30 (m, 2H), 7.31-7.38 (m, 2H), 7.42-7.49 (m, 2H) 7.69 (d, *J =* 15.6 Hz, 1H), 7.94 (d, *J =* 7.7 Hz, 1H).

**MS (ESI):** [M + H]⁺ = 454.1.

### Example 104: (S,E)-2-(3-((1-(2-chlorophenyl)-2-oxocyclohexyl)(methyl)amino)-3-oxoprop-1-en-1-yl)phenyl acetate (A-104)

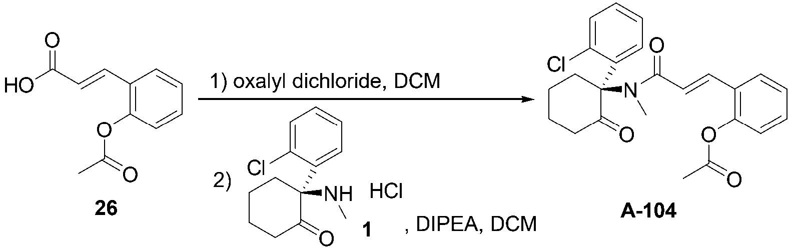

To a solution of compound **26** (51.5 mg, 0.25 mmol) in DCM (2 mL) was added oxalyl chloride (0.043 mL, 0.5 mmol). The reaction was stirred at 25 °C for 4 h, and the solvent was removed under vacuum. The residue was dissolved with DCM (2 mL), and a solution of compound **1** (68 mg, 0.25 mmol) and DIPEA (0.065 mL, 0.375 mmol) in DCM (1 mL) was added. The reaction was stirred at 25 °C for 16 h. The reaction was diluted with DCM (10 mL), washed with H₂O (10 mL). The organic layer was dried over MgSO₄, filtered and concentrated to get an oil, which was purified on silica gel column eluting with hexane/EA (7/3) to afford 38 mg (36% yield) of the titled compound (**A-104**) as a white solid.

**¹HNMR** (500 MHz, CDCl₃) δ = 1.71-1.80 (m, 2H), 1.82-1.94 (m, 1H), 2.00-2.12 (m, 1H), 2.35 (s, 3H), 2.44-2.51 (m, 1H), 2.52-2.60 (m, 1H), 2.62-2.73 (m, 1H), 3.18 (s, 3H), 3.30-3.41 (m, 1H), 6.90-7.05 (m, 2H), 7.10-7.18 (m, 1H), 7.22-7.32 (m, 3H), 7.38-7.43 (m, 1H), 7.44-7.50 (m, 1H), 7.66-7.76 (m, 1H), 7.80-7.87 (m, 1H).

**MS (ESI):** [M + H]⁺ = 426.1.

### Example 105: (E)-N-((S)-1-(2-chlorophenyl)-2-oxocyclohexyl)-3-(2-hydroxyphenyl)-N-methylacrylamide (A-105)

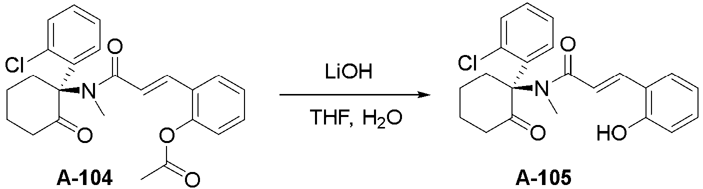

To a solution of compound (**A-104**) (20 mg, 0.047 mmol) in THF (1 mL) was added aqueous LiOH solution (0.8 mL, 1 M) at 0 °C. The reaction was stirred at 25 °C for 2 h and then concentrated. The mixture was diluted with H₂O (3 mL) and adjusted to pH=3 with aqueous HCl solution (1 M) and then extracted with DCM (5 mL). The organic layer was dried over MgSO₄, filtered and concentrated to get an oil, which was recrystallized from DCM and hexane to afford 12 mg (66% yield) of the titled compound (**A-105**) as a white solid.

**¹HNMR** (500 MHz, acetone-*d₆*) δ = 1.71-1.90 (m, 4H), 2.29-2.36 (m, 1H), 2.44-2.50 (m, 1H), 2.57-2.66 (m, 1H), 3.28 (s, 3H), 3.31-3.43 (m, 1H), 6.86-6.93 (m, 1H), 6.98 (d, *J =* 8.1 Hz, 1H), 7.04-7.09 (m, 1H), 7.21-7.30 (m, 3H), 7.32-7.39 (m, 1H), 7.40-7.47 (m, 1H) 7.68 (d, *J =* 7.7 Hz, 1H), 7.96 (d, *J =* 15.6 Hz, 1H).

**MS (ESI):** [M + H]⁺ = 384.1.

### Example 106: (S,Z)-4-((1-(2-chlorophenyl)-2-oxocyclohexyl)(methyl)amino)-4-oxobut-2-enoic acid (A-106)

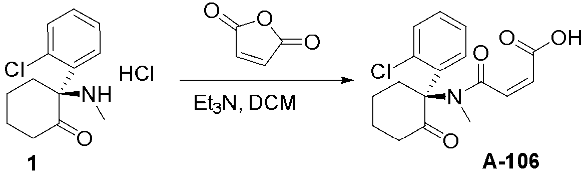

To a solution of compound **1** (137 mg, 0.5 mmol) and furan-2,5-dione (392 mg, 4 mmol) in DCM (5 mL) was added Et₃N (0.21 mL, 1.5 mmol) at 0 °C. The reaction was stirred at 25 °C for 22 h. The mixture was diluted with DCM (5 mL) and washed with aqueous NaOH solution (20 mL, 1 M). The aqueous layer was adjusted to pH=3 with aqueous HCl solution (1 M) and then extracted with DCM (20 mL). The organic layer was dried over MgSO₄, filtered and concentrated to afford 76 mg (45% yield) of the titled compound (**A-106**) as a light yellow solid.

**¹HNMR** (500 MHz, CDCl₃) δ = 1.60-1.64 (m, 1H), 1.79-1.88 (m, 2H), 1.98-2.01 (m, 1H), 2.57-2.66 (m, 2H), 2.77-2.80 (m, 1H), 3.06 (s, 3H), 3.17-3.21 (m, 1H), 6.32-6.35 (m, 1H), 6.66-6.69 (m, 1H), 7.15-7.18 (m, 1H), 7.25-7.29 (m, 2H), 7.44-7.47 (m, 1H).

**MS (ESI):** [M + Na]⁺ = 358.0.

### Example 107: (S,Z)-4-((1-(2-chlorophenyl)-2-oxocyclohexyl)(methyl)amino)-4-oxobut-2-enoic acid (A-107)

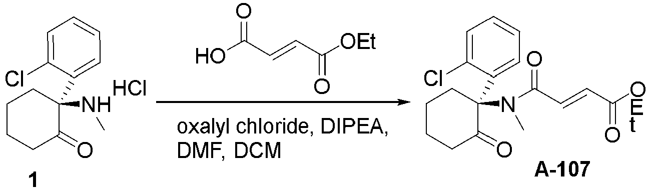

To a solution of (*E*)-4-ethoxy-4-oxobut-2-enoic acid (216 mg, 1.5 mmol) in DCM (5 mL) was added oxalyl chloride (0.26 mL, 3 mmol) and a few drops of DMF. The mixture was stirred at 25 °C for 1 h and then dried under vacuum. The residue was dissolved in DCM (3 mL) and a solution of compound **1** (137 mg, 0.5 mmol) and DIPEA (0.26 mL, 1.5 mmol) in DCM (3 mL) was added at 0 °C. The reaction was stirred at 25 °C for 2 h. The mixture was diluted with DCM (5 mL) and washed with water (10 mL) and brine (10 mL). The organic layer was dried over MgSO₄, filtered and concentrated to get an oil, which was purified on silica gel column eluting with hexane/EA (4/1) to afford 76 mg (40% yield) of the titled compound (**A-107**) as a light yellow foam.

**¹HNMR** (500 MHz, CD₃OD) δ = 1.32 (t, *J =* 12.0 Hz, 3H), 1.76-1.84 (m, 3H), 2.03-2.06 (m, 1H), 2.43-2.46 (m, 2H), 2.62-2.65 (m, 1H), 3.18 (s, 3H), 3.31-2.34 (m, 1H), 4.27 (q, *J =* 12.0 Hz, 2H), 6.67-6.70 (m, 1H), 7.03-7.05 (m, 1H), 7.27-7.31 (m, 2H), 7.46-7.48 (m, 1H), 7.54-7.57 (m, 1H).

**MS (ESI):** [M + H]⁺ = 364.1.

### Example 108: (((S)-1-(2-chlorophenyl)-2-oxocyclohexyl)(methyl)carbamoyl)-L-alanyl-L-proline (A-108)

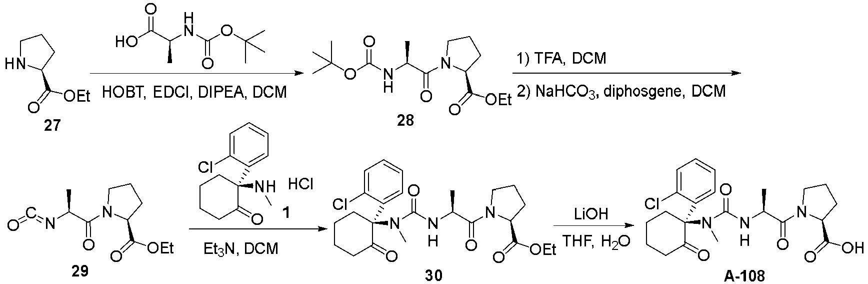

To a solution of compound **27** (1.3 g, 9.08 mmol) in DCM (25 mL) was dropwise to a solution of (*tert*-butoxycarbonyl)-*L*-alanine (2.06 g, 10.9 mmol), HOBT (2.21 g, 16.3 mmol), EDCI (3.13 g, 16.3 mmol) and DIPEA (5.69 mL, 32.7 mmol) in DCM (25 mL). The reaction was stirred for 4 h at 25 °C. The reaction was diluted with DCM (100 mL), washed with H₂O (25 mL). The organic layer was separated and washed with aqueous HCl solution (1 M), then aqueous saturated NaHCO₃ solution (20 mL) and brine (20 mL). The organic layer was dried over MgSO₄, filtered and concentrated to get an oil, which was purified on silica gel column eluting with hexane/EA (7/3) to afford 1.71 g (60% yield) of compound **28** as a sticky oil.

**¹HNMR** (500 MHz, CDCl₃) δ = 1.25 (t, *J =* 7.1 Hz, 3H), 1.35 (d, *J =* 6.9 Hz, 3H), 1.42 (s, 9H), 1.86-2.12 (m, 3H), 2.16-2.29 (m, 1H), 3.55-3.64 (m, 1H), 3.65-3.76 (m, 1H), 4.08-4.24 (m, 2H), 4.37-4.57 (m, 2H).

**MS (ESI):** [M + H]⁺ = 315.3.

To a solution of compound **28** (527 mg, 1.67 mmol) in DCM (10 mL) was added TFA (5.0 mL). The reaction was stirred at 25 °C for 16 h and then concentrated. The reaction was diluted with DCM (10 mL), washed with H₂O (5 mL). The organic layer was dried over MgSO₄, filtered and concentrated to afford a sticky oil (360 mg), which was dissolved in DCM (8 mL) and aqueous saturated NaHCO₃ solution (12 mL) was added. To the solution diphosgene (166 mg, 0.84 mmol) was added slowly at 0 °C. The mixture was stirred at 25 °C for 5 h. The reaction was diluted with DCM (10 mL), washed with H₂O (5 mL). The organic layer was dried over MgSO₄, filtered and concentrated to afford compound **29** (373 mg) as a sticky oil.

To a solution of compound **29** (373 mg, 1.55 mmol) in DCM (10 mL) was added compound **1** (118 mg, 0.43 mmol) and Et₃N (0.301 mL, 2.16 mmol). The reaction was heated to 70 °C for overnight. The reaction was diluted with DCM (10 mL), washed with H₂O (5 mL). The organic layer was dried over MgSO₄, filtered and concentrated to get an oil, which was purified on silica gel column eluting with hexane/EA (6/4) to afford 192 mg (94% yield) of compound **30** as a white solid.

**¹HNMR** (500 MHz, DMSO-*d*₆) δ = 1.07-1.21 (m, 4H), 1.23-1.33 (m, 1H), 1.37-1.56 (m, 5H), 1.56-1.71 (m, 3H), 1.73-2.06 (m, 6H), 2.07-2.22 (m, 1H), 2.82-3.06 (m, 1H), 3.49-3.63 (m, 1H), 3.64-3.77 (m, 1H), 3.95-4.13 (m, 2H), 4.19-4.31 (m, 1H), 4.76-4.89 (m, 1H), 6.12-6.25 (m, 1H), 7.22-7.48 (m, 4H).

**MS (ESI):** [M + Na]⁺ = 500.3.

To a solution of compound **30** (187 mg, 0.39 mmol) in THF (4 mL) was added aqueous LiOH solution (2 mL, 1 M). The reaction was stirred for 1 h at 25 °C and then concentrated. The reaction was diluted with DCM (10 mL), washed with pH 3 HCl solution (10 mL). The organic layer was dried over MgSO₄, filtered and concentrated to get an oil, which was purified on silica gel column eluting with hexane/EA (1/1) to afford 110 mg (63% yield) of the titled compound (**A-108**) as a white solid.

**¹HNMR** (500 MHz, DMSO-*d*₆) δ = 1.32 (d, *J =* 6.9 Hz, 4H), 1.35-1.52 (m, 3H), 1.55-1.64 (m, 1H), 1.77-1.99 (m, 4H), 2.03-2.23 (m, 2H), 2.61 (s, 3H), 3.39-3.46 (m, 1H), 3.50-3.58 (m, 1H), 4.23 (dd, *J =* 3.9, 8.7 Hz, 1H), 4.91 (q, *J =* 6.9, 13.9 Hz, 1H), 5.08 (dd, *J =* 3.2, 6.8 Hz, 1H), 7.33-7.38 (m, 2H), 7.42-7.48 (m, 1H), 7.49-7.54 (m, 1H).

**MS (ESI):** [M + Na]⁺ = 472.2.

### Example 109: Metabolic stability assay of the test compounds

### Mouse, Rat, Dog, Monkey and Human Liver S9 Fractions Metabolic Stability Assay

The protocol for mouse, rat, dog, monkey or human liver S9 fractions metabolic stability assay is employed to determine the half-life (T_{1/2}) of the compounds of the present disclosure and their releasing efficiency of conversion from the prodrugs to *S*-ketamine *in vitro.*

The following is the study outline for S9 assay: 1) For S-ketamine releasing efficiency assay, pooled liver S9 fractions (mouse, rat, dog, monkey or human) were obtained from commercial vendors (e.g., CD-1 male mouse liver S9, SD male rat liver S9, Beagle male dog liver S9, and mixed-gender pooled human liver S9 were purchased from Corning (Woburn, MA, USA); Cynomolgus male monkey liver S9 was purchased from Gibco (Thermo Fisher Scientific Inc. USA)) and stored at -80 °C prior to use. 2) Potassium phosphate buffer (100 mM, pH 7.4) containing 3 mM MgCl₂ was pre-incubated in triplicate with test article (3 µM, final acetonitrile concentration 0.1%) in a 37 °C incubator for 10 min. 3) The reactions were initiated by adding pre-warmed S9 fractions (1.0 mg/mL) in the presence of 2 mM NADPH. The final incubation mixture volume was 200 µL. 4) All reactions were terminated using five volumes of extraction solvent at the pre-defined time points (0 to 60 min). 5) Aliquots of terminated incubation mixtures were centrifuged at 20,000 x g for 5 min. 6) The supernatants were analyzed with LC-MS/MS for the amount of the test article remaining and S-ketamine formation. Data are shown as below in Table 1.

**Table 1: Metabolic Stability of Test Compounds in Mouse, Rat, Dog, Monkey and Human Liver S9 Fractions**

| **Test Article** | **Species** | **T_{1/2} (min)** | ***S*-ketamine Releasing Efficiency (%)** |
|---|---|---|---|
| Compound (A-1) | Rat | 14.30 | 51.69 |
| Compound (A-3) | Dog | 12.02 | 39.49 |
| | Human | 4.74 | 62.67 |
| | Monkey | 3.84 | 38.97 |
| | Mouse | <1 | 54.19 |
| | Rat | 6.97 | 83.67 |
| Compound (A-4) | Dog | 2.56 | 50.26 |
| | Human | 2.33 | 64.76 |
| | Monkey | <1 | 91.36 |
| | Mouse | 1.10 | 44.16 |
| | Rat | 4.59 | 91.39 |
| Compound (A-5) | Rat | 3.67 | 49.22 |
| Compound (A-7) | Rat | 15.83 | 40.55 |
| Compound (A-10) | Mouse | <1 | 64.37 |
| Compound (A-12) | Dog | 6.16 | 41.62 |
| | Mouse | 0.82 | 71.44 |
| Compound (A-14) | Human | <1 | 18.44 |
| | Mouse | <1 | 30.89 |
| Compound (A-28) | Human | 21.64 | 22.17 |
| | Mouse | 6.34 | 30.50 |
| Compound (A-16) | Mouse | 1.21 | 65.12 |
| Compound (A-17) | Mouse | <1 | 60.65 |
| Compound (A-18) | Mouse | <1 | 50.13 |
| Compound (A-19) | Mouse | <1 | 60.76 |
| Compound (A-20) | Monkey | <1 | 41.40 |
| | Mouse | <1 | 57.46 |
| | Rat | 2.08 | 68.26 |
| Compound (A-22) | Mouse | 1.21 | 56.30 |
| Compound (A-24) | Mouse | 1.35 | 34.90 |
| Compound (A-25) | Mouse | <1 | 113.75 |
| | Rat | 2.37 | 69.17 |
| Compound (A-26) | Mouse | 1.21 | 46.09 |
| Compound (A-27) | Monkey | <1 | 47.14 |
| | Mouse | 1.01 | 47.70 |
| | Rat | <1 | 93.40 |
| Compound (A-30) | Mouse | 3.25 | 19.05 |
| | Rat | 2.20 | 12.62 |
| Compound (A-33) | Mouse | <1 | 64.83 |
| Compound (A-34) | Mouse | 2.16 | 39.24 |
| Compound (A-35) | Mouse | 1.31 | 91.63 |
| Compound (A-37) | Mouse | 1.93 | 54.58 |
| | Rat | <1 | 75.56 |
| Compound (A-52) | Rat | <1 | 56.62 |
| Compound (A-53) | Rat | <1 | 72.61 |
| S-ketamine | Dog | 5.14 | 100.00 |
| | Human | 111.36 | 100.00 |
| | Monkey | 6.88 | 100.00 |
| | Mouse | 44.65 | 100.00 |
| | Rat | 10.38 | 100.00 |

The *in vitro S*-ketamine releasing efficiency assay employing mouse, rat, dog, monkey and human liver S9 fractions had shown that the prodrug compounds could be converted to *S*-ketamine with variable releasing efficiencies, which suggested that they would be converted into S-ketamine in the systemic circulation after being administered to mouse, rat, dog, monkey and human.

### Mouse, Rat, Dog, Monkey and Human Whole Blood Metabolic Stability Assay

The protocol for mouse, rat, dog, monkey and human whole blood metabolic stability assay is employed to determine the releasing efficiency of the compounds of the present disclosure conversion from the prodrugs to *S*-ketamine *in vitro.*

The following is the study outline for whole blood assay: 1) For S-ketamine releasing efficiency assay, rat whole blood in mixed gender was obtained from commercial vendors (*e.g*., heparinized CD-1 mouse whole blood pools (N>5) and SD rat whole blood pools (N>5) were purchased from BioLASCO (Yi-Lan, Taiwan); heparinized beagle dog whole blood pools (N=3) were purchased from Center of Toxicology and Preclinical Sciences (CTPS, QPS Taiwan); heparinized cynomolgus monkey whole blood pools (N=3) were purchased from the Laboratory Animal Center (LAC) of National Defense Medical Center (NDMC); and fresh heparinized human whole blood were obtained from healthy donors (N>6)), stored at 4 °C prior to use. 2) Test article was incubated in 37 °C pre-warmed rat whole blood at 3 µM (final acetonitrile concentration 1%) for up to 60 minutes at 37 °C. 3) Aliquots of 100 µL spiked sample solutions were taken at the pre-defined time points (0 to 60 min) post incubation, and were immediately extracted by adding five volumes of extraction solvent and then centrifuged at 20,000 x g for 5 minutes. 4) The supernatant fractions were analyzed with LC-MS/MS for the amount of the test article remaining and ketamine formation. The measurement results were then used for calculation of conversion efficiency the test compounds into *S*-ketamine in whole blood. Data are shown as below in Table 2.

**Table 2. Metabolic Stability of Test Compounds in Mouse, Rat, Dog, Monkey and Human Whole Blood**

| **Test Article** | **Species** | **T_{1/2} (min)** | ***S*-ketamine Releasing Efficiency (%)** |
|---|---|---|---|
| Compound (A-1) | Rat | <1 | 45.71 |
| Compound (A-3) | Dog | 1.01 | 82.14 |
| | Human | 1.41 | 74.11 |
| | Monkey | <1 | 75.11 |
| | Mouse | <1 | 67.47 |
| | Rat | <1 | 94.43 |
| Compound (A-4) | Dog | >120 | 2.29 |
| | Human | >120 | 2.56 |
| | Monkey | >120 | 12.88 |
| | Mouse | <1 | 122.58 |
| | Rat | 1.06 | 97.34 |
| Compound (A-5) | Rat | <1 | 89.13 |
| Compound (A-7) | Rat | 8.68 | 65.17 |
| Compound (A-10) | Mouse | <1 | 78.13 |
| Compound (A-12) | Dog | 35.64 | 33.52 |
| | Monkey | 30.43 | 60.11 |
| | Mouse | <1 | 119.76 |
| Compound (A-14) | Human | 67.61 | 28.38 |
| | Monkey | 44.18 | 34.25 |
| | Mouse | <1 | 148.78 |
| Compound (A-28) | Human | >120 | 3.46 |
| | Mouse | >120 | 8.25 |
| Compound (A-16) | Monkey | 21.82 | 55.26 |
| | Mouse | <1 | 150.78 |
| Compound (A-17) | Monkey | 4.95 | 74.36 |
| | Mouse | <1 | 136.03 |
| Compound (A-18) | Mouse | <1 | 98.97 |
| Compound (A-19) | Mouse | <1 | 125.27 |
| Compound (A-20) | Monkey | 5.65 | 62.86 |
| | Mouse | <1 | 107.83 |
| | Rat | 1.12 | 82.55 |
| Compound (A-22) | Mouse | <1 | 117.55 |
| Compound (A-24) | Mouse | <1 | 101.54 |
| Compound (A-25) | Monkey | 1.58 | 116.70 |
| | Mouse | <1 | 187.52 |
| | Rat | <1 | 94.75 |
| Compound (A-26) | Mouse | <1 | 110.63 |
| Compound (A-27) | Monkey | 1.54 | 77.88 |
| | Mouse | <1 | 113.11 |
| | Rat | <1 | 101.22 |
| Compound (A-30) | Mouse | 3.25 | 117.02 |
| | Rat | <1 | 85.49 |
| Compound (A-33) | Mouse | 1.08 | 143.70 |
| Compound (A-34) | Mouse | 9.95 | 134.67 |
| Compound (A-35) | Mouse | 1.06 | 141.89 |
| Compound (A-37) | Mouse | >120 | 20.18 |
| | Rat | >120 | 6.48 |
| Compound (A-52) | Rat | 3.29 | 87.68 |
| Compound (A-53) | Rat | 2.41 | 96.75 |
| S-ketamine | Dog | >120 | 100.00 |
| | Human | >120 | 100.00 |
| | Monkey | >120 | 100.00 |
| | Mouse | >120 | 100.00 |
| | Rat | >120 | 100.00 |

The *in vitro* S-ketamine releasing efficiency assay employing mouse, rat, dog, monkey and human whole blood had shown that prodrug compounds could be converted to S-ketamine with variable releasing efficiencies, which suggested that they would be converted into *S*-ketamine in the systemic circulation after being administered to mouse, rat, dog, monkey and human.

### Example 110: Pharmacokinetic studies

The mouse/rat pharmacokinetic profiles of the test articles were evaluated following (*S-*ketamine) or oral (*S*-ketamine or prodrugs) administrations in CD-1 mice and SD rats. Blood samples were collected from the facial veins using heparinated tubes at pre-dose and 3 min, 10 min, 30 min, 1 h, 2 h, 3 h, 4 h, 6 h, and 8 h post-dose after intravenous administration (IV), and withdrawn at pre-dose and 10 min, 30 min, 1 h, 2 h, 3 h, 4 h, 5 h, 6 h, and 8 h post-dose after oral administration (PO). In mouse PK study, mice were sub-grouped for a sparse sampling strategy. Each mouse provided two blood samples at different collection times. Blood samples were collected from alternating groups of three mice per time point. To prevent compound degradation, blood samples once drawn were immediately mixed in a ratio of 1:3 (v/v) with acetonitrile (containing 0.1% formic acid). The de-proteinized samples were temporarily held in ice following by storing at -70 °C before bioanalysis. The concentrations of analytes in blood were determined by LC-MS/MS. Various pharmacokinetic parameters were calculated using Phoenix^{™} WinNonlin^{®} software. To quantify the bioconversion efficiency of the test compounds in the circulation system, the relative bioavailability of *S*-ketamine after oral administration was calculated. The values of relative bioavailability were expressed as the ratio of the AUC of *S*-ketamine converted from the test compounds versus the AUC of *S*-ketamine HCl salt administrated *via* intravenous alone adjusted by dose. Data are shown as below in Table 3 and Table 4.

**Table 3. Mouse pharmacokinetic parameters of S-ketamine and representative compounds**

| | | **AUCₗₐₛₜ (h*µg/mL)** | **Tₘₐₓ (min)** | **Cₘₐₓ (µg/mL)** | **Bioavailability F (%)** |
|---|---|---|---|---|---|
| S-ketamine HCl @ 10 µmol/Kg | IV | 687 | | | 100.0 |
| S-ketamine HCl @ 20 µmol/Kg | PO | 318 | 10 | 525 | 23.1** |
| Compound (A-3) @ 20 µmol/Kg | PO* | 323 | 10 | 739 | 23.5** |
| Compound (A-4) @ 20 µmol/Kg | PO* | 211 | 10 | 271 | 15.3** |
| Compound (A-12) @ 20 µmol/Kg | PO* | 178 | 10 | 271 | 13.0** |
| Compound (A-20) @ 20 µmol/Kg | PO* | 190 | 10 | 282 | 13.8** |
| Compound (A-27) @ 20 µmol/Kg | PO* | 425 | 10 | 808 | 30.9** |
| Compound (A-37) @ 20 µmol/Kg | PO* | 270 | 10 | 532 | 19.7** |
| Compound (A-51) @ 20 µmol/Kg | PO* | 323 | 10 | 786 | 223.5** |

| | | | | | |
|---|---|---|---|---|---|
| Note: * measured and calculated based on *S*-ketamine, ** relative bioavailability | | | | | |

**Table 4. Rat pharmacokinetic parameters of S-ketamine and representative compounds**

| | | AUCₗₐₛₜ (h*nM) | Tₘₐₓ(min) | Cₘₐₓ (nM) | Bioavailability F (%) |
|---|---|---|---|---|---|
| S-ketamine HCl @ 10 µmol/Kg | IV | 739.0 | | | 100.0 |
| S-ketamine HCl @ 20 µmol/Kg | PO | 72.1 | 10.0 | 116.0 | 4.9** |
| Compound (A-3) @ 20 µmol/Kg | PO* | 74.3 | 31.8 | 47.8 | 5.0** |
| Compound (A-4) @ 20 µmol/Kg | PO* | 127.0 | 25.8 | 88.0 | 8.6** |
| Compound (A-20) @ 20 µmol/Kg | PO* | 90.3 | 22.2 | 69.7 | 6.1** |
| Compound (A-27) @ 20 µmol/Kg | PO* | 99.4 | 30.0 | 85.5 | 6.7** |
| Compound (A-37) @ 20 µmol/Kg | PO* | 97.2 | 18.0 | 96.2 | 6.6** |
| Compound (A-55) @ 20 µmol/Kg | PO* | 97.5 | 18.0 | 79.9 | 6.6** |
| Compound (A-58) @ 20 µmol/Kg | PO* | 112 | 18.0 | 73.2 | 7.6** |

| | | | | | |
|---|---|---|---|---|---|
| Note: * measured and calculated based on *S*-ketamine, ** relative bioavailability | | | | | |

For dog pharmacokinetic studies, male Beagle dogs were housed individually. Dogs in oral administration groups were fasted overnight before use but with free access to water supply. Dogs in IV groups have free access to food and water. For *S*-ketamine HCl salt, a single dose of 3.75 µmol/kg was administered to each dog *via* intravenous (IV) administration. The vehicle used for S-ketamine HCl salt is saline. For other test compounds, a single dose of each test compound was administered to each dog *via* oral gavage (n=3/group). The dosage of each test compound is listed in the Table 5. Blood samples were collected at specified time-points (pre-dose, 10 min, 30 min, 1 h, 2 h, 3 h, 4 h, 5 h, 6 h, 8 h, post-dose) following administration to individual dogs within IV and PO group. To prevent compound degradation, blood samples once drawn were immediately mixed in a ratio of 1:3 (v/v) with acetonitrile (containing 0.1% formic acid). The de-proteinized samples were temporarily held in ice following by storing at -70 °C before bioanalysis. The concentrations of analytes in blood were determined by LC-MS/MS. Various pharmacokinetic parameters were calculated using Phoenix^{™} WinNonlin^{®} software. To quantify the bioconversion efficiency of the test compounds in the circulation system, the bioavailability of S-ketamine after PO administration was calculated. Data are shown as below in Table 5.

**Table 5. Dog pharmacokinetic parameters of S-ketamine and representative compounds**

| | | AUCₗₐₛₜ (nM*h) | Tₘₐₓ (h) | Cₘₐₓ (nM/mL) | Bioavailability F (%) |
|---|---|---|---|---|---|
| S-ketamine HCl @ 3.75 µmol/kg | IV | 244 | | | 100.00 |
| S-ketamine HCl @ 15 µmol/kg | PO* | 17.2 | 0.44 | 9.0 | 1.8** |
| Compound (A-3) @ 15 µmol/kg | PO* | 54.5 | 0.28 | 53.7 | 5.6** |
| Compound (A-4) @ 15 µmol/kg | PO* | 42.3 | 0.67 | 27.0 | 4.3** |

| | | | | | |
|---|---|---|---|---|---|
| Note: * measured and calculated based on *S*-ketamine, ** relative bioavailability | | | | | |

For Monkey pharmacokinetic studies, three cynomolgus monkeys (two males, one female, Macaca fascicularis), from the colony at the Laboratory Animal Center (LAC) of National Defense Medical Center (NDMC), were studied. The mean age of the subjects was 6 years with a mean weight of 6.6 kg (6 to 7 kg). Each treatment was conducted at least 7 days washout between treatments. On the day of *in vivo* experiments, monkeys were sedated by intramuscular injection of Alfaxan (5 mg/kg) and Dexmedetomidine (10 mcg/kg). For intravenous administration, *S-*ketamine HCl solution was administered as a bolus injection slowly *via* a cephalic vein at a dose of 3.2 µmol/kg. For oral administration, the dosage of each test compound is listed in the Table 6 and was administrated *via* oral gavage. Monkeys in intravenous treatment group were free access to laboratory diet, and monkeys in oral treatment groups were fasted overnight prior to the treatment and fed at 2 to 3 hours after the test article administration. Drinking water was supplied *ad libitum* during the study period. Blood samples (0.35 mL/each) were collected from monkeys through the saphenous vein. The collected blood samples were placed into tubes containing heparin as the anticoagulant. Blood samples of intravenous (IV) group were collected at pre-dose, 10 min, 30 min, 1 h, 1.5 h, 2 h, 3 h, 4 h, 6 h, and 8 h post-dose. For PO group, blood samples were collected at pre-dose, 30 min, 1 h, 1.5 h, 2 h, 3 h, 4 h, 5 h, 6 h, and 8 h post-dose. To prevent compound degradation, 100 µL of blood samples once drawn from monkeys were immediately mixed with 300 µL of acetonitrile (containing 0.1% formic acid) in a ratio of 1:3 (v/v). The de-proteinized samples were temporarily held in ice following by storing at -70 °C before bioanalysis. The concentrations of analytes in blood were determined by LC-MS/MS.

**Table 6. Monkey pharmacokinetic parameters of S-ketamine and representative compounds**

| | | AUCₗₐₛₜ (nM*h) | Tₘₐₓ (h) | Cₘₐₓ (nM/mL) | Bioavailability F (%) |
|---|---|---|---|---|---|
| S-ketamine HCl @ 3.2 µmol/kg | IV | 2252.0 | | | 100.0 |
| S-ketamine HCl @ 6.4 µmol/kg | PO* | 23.4 | 4.0 | 12.7 | 0.5** |
| Compound (A-3) @ 6.4 µmol/kg | PO* | 83.1 | 3.7 | 22.8 | 1.8** |

| | | | | | |
|---|---|---|---|---|---|
| Note: * measured and calculated based on *S*-ketamine, ** relative bioavailability | | | | | |

Finally, it should be noted that there are other ways to practice the invention. Accordingly, embodiments of the present invention are to be described as examples, but the present invention is not limited to the contents described, further modifications may be made within the scope of the present invention or the equivalents added in the claims.

All publications or patents cited herein are incorporated by reference in this invention.

Reference throughout this specification to "an embodiment", "some embodiments", "one embodiment", "another example", "an example", "a specific example" or "some examples" means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example, "in an example," "in a specific example," or "in some examples," in various places throughout this specification are not necessarily referring to the same embodiment or example of the present disclosure. Furthermore, the particular features, structures, materials, or characteristics may be combined in any suitable manner in one or more embodiments or examples.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.
The present invention may additionally be defined by the following numbered clauses:
Clause 1. A compound having the structure of Formula (Ia) or (Ib), or a stereoisomer, an *N-*oxide, a solvate, a metabolite, a pharmaceutically acceptable salt or a prodrug thereof: 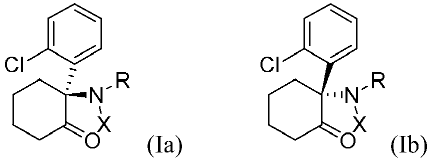 wherein R is -C(=O)R¹, -C(=O)OR², -C(=O)O(CHR³)OC(=O)R⁴ or -CD₃; and X is -CH₃ or - CD₃.
Clause 2. A compound having the structure of Formula (IIa) or (IIb), or a stereoisomer, an *N-*oxide, a solvate, a metabolite, a pharmaceutically acceptable salt or a prodrug thereof: 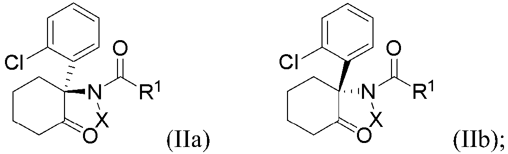 wherein R¹ is, optionally, substituted or unsubstituted aryl-OH, aryl-NH₂, alkenyl-OH, alkenyl-NH₂, alkyl-NH₂, alkyl-OH, carbocyclyl or heterocyclyl containing one or more N or O; and X is -CH₃ or -CD₃.
Clause 3. The compound of clause 2, wherein R¹ is aminoC₁₋₆ alkyl, -R^{1a}NHCOR^{1b}, - R^{1a}OCOR^{1b}, -R^{1a}COOR^{1b}, 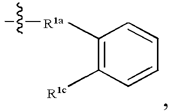 or C₃₋₆ heterocyclyl,
   wherein R¹ is optionally substituted with C₁₋₆ alkyl, -OH or oxo(=O),
   wherein R^{1a} and R^{1b} is independently H, C₁₋₆ alkyl or C₂₋₆ alkenyl, and
   R^{1c} is -OH, C₁₋₃ hydroxyalkyl, -OCOR^{1b} or -CH₂ OCOR^{1b}.
Clause 4. The compound of clause 2, wherein the heterocyclyl containing one or more N or O is 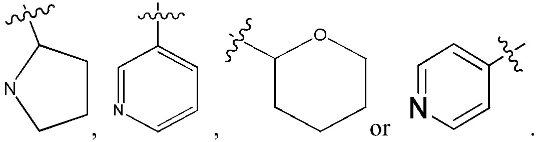
Clause 5. A compound selected from the group consisting of 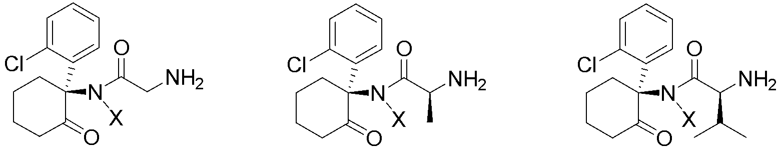 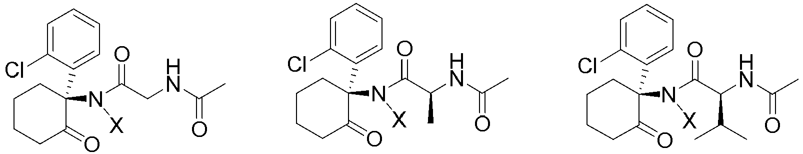 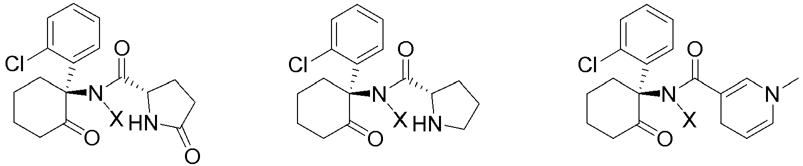 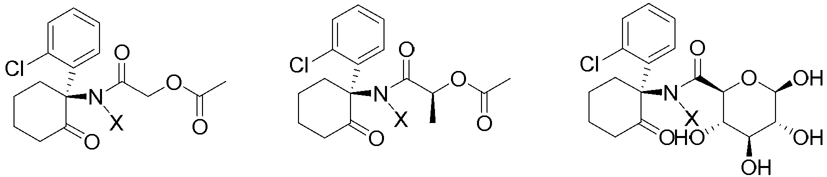 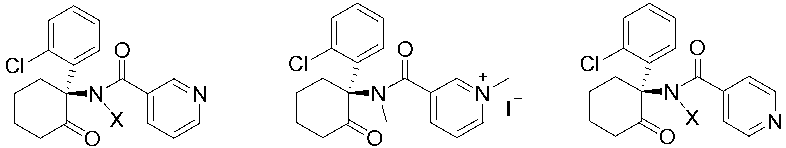 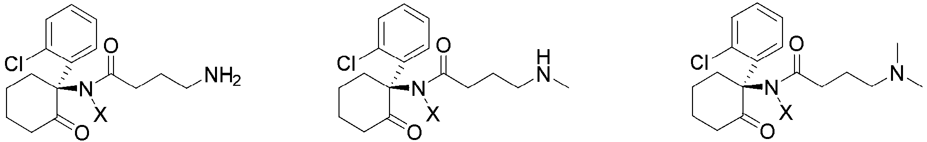 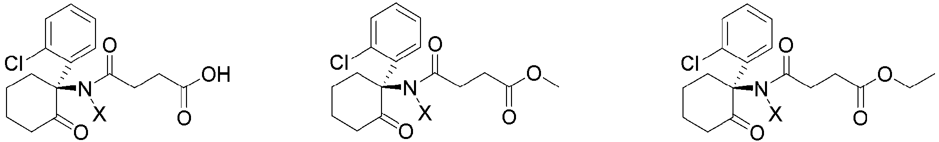 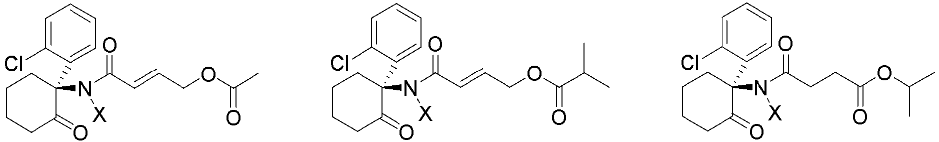 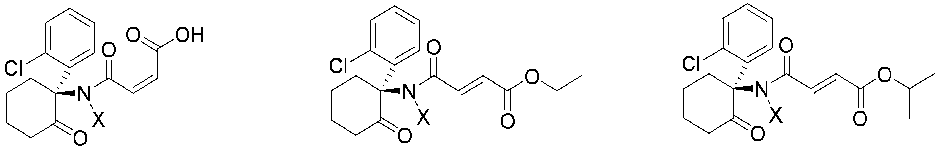 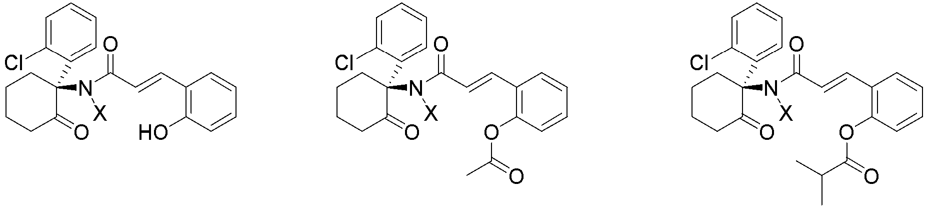 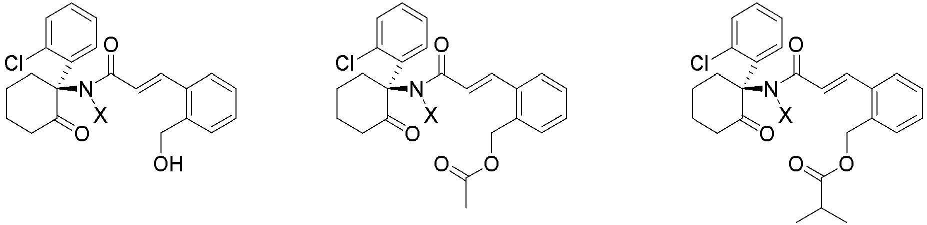 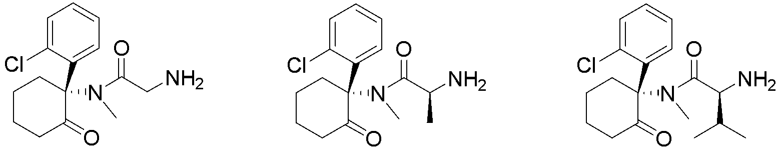 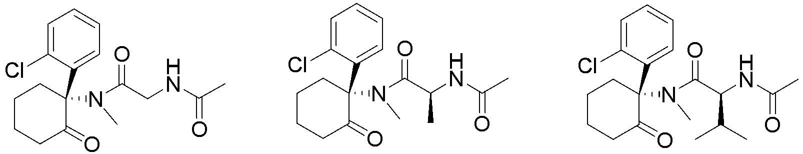 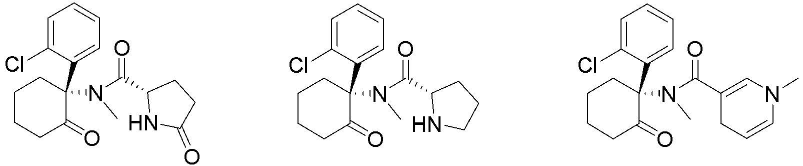 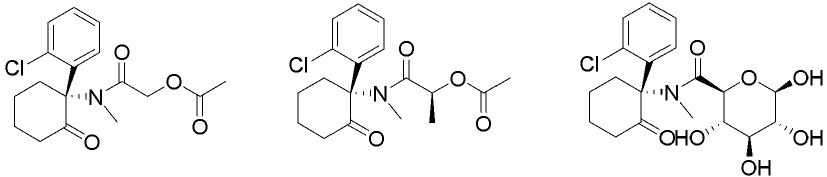 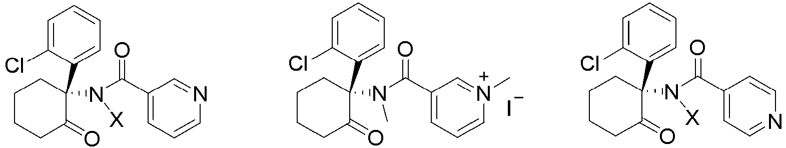 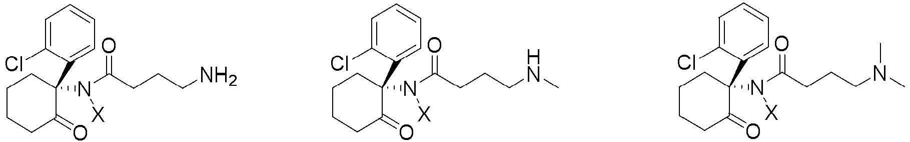 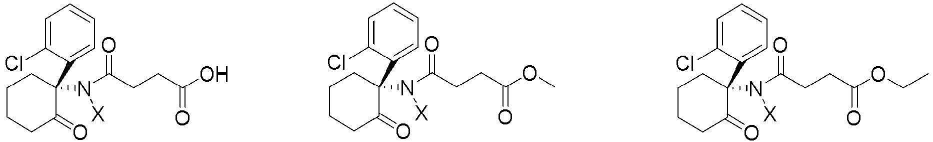 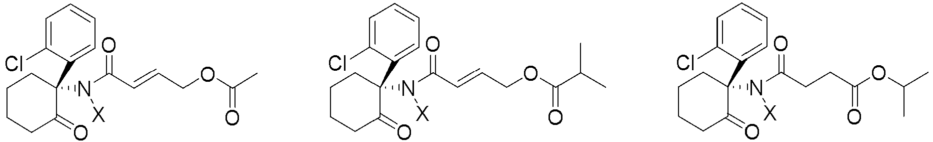 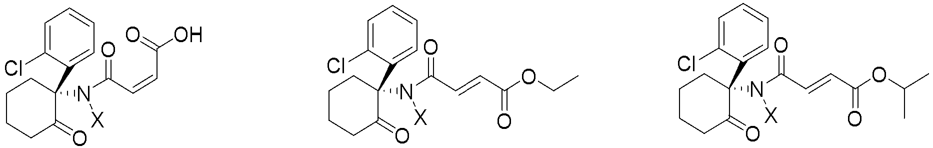 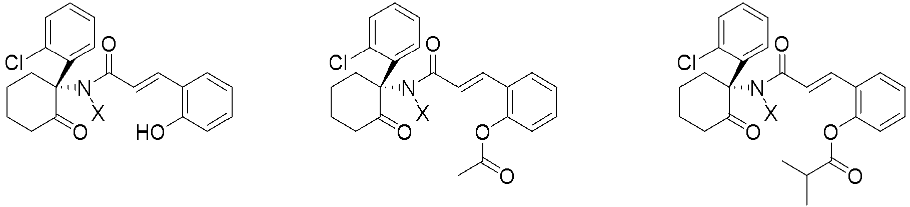 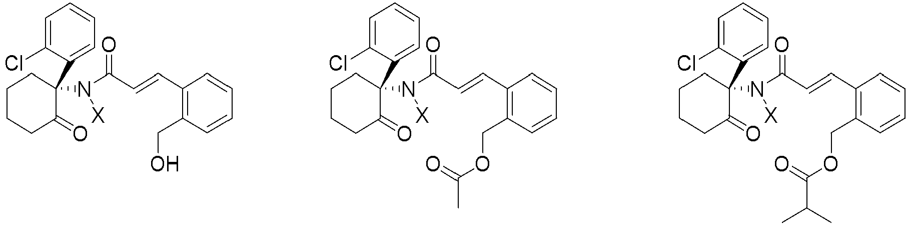 wherein X is -CH₃ or -CD₃.
Clause 6. A compound having the structure of Formula (IIIa) or (IIIb), or a stereoisomer, an *N*-oxide, a solvate, a metabolite, a pharmaceutically acceptable salt or a prodrug thereof: 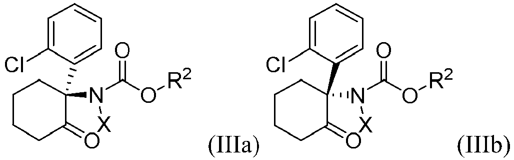 wherein R² is optionally substituted or unsubstituted alkyl, aryl, carbocyclyl or heterocyclyl containing one or more O; and X is -CH₃ or -CD₃.
Clause 7. The compound of clause 6, wherein R² is C₁₋₆ alkyl, C₁₋₆ hydroxyalkyl, aminoC₁₋₆ alkyl, -R^{2a}S(O)ₙ₁R^{2b}, -R^{2a}COOR^{2b}, C₃₋₆ aryl or C₃₋₆ heterocyclyl,
   wherein R² is optionally substituted with C₁₋₆ alkyl, -OH, C₁₋₆ hydroxyalkyl, 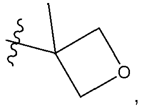 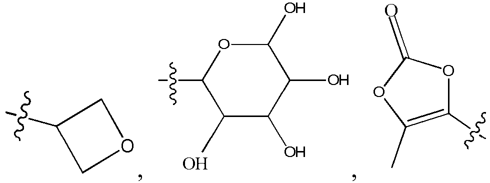 or -R^{2a}COOR^{2b}, given that C₁₋₆ alkyl is substituted with 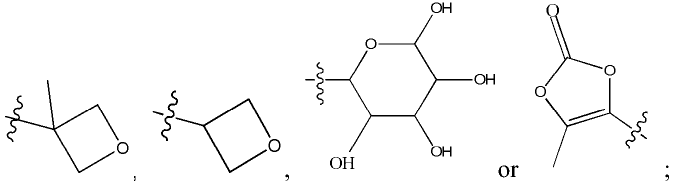
   R^{2a} is C₁₋₆ alkyl, wherein R^{2a} is optionally substituted with C₁₋₆ alkyl or -NH₂;
   R^{2b} is H or C₁₋₆ alkyl; and
   n₁ is 0, 1, 2.
   Clause 8. A compound selected from the group consisting of 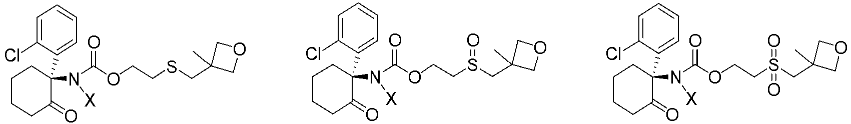 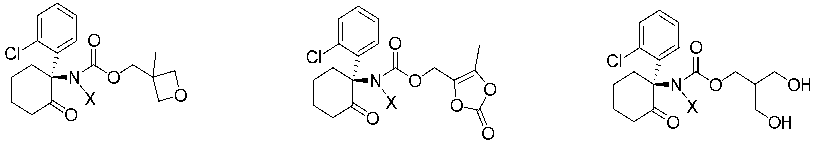 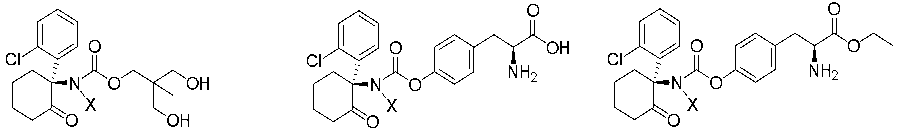 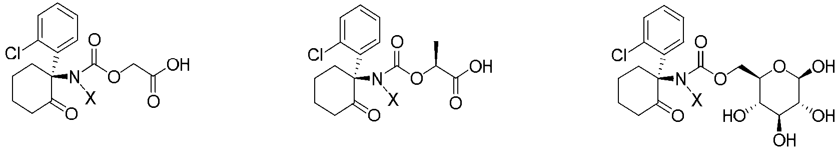 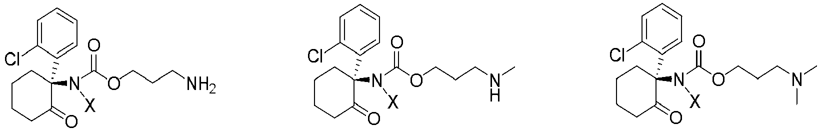 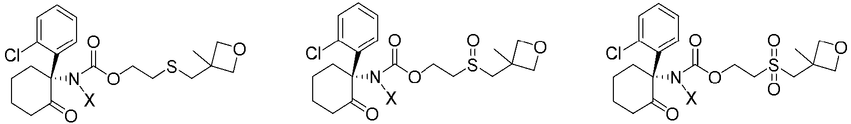 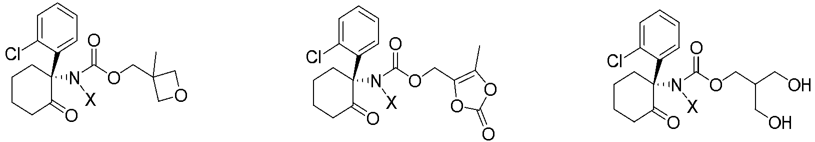 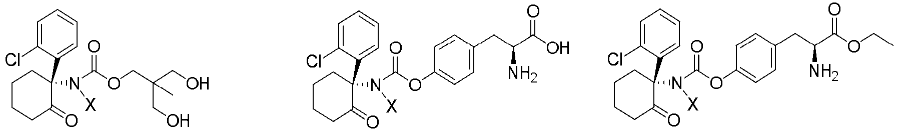 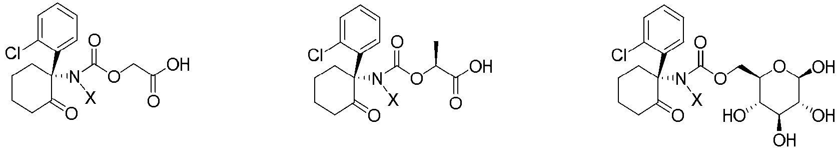 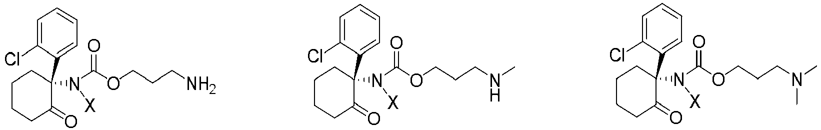 wherein X is -CH₃ or -CD₃.
Clause 9. A compound having the structure of Formula (IVa) or (IVb), or a stereoisomer, an N-oxide, a solvate, a metabolite, a pharmaceutically acceptable salt or a prodrug thereof: 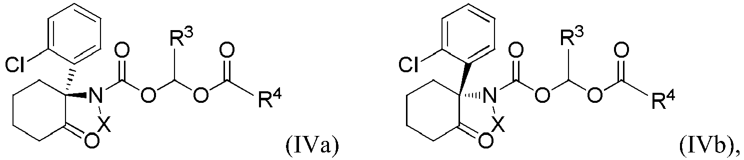 wherein R⁴ is independently substituted or unsubstituted alkyl, aryl, azaaryl, carbocyclyl or heterocyclyl containing one or more O or N, while R³ is H or substituted or unsubstituted alkyl; and X is -CH₃ or -CD₃.
Clause 10. The compound of clause 9, wherein R³ is H or C₁₋₆ alkyl.
Clause 11. The compound of clause 9, wherein R⁴ is C₁₋₆ alkyl, aminoC₁₋₆ alkyl, C₁₋₆ hydroxyalkyl, -R^{4a}NCOR^{4b}, -R^{4a}OCOR^{4b}, -R^{4a}S(O)ₙ₂R⁴⁶, C₁₋₆ heterocyclyl, C₁₋₅ azaaryl or 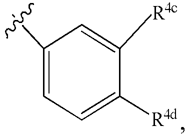
   wherein R⁴ is optionally substituted with C₁₋₆ alkyl, -NH₂, oxo(=O), C₁₋₆ hydroxyalkyl, 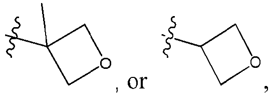 given that C₁₋₆ alkyl is substituted with 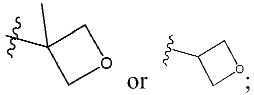
   wherein R^{4a} is C₁₋₆ alkyl, R^{4b} is C₁₋₆ alkyl or C₁₋₆ haloalkyl;
   R^{4c} is benzyl, R^{4d} is H, or R^{4c} and R^{4d}, together with the carbon atoms to which they are attached, form a C₅₋₆ heterocyclyl; and
   n₂ is 0,1 or 2.
Clause 12. The compound of clause 11, wherein C₁₋₆ heterocyclyl is 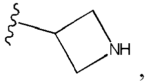 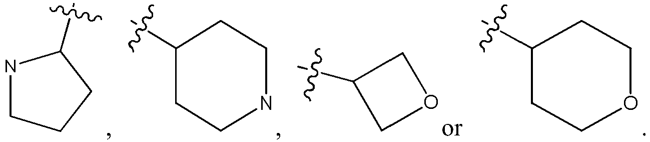
Clause 13. The compound of clause 11, wherein C₁₋₅ azaaryl is 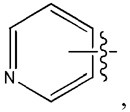 wherein 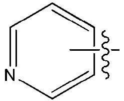 is optionally substituted with one or more methyl or -NH₂, or the combination thereof.
Clause 14. The compound of clause 11, wherein 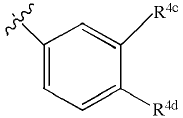 is 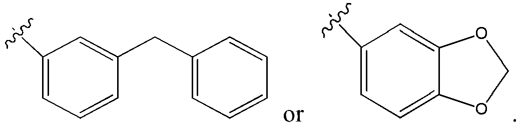
Clause 15. A Compound selected from the group consisting of 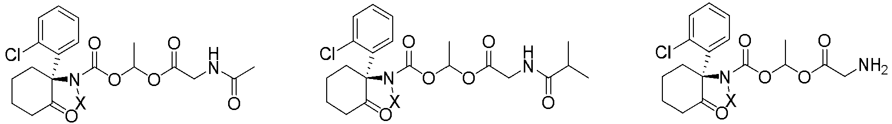 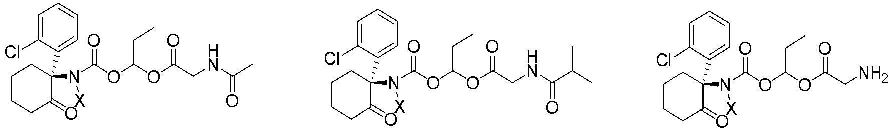 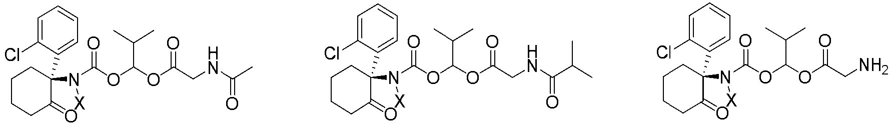 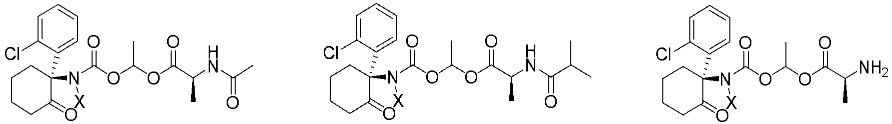 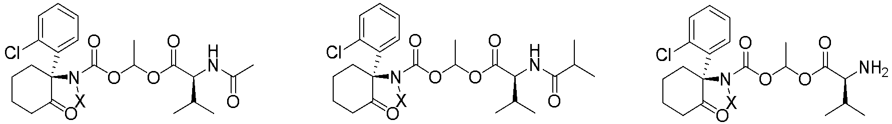 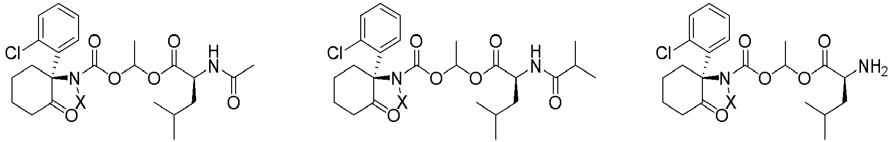 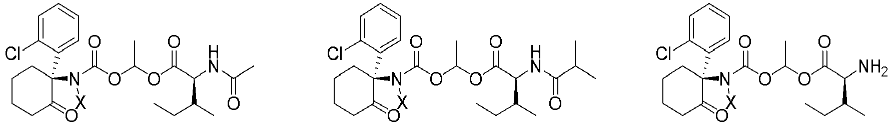 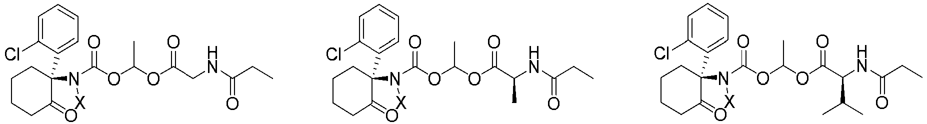 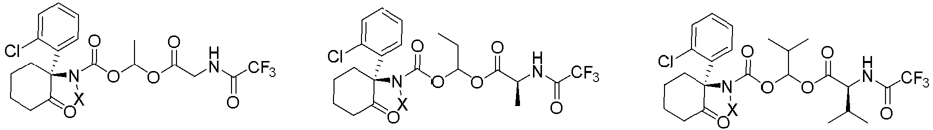 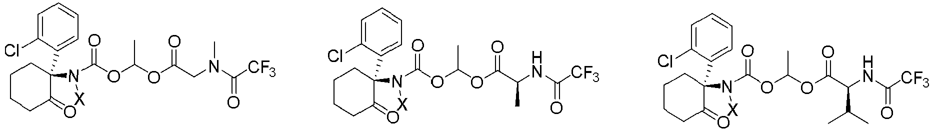 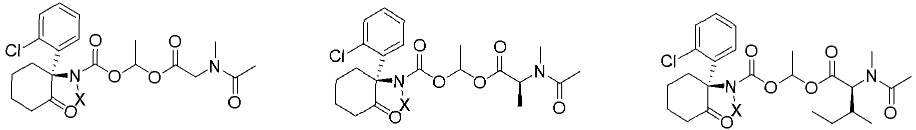 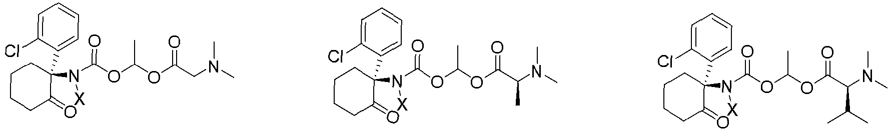 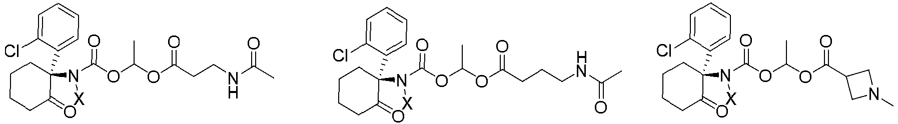 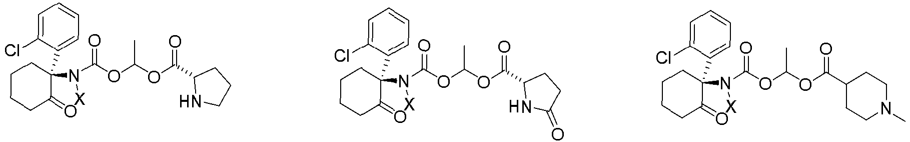 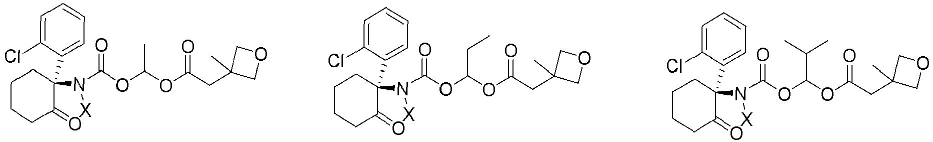 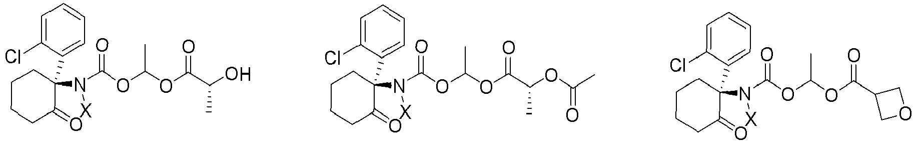 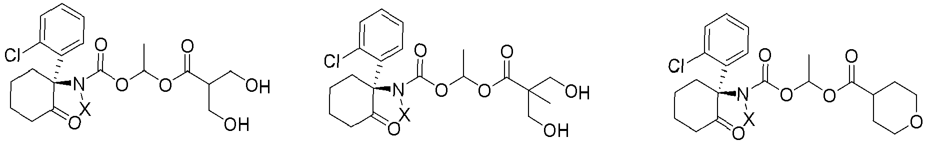 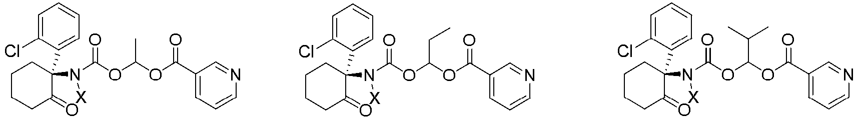 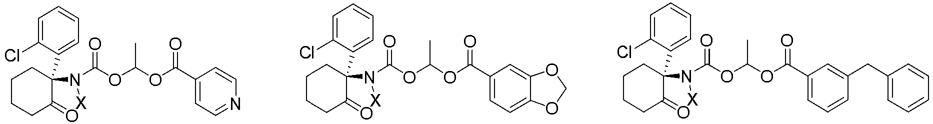 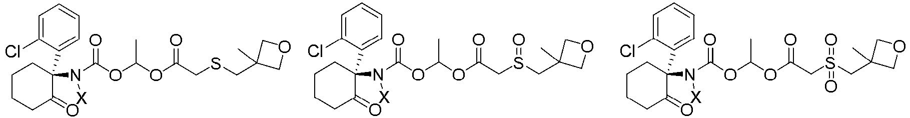 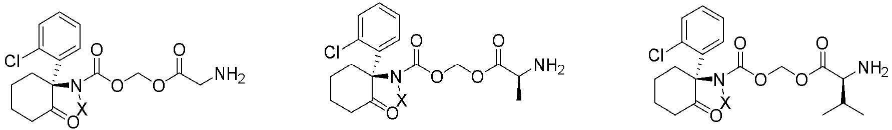 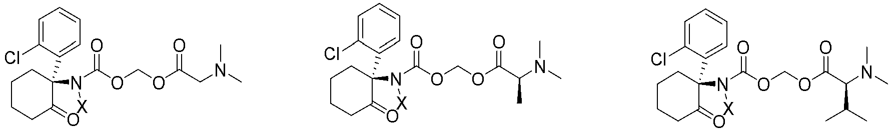 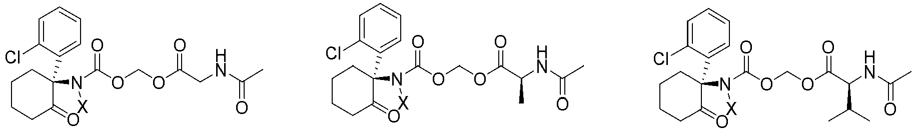 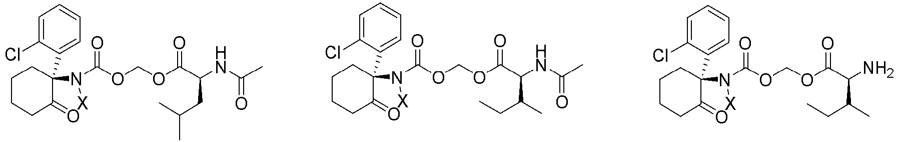 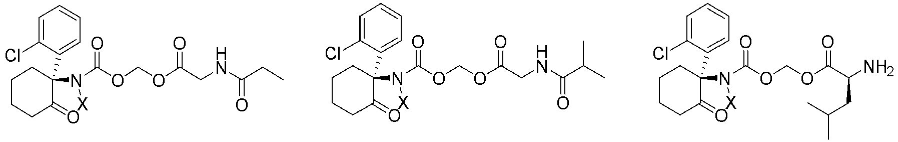 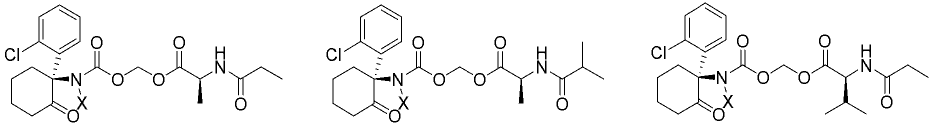 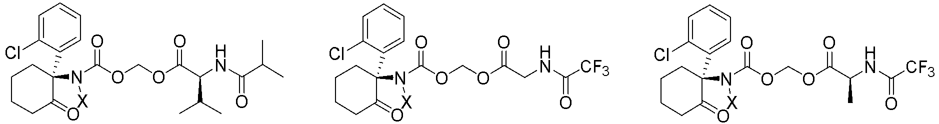 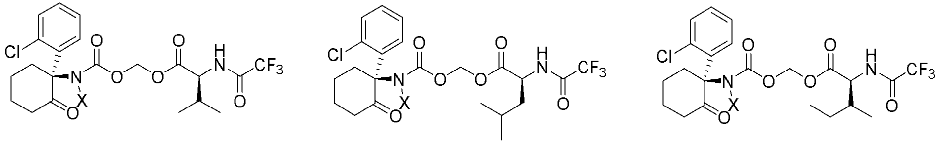 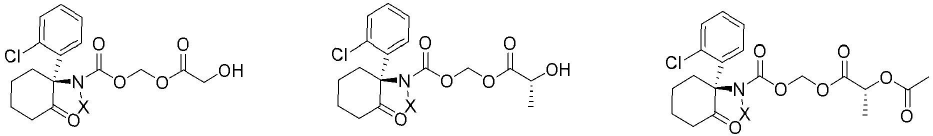 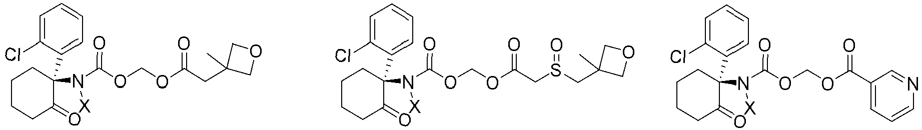 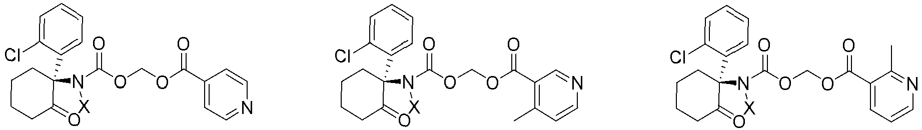 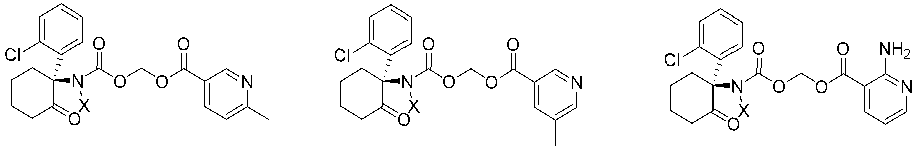 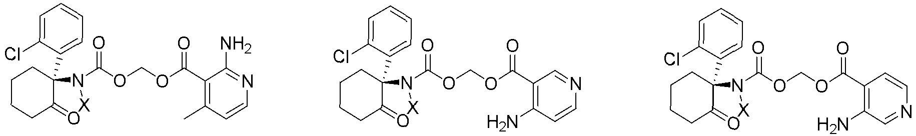 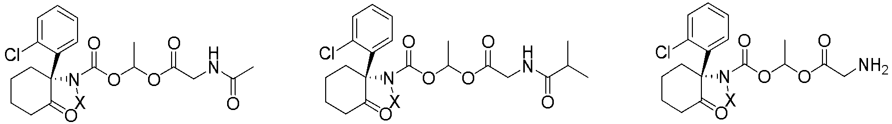 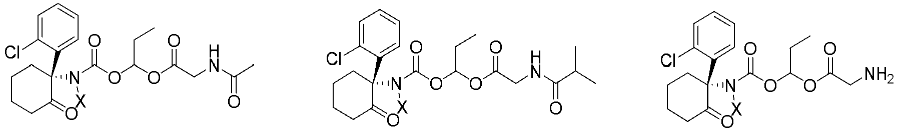 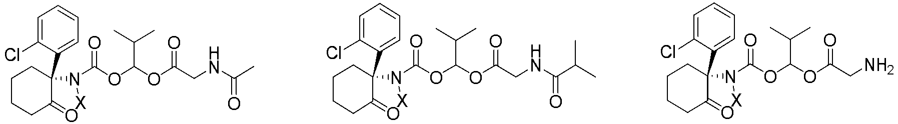 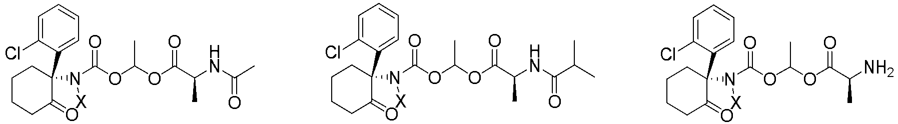 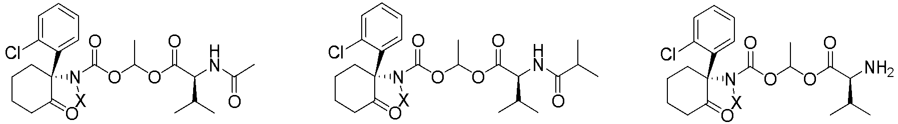 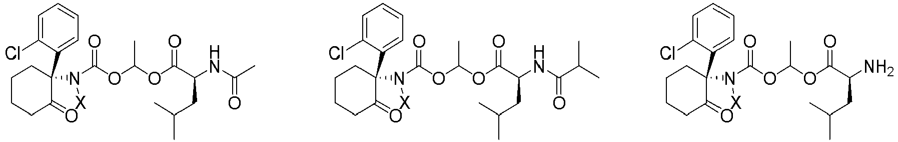 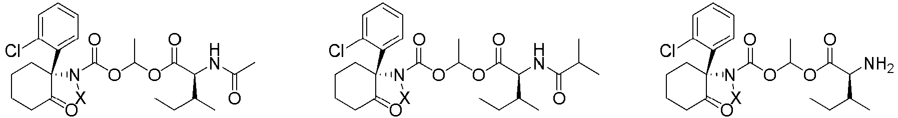 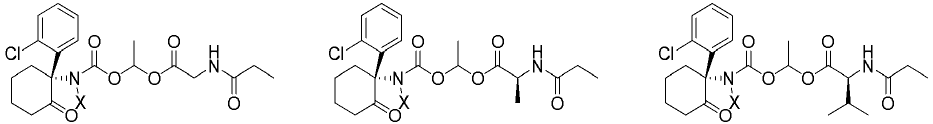 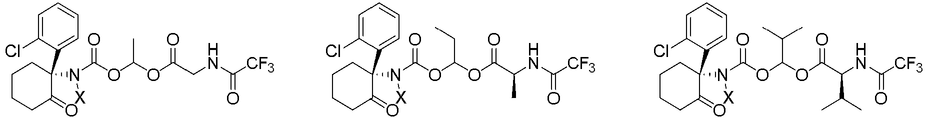 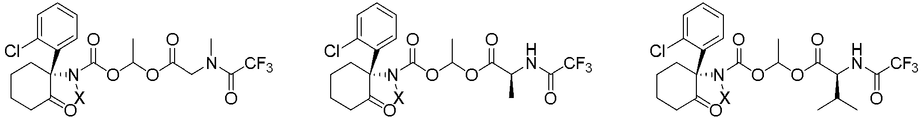 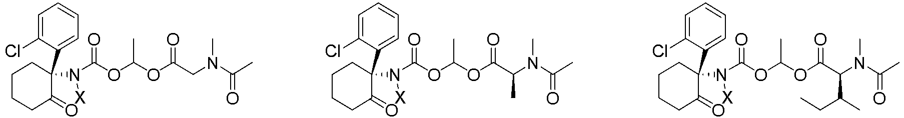 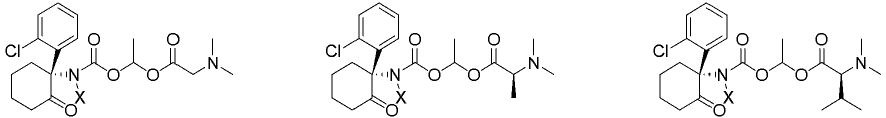 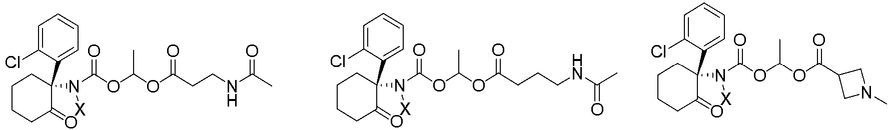 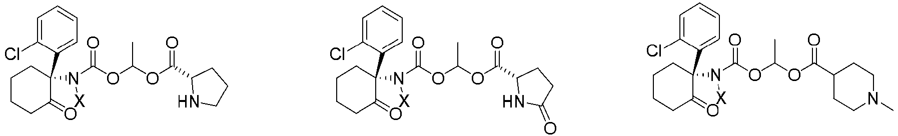 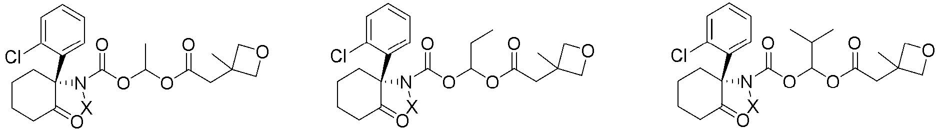 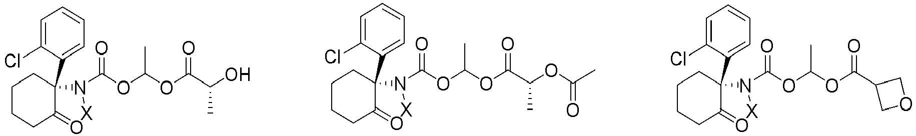 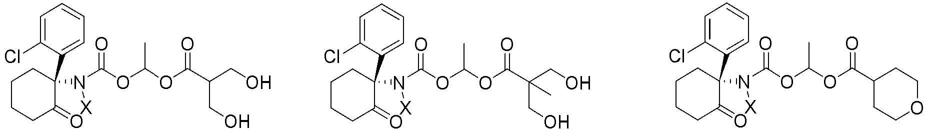 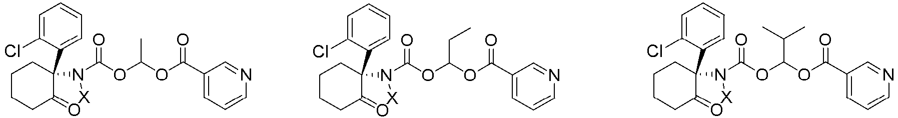 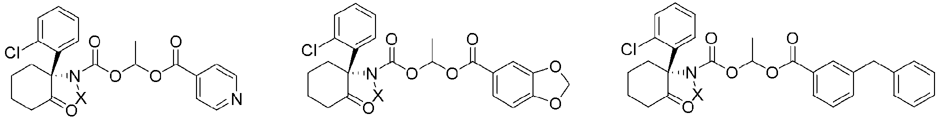 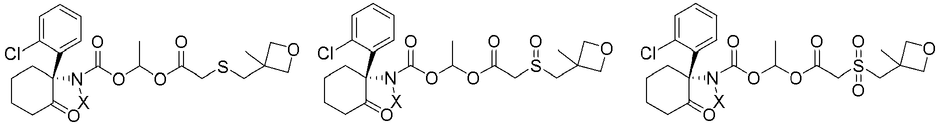 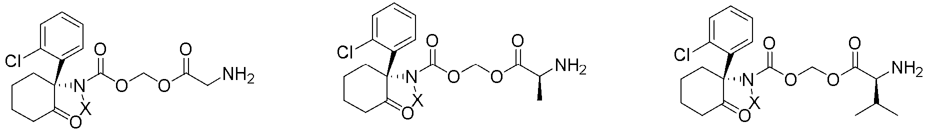 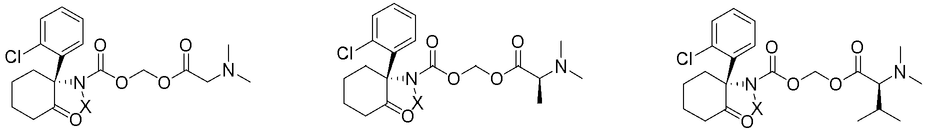 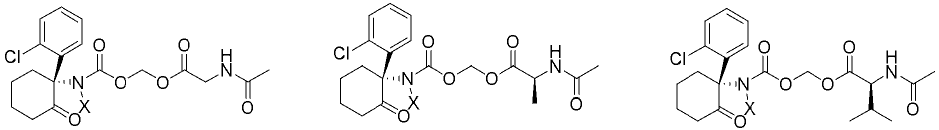 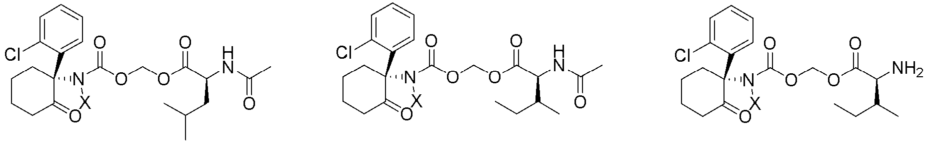 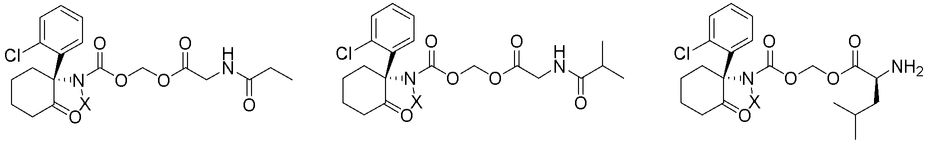 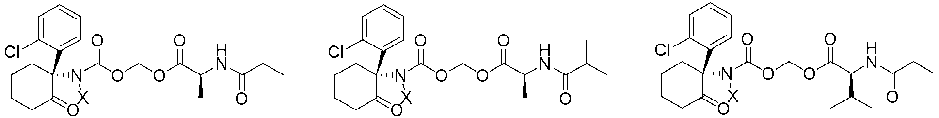 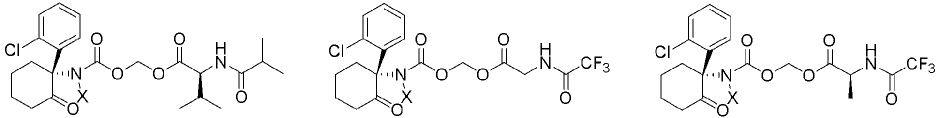 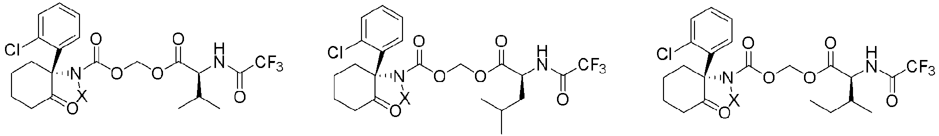 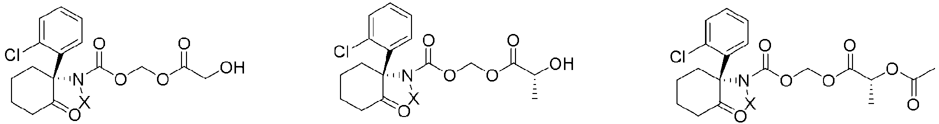 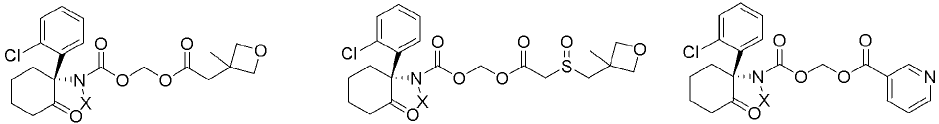 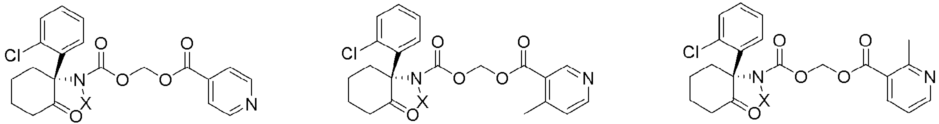 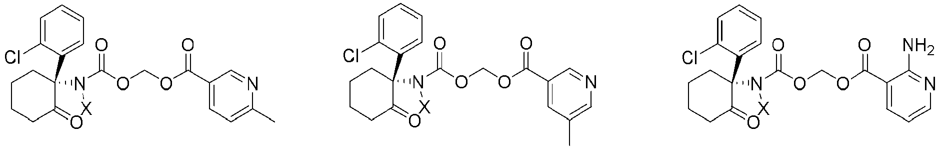 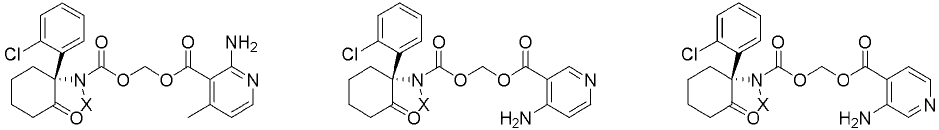 wherein X is -CH₃ or -CD₃.
Clause 16. A compound having the structure of Formula (Va) or (Vb) or (Vc) or (Vd), or a stereoisomer, an N-oxide, a solvate, a metabolite, a pharmaceutically acceptable salt or a prodrug thereof: 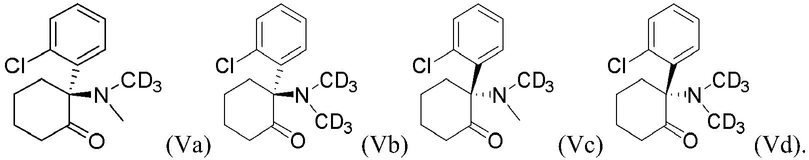
Clause 17. A pharmaceutical composition comprising the compound of any one of clauses 1 to 16.
Clause 18. The pharmaceutical composition of clause 17 further comprising at least one pharmaceutically acceptable excipient carrier, adjuvant, vehicle or a combination thereof.
Clause 19. The pharmaceutical composition of clause 18 further comprising one or more adjunctive therapeutic agents in a pharmaceutically effective amount, and wherein the adjunctive therapeutic agent is used in treating a neurological and psychiatric disorder or disease of the central nervous system.
Clause 20. The pharmaceutical composition of clause 19, wherein the neurological and psychiatric disorder or disease of the central nervous system is depression or pain.
Clause 21. The pharmaceutical composition of clause 19, wherein the adjunctive therapeutic agent is selected from the group consisting of at least one member of lithium, a pharmaceutical or an herbal antidepressant, an anticonvulsant, a mood stabilizer, an antipsychotic agent, and a benzodiazepine.
Clause 22. Use of the compound of any one of clauses 1 to 16 or the pharmaceutical composition of any one of clauses 17 to 21 in the manufacture of a medicament for preventing, managing, treating or lessening a neurological and psychiatric disorder or disease of the central nervous system in a patient.
Clause 23. Use of the compound of any one of clauses 1 to 16 or the pharmaceutical composition of any one of clauses 17 to 21 in the manufacture of a medicament for antagonizing the NMDA receptor.
Clause 24. The compound of any one of clauses 1 to 16 or the pharmaceutical composition of any one of clauses 17 to 21 for use in preventing, managing, treating or lessening a neurological and psychiatric disorder or disease of the central nervous system in a patient.
Clause 25. The compound of any one of clauses 1 to 16 or the pharmaceutical composition of any one of clauses 17 to 21 for use in antagonizing the NMDA receptor.
Clause 26. A method of preventing, managing, treating or lessening a neurological and psychiatric disorder or disease of the central nervous system in a patient, comprising administering to the patient in need thereof a therapeutically effective amount of the compound of any one of clauses 1 to 16 or the pharmaceutical composition of any one of clauses 17 to 21.
Clause 27. A method of antagonizing the NMDA receptor in a patient, comprising administering to the patient in need thereof a therapeutically effective amount of the compound of any one of clauses 1 to 16 or the pharmaceutical composition of any one of clauses 17 to 21.

## Claims

1. A compound having the structure of Formula A12, A16, A27, A52, or A53, or a pharmaceutically acceptable salt thereof:
| | |
|---|---|
| A12 | 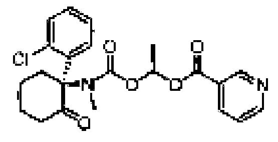 |
| A16 | 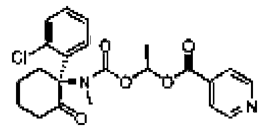 |
| A27 | 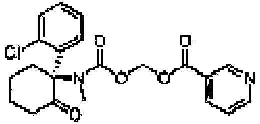 |
| A52 | 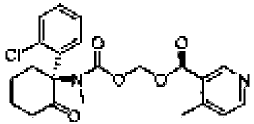 |
| A53 | 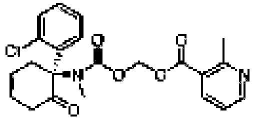 |

2. The compound according to claim 1, wherein the compound is selected from:
1-((((*S*)-1-(2-chlorophenyl)-2-oxocyclohexyl)(methyl)carbamoyl)oxy)ethyl nicotinate (A-12);
1-(isonicotinoyloxy)ethyl (*S*)-1-(2-chlorophenyl)-2-oxocyclohexylmethylcarbamate (A-16);
(nicotinoyloxy)methyl (*S*)-1-(2-chlorophenyl)-2-oxocyclohexylmethylcarbamate (A-27);
(4-methylpyridine-3-carboxyloyloxy)methyl (*S*)-1-(2-chlorophenyl)-2-oxocyclohexylmethylcarbamate (A-52); and
(2-methylpyridine-3-carboxyloyloxy)methyl (*S*)-1-(2-chlorophenyl)-2-oxocyclohexylmethylcarbamate (A-53),
or a pharmaceutically acceptable salt of any of the foregoing.

3. A pharmaceutical composition comprising the compound according to claim 1 or 2.

4. The pharmaceutical composition according to claim 3, wherein the pharmaceutical composition is an oral dosage form.

5. A compound according to claim 1 or 2, or a pharmaceutical composition according to claim 3 or 4, for use in the treatment of pain.

6. A compound according to claim 1 or 2, or a pharmaceutical composition according to claim 3 or 4, for use in the treatment of depression.

7. A compound according to claim 1 or 2, or a pharmaceutical composition according to claim 3 or 4, for use as an NMDA receptor antagonist in treating a neurological disorder, a psychiatric disorder, or a disease of the central nervous system related to NMDA receptors.

8. A compound according to claim 1 or 2, or a pharmaceutical composition according to claim 3 or 4, for use as an NMDA receptor antagonist in treating a condition related to NMDA receptors selected from the group consisting of an anxiety disorder, a seasonal affective disorder, mania, a bipolar disorder, obsessive-compulsive disorder, insomnia and fatigue resulting from jet lag, mental schizophrenia, seizure, panic attack, melancholia, alcohol addiction, drug addiction, alcoholism, substance abuse, drug addiction withdrawal symptoms, insomnia, a psychotic disorder, epilepsy, somnipathy, sleep disorder, sleep apnea syndrome, a mandatory eating disorder, fibromyalgia, stress, obesity, Parkinson's disease, a cognitive disorder, a memory disorder, premenstrual tension syndrome, a migraine headache, memory loss, Alzheimer silent disease, or a disorder related to normal or pathological aging.
